# EUROPEAN PATENT SPECIFICATION

(11) **EP 1 261 712 B1**
(45) Date of publication and mention of the grant of the patent: **13.12.2006**
(21) Application number: 01920218.3
(22) Date of filing: 05.03.2001
(51) Int. Cl.: C12N 15/12, C07K 14/47, C07K 14/705, C07K 16/18, C07K 16/28, G01N 33/68, C12Q 1/68, A61K 38/17, A61K 31/70, A61K 39/395, C12N 1/21, C12N 5/10

(54) **PROTEINS NAMED FCTRX AND NUCLEIC ACIDS ENCODING SAME**
FCTRX GENANNTE PROTEINE UND DAFÜR KODIERENDE NUKLEIN SÄURE
NOUVELLES PROTEINES ET ACIDES NUCLEIQUES CODANT LESDITES PROTEINES

(30) Priority: 03.03.2000 US 186592 P; 03.03.2000 US 186718 P; 06.03.2000 US 187293 P; 06.03.2000 US 187294 P; 17.03.2000 US 190400 P; 07.04.2000 US 196018 P; 03.01.2001 US 259548 P
(43) Date of publication of application: 04.12.2002
(73) Proprietor: Curagen Corporation, New Haven, CT 06511 (US)
(72) Inventor: VERNET, Corine, A., M., Branford, CO 06471 (US); FERNANDES, Elma, Branford, CT 06405 (US); SHIMKETS, Richard, A., Guilford, CT 06437 (US); HERRMANN, John, L., Guilford, CT 06437 (US); MAJUMDER, Kumud, Stamford, CT 06903 (US); MACDOUGALL, John, Hamden, CT 06517 (US); MISHRA, Vishnu, Gainesville, Florida 32608 (US); MEZES, Peter, S., Old Lyme, CT 06371 (US); RASTELLI, Luca, Guilford, CT 06437 (US)
(74) Representative: MacLean, Martin Robert
(86) International application number: PCT/US2001/007160
(87) International publication number: WO 2001/066747

(56) References cited:
- WO-A-00/36105
- WO-A-00/75321
- WO-A-01/19856
- US-A- 6 004 775
- DATABASE EMBL [Online] BAA21725, accession number BAA21725, 7 August 1997 (1997-08-07) N. FUJIMOTO ET AL: "cloning of a gene specifically expressed in the neurostem cell and developing central nervous system" XP002183301 cited in the application & UNPUBLISHED,
- DATABASE EMBL [Online] HS74397, accession number R30743, 3 May 1995 (1995-05-03) D.M. HWANG ET AL: "F9800 FETAL HEART HOMO SAPIENS CDNA CLONE F9800 5' END, MRNA SEQUENCE" XP002183302 & D.M. HWANG ET AL: "Analysis of expressed sequence tags from a fetal human heart cDNA library" GENOMICS, vol. 30, no. 2, 1995, pages 293-298,
- DATABASE EMBL [Online] HSM802230, accession number AL137500, 27 January 2000 (2000-01-27) B. OTTENWAELDER ET AL: "Homo saiens mRNA; cDNA DKFZp761F171 (from clone DKFZp761F171); partial cds" XP002190348
- DATABASE EMBL [Online] accession number AF086607, 3 August 1999 (1999-08-03) J.M. OTAKI ET AL: "Rattus norvegicus Neurestin alpha mRNA, complete cds." XP002190349 cited in the application -& J.M. OTAKI ET AL : "Neurestin: Putative transmembrane molecule implicated in neuronal development" DEVELOPMENTAL BIOLOGY, vol. 212, no. 1, 1999, pages 165-181, XP002190346
- DATABASE EMBL [Online] accession number AB025411, 10 May 1999 (1999-05-10) T. OOHASHI ET AL: "Mus musculus mRNA for Ten-m2, complete cdcs" XP002190350 cited in the application & T. OOHASHI ET AL: "Mouse Ten-m/Odz is a new family of dimeric type II transmembrane proteins expressed in many tissues" J.CELL BIOL., vol. 0, no. 0, 1999,
- DATABASE EMBL [Online] GGA245711, accession number AJ245711, 1 October 1999 (1999-10-01) R. CHIQUET-EHRISMANN: "Gallus gallus mRNA for teneurin-2, short splice variant (ten2 gene) short splice variant; ten2 gene; teneurin-2; Type II transmembrane protein" XP002190351 cited in the application -& B.P. RUBIN ET AL: "Teneurins: a novel family of Neuronal cell surface proteins in vertebrates, homologous to the Drosophila pair-rule gene product Ten-m" DEVELOMENTAL BIOLOGY, vol. 216, 1999, pages 195-209, XP002190393
- DATABASE EMBL [Online] accession number AB032953, 11 November 1999 (1999-11-11) O. OHARA ET AL: "Homo sapiens mRNA for KIAA1127 protein , partial cds" XP002190352 cited in the application -& M. HIROSAWA ET AL: "Characterization of cDNA clones selected by GneMark analysis from size-frationated cDNA libraries from human brain" DNA RESEARCH, vol. 6, 1999, pages 329-336, XP000924951
- YAMAUCHI T ET AL: "PURIFICATION AND MOLECULAR CLONING OF PROSTACYCLIN-STIMULATING FACTOR FROM SERUM-FREE CONDITIONED MEDIUM OF HUMAN DIPLOID FIBROBLAST CELLS" BIOCHEMICAL JOURNAL, PORTLAND PRESS, LONDON, GB, vol. 303, 1994, pages 591-598, XP002923572 ISSN: 0264-6021 cited in the application
- DATABASE EMBL [Online] acession number AC008642, 4 August 1999 (1999-08-04) DOE JOINT GENOME INSTITUTE: "Homo sapiens chromosome 5 clone CTB-180C19, complete sequence" XP002190385 & UNPUBLISHED,
- DATABASE EMBL [Online] Q61581, accession number Q61581, 1 November 1996 (1996-11-01) M.V. KATO ET AL: "a follistatin-like gene, mac-25, may act as a growth suppressor of osteosarcoma cells" XP002183303 cited in the application & ONCOGENE, vol. 12, 1996, pages 1361-1364,
- ZWIJSEN ET AL: "Characterization of a rat C6 glioma-secreted follistatin-related protein (FRP);cloning and sequencing of the human homologue" EUROPEAN JOURNAL OF BIOCHEMISTRY, BERLIN, DE, vol. 225, no. 3, November 1994 (1994-11), pages 937-946, XP002103181 ISSN: 0014-2956 cited in the application
- DATABASE EMBL [Online] AV292454, accession number AV292454, 3 November 1999 (1999-11-03) T. ARAKAWA ET AL: "Mus musculus 6 days neonate head cDNA, RIKEN full-length enriched library clone: 543042E24, 3' end partial sequence" XP002183304 & I. YAMANAKA ET AL: "Mapping of 19032 mouse cDNAs on mouse chromosomes" J.STR. FUNCT. GENOMICS, vol. 2, 2001, pages L72-L86,
- DATABASE EMBL [Online] accession number AF195419, 27 January 2000 (2000-01-27) T. BEN-ZUR ET AL: "Mus musculus ODZ2 (Odz2) mRNA, partial cds" XP002190353 cited in the application -& T. BEN-ZUR ET AL: "The mammalian Odz gene family: Homologs of a drosophila pair rule gene with expression implying distinct yet overlapping developmental roles" DEVELOPMENT BIOLOGY, vol. 117, January 2000 (2000-01), pages 107-120, XP002190347

### SEQUENCE LISTING

<110> Curagen Corporation
   Vernet, Cornie AM
   Fernandes, Elma
   Shimkets, Richard A
   Herrmann, John L
   Majumder, Kumud
   Mishra, Vishna
   Mezes, Peter S
   Rastelli, Luca
<120> NOVEL PROTEINS AND NUCLEIC ACIDS ENCODING SAME
<130> 15966-697-061
<140> PCT/US01/07160
   <141> 2001-03-05
<150> 60/186,592
   <151> 2000-03-03
<150> 60/187,293
   <151> 2000-03-06
<150> 60/186,718
   <151> 2000-03-03
<160> 98
<170> PatentIn Ver. 2.1
<210> 1
   <211> 771
   <212> DNA
   <213> Homo sapiens
<220>
   <221> CDS
   <222> (438)..(752)
<400> 1
   ggtcctcacc cccttcctct ctcccagcct cggtgtctgg ttacggctcc tctgctcgca 60
   ttgtgacttt gggccaggct gggggaaatg acccgggagg gtcccatgcg gctacataaa 120
   attggcagcc ttagaactag tgggaaggcg ggtgcgcgaa gtcgaggggc ggagagaggg 180
   ggccggagga gctgctttct gaatccaagt tcgtgggctc tctcagaagt cctcaggacg 240
   gagcagaggt ggccggcggg cccggctgac tgcgcctctg ctttctttcc ataacctttt 300
   ctttcggact cgaatcacgg ctgctgcgaa gggtctagtt ccggacacta gggccccaga 360
   tcgtgtcaca tccatatgac acttggaatg tgacagggca ggatgtgatc tttggctgtg 420
   aagtgtttgc ctacccc atg gcc tcc atc gag tgg agg aag gat ggc ttg    470
                      Met Ala Ser Ile Glu Trp Arg Lys Asp Gly Leu
                       1               5                  10
   gac atc cag ctg cca ggg gat gac ccc cac atc tct gtg cag ttt agg    518
   Asp Ile Gln Leu Pro Gly Asp Asp Pro His Ile Ser Val Gln Phe Arg
               15                  20                  25
   ggt gga ccc cag agg ttt gag gtg act ggc tgg ctg cag atc cag gct    566
   Gly Gly Pro Gln Arg Phe Glu Val Thr Gly Trp Leu Gln Ile Gln Ala
           30                  35                  40
   gtg cgt ccc agt gat gag ggc act tac cgc tgc ctt gcc cgc aat gcc    614
   Val Arg Pro Ser Asp Glu Gly Thr Tyr Arg Cys Leu Ala Arg Asn Ala
       45                  50                  55
   ctg ggt caa gtg gag gcc cct gct agc ttg aca gtg ctc aca cct gac    662
   Leu Gly Gln Val Glu Ala Pro Ala Ser Leu Thr Val Leu Thr Pro Asp
   60                  65                  70                  75
   cag ctg aac tct aca ggc atc ccc cag ctg cga tca cta aac ctg gtt    710
   Gln Leu Asn Ser Thr Gly Ile Pro Gln Leu Arg Ser Leu Asn Leu Val
                   80                  85                  90
   cct gag gag gag gct gag agt gaa gag aat gac gat tac tac            752
   Pro Glu Glu Glu Ala Glu Ser Glu Glu Asn Asp Asp Tyr Tyr
               95                  100                 105
   taggtccaga gctctggcc                                               771
<210> 2
   <211> 105
   <212> PRT
   <213> Homo sapiens
<400> 2
   Met Ala Ser Ile Glu Trp Arg Lys Asp Gly Leu Asp Ile Gln Leu Pro
   1 5 10 15
   Gly Asp Asp Pro His Ile Ser Val Gln Phe Arg Gly Gly Pro Gln Arg
   20 25 30
   Phe Glu Val Thr Gly Trp Leu Gln Ile Gln Ala Val Arg Pro Ser Asp
   35 40 45
   Glu Gly Thr Tyr Arg Cys Leu Ala Arg Asn Ala Leu Gly Gln Val Glu
   50 55 60
   Ala Pro Ala Ser Leu Thr Val Leu Thr Pro Asp Gln Leu Asn Ser Thr
   65 70 75 80
   Gly Ile Pro Gln Leu Arg Ser Leu Asn Leu Val Pro Glu Glu Glu Ala
   85 90 95
   Glu Ser Glu Glu Asn Asp Asp Tyr Tyr
   100 105
<210> 3
   <211> 5502
   <212> DNA
   <213> Homo sapiens
<220>
   <221> CDS
   <222> (420)..(2864)
<400> 3
   caatttcaca caggaaacag ctatgccatg attacgcaag ttggtaccga gctcggatcc 60
   actagtaacg gccgccagtg tgctggaatt cggcttactc actatagggc tcgagcggct 120
   gcccgggcag gtcattaatt ccatttcttt ttagagtatc acagctttct ccttcactga 180
   ccaccctttg cttcctgtca gaaagccctg gacagaactc tctgtgggat tctgcccatg 240
   tttctgagat atcgcctcaa ttgtcctggc tgggctgtcg ggtctgcccg ttttacagat 300
   gggcaaactg gagtgggaag tatccgggtg gcttcctcag gcctgcagct ggtggagcag 360
   ctactgaaac aatcaggagc ccagaagctt tgaagtcaca agaagagaag actcccaga 419
   atg cag tgt gat gtt ggt gat gga cgc ctg ttt cgc ctt tca ctt aaa 467
   Met Gln Cys Asp Val Gly Asp Gly Arg Leu Phe Arg Leu Ser Leu Lys
   1 5 10 15
   cgt gcc ctt tcc agc tgc cct gac ctc ttt ggg ctt tcc agc cgc aac    515
   Arg Ala Leu Ser Ser Cys Pro Asp Leu Phe Gly Leu Ser Ser Arg Asn
                   20                  25                  30
   gag ctg ctg gcc tcc tgc ggg aag aag ttc tgc agc cga ggg agc cgg    563
   Glu Leu Leu Ala Ser Cys Gly Lys Lys Phe Cys Ser Arg Gly Ser Arg
               35                  40                  45
   tgc gtg ctc agc agg aag aca ggg gag ccc gaa tgc cag tgc ctg gag    611
   Cys Val Leu Ser Arg Lys Thr Gly Glu Pro Glu Cys Gln Cys Leu Glu
           50                  55                  60
   gca tgc agg ccc agc tac gtg cct gtg tgc ggc tct gat ggg agg ttt    659
   Ala Cys Arg Pro Ser Tyr Val Pro Val Cys Gly Ser Asp Gly Arg Phe
       65                  70                  75                  80
   tat gaa aac cac tgt aag ctc cac cgt gct gct tgc ctc ctg gga aag    707
   Tyr Glu Asn His Cys Lys Leu His Arg Ala Ala Cys Leu Leu Gly Lys
                       85                  90                  95
   agg atc acc gtc atc cac agc aag gac tgt ttc ctc aaa ggt gac acg    755
   Arg Ile Thr Val Ile His Ser Lys Asp Cys Phe Leu Lys Gly Asp Thr
                   100                 105                 110
   tgc acc atg gcc ggc tac gcc cgc ttg aag aat gtc ctt ctg gca ctc    803
   Cys Thr Met Ala Gly Tyr Ala Arg Leu Lys Asn Val Leu Leu Ala Leu
               115                 120                 125
   cag acc cgt ctg cag cca ctc caa gaa gga gac agc aga caa gac cct    851
   Gln Thr Arg Leu Gln Pro Leu Gln Glu Gly Asp Ser Arg Gln Asp Pro
           130                 135                 140
   gcc tcc cag aag cgc ctc ctg gtg gaa tct ctg ttc agg gac tta gat    899
   Ala Ser Gln Lys Arg Leu Leu Val Glu Ser Leu Phe Arg Asp Leu Asp
   145                 150                 155                 160
   gca gat ggc aat ggc cac ctc agc agc tcc gaa ctg gct cag cat gtg    947
   Ala Asp Gly Asn Gly His Leu Ser Ser Ser Glu Leu Ala Gln His Val
                       165                 170                 175
   ctg aag aag cag gac ctg gat gaa gac tta ctt ggt tgc tca cca ggt    995
   Leu Lys Lys Gln Asp Leu Asp Glu Asp Leu Leu Gly Cys Ser Pro Gly
                   180                 185                 190
   gac ctc ctc cga ttt gac gat tac aac agt gac agc tcc ctg acc ctc    1043
   Asp Leu Leu Arg Phe Asp Asp Tyr Asn Ser Asp Ser Ser Leu Thr Leu
               195                 200                 205
   cgc gag ttc tac atg gcc ttc caa gtg gtt cag ctc agc ctc gcc ccc    1091
   Arg Glu Phe Tyr Met Ala Phe Gln Val Val Gln Leu Ser Leu Ala Pro
       210                 215                 220
   gag gac agg gtc agt gtg acc aca gtg acc gtg ggg ctg agc aca gtg    1139
   Glu Asp Arg Val Ser Val Thr Thr Val Thr Val Gly Leu Ser Thr Val
   225                 230                 235                 240
   ctg acc tgc gcc gtc cat gga gac ctg agg cca cca atc atc tgg aag    1187
   Leu Thr Cys Ala Val His Gly Asp Leu Arg Pro Pro Ile Ile Trp Lys
                   245                 250                 255
   cgc aac ggg ctc acc ctg aac ttc ctg gac ttg gaa gac atc aat gac    1235
   Arg Asn Gly Leu Thr Leu Asn Phe Leu Asp Leu Glu Asp Ile Asn Asp
               260                 265                 270
   ttt gga gag gat gat tcc ctg tac atc acc aag gtg acc acc atc cac    1283
   Phe Gly Glu Asp Asp Ser Leu Tyr Ile Thr Lys Val Thr Thr Ile His
           275                 280                 285
   atg ggc aat tac acc tgc cat gct tcc ggc cac gag cag ctg ttc cag    1331
   Met Gly Asn Tyr Thr Cys His Ala Ser Gly His Glu Gln Leu Phe Gln
       290                 295                 300
   acc cac gtc ctg cag gtg aat gtg ccg cca gtc atc cgt gtc tat cca    1379
   Thr His Val Leu Gln Val Asn Val Pro Pro Val Ile Arg Val Tyr Pro
   305                 310                 315                 320
   gag agc cag gca cag gag cct gga gtg gca gcc agc cta aga tgc cat    1427
   Glu Ser Gln Ala Gln Glu Pro Gly Val Ala Ala Ser Leu Arg Cys His
                   325                 330                 335
   gct gag ggc att ccc atg ccc aga atc act tgg ctg aaa aac ggc gtg    1475
   Ala Glu Gly Ile Pro Met Pro Arg Ile Thr Trp Leu Lys Asn Gly Val
               340                 345                 350
   gat gtc tca act cag atg tcc aaa cag ctc tcc ctt tta gcc aat ggg    1523
   Asp Val Ser Thr Gln Met Ser Lys Gln Leu Ser Leu Leu Ala Asn Gly
           355                 360                 365
   agc gaa ctc cac atc agc agt gtt cgg tat gaa gac aca ggg gca tac    1571
   Ser Glu Leu His Ile Ser Ser Val Arg Tyr Glu Asp Thr Gly Ala Tyr
       370                 375                 380
   acc tgc att gcc aaa aat gaa gtg ggt gtg gat gaa gat atc tcc tcg    1619
   Thr Cys Ile Ala Lys Asn Glu Val Gly Val Asp Glu Asp Ile Ser Ser
   385                 390                 395                 400
   ctc ttc att gaa gac tca gct aga aag acc ctt gca aac atc ctg tgg     1667
   Leu Phe Ile Glu Asp Ser Ala Arg Lys Thr Leu Ala Asn Ile Leu Trp
                   405                 410                 415
   cga gag gaa ggc ctc agc gtg gga aac atg ttc tat gtc ttc tcc gac     1715
   Arg Glu Glu Gly Leu Ser Val Gly Asn Met Phe Tyr Val Phe Ser Asp
               420                 425                 430
   gac ggt atc atc gtc atc cat cct gtg gac tgt gag atc cag agg cac     1763
   Asp Gly Ile Ile Val Ile His Pro Val Asp Cys Glu Ile Gln Arg His
           435                 440                 445
   ctc aaa ccc acg gaa aag att ttc atg agc tat gaa gaa atc tgt cct     1811
   Leu Lys Pro Thr Glu Lys Ile Phe Met Ser Tyr Glu Glu Ile Cys Pro
       450                 455                 460
   caa aga gaa aaa aat gca acc cag ccc tgc cag tgg gta tct gca gtc     1859
   Gln Arg Glu Lys Asn Ala Thr Gln Pro Cys Gln Trp Val Ser Ala Val
   465                 470                 475                 480
   aat gtc cgg aac cgg tac atc tat gtg gcc cag cca gca ctg agc aga     1907
   Asn Val Arg Asn Arg Tyr Ile Tyr Val Ala Gln Pro Ala Leu Ser Arg
                   485                 490                 495
   gtc ctt gtg gtc gac atc caa gcc cag aaa gtc cta cag tcc ata ggt     1955
   Val Leu Val Val Asp Ile Gln Ala Gln Lys Val Leu Gln Ser Ile Gly
               500                 505                 510
   gtg gac cct ctg ccg gct aag ctg tcc tat gac aag tca cat gac caa     2003
   Val Asp Pro Leu Pro Ala Lys Leu Ser Tyr Asp Lys Ser His Asp Gln
           515                 520                 525
   gtg tgg gtc ctg agc tgg ggg gac gtg cac aag tcc cga cca agt ctc     2051
   Val Trp Val Leu Ser Trp Gly Asp Val His Lys Ser Arg Pro Ser Leu
       530                 535                 540
   cag gtg atc aca gaa gcc agc acc ggc cag agc cag cac ctc atc cgc     2099
   Gln Val Ile Thr Glu Ala Ser Thr Gly Gln Ser Gln His Leu Ile Arg
   545                 550                 555                 560
   aca ccc ttt gca gga gtg gat gat ttc ttc att ccc cca aca aac ctc     2147
   Thr Pro Phe Ala Gly Val Asp Asp Phe Phe Ile Pro Pro Thr Asn Leu
                   565                 570                 575
   atc atc aac cac atc agg ttt ggc ttc atc ttc aac aag tct gat cct     2195
   Ile Ile Asn His Ile Arg Phe Gly Phe Ile Phe Asn Lys Ser Asp Pro
               580                 585                 590
   gca gtc cac aag gtg gac ctg gaa aca atg atg ccc ctc aag acc atc     2243
   Ala Val His Lys Val Asp Leu Glu Thr Met Met Pro Leu Lys Thr Ile
           595                 600                 605
   ggc ctg cac cac cat ggc tgc gtg ccc cag gcc atg gca cac acc cac     2291
   Gly Leu His His His Gly Cys Val Pro Gln Ala Met Ala His Thr His
       610                 615                 620
   ctg ggc ggc tac ttc ttc atc cag tgc cga cag gac agc ccc gcc tct     2339
   Leu Gly Gly Tyr Phe Phe Ile Gln Cys Arg Gln Asp Ser Pro Ala Ser
   625                 630                 635                 640
   gct gcc cga cag ctg ctc gtt gac agt gtc aca gac tct gtg ctt ggc     2387
   Ala Ala Arg Gln Leu Leu Val Asp Ser Val Thr Asp Ser Val Leu Gly
                   645                 650                 655
   ccc aat ggt gat gta aca ggc acc cca cac aca tcc ccc gac ggg cgc     2435
   Pro Asn Gly Asp Val Thr Gly Thr Pro His Thr Ser Pro Asp Gly Arg
               660                 665                 670
   ttc ata gtc agt gct gca gct gac agc ccc tgg ctg cac gtg cag gag     2483
   Phe Ile Val Ser Ala Ala Ala Asp Ser Pro Trp Leu His Val Gln Glu
           675                 680                 685
   atc aca gtg cgg ggc gag atc cag acc ctg tat gac ctg caa ata aac     2531
   Ile Thr Val Arg Gly Glu Ile Gln Thr Leu Tyr Asp Leu Gln Ile Asn
       690                 695                 700
   tcg ggc atc tca gac ttg gcc ttc cag cgc tcc ttc act gaa agc aat     2579
   Ser Gly Ile Ser Asp Leu Ala Phe Gln Arg Ser Phe Thr Glu Ser Asn
   705                 710                 715                 720
   caa tac aac atc tac gcg gct ctg cac acg gag ccg gac ctg ctg ttc     2627
   Gln Tyr Asn Ile Tyr Ala Ala Leu His Thr Glu Pro Asp Leu Leu Phe
                   725                 730                 735
   ctg gag ctg tcc acg ggg aag gtg ggc atg ctg aag aac tta aag gag     2675
   Leu Glu Leu Ser Thr Gly Lys Val Gly Met Leu Lys Asn Leu Lys Glu
               740                 745                 750
   cca ccc gca ggg cca gct cag ccc tgg ggg ggt acc cac aga atc atg     2723
   Pro Pro Ala Gly Pro Ala Gln Pro Trp Gly Gly Thr His Arg Ile Met
           755                 760                 765
   agg gac agt ggg ctg ttt gga cag tac ctc ctc aca cca gcc cga gag     2771
   Arg Asp Ser Gly Leu Phe Gly Gln Tyr Leu Leu Thr Pro Ala Arg Glu
       770                 775                 780
   tca ctg ttc ctc atc aat ggg aga caa aac acg ctg cgg tgt gag gtg     2819
   Ser Leu Phe Leu Ile Asn Gly Arg Gln Asn Thr Leu Arg Cys Glu Val
   785                 790                 795                 800
   tca ggt ata aag ggg ggg acc aca gtg gtg tgg gtg ggt gag gta         2864
   Ser Gly Ile Lys Gly Gly Thr Thr Val Val Trp Val Gly Glu Val
                   805                 810                 815
   tgaagggccc agagcagagc cctgggccaa ggaacacccc ctagtcctga cactgcagcc 2924
   tcaagcaggt acgctgtaca tttttacaga caaaagcaaa aacctgtact cgctttgtgg 2984
   ttcaacactg gtctccttgc aagtttccta gtataaggta tgcgctgcta ccaagattgg 3044
   ggttttttcg ttaggaagta tgatttatgc cttgagctac gatgagaaca tatgctgctg 3104
   tgtaaaggga tcatttctgt gccaagctgc acaccgagtg acctggggac atcatggaac 3164
   caagggatcc tgctctccaa gcagacacct ctgtcagttg ccttcacata gtcattgtcc 3224
   cttactgcca gacccagcca gactttgccc tgacggagtg gcccggaagc agaggccgac 3284
   caggagcagg ggcctccctc ccgaactgaa agcccatccg tcctcgcgtg ggaccgcatc 3344
   ttctccctcg cagctgcttc ttgcttttct ttccatttga cttgctgtaa gcctgaggga 3404
   gagccaacaa gacttactgc atcttggggg atggggaaat cactcacttt attttggaaa 3464
   tttttgatta aaaaaaaatt ttataatctc aaatgctagt aagcagaaag atgctctccg 3524
   aggtccaact atatccttcc ctgccttagg ccgagtctcg ggggtggtca caaccccaca 3584
   tcccacagcc agaaagaaca atggtcatct gagaatactg gccctgtcga ctattgccac 3644
   cctgcttctc caagagcaga ccaggccacc tcatccgtaa ggactcggtt ctgtgttggg 3704
   accccaaaaa accagaacaa gttctgtgtg cctcctttca gcacagaagg gagacatctc 3764
   attagtcagg tctggtaccc cagattcagg gcagactggg cttgcctggc aaggtatggg 3824
   tggcctccag gctcaatgca gaaaccccaa ggacacgagt ggggccaggt gagttcctga 3884
   agctatacct tttcaaaaca gattttgttt tcctacctgt ggcccatcca ctcctctctg 3944
   gtaccccatc cccgcatcag cactgcagag agaacacatt tcggcgaggg ttttcttacc 4004
   cacattcccc aatcaataca cacacactgc agaacccaga acagaaggcc acaggctggc 4064
   actactgcat tctccttatg tgtctcaggc tgtggtgact ctcacatggg catcgaagaa 4124
   gtacaaccca catagccctc tggagaccgc ctagatcaga gactcagcaa aaacaggctc 4184
   gccttccctc tcccacatat gagtggaact tacatgtgtc ctggtttgaa tgatcatttt 4244
   gcaagccaca cgggttggga gaggtggtct caccacagac gtctttgcta atttggccac 4304
   cttcacctac tgacatgacc aggattttcc tttgccatta aggaatgaac tctttcaagg 4364
   agaggaaacc ctagactctg tgtcactctc aacacacaca gctcctttca ctcctgcctg 4424
   actgccaagc cacctgcatc ccccgcccca gatctcatga gatcaatcac ttgtatgtct 4484
   cacgcaactt ggtccaccaa acgcctgtcc cctgtaactc ctaggggtgc gcctagacag 4544
   gtacgtctgt tttttatttt aaaagatatg ctatgtagat ataagttgag gaagctcacc 4604
   tcaaaagcct agaatgcagt ttcacagtag ctgggatgca tggatgaccc atctcacccc 4664
   tttttttttc ctgcctcaat atcttgatat gttatgttta ctcccaatct cccattttta 4724
   ccactaaaat tctccaactt tcataaactt ttttttggaa aaatttccat tgtatcagcc 4784
   cctgacagaa aaaggatctc tgagcctaaa ggaggaaaag tcccaccaac taccagacca 4844
   gaacacgagc ccctctgggc agcaggattc ctaagtcaaa gaccagtttg acccaaactg 4904
   gccttttaaa ataatcagga gtgacagagt caacttctgc agcacctgct tctcccccac 4964
   tgtcccttcc atcttggaat gtgtctaaaa aagcatagct gccctttgct gtcctcagag 5024
   tgcatttcct ggagacggca ggcttaggtc tcactgacag catgccagac acaactgaat 5084
   cgaagcaggc ctgaagccta ggtcagggtt tcaggagtcc agccccagga ggcaaagtca 5144
   ccaatgcagg gaggtaaatg ccttttggca ggaaaaccaa tagagttggt tgggtgggga 5204
   gtcaggggtg ggaggagaag gaggaagagg aggaaggcca gactggcctg ccctttctcc 5264
   catacttcac cccagcagag gttcatggga cacagttgga aagccactgg gaggaaatgc 5324
   ctcactacag gggggcctcc tgtagcaagc ccagccggta atcctcctaa tgaacccaca 5384
   aggtcaattc acaactgata tcttagctat taaagaagta ctgactttac caaaagaatc 5444
   atcaagaaag ctatttatat aaaccccctc agtcattttg aaataaaatt aattttac 5502
<210> 4
   <211> 815
   <212> PRT
   <213> Homo sapiens
<400> 4
   Met Gln Cys Asp Val Gly Asp Gly Arg Leu Phe Arg Leu Ser Leu Lys
   1               5                   10                  15
   Arg Ala Leu Ser Ser Cys Pro Asp Leu Phe Gly Leu Ser Ser Arg Asn
               20                  25                  30
   Glu Leu Leu Ala Ser Cys Gly Lys Lys Phe Cys Ser Arg Gly Ser Arg
           35                  40                  45
   Cys Val Leu Ser Arg Lys Thr Gly Glu Pro Glu Cys Gln Cys Leu Glu
       50                  55                  60
   Ala Cys Arg Pro Ser Tyr Val Pro Val Cys Gly Ser Asp Gly Arg Phe
   65                  70                  75                  80
   Tyr Glu Asn His Cys Lys Leu His Arg Ala Ala Cys Leu Leu Gly Lys
                   85                  90                  95
   Arg Ile Thr Val Ile His Ser Lys Asp Cys Phe Leu Lys Gly Asp Thr
               100                 105                 110
   Cys Thr Met Ala Gly Tyr Ala Arg Leu Lys Asn Val Leu Leu Ala Leu
           115                 120                 125
   Gln Thr Arg Leu Gln Pro Leu Gln Glu Gly Asp Ser Arg Gln Asp Pro
       130                 135                 140
   Ala Ser Gln Lys Arg Leu Leu Val Glu Ser Leu Phe Arg Asp Leu Asp
   145                 150                 155                 160
   Ala Asp Gly Asn Gly His Leu Ser Ser Ser Glu Leu Ala Gln His Val
                   165                 170                 175
   Leu Lys Lys Gln Asp Leu Asp Glu Asp Leu Leu Gly Cys Ser Pro Gly
               180                 185                 190
   Asp Leu Leu Arg Phe Asp Asp Tyr Asn Ser Asp Ser Ser Leu Thr Leu
           195                 200                 205
   Arg Glu Phe Tyr Met Ala Phe Gln Val Val Gln Leu Ser Leu Ala Pro
       210                 215                 220
   Glu Asp Arg Val Ser Val Thr Thr Val Thr Val Gly Leu Ser Thr Val
   225 230 235 240
   Leu Thr Cys Ala Val His Gly Asp Leu Arg Pro Pro Ile Ile Trp Lys
   245 250 255
   Arg Asn Gly Leu Thr Leu Asn Phe Leu Asp Leu Glu Asp Ile Asn Asp
   260 265 270
   Phe Gly Glu Asp Asp Ser Leu Tyr Ile Thr Lys Val Thr Thr Ile His
   275 280 285
   Met Gly Asn Tyr Thr Cys His Ala Ser Gly His Glu Gln Leu Phe Gln
   290 295 300
   Thr His Val Leu Gln Val Asn Val Pro Pro Val Ile Arg Val Tyr Pro
   305 310 315 320
   Glu Ser Gln Ala Gln Glu Pro Gly Val Ala Ala Ser Leu Arg Cys His
   325 330 335
   Ala Glu Gly Ile Pro Met Pro Arg Ile Thr Trp Leu Lys Asn Gly Val
   340 345 350
   Asp Val Ser Thr Gln Met Ser Lys Gln Leu Ser Leu Leu Ala Asn Gly
   355 360 365
   Ser Glu Leu His Ile Ser Ser Val Arg Tyr Glu Asp Thr Gly Ala Tyr
   370 375 380
   Thr Cys Ile Ala Lys Asn Glu Val Gly Val Asp Glu Asp Ile Ser Ser
   385 390 395 400
   Leu Phe Ile Glu Asp Ser Ala Arg Lys Thr Leu Ala Asn Ile Leu Trp
   405 410 415
   Arg Glu Glu Gly Leu Ser Val Gly Asn Met Phe Tyr Val Phe Ser Asp
   420 425 430
   Asp Gly Ile Ile Val Ile His Pro Val Asp Cys Glu Ile Gln Arg His
   435 440 445
   Leu Lys Pro Thr Glu Lys Ile Phe Met Ser Tyr Glu Glu Ile Cys Pro
   450 455 460
   Gln Arg Glu Lys Asn Ala Thr Gln Pro Cys Gln Trp Val Ser Ala Val
   465 470 475 480
   Asn Val Arg Asn Arg Tyr Ile Tyr Val Ala Gln Pro Ala Leu Ser Arg
   485 490 495
   Val Leu Val Val Asp Ile Gln Ala Gln Lys Val Leu Gln Ser Ile Gly
   500 505 510
   Val Asp Pro Leu Pro Ala Lys Leu Ser Tyr Asp Lys Ser His Asp Gln
   515 520 525
   Val Trp Val Leu Ser Trp Gly Asp Val His Lys Ser Arg Pro Ser Leu
   530 535 540
   Gln Val Ile Thr Glu Ala Ser Thr Gly Gln Ser Gln His Leu Ile Arg
   545 550 555 560
   Thr Pro Phe Ala Gly Val Asp Asp Phe Phe Ile Pro Pro Thr Asn Leu
   565 570 575
   Ile Ile Asn His Ile Arg Phe Gly Phe Ile Phe Asn Lys Ser Asp Pro
   580 585 590
   Ala Val His Lys Val Asp Leu Glu Thr Met Met Pro Leu Lys Thr Ile
   595 600 605
   Gly Leu His His His Gly Cys Val Pro Gln Ala Met Ala His Thr His
   610 615 620
   Leu Gly Gly Tyr Phe Phe Ile Gln Cys Arg Gln Asp Ser Pro Ala Ser
   625 630 635 640
   Ala Ala Arg Gln Leu Leu Val Asp Ser Val Thr Asp Ser Val Leu Gly
   645 650 655
   Pro Asn Gly Asp Val Thr Gly Thr Pro His Thr Ser Pro Asp Gly Arg
   660 665 670
   Phe Ile Val Ser Ala Ala Ala Asp Ser Pro Trp Leu His Val Gln Glu
   675 680 685
   Ile Thr Val Arg Gly Glu Ile Gln Thr Leu Tyr Asp Leu Gln Ile Asn
   690 695 700
   Ser Gly Ile Ser Asp Leu Ala Phe Gln Arg Ser Phe Thr Glu Ser Asn
   705 710 715 720
   Gln Tyr Asn Ile Tyr Ala Ala Leu His Thr Glu Pro Asp Leu Leu Phe
   725 730 735
   Leu Glu Leu Ser Thr Gly Lys Val Gly Met Leu Lys Asn Leu Lys Glu
               740                 745                 750
   Pro Pro Ala Gly Pro Ala Gln Pro Trp Gly Gly Thr His Arg Ile Met
           755                 760                 765
   Arg Asp Ser Gly Leu Phe Gly Gln Tyr Leu Leu Thr Pro Ala Arg Glu
       770                 775                 780
   Ser Leu Phe Leu Ile Asn Gly Arg Gln Asn Thr Leu Arg Cys Glu Val
   785                 790                 795                 800
   Ser Gly Ile Lys Gly Gly Thr Thr Val Val Trp Val Gly Glu Val
                   805                 810                 815
<210> 5
   <211> 1430
   <212> DNA
   <213> Homo sapiens
<220>
   <221> CDS
   <222> (69)..(1211)
<400> 5
   aaaaaaggcg gggggtggac ttagcagtgt aatttgagac cggtggtaag gattggagcg 60
   agctagag atg ctg cac gct gct aac aag gga agg aag cct tca gct gag  110
            Met Leu His Ala Ala Asn Lys Gly Arg Lys Pro Ser Ala Glu
            1               5                   10
   gca ggt cgt ccc att cca cct aca tcc tcg cct agt ctc ctc cca tct   158
   Ala Gly Arg Pro Ile Pro Pro Thr Ser Ser Pro Ser Leu Leu Pro Ser
   15                  20                  25                  30
   gct cag ctg cct agc tcc cat aat cct cca cca gtt agc tgc cag atg   206
   Ala Gln Leu Pro Ser Ser His Asn Pro Pro Pro Val Ser Cys Gln Met
                   35                  40                  45
   cca ttg cta gac agc aac acc tcc cat caa atc atg gac acc aac cct   254
   Pro Leu Leu Asp Ser Asn Thr Ser His Gln Ile Met Asp Thr Asn Pro
               50                  55                  60
   gat gag gaa ttc tcc ccc aat tca tac ctg ctc aga gca tgc tca ggg   302
   Asp Glu Glu Phe Ser Pro Asn Ser Tyr Leu Leu Arg Ala Cys Ser Gly
   65 70 75
   ccc cag caa gcc tcc agc agt ggc cct ccg aac cac cac agc cag tcg 350
   Pro Gln Gln Ala Ser Ser Ser Gly Pro Pro Asn His His Ser Gln Ser
   80 85 90
   act ctg agg ccc cct ctc cca ccc cct cac aac cac acg ctg tcc cat 398
   Thr Leu Arg Pro Pro Leu Pro Pro Pro His Asn His Thr Leu Ser His
   95 100 105 110
   cac cac tcg tcc gcc aac tcc ctc aac agg aac tca ctg acc aat cgg 446
   His His Ser Ser Ala Asn Ser Leu Asn Arg Asn Ser Leu Thr Asn Arg
   115 120 125
   cgg agt cag atc cac gcc ccg gcc cca gcg ccc aat gac ctg gcc acc 494
   Arg Ser Gln Ile His Ala Pro Ala Pro Ala Pro Asn Asp Leu Ala Thr
   130 135 140
   aca cca gag tcc gtt cag ctt cag gac agc tgg gtg cta aac agc aac 542
   Thr Pro Glu Ser Val Gln Leu Gln Asp Ser Trp Val Leu Asn Ser Asn
   145 150 155
   gtg cca ctg gag acc cgg cac ttc ctc ttc aag acc tcc tcg ggg agc 590
   Val Pro Leu Glu Thr Arg His Phe Leu Phe Lys Thr Ser Ser Gly Ser
   160 165 170
   aca ccc ttg ttc agc agc tct tcc ccg gga tac cct ttg acc tca gga 638
   Thr Pro Leu Phe Ser Ser Ser Ser Pro Gly Tyr Pro Leu Thr Ser Gly
   175 180 185 190
   acg gtt tac acg ccc ccg ccc cgc ctg ctg ccc agg aat act ttc tcc 686
   Thr Val Tyr Thr Pro Pro Pro Arg Leu Leu Pro Arg Asn Thr Phe Ser
   195 200 205
   agg aag gct ttc aag ctg aag aag ccc tcc aaa tac tgc agc tgg aaa 734
   Arg Lys Ala Phe Lys Leu Lys Lys Pro Ser Lys Tyr Cys Ser Trp Lys
   210 215 220
   tgt gct gcc ctc tcc gcc att gcc gcg gcc ctc ctc ttg gct att ttg 782
   Cys Ala Ala Leu Ser Ala Ile Ala Ala Ala Leu Leu Leu Ala Ile Leu
   225 230 235
   ctg gcg tat ttc ata gtg ccc tgg tcg ttg aaa aac agc agc ata gac 830
   Leu Ala Tyr Phe Ile Val Pro Trp Ser Leu Lys Asn Ser Ser Ile Asp
   240 245 250
   agt ggt gaa gca gaa gtt ggt cgg cgg gta aca caa gaa gtc cca cca 878
   Ser Gly Glu Ala Glu Val Gly Arg Arg Val Thr Gln Glu Val Pro Pro
   255 260 265 270
   ggg gtg ttt tgg agg tca caa att cac atc agt cag ccc cag ttc tta 926
   Gly Val Phe Trp Arg Ser Gln Ile His Ile Ser Gln Pro Gln Phe Leu
   275 280 285
   aag ttc aac atc tcc ctc ggg aag gac gct ctc ttt ggt gtt tac ata 974
   Lys Phe Asn Ile Ser Leu Gly Lys Asp Ala Leu Phe Gly Val Tyr Ile
   290 295 300
   aga aga gga ctt cca cca tct cat gcc cag tat gac ttc atg gaa cgt 1022
   Arg Arg Gly Leu Pro Pro Ser His Ala Gln Tyr Asp Phe Met Glu Arg
   305 310 315
   ctg gac ggg aag gag aag tgg agt gtg gtt gag tct ccc agg gaa cgc 1070
   Leu Asp Gly Lys Glu Lys Trp Ser Val Val Glu Ser Pro Arg Glu Arg
   320 325 330
   cgg agc ata cag acc ttg gtt cag aat gaa gcc gtg ttt gtg cag tac 1118
   Arg Ser Ile Gln Thr Leu Val Gln Asn Glu Ala Val Phe Val Gln Tyr
   335 340 345 350
   ctg gat gtg ggc ctg tgg cat ctg gcc ttc tac aat gat gga aaa gac 1166
   Leu Asp Val Gly Leu Trp His Leu Ala Phe Tyr Asn Asp Gly Lys Asp
   355 360 365
   aaa gag atg gtt tcc ttc aat act gtt gtc cta gat ggg acc atc 1211
   Lys Glu Met Val Ser Phe Asn Thr Val Val Leu Asp Gly Thr Ile
   370 375 380
   tagttgcaga aaaacaagct cagggcgccc actgatttga cattatgatt cagtgcagga 1271
   ctgtccacgt aactgccatg ggaatggtga atgtgtgtcc ggggtgtgtc actgtttccc 1331
   aggatttcta ggagcagact gtgctaaaga ccttcctgcc ttgactttct gcaagacaat 1391
   cattaataaa gctgctctgt aaatactaaa aaaaaaaca 1430
<210> 6
   <211> 381
   <212> PRT
   <213> Homo sapiens
<400> 6
   Met Leu His Ala Ala Asn Lys Gly Arg Lys Pro Ser Ala Glu Ala Gly
   1 5 10 15
   Arg Pro Ile Pro Pro Thr Ser Ser Pro Ser Leu Leu Pro Ser Ala Gln
   20 25 30
   Leu Pro Ser Ser His Asn Pro Pro Pro Val Ser Cys Gln Met Pro Leu
   35 40 45
   Leu Asp Ser Asn Thr Ser His Gln Ile Met Asp Thr Asn Pro Asp Glu
   50 55 60
   Glu Phe Ser Pro Asn Ser Tyr Leu Leu Arg Ala Cys Ser Gly Pro Gln
   65 70 75 80
   Gln Ala Ser Ser Ser Gly Pro Pro Asn His His Ser Gln Ser Thr Leu
   85 90 95
   Arg Pro Pro Leu Pro Pro Pro His Asn His Thr Leu Ser His His His
   100 105 110
   Ser Ser Ala Asn Ser Leu Asn Arg Asn Ser Leu Thr Asn Arg Arg Ser
   115 120 125
   Gln Ile His Ala Pro Ala Pro Ala Pro Asn Asp Leu Ala Thr Thr Pro
   130 135 140
   Glu Ser Val Gln Leu Gln Asp Ser Trp Val Leu Asn Ser Asn Val Pro
   145 150 155 160
   Leu Glu Thr Arg His Phe Leu Phe Lys Thr Ser Ser Gly Ser Thr Pro
   165 170 175
   Leu Phe Ser Ser Ser Ser Pro Gly Tyr Pro Leu Thr Ser Gly Thr Val
   180 185 190
   Tyr Thr Pro Pro Pro Arg Leu Leu Pro Arg Asn Thr Phe Ser Arg Lys
   195 200 205
   Ala Phe Lys Leu Lys Lys Pro Ser Lys Tyr Cys Ser Trp Lys Cys Ala
   210 215 220
   Ala Leu Ser Ala Ile Ala Ala Ala Leu Leu Leu Ala Ile Leu Leu Ala
   225 230 235 240
   Tyr Phe Ile Val Pro Trp Ser Leu Lys Asn Ser Ser Ile Asp Ser Gly
   245 250 255
   Glu Ala Glu Val Gly Arg Arg Val Thr Gln Glu Val Pro Pro Gly Val
   260 265 270
   Phe Trp Arg Ser Gln Ile His Ile Ser Gln Pro Gln Phe Leu Lys Phe
   275 280 285
   Asn Ile Ser Leu Gly Lys Asp Ala Leu Phe Gly Val Tyr Ile Arg Arg
   290 295 300
   Gly Leu Pro Pro Ser His Ala Gln Tyr Asp Phe Met Glu Arg Leu Asp
   305 310 315 320
   Gly Lys Glu Lys Trp Ser Val Val Glu Ser Pro Arg Glu Arg Arg Ser
   325 330 335
   Ile Gln Thr Leu Val Gln Asn Glu Ala Val Phe Val Gln Tyr Leu Asp
   340 345 350
   Val Gly Leu Trp His Leu Ala Phe Tyr Asn Asp Gly Lys Asp Lys Glu
   355 360 365
   Met Val Ser Phe Asn Thr Val Val Leu Asp Gly Thr Ile
   370 375 380
<210> 7
   <211> 9826
   <212> DNA
   <213> Homo sapiens
<220>
   <221> CDS
   <222> (280)..(8478)
<400> 7
<210> 8
   <211> 2733
   <212> PRT
   <213> Homo sapiens
<400> 8
<210> 9
   <211> 201
   <212> DNA
   <213> Homo sapiens
<400> 9
   gctccaaagc gagctgggac cgaagactct aggctaagtt atctatgtag atggtgtcag 60
   ggagcgaagc tactgaccga gctgctgtta catccagctt tttaattgcc taagcggtct 120
   ggggcttgct tcgtcatttg gctttgctgt ggagcactcc tgtaaagcca gctgaattgt 180
   acatcgaaga tccacccttt t                                           201
<210> 10
   <211> 201
   <212> DNA
   <213> Homo sapiens
<400> 10
   gctccaaagc gagctgggac cgaagactct aggctaagtt atctatgtag atggtgtcag 60
   ggagcgaagc tactgaccga gctgctgtta catccagctt tttaattgcc taagcggtct 120
   ggggcttgct tcgtcatttg gctttgctgt ggagcactcc tgtaaagcca gctgaattgt 180
   acatcgaaga tccacccttt t                                           201
<210> 11
   <211> 134
   <212> DNA
   <213> Homo sapiens
<400> 11
   ccagcattag atgagttgac aaaaatgcag tttcagctct gaaggtctga aagattctgc 60
   tgcaactaaa gctctgaaga ttctgctaca actatgacat ccattttctc ccacttcaga 120
   caggatgaat acaa                                                   134
<210> 12
   <211> 9729
   <212> DNA
   <213> Homo sapiens
<220>
   <221> CDS
   <222> (210)..(8381)
<400> 12
   ccagcattag atgagttgac aaaaatgcag tttcagctct gaaggtctga aagattctgc 60
   tgcaactaaa gctctgaaga ttctgctaca actatgacat ccattttctc ccacttcaga 120
   caggatgaat acaaggtggc aaagtgacaa gtgccaaaac tcaggcctga ctttcctgaa 180
   aacatcagca ttctgccata tctggaata atg gat gta aag gac cgg cga cac   233
                                   Met Asp Val Lys Asp Arg Arg His
                                    1               5
   cgc tct ttg acc aga gga cgc tgt ggc aaa gag tgt cgc tac aca agc   281
   Arg Ser Leu Thr Arg Gly Arg Cys Gly Lys Glu Cys Arg Tyr Thr Ser
        10                  15                  20
   tcc tct ctg gac agt gag gac tgc cgg gtg ccc aca cag aaa tcc tac   329
   Ser Ser Leu Asp Ser Glu Asp Cys Arg Val Pro Thr Gln Lys Ser Tyr
    25                  30                  35                  40
   agc tcc agt gag act ctg aag gcc tat gac cat gac agc agg atg cac   377
   Ser Ser Ser Glu Thr Leu Lys Ala Tyr Asp His Asp Ser Arg Met His
                    45                  50                  55
   tat gga aac cga gtc aca gac ctc atc cac cgg gag tca gat gag ttt   425
   Tyr Gly Asn Arg Val Thr Asp Leu Ile His Arg Glu Ser Asp Glu Phe
                60                  65                  70
   cct aga caa gga acc aac ttc acc ctt gcc gaa ctg ggc atc tgt gag   473
   Pro Arg Gln Gly Thr Asn Phe Thr Leu Ala Glu Leu Gly Ile Cys Glu
            75                  80                  85
   ccc tcc cca cac cga agc ggc tac tgc tcc gac atg ggg atc ctt cac   521
   Pro Ser Pro His Arg Ser Gly Tyr Cys Ser Asp Met Gly Ile Leu His
        90                  95                 100
   cag ggc tac tcc ctt agc aca ggg tct gac gcc gac tcc gac acc gag   569
   Gln Gly Tyr Ser Leu Ser Thr Gly Ser Asp Ala Asp Ser Asp Thr Glu
   105                 110                 115                 120
   gga ggg atg tct cca gaa cac gcc atc aga ctg tgg ggc aga ggg ata   617
   Gly Gly Met Ser Pro Glu His Ala Ile Arg Leu Trp Gly Arg Gly Ile
                   125                 130                 135
   aaa tcc agg cgc agt tcc ggc ctg tcc agt cgt gaa aac tcg gcc ctt   665
   Lys Ser Arg Arg Ser Ser Gly Leu Ser Ser Arg Glu Asn Ser Ala Leu
               140                 145                 150
   acc ctg act gac tct gac aac gaa aac aaa tca gat gat gag aac ggt   713
   Thr Leu Thr Asp Ser Asp Asn Glu Asn Lys Ser Asp Asp Glu Asn Gly
           155                 160                 165
   cgt ccc att cca cct aca tcc tcg cct agt ctc ctc cca tct gct cag   761
   Arg Pro Ile Pro Pro Thr Ser Ser Pro Ser Leu Leu Pro Ser Ala Gln
       170                 175                 180
   ctg cct agc tcc cat aat cct cca cca gtt agc tgc cag atg cca ttg    809
   Leu Pro Ser Ser His Asn Pro Pro Pro Val Ser Cys Gln Met Pro Leu
   185                 190                 195                 200
   cta gac agc aac acc tcc cat caa atc atg gac acc aac cct gat gag    857
   Leu Asp Ser Asn Thr Ser His Gln Ile Met Asp Thr Asn Pro Asp Glu
                   205                 210                 215
   gaa ttc tcc ccc aat tca tac ctg ctc aga gca tgc tca ggg ccc cag    905
   Glu Phe Ser Pro Asn Ser Tyr Leu Leu Arg Ala Cys Ser Gly Pro Gln
               220                 225                 230
   caa gcc tcc agc agt ggc cct ccg aac cac cac agc cag tcg act ctg    953
   Gln Ala Ser Ser Ser Gly Pro Pro Asn His His Ser Gln Ser Thr Leu
           235                 240                 245
   agg ccc cct ctc cca ccc cct cac aac cac acg ctg tcc cat cac cac    1001
   Arg Pro Pro Leu Pro Pro Pro His Asn His Thr Leu Ser His His His
       250                 255                 260
   tcg tcc gcc aac tcc ctc aac agg aac tca ctg acc aat cgg cgg agt    1049
   Ser Ser Ala Asn Ser Leu Asn Arg Asn Ser Leu Thr Asn Arg Arg Ser
   265                 270                 275                 280
   cag atc cac gcc ccg gcc cca gcg ccc aat gac ctg gcc acc aca cca    1097
   Gln Ile His Ala Pro Ala Pro Ala Pro Asn Asp Leu Ala Thr Thr Pro
                   285                 290                 295
   gag tcc gtt cag ctt cag gac agc tgg gtg cta aac agc aac gtg cca    1145
   Glu Ser Val Gln Leu Gln Asp Ser Trp Val Leu Asn Ser Asn Val Pro
               300                 305                 310
   ctg gag acc cgg cac ttc ctc ttc aag acc tcc tcg ggg agc aca ccc    1193
   Leu Glu Thr Arg His Phe Leu Phe Lys Thr Ser Ser Gly Ser Thr Pro
           315                 320                 325
   ttg ttc agc agc tct tcc ccg gga tac cct ttg acc tca gga acg gtt    1241
   Leu Phe Ser Ser Ser Ser Pro Gly Tyr Pro Leu Thr Ser Gly Thr Val
       330                 335                 340
   tac acg ccc ccg ccc cgc ctg ctg ccc agg aat act ttc tcc agg aag    1289
   Tyr Thr Pro Pro Pro Arg Leu Leu Pro Arg Asn Thr Phe Ser Arg Lys
   345                 350                 355                 360
   gct ttc aag ctg aag aag ccc tcc aaa tac tgc agc tgg aaa tgt gct    1337
   Ala Phe Lys Leu Lys Lys Pro Ser Lys Tyr Cys Ser Trp Lys Cys Ala
                       365                 370                 375
   gcc ctc tcc gcc att gcc gcg gcc ctc ctc ttg gct att ttg ctg gcg    1385
   Ala Leu Ser Ala Ile Ala Ala Ala Leu Leu Leu Ala Ile Leu Leu Ala
                   380                 385                 390
   tat ttc ata gtg ccc tgg tcg ttg aaa aac agc agc ata gac agt ggt    1433
   Tyr Phe Ile Val Pro Trp Ser Leu Lys Asn Ser Ser Ile Asp Ser Gly
               395                 400                 405
   gaa gca gaa gtt ggt cgg cgg gta aca caa gaa gtc cca cca ggg gtg    1481
   Glu Ala Glu Val Gly Arg Arg Val Thr Gln Glu Val Pro Pro Gly Val
           410                 415                 420
   ttt tgg agg tca caa att cac atc agt cag ccc cag ttc tta aag ttc    1529
   Phe Trp Arg Ser Gln Ile His Ile Ser Gln Pro Gln Phe Leu Lys Phe
   425                 430                 435                 440
   aac atc tcc ctc ggg aag gac gct ctc ttt ggt gtt tac ata aga aga    1577
   Asn Ile Ser Leu Gly Lys Asp Ala Leu Phe Gly Val Tyr Ile Arg Arg
                       445                 450                 455
   gga ctt cca cca tct cat gcc cag tat gac ttc atg gaa cgt ctg gac    1625
   Gly Leu Pro Pro Ser His Ala Gln Tyr Asp Phe Met Glu Arg Leu Asp
                   460                 465                 470
   ggg aag gag aag tgg agt gtg gtt gag tct ccc agg gaa cgc cgg agc    1673
   Gly Lys Glu Lys Trp Ser Val Val Glu Ser Pro Arg Glu Arg Arg Ser
               475                 480                 485
   ata cag acc ttg gtt cag aat gaa gcc gtg ttt gtg cag tac ctg gat    1721
   Ile Gln Thr Leu Val Gln Asn Glu Ala Val Phe Val Gln Tyr Leu Asp
           490                 495                 500
   gtg ggc ctg tgg cat ctg gcc ttc tac aat gat gga aaa gac aaa gag    1769
   Val Gly Leu Trp His Leu Ala Phe Tyr Asn Asp Gly Lys Asp Lys Glu
   505                 510                 515                 520
   atg gtt tcc ttc aat act gtt gtc cta gat tca gtg cag gac tgt cca    1817
   Met Val Ser Phe Asn Thr Val Val Leu Asp Ser Val Gln Asp Cys Pro
                       525                 530                 535
   cgt aac tgc cat ggg aat ggt gaa tgt gtg tcc ggg gtg tgt cac tgt    1865
   Arg Asn Cys His Gly Asn Gly Glu Cys Val Ser Gly Val Cys His Cys
                   540                 545                 550
   ttc cca gga ttt cta gga gca gac tgt gct aaa gct gcc tgc cct gtc    1913
   Phe Pro Gly Phe Leu Gly Ala Asp Cys Ala Lys Ala Ala Cys Pro Val
               555                 560                 565
   ctg tgc agt ggg aat gga caa tat tct aaa ggg acg tgc cag tgc tac   1961
   Leu Cys Ser Gly Asn Gly Gln Tyr Ser Lys Gly Thr Cys Gln Cys Tyr
           570                 575                 580
   agc ggc tgg aaa ggt gca gag tgc gac gtg ccc atg aat cag tgc atc   2009
   Ser Gly Trp Lys Gly Ala Glu Cys Asp Val Pro Met Asn Gln Cys Ile
   585                 590                 595                 600
   gat cct tcc tgc ggg ggc cac ggc tcc tgc att gat ggg aac tgt gtc   2057
   Asp Pro Ser Cys Gly Gly His Gly Ser Cys Ile Asp Gly Asn Cys Val
                   605                 610                 615
   tgc tct gct ggc tac aaa ggc gag cac tgt gag gaa gtt gat tgc ttg   2105
   Cys Ser Ala Gly Tyr Lys Gly Glu His Cys Glu Glu Val Asp Cys Leu
               620                 625                 630
   gat ccc acc tgc tcc agc cac gga gtc tgt gtg aat gga gaa tgc ctg   2153
   Asp Pro Thr Cys Ser Ser His Gly Val Cys Val Asn Gly Glu Cys Leu
           635                 640                 645
   tgc agc cct ggc tgg ggt ggt ctg aac tgt gag ctg gcg agg gtc cag   2201
   Cys Ser Pro Gly Trp Gly Gly Leu Asn Cys Glu Leu Ala Arg Val Gln
       650                 655                 660
   tgc cca gac cag tgc agt ggg cat ggc acg tac ctg cct gac acg ggc   2249
   Cys Pro Asp Gln Cys Ser Gly His Gly Thr Tyr Leu Pro Asp Thr Gly
   665                 670                 675                 680
   ctc tgc agc tgc gat ccc aac tgg atg ggt ccc gac tgc tct gtt gaa   2297
   Leu Cys Ser Cys Asp Pro Asn Trp Met Gly Pro Asp Cys Ser Val Glu
                   685                 690                 695
   gtg tgc tca gta gac tgt ggc act cac ggc gtc tgc atc ggg gga gcc   2345
   Val Cys Ser Val Asp Cys Gly Thr His Gly Val Cys Ile Gly Gly Ala
               700                 705                 710
   tgc cgc tgt gaa gag ggc tgg aca ggc gca gcg tgt gac cag cgc gtg   2393
   Cys Arg Cys Glu Glu Gly Trp Thr Gly Ala Ala Cys Asp Gln Arg Val
           715                 720                 725
   tgc cac ccc cgc tgc att gag cat ggg acc tgt aaa gat ggc aaa tgt   2441
   Cys His Pro Arg Cys Ile Glu His Gly Thr Cys Lys Asp Gly Lys Cys
       730                 735                 740
   gaa tgc cga gag ggc tgg aat ggt gaa cac tgc acc att gat ggc tgc   2489
   Glu Cys Arg Glu Gly Trp Asn Gly Glu His Cys Thr Ile Asp Gly Cys
   745                 750                 755                 760
   cct gac ttg tgc aac ggt aac ggg aga tgc aca ctg ggt cag aac agc    2537
   Pro Asp Leu Cys Asn Gly Asn Gly Arg Cys Thr Leu Gly Gln Asn Ser
                   765                 770                 775
   tgg cag tgt gtc tgc cag acc ggc tgg aga ggg ccc gga tgc aac gtt    2585
   Trp Gln Cys Val Cys Gln Thr Gly Trp Arg Gly Pro Gly Cys Asn Val
               780                 785                 790
   gcc atg gaa act tcc tgt gct gat aac aag gat aat gag gga gat ggc    2633
   Ala Met Glu Thr Ser Cys Ala Asp Asn Lys Asp Asn Glu Gly Asp Gly
           795                 800                 805
   ctg gtg gat tgt ttg gac cct gac tgc tgc ctg cag tca gcc tgt cag    2681
   Leu Val Asp Cys Leu Asp Pro Asp Cys Cys Leu Gln Ser Ala Cys Gln
       810                 815                 820
   aac agc ctg ctc tgc cgg ggg tcc cgg gac cca ctg gac atc att cag    2729
   Asn Ser Leu Leu Cys Arg Gly Ser Arg Asp Pro Leu Asp Ile Ile Gln
   825                 830                 835                 840
   cag ggc cag acg gat tgg ccc gca gtg aag tcc ttc tat gac cgt atc    2777
   Gln Gly Gln Thr Asp Trp Pro Ala Val Lys Ser Phe Tyr Asp Arg Ile
                   845                 850                 855
   aag ctc ttg gca ggc aag gat agc acc cac atc att cct gga gag aac    2825
   Lys Leu Leu Ala Gly Lys Asp Ser Thr His Ile Ile Pro Gly Glu Asn
               860                 865                 870
   cct ttc aac agc agc ttg gtt tct ctc atc cga ggc caa gta gta act    2873
   Pro Phe Asn Ser Ser Leu Val Ser Leu Ile Arg Gly Gln Val Val Thr
           875                 880                 885
   aca gat gga act ccc ctg gtc ggt gtg aac gtg tct ttt gtc aag tac    2921
   Thr Asp Gly Thr Pro Leu Val Gly Val Asn Val Ser Phe Val Lys Tyr
       890                 895                 900
   cca aaa tac ggc tac acc atc acc cgc cag gat ggc acg ttc gac ctg    2969
   Pro Lys Tyr Gly Tyr Thr Ile Thr Arg Gln Asp Gly Thr Phe Asp Leu
   905                 910                 915                 920
   atc gca aat gga ggt gct tcc ttg act cta cac ttt gag cga gcc ccg    3017
   Ile Ala Asn Gly Gly Ala Ser Leu Thr Leu His Phe Glu Arg Ala Pro
                   925                 930                 935
   ttc atg agc cag gag cgc act gtg tgg ctg ccg tgg aac agc ttt tac    3065
   Phe Met Ser Gln Glu Arg Thr Val Trp Leu Pro Trp Asn Ser Phe Tyr
   940 945 950
   gcc atg gac acc ctg gtg atg aag acc gag gag aac tcc atc ccc agc 3113
   Ala Met Asp Thr Leu Val Met Lys Thr Glu Glu Asn Ser Ile Pro Ser
   955 960 965
   tgt gac ctc agt ggc ttt gtc cgg cct gat cca atc atc atc tcc tcc 3161
   Cys Asp Leu Ser Gly Phe Val Arg Pro Asp Pro Ile Ile Ile Ser Ser
   970 975 980
   cca ctg tcc acc ttc ttt agt gct gcc cct ggg cag aat ccc atc gtg 3209
   Pro Leu Ser Thr Phe Phe Ser Ala Ala Pro Gly Gln Asn Pro Ile Val
   985 990 995 1000
   cct gag acc cag gtt ctt cat gaa gaa atc gag ctc cct ggt tcc aat 3257
   Pro Glu Thr Gln Val Leu His Glu Glu Ile Glu Leu Pro Gly Ser Asn
   1005 1010 1015
   gtg aaa ctt cgc tat ctg agc tct aga act gca ggg tac aag tca ctg 3305
   Val Lys Leu Arg Tyr Leu Ser Ser Arg Thr Ala Gly Tyr Lys Ser Leu
   1020 1025 1030
   ctg aag atc acc atg acc cag tcc aca gtg ccc ctg aac ctc att agg 3353
   Leu Lys Ile Thr Met Thr Gln Ser Thr Val Pro Leu Asn Leu Ile Arg
   1035 1040 1045
   gtt cac ctg atg gtg gct gtc gag ggg cat ctc ttc cag aag tca ttc 3401
   Val His Leu Met Val Ala Val Glu Gly His Leu Phe Gln Lys Ser Phe
   1050 1055 1060
   cag gct tct ccc aac ctg gcc tcc acc ttc atc tgg gac aag aca gat 3449
   Gln Ala Ser Pro Asn Leu Ala Ser Thr Phe Ile Trp Asp Lys Thr Asp
   1065 1070 1075 1080
   gcg tat ggc caa agg gtg tat gga ctc tca gat gct gtt gtg tct gtc 3497
   Ala Tyr Gly Gln Arg Val Tyr Gly Leu Ser Asp Ala Val Val Ser Val
   1085 1090 1095
   ggg ttt gaa tat gag acc tgt ccc agt cta att ctc tgg gag aaa agg 3545
   Gly Phe Glu Tyr Glu Thr Cys Pro Ser Leu Ile Leu Trp Glu Lys Arg
   1100 1105 1110
   aca gcc ctc ctt cag gga ttc gag ctg gac ccc tcc aac ctc ggt ggc 3593
   Thr Ala Leu Leu Gln Gly Phe Glu Leu Asp Pro Ser Asn Leu Gly Gly
   1115 1120 1125
   tgg tcc cta gac aaa cac cac atc ctc aat gtt aaa agt gga atc cta 3641
   Trp Ser Leu Asp Lys His His Ile Leu Asn Val Lys Ser Gly Ile Leu
   1130 1135 1140
   cac aaa ggc act ggg gaa aac cag ttc ctg acc cag cag cct gcc atc 3689
   His Lys Gly Thr Gly Glu Asn Gln Phe Leu Thr Gln Gln Pro Ala Ile
   1145 1150 1155 1160
   atc acc agc atc atg ggc aat ggt cgc cgc cgg agc att tcc tgt ccc 3737
   Ile Thr Ser Ile Met Gly Asn Gly Arg Arg Arg Ser Ile Ser Cys Pro
   1165 1170 1175
   agc tgc aac ggc ctt gct gaa ggc aac aag ctg ctg gcc cca gtg gct 3785
   Ser Cys Asn Gly Leu Ala Glu Gly Asn Lys Leu Leu Ala Pro Val Ala
   1180 1185 1190
   ctg gct gtt gga atc gat ggg agc ctc tat gtg ggt gac ttc aat tac 3833
   Leu Ala Val Gly Ile Asp Gly Ser Leu Tyr Val Gly Asp Phe Asn Tyr
   1195 1200 1205
   atc cga cgc atc ttt ccc tct cga aat gtg acc agc atc ttg gag tta 3881
   Ile Arg Arg Ile Phe Pro Ser Arg Asn Val Thr Ser Ile Leu Glu Leu
   1210 1215 1220
   cga aat aaa gag ttt aaa cat agc aac aac cca gca cac aag tac tac 3929
   Arg Asn Lys Glu Phe Lys His Ser Asn Asn Pro Ala His Lys Tyr Tyr
   1225 1230 1235 1240
   ttg gca gtg gac ccc gtg tcc ggc tcg ctc tac gtg tcc gac acc aac 3977
   Leu Ala Val Asp Pro Val Ser Gly Ser Leu Tyr Val Ser Asp Thr Asn
   1245 1250 1255
   agc agg aga atc tac cgc gtc aag tct ctg agt gga acc aaa gac ctg 4025
   Ser Arg Arg Ile Tyr Arg Val Lys Ser Leu Ser Gly Thr Lys Asp Leu
   1260 1265 1270
   gct ggg aat tcg gaa gtt gtg gca ggg acg gga gag cag tgt cta ccc 4073
   Ala Gly Asn Ser Glu Val Val Ala Gly Thr Gly Glu Gln Cys Leu Pro
   1275 1280 1285
   ttt gat gaa gcc cgc tgc ggg gat gga ggg aag gcc ata gat gca acc 4121
   Phe Asp Glu Ala Arg Cys Gly Asp Gly Gly Lys Ala Ile Asp Ala Thr
   1290 1295 1300
   ctg atg agc ccg aga ggt att gca gta gac aag aat ggg ctc atg tac 4169
   Leu Met Ser Pro Arg Gly Ile Ala Val Asp Lys Asn Gly Leu Met Tyr
   1305 1310 1315 1320
   ttt gtc gat gcc acc atg atc cgg aag gtt gac cag aat gga atc atc 4217
   Phe Val Asp Ala Thr Met Ile Arg Lys Val Asp Gln Asn Gly Ile Ile
               1325                1330                1335
   tcc acc ctg ctg ggc tcc aat gac ctc act gcc gtc cgg ccg ctg agc    4265
   Ser Thr Leu Leu Gly Ser Asn Asp Leu Thr Ala Val Arg Pro Leu Ser
           1340                1345                1350
   tgt gat tcc agc atg gat gta gcc cag gtt cgt ctg gag tgg cca aca    4313
   Cys Asp Ser Ser Met Asp Val Ala Gln Val Arg Leu Glu Trp Pro Thr
       1355                1360                1365
   gac ctt gct gtc aat ccc atg gat aac tcc ttg tat gtt cta gag aac    4361
   Asp Leu Ala Val Asn Pro Met Asp Asn Ser Leu Tyr Val Leu Glu Asn
   1370                1375                1380
   aat gtc atc ctt cga atc acc gag aac cac caa gtc agc atc att gcg    4409
   Asn Val Ile Leu Arg Ile Thr Glu Asn His Gln Val Ser Ile Ile Ala
   1385                1390                1395                1400
   gga cgc ccc atg cac tgc caa gtt cct ggc att gac tac tca ctc agc    4457
   Gly Arg Pro Met His Cys Gln Val Pro Gly Ile Asp Tyr Ser Leu Ser
               1405                1410                1415
   aaa cta gcc att cac tct gcc ctg gag tca gcc agt gcc att gcc att    4505
   Lys Leu Ala Ile His Ser Ala Leu Glu Ser Ala Ser Ala Ile Ala Ile
           1420                1425                1430
   tct cac act ggg gtc ctc tac atc act gag aca gat gag aag aag att    4553
   Ser His Thr Gly Val Leu Tyr Ile Thr Glu Thr Asp Glu Lys Lys Ile
       1435                1440                1445
   aac cgt cta cgc cag gta aca acc aac ggg gag atc tgc ctt tta gct    4601
   Asn Arg Leu Arg Gln Val Thr Thr Asn Gly Glu Ile Cys Leu Leu Ala
   1450                1455                1460
   ggg gca gcc tcg gac tgc gac tgc aaa aac gat gtc aat tgc aac tgc    4649
   Gly Ala Ala Ser Asp Cys Asp Cys Lys Asn Asp Val Asn Cys Asn Cys
   1465                1470                1475                1480
   tat tca gga gat gat gcc tac gcg act gat gcc atc ttg aat tcc cca    4697
   Tyr Ser Gly Asp Asp Ala Tyr Ala Thr Asp Ala Ile Leu Asn Ser Pro
               1485                1490                1495
   tca tcc tta gct gta gct cca gat ggt acc att tac att gca gac ctt    4745
   Ser Ser Leu Ala Val Ala Pro Asp Gly Thr Ile Tyr Ile Ala Asp Leu
           1500                1505                1510
   gga aat att cgg atc agg gcg gtc agc aag aac aag cct gtt ctt aat    4793
   Gly Asn Ile Arg Ile Arg Ala Val Ser Lys Asn Lys Pro Val Leu Asn
           1515                1520                1525
   gcc ttc aac cag tat gag gct gca tcc ccc gga gag cag gag tta tat    4841
   Ala Phe Asn Gln Tyr Glu Ala Ala Ser Pro Gly Glu Gln Glu Leu Tyr
       1530                1535                1540
   gtt ttc aac gct gat ggc atc cac caa tac act gtg agc ctg gtg aca    4889
   Val Phe Asn Ala Asp Gly Ile His Gln Tyr Thr Val Ser Leu Val Thr
   1545                1550                1555                1560
   ggg gag tac ttg tac aat ttc aca tat agt act gac aat gat gtc act    4937
   Gly Glu Tyr Leu Tyr Asn Phe Thr Tyr Ser Thr Asp Asn Asp Val Thr
                   1565                1570                1575
   gaa ttg att gac aat aat ggg aat tcc ctg aag atc cgt cgg gac agc    4985
   Glu Leu Ile Asp Asn Asn Gly Asn Ser Leu Lys Ile Arg Arg Asp Ser
               1580                1585                1590
   agt ggc atg ccc cgt cac ctg ctc atg cct gac aac cag atc atc acc    5033
   Ser Gly Met Pro Arg His Leu Leu Met Pro Asp Asn Gln Ile Ile Thr
           1595                1600                1605
   ctc acc gtg ggc acc aat gga ggc ctc aaa gtc gtg tcc aca cag aac    5081
   Leu Thr Val Gly Thr Asn Gly Gly Leu Lys Val Val Ser Thr Gln Asn
       1610                1615                1620
   ctg gag ctt ggt ctc atg acc tat gat ggc aac act ggg ctc ctg gcc    5129
   Leu Glu Leu Gly Leu Met Thr Tyr Asp Gly Asn Thr Gly Leu Leu Ala
   1625                1630                1635                1640
   acc aag agc gat gaa aca gga tgg acg act ttc tat gac tat gac cac    5177
   Thr Lys Ser Asp Glu Thr Gly Trp Thr Thr Phe Tyr Asp Tyr Asp His
                   1645                1650                1655
   gaa ggc cgc ctg acc aac gtg acg cgc ccc acg ggg gtg gta acc agt    5225
   Glu Gly Arg Leu Thr Asn Val Thr Arg Pro Thr Gly Val Val Thr Ser
               1660                1665                1670
   ctg cac cgg gaa atg gag aaa tct att acc att gac att gag aac tcc    5273
   Leu His Arg Glu Met Glu Lys Ser Ile Thr Ile Asp Ile Glu Asn Ser
           1675                1680                1685
   aac cgt gat gat gac gtc act gtc atc acc aac ctc tct tca gta gag    5321
   Asn Arg Asp Asp Asp Val Thr Val Ile Thr Asn Leu Ser Ser Val Glu
       1690                1695                1700
   gcc tcc tac aca gtg gta caa gat caa gtt cgg aac agc tac cag ctc    5369
   Ala Ser Tyr Thr Val Val Gln Asp Gln Val Arg Asn Ser Tyr Gln Leu
   1705                1710                1715                1720
   tgt aat aat ggt acc ctg agg gtg atg tat gct aat ggg atg ggt atc    5417
   Cys Asn Asn Gly Thr Leu Arg Val Met Tyr Ala Asn Gly Met Gly Ile
                  1725                1730                1735
   agc ttc cac agc gag ccc cat gtc cta gcg ggc acc atc acc ccc acc    5465
   Ser Phe His Ser Glu Pro His Val Leu Ala Gly Thr Ile Thr Pro Thr
              1740                1745                1750
   att gga cgc tgc aac atc tcc ctg cct atg gag aat ggc tta aac tcc    5513
   Ile Gly Arg Cys Asn Ile Ser Leu Pro Met Glu Asn Gly Leu Asn Ser
          1755                1760                1765
   att gag tgg cgc cta aga aag gaa cag att aaa ggc aaa gtc acc atc    5561
   Ile Glu Trp Arg Leu Arg Lys Glu Gln Ile Lys Gly Lys Val Thr Ile
      1770                1775                1780
   ttt ggc agg aag ctc cgg gtc cat gga aga aat ctc ttg tcc att gac    5609
   Phe Gly Arg Lys Leu Arg Val His Gly Arg Asn Leu Leu Ser Ile Asp
   1785                1790                1795                1800
   tat gat cga aat att cgg act gaa aag atc tat gat gac cac cgg aag    5657
   Tyr Asp Arg Asn Ile Arg Thr Glu Lys Ile Tyr Asp Asp His Arg Lys
                  1805                1810                1815
   ttc acc ctg agg atc att tat gac cag gtg ggc cgc ccc ttc ctc tgg    5705
   Phe Thr Leu Arg Ile Ile Tyr Asp Gln Val Gly Arg Pro Phe Leu Trp
              1820                1825                1830
   ctg ccc agc agc ggg ctg gca gct gtc aac gtg tca tac ttc ttc aat    5753
   Leu Pro Ser Ser Gly Leu Ala Ala Val Asn Val Ser Tyr Phe Phe Asn
          1835                1840                1845
   ggg cgc ctg gct ggg ctt cag cgt ggg gcc atg agc gag agg aca gac    5801
   Gly Arg Leu Ala Gly Leu Gln Arg Gly Ala Met Ser Glu Arg Thr Asp
      1850                1855                1860
   atc gac aag caa ggc cgc atc gtg tcc cgc atg ttc gct gac ggg aaa    5849
   Ile Asp Lys Gln Gly Arg Ile Val Ser Arg Met Phe Ala Asp Gly Lys
   1865                1870                1875                1880
   gtg tgg agc tac tcc tac ctt gac aag tcc atg gtc ctc ctg ctt cag    5897
   Val Trp Ser Tyr Ser Tyr Leu Asp Lys Ser Met Val Leu Leu Leu Gln
                  1885                1890                1895
   agc caa cgt cag tat ata ttt gag tat gac tcc tct gac cgc ctc ctt    5945
   Ser Gln Arg Gln Tyr Ile Phe Glu Tyr Asp Ser Ser Asp Arg Leu Leu
   1900 1905 1910
   gcc gtc acc atg ccc agc gtg gcc cgg cac agc atg tcc aca cac acc 5993
   Ala Val Thr Met Pro Ser Val Ala Arg His Ser Met Ser Thr His Thr
   1915 1920 1925
   tcc atc ggc tac atc cgt aat att tac aac ccg cct gaa agc aat gct 6041
   Ser Ile Gly Tyr Ile Arg Asn Ile Tyr Asn Pro Pro Glu Ser Asn Ala
   1930 1935 1940
   tcg gtc atc ttt gac tac agt gat gac ggc cgc atc ctg aag acc tcc 6089
   Ser Val Ile Phe Asp Tyr Ser Asp Asp Gly Arg Ile Leu Lys Thr Ser
   1945 1950 1955 1960
   ttt ttg ggc acc gga cgc cag gtg ttc tac aag tat ggg aaa ctc tcc 6137
   Phe Leu Gly Thr Gly Arg Gln Val Phe Tyr Lys Tyr Gly Lys Leu Ser
   1965 1970 1975
   aag tta tca gag att gtc tac gac agt acc gcc gtc acc ttc ggg tat 6185
   Lys Leu Ser Glu Ile Val Tyr Asp Ser Thr Ala Val Thr Phe Gly Tyr
   1980 1985 1990
   gac gag acc act ggt gtc ttg aag atg gtc aac ctc caa agt ggg ggc 6233
   Asp Glu Thr Thr Gly Val Leu Lys Met Val Asn Leu Gln Ser Gly Gly
   1995 2000 2005
   ttc tcc tgc acc atc agg tac cgg aag att ggc ccc ctg gtg gac aag 6281
   Phe Ser Cys Thr Ile Arg Tyr Arg Lys Ile Gly Pro Leu Val Asp Lys
   2010 2015 2020
   cag atc tac agg ttc tcc gag gaa ggc atg gtc aat gcc agg ttt gac 6329
   Gln Ile Tyr Arg Phe Ser Glu Glu Gly Met Val Asn Ala Arg Phe Asp
   2025 2030 2035 2040
   tac acc tat cat gac aac agc ttc cgc atc gca agc atc aag ccc gtc 6377
   Tyr Thr Tyr His Asp Asn Ser Phe Arg Ile Ala Ser Ile Lys Pro Val
   2045 2050 2055
   ata agt gag act ccc ctc ccc gtt gac ctc tac cgc tat gat gag att 6425
   Ile Ser Glu Thr Pro Leu Pro Val Asp Leu Tyr Arg Tyr Asp Glu Ile
   2060 2065 2070
   tct ggc aag gtg gaa cac ttt ggt aag ttt gga gtc atc tat tat gac 6473
   Ser Gly Lys Val Glu His Phe Gly Lys Phe Gly Val Ile Tyr Tyr Asp
   2075 2080 2085
   atc aac cag atc atc acc act gcc gtg atg acc ctc agc aaa cac ttc 6521
   Ile Asn Gln Ile Ile Thr Thr Ala Val Met Thr Leu Ser Lys His Phe
   2090 2095 2100
   gac acc cat ggg cgg atc aag gag gtc cag tat gag atg ttc cgg tcc 6569
   Asp Thr His Gly Arg Ile Lys Glu Val Gln Tyr Glu Met Phe Arg Ser
   2105 2110 2115 2120
   ctc atg tac tgg atg acg gtg caa tat gac agc atg ggc agg gtg atc 6617
   Leu Met Tyr Trp Met Thr Val Gln Tyr Asp Ser Met Gly Arg Val Ile
   2125 2130 2135
   aag agg gag cta aaa ctg ggg ccc tat gcc aat acc acg aag tac acc 6665
   Lys Arg Glu Leu Lys Leu Gly Pro Tyr Ala Asn Thr Thr Lys Tyr Thr
   2140 2145 2150
   tat gac tac gat ggg gac ggg cag ctc cag agc gtg gcc gtc aat gac 6713
   Tyr Asp Tyr Asp Gly Asp Gly Gln Leu Gln Ser Val Ala Val Asn Asp
   2155 2160 2165
   cgc ccg acc tgg cgc tac agc tat gac ctt aat ggg aat ctc cac tta 6761
   Arg Pro Thr Trp Arg Tyr Ser Tyr Asp Leu Asn Gly Asn Leu His Leu
   2170 2175 2180
   ctg aac cca ggc aac agt gtg cgc ctc atg ccc ttg cgc tat gac ctc 6809
   Leu Asn Pro Gly Asn Ser Val Arg Leu Met Pro Leu Arg Tyr Asp Leu
   2185 2190 2195 2200
   cgg gat cgg ata acc aga ctc ggg gat gtg cag tac aaa att gac gac 6857
   Arg Asp Arg Ile Thr Arg Leu Gly Asp Val Gln Tyr Lys Ile Asp Asp
   2205 2210 2215
   gat ggc tat ctg tgc cag aga ggg tct gac atc ttc gaa tac aat tcc 6905
   Asp Gly Tyr Leu Cys Gln Arg Gly Ser Asp Ile Phe Glu Tyr Asn Ser
   2220 2225 2230
   aag ggc ctc cta aca aga gcc tac aac aag gcc agc ggg tgg agt gtc 6953
   Lys Gly Leu Leu Thr Arg Ala Tyr Asn Lys Ala Ser Gly Trp Ser Val
   2235 2240 2245
   cag tac cgc tat gat ggc gta gga cgg cgg gct tcc tac aag acc aac 7001
   Gln Tyr Arg Tyr Asp Gly Val Gly Arg Arg Ala Ser Tyr Lys Thr Asn
   2250 2255 2260
   ctg ggc cac cac ctg cag tac ttc tac tct gac ctc cac aac ccg acg 7049
   Leu Gly His His Leu Gln Tyr Phe Tyr Ser Asp Leu His Asn Pro Thr
   2265 2270 2275 2280
   cgc atc acc cat gtc tac aat cac tcc aac tcg gag att acc tca ctg 7097
   Arg Ile Thr His Val Tyr Asn His Ser Asn Ser Glu Ile Thr Ser Leu
   2285 2290 2295
   tac tac gac ctc cag ggc cac ctc ttt gcc atg gag agc agc agt ggg 7145
   Tyr Tyr Asp Leu Gln Gly His Leu Phe Ala Met Glu Ser Ser Ser Gly
   2300 2305 2310
   gag gag tac tat gtt gcc tct gat aac aca ggg act cct ctg gct gtg 7193
   Glu Glu Tyr Tyr Val Ala Ser Asp Asn Thr Gly Thr Pro Leu Ala Val
   2315 2320 2325
   ttc agc atc aac ggc ctc atg atc aaa cag ctg cag tac acg gcc tat 7241
   Phe Ser Ile Asn Gly Leu Met Ile Lys Gln Leu Gln Tyr Thr Ala Tyr
   2330 2335 2340
   ggg gag att tat tat gac tcc aac ccc gac ttc cag atg gtc att ggc 7289
   Gly Glu Ile Tyr Tyr Asp Ser Asn Pro Asp Phe Gln Met Val Ile Gly
   2345 2350 2355 2360
   ttc cat ggg gga ctc tat gac ccc ctg acc aag ctg gtc cac ttc act 7337
   Phe His Gly Gly Leu Tyr Asp Pro Leu Thr Lys Leu Val His Phe Thr
   2365 2370 2375
   cag cgt gat tat gat gtg ctg gca gga cga tgg acc tcc cca gac tat 7385
   Gln Arg Asp Tyr Asp Val Leu Ala Gly Arg Trp Thr Ser Pro Asp Tyr
   2380 2385 2390
   acc atg tgg aaa aac gtg ggc aag gag ccg gcc ccc ttt aac ctg tat 7433
   Thr Met Trp Lys Asn Val Gly Lys Glu Pro Ala Pro Phe Asn Leu Tyr
   2395 2400 2405
   atg ttc aag agc aac aat cct ctc agc agt gag cta gat ttg aag aac 7481
   Met Phe Lys Ser Asn Asn Pro Leu Ser Ser Glu Leu Asp Leu Lys Asn
   2410 2415 2420
   tac gtg aca gat gtg aaa agc tgg ctt gtg atg ttt gga ttt cag ctt 7529
   Tyr Val Thr Asp Val Lys Ser Trp Leu Val Met Phe Gly Phe Gln Leu
   2425 2430 2435 2440
   agc aac atc att cct ggc ttc ccg aga gcc aaa atg tat ttc gtg cct 7577
   Ser Asn Ile Ile Pro Gly Phe Pro Arg Ala Lys Met Tyr Phe Val Pro
   2445 2450 2455
   cct ccc tat gaa ttg tca gag agt caa gca agt gag aat gga cag ctc 7625
   Pro Pro Tyr Glu Leu Ser Glu Ser Gln Ala Ser Glu Asn Gly Gln Leu
   2460 2465 2470
   att aca ggt gtc caa cag aca aca gag aga cat aac cag gcc ttc atg 7673
   Ile Thr Gly Val Gln Gln Thr Thr Glu Arg His Asn Gln Ala Phe Met
           2475                2480                2485
   gct ctg gaa gga cag gtc att act aaa aag ctc cac gcc agc atc cga    7721
   Ala Leu Glu Gly Gln Val Ile Thr Lys Lys Leu His Ala Ser Ile Arg
       2490                2495                2500
   gag aaa gca ggt cac tgg ttt gcc acc acc acg ccc atc att ggc aaa    7769
   Glu Lys Ala Gly His Trp Phe Ala Thr Thr Thr Pro Ile Ile Gly Lys
   2505                2510                2515                2520
   ggc atc atg ttt gcc atc aaa gaa ggg cgg gtg acc acg ggc gtg tcc    7817
   Gly Ile Met Phe Ala Ile Lys Glu Gly Arg Val Thr Thr Gly Val Ser
                   2525                2530                2535
   agc atc gcc agc gaa gat agc cgc aag gtg gca tct gtg ctg aac aac    7865
   Ser Ile Ala Ser Glu Asp Ser Arg Lys Val Ala Ser Val Leu Asn Asn
               2540                2545                2550
   gcc tac tac ctg gac aag atg cac tac agc atc gag ggc aag gac acc    7913
   Ala Tyr Tyr Leu Asp Lys Met His Tyr Ser Ile Glu Gly Lys Asp Thr
           2555                2560                2565
   cac tac ttt gtg aag att ggc tca gcc gat ggc gac ctg gtc aca cta    7961
   His Tyr Phe Val Lys Ile Gly Ser Ala Asp Gly Asp Leu Val Thr Leu
       2570                2575                2580
   ggc acc acc atc ggc cgc aag gtg cta gag agc ggg gtg aac gtg acc    8009
   Gly Thr Thr Ile Gly Arg Lys Val Leu Glu Ser Gly Val Asn Val Thr
   2585                2590                2595                2600
   gtg tcc cag ccc acg ctg ctg gtc aac ggc agg act cga agg ttc acg    8057
   Val Ser Gln Pro Thr Leu Leu Val Asn Gly Arg Thr Arg Arg Phe Thr
                   2605                2610                2615
   aac att gag ttc cag tac tcc acg ctg ctg ctc agc atc cgc tat ggc    8105
   Asn Ile Glu Phe Gln Tyr Ser Thr Leu Leu Leu Ser Ile Arg Tyr Gly
               2620                2625                2630
   ctc acc ccc gac acc ctg gac gaa gag aag gcc cgc gtc ctg gac cag    8153
   Leu Thr Pro Asp Thr Leu Asp Glu Glu Lys Ala Arg Val Leu Asp Gln
           2635                2640                2645
   gcg aga cag agg gcc ctg ggc acg gcc tgg gcc aag gag cag cag aaa    8201
   Ala Arg Gln Arg Ala Leu Gly Thr Ala Trp Ala Lys Glu Gln Gln Lys
       2650                2655                2660
   gcc agg gac ggg aga gag ggg agc cgc ctg tgg act gag ggc gag aag    8249
   Ala Arg Asp Gly Arg Glu Gly Ser Arg Leu Trp Thr Glu Gly Glu Lys
   2665                2670                2675                2680
   cag cag ctt ctg agc acc ggg cgc gtg caa ggg tac gag gga tat tac     8297
   Gln Gln Leu Leu Ser Thr Gly Arg Val Gln Gly Tyr Glu Gly Tyr Tyr
                   2685                2690                2695
   gtg ctt ccc gtg gag caa tac cca gag ctt gca gac agt agc agc aac     8345
   Val Leu Pro Val Glu Gln Tyr Pro Glu Leu Ala Asp Ser Ser Ser Asn
               2700                2705                2710
   atc cag ttt tta aga cag aat gag atg gga aag agg taacaaaata          8391
   Ile Gln Phe Leu Arg Gln Asn Glu Met Gly Lys Arg
           2715                2720
   atctgctgcc attccttgtc tgaatggctc agcaggagta actgttatct cctctcctaa 8451
   ggagatgaag acctaacagg ggcactgcgg ctgggctgct ttaggagacc aagtggcaag 8511
   aaagctcaca ttttttgagt tcaaatgcta ctgtccaagc gagaagtccc tcatcctgaa 8571
   gtagactaaa gcccggctga aaattccgag gaaaacaaaa caaacgaatg aatgaacaga 8631
   cacacacaat gttccaagtt cccctaaaat atgacccact tgttctgggt ctacgcagaa 8691
   aagagacgca aagtgtccaa aaggaacaaa agaacaaaaa cgaataagca aagaagaaaa 8751
   caaacaaaaa caaaacaaaa caaacacacg gaccgataaa caaagaagcg aagataagaa 8811
   agaaggcctc atatccaatt acctcactca ttcacatgtg agcgacacgc agacatccgc 8871
   gagggccagc gtcaccagac cagctgcggg acaaaccact cagactgctt gtaggacaaa 8931
   tacttctgac attttcgttt aagcaaatac aggtgcattt aaaacacgac tttgggggtg 8991
   atttgtgtgt agcgcctggg gaggggggat aaaagaggag gagtgagcac tggaaatact 9051
   ttttaaagaa aaaaaaacat gagggaataa aagaaattcc tatcaaaaat caaagtgaaa 9111
   taataccatc cagcacttaa ctctcaggtc ccaactaagt ctggcctgag ctaatttatt 9171
   tgagcgcaga gtgtaaaatt taattcaaaa tggtggctat aatcactaca gataaatttc 9231
   atactctttt gtctttggag attccattgt ggacagtaat acgcagttac agggtgtagt 9291
   ctgtttagat tccgtagttc gtgggtatca gtttcggtag aggtgcagca tcgtgacact 9351
   tttgctaaca ggtaccactt ctgatcaccc tgtacataca tgagccgaaa ggcacaatca 9411
   ctgtttcaga tttaaaatta ttagtgtgtt tgtttggtcc agaaactgag acaatcacat 9471
   gacagtcacc acgaggagag aaaatttaaa aaataaaaat aaaaacaaaa aaaattttaa 9531
   aaattaaaaa aacaaaaata aagtctaata agaactttgg tacaggaact tttttgtaat 9591
   atacatgtat gaattgttca tcgagttttt atattaattt taatttgctg ctaagcaaag 9651
   actagggaca ggcaaagata atttatggca aagtgtttaa attgtttata cataaataaa 9711
   gtctctaaaa ctcctgtg 9729
<210> 13
   <211> 2724
   <212> PRT
   <213> Homo sapiens
<400> 13
   Met Asp Val Lys Asp Arg Arg His Arg Ser Leu Thr Arg Gly Arg Cys
   1               5                   10                  15
   Gly Lys Glu Cys Arg Tyr Thr Ser Ser Ser Leu Asp Ser Glu Asp Cys
               20                  25                  30
   Arg Val Pro Thr Gln Lys Ser Tyr Ser Ser Ser Glu Thr Leu Lys Ala
           35                  40                  45
   Tyr Asp His Asp Ser Arg Met His Tyr Gly Asn Arg Val Thr Asp Leu
       50                  55                  60
   Ile His Arg Glu Ser Asp Glu Phe Pro Arg Gln Gly Thr Asn Phe Thr
   65                  70                  75                  80
   Leu Ala Glu Leu Gly Ile Cys Glu Pro Ser Pro His Arg Ser Gly Tyr
                   85                  90                  95
   Cys Ser Asp Met Gly Ile Leu His Gln Gly Tyr Ser Leu Ser Thr Gly
               100                 105                 110
   Ser Asp Ala Asp Ser Asp Thr Glu Gly Gly Met Ser Pro Glu His Ala
           115                 120                 125
   Ile Arg Leu Trp Gly Arg Gly Ile Lys Ser Arg Arg Ser Ser Gly Leu
       130                 135                 140
   Ser Ser Arg Glu Asn Ser Ala Leu Thr Leu Thr Asp Ser Asp Asn Glu
   145                 150                 155                 160
   Asn Lys Ser Asp Asp Glu Asn Gly Arg Pro Ile Pro Pro Thr Ser Ser
   165 170 175
   Pro Ser Leu Leu Pro Ser Ala Gln Leu Pro Ser Ser His Asn Pro Pro
   180 185 190
   Pro Val Ser Cys Gln Met Pro Leu Leu Asp Ser Asn Thr Ser His Gln
   195 200 205
   Ile Met Asp Thr Asn Pro Asp Glu Glu Phe Ser Pro Asn Ser Tyr Leu
   210 215 220
   Leu Arg Ala Cys Ser Gly Pro Gln Gln Ala Ser Ser Ser Gly Pro Pro
   225 230 235 240
   Asn His His Ser Gln Ser Thr Leu Arg Pro Pro Leu Pro Pro Pro His
   245 250 255
   Asn His Thr Leu Ser His His His Ser Ser Ala Asn Ser Leu Asn Arg
   260 265 270
   Asn Ser Leu Thr Asn Arg Arg Ser Gln Ile His Ala Pro Ala Pro Ala
   275 280 285
   Pro Asn Asp Leu Ala Thr Thr Pro Glu Ser Val Gln Leu Gln Asp Ser
   290 295 300
   Trp Val Leu Asn Ser Asn Val Pro Leu Glu Thr Arg His Phe Leu Phe
   305 310 315 320
   Lys Thr Ser Ser Gly Ser Thr Pro Leu Phe Ser Ser Ser Ser Pro Gly
   325 330 335
   Tyr Pro Leu Thr Ser Gly Thr Val Tyr Thr Pro Pro Pro Arg Leu Leu
   340 345 350
   Pro Arg Asn Thr Phe Ser Arg Lys Ala Phe Lys Leu Lys Lys Pro Ser
   355 360 365
   Lys Tyr Cys Ser Trp Lys Cys Ala Ala Leu Ser Ala Ile Ala Ala Ala
   370 375 380
   Leu Leu Leu Ala Ile Leu Leu Ala Tyr Phe Ile Val Pro Trp Ser Leu
   385 390 395 400
   Lys Asn Ser Ser Ile Asp Ser Gly Glu Ala Glu Val Gly Arg Arg Val
   405 410 415
   Thr Gln Glu Val Pro Pro Gly Val Phe Trp Arg Ser Gln Ile His Ile
   420 425 430
   Ser Gln Pro Gln Phe Leu Lys Phe Asn Ile Ser Leu Gly Lys Asp Ala
   435 440 445
   Leu Phe Gly Val Tyr Ile Arg Arg Gly Leu Pro Pro Ser His Ala Gln
   450 455 460
   Tyr Asp Phe Met Glu Arg Leu Asp Gly Lys Glu Lys Trp Ser Val Val
   465 470 475 480
   Glu Ser Pro Arg Glu Arg Arg Ser Ile Gln Thr Leu Val Gln Asn Glu
   485 490 495
   Ala Val Phe Val Gln Tyr Leu Asp Val Gly Leu Trp His Leu Ala Phe
   500 505 510
   Tyr Asn Asp Gly Lys Asp Lys Glu Met Val Ser Phe Asn Thr Val Val
   515 520 525
   Leu Asp Ser Val Gln Asp Cys Pro Arg Asn Cys His Gly Asn Gly Glu
   530 535 540
   Cys Val Ser Gly Val Cys His Cys Phe Pro Gly Phe Leu Gly Ala Asp
   545 550 555 560
   Cys Ala Lys Ala Ala Cys Pro Val Leu Cys Ser Gly Asn Gly Gln Tyr
   565 570 575
   Ser Lys Gly Thr Cys Gln Cys Tyr Ser Gly Trp Lys Gly Ala Glu Cys
   580 585 590
   Asp Val Pro Met Asn Gln Cys Ile Asp Pro Ser Cys Gly Gly His Gly
   595 600 605
   Ser Cys Ile Asp Gly Asn Cys Val Cys Ser Ala Gly Tyr Lys Gly Glu
   610 615 620
   His Cys Glu Glu Val Asp Cys Leu Asp Pro Thr Cys Ser Ser His Gly
   625 630 635 640
   Val Cys Val Asn Gly Glu Cys Leu Cys Ser Pro Gly Trp Gly Gly Leu
   645 650 655
   Asn Cys Glu Leu Ala Arg Val Gln Cys Pro Asp Gln Cys Ser Gly His
   660 665 670
   Gly Thr Tyr Leu Pro Asp Thr Gly Leu Cys Ser Cys Asp Pro Asn Trp
   675 680 685
   Met Gly Pro Asp Cys Ser Val Glu Val Cys Ser Val Asp Cys Gly Thr
   690 695 700
   His Gly Val Cys Ile Gly Gly Ala Cys Arg Cys Glu Glu Gly Trp Thr
   705 710 715 720
   Gly Ala Ala Cys Asp Gln Arg Val Cys His Pro Arg Cys Ile Glu His
   725 730 735
   Gly Thr Cys Lys Asp Gly Lys Cys Glu Cys Arg Glu Gly Trp Asn Gly
   740 745 750
   Glu His Cys Thr Ile Asp Gly Cys Pro Asp Leu Cys Asn Gly Asn Gly
   755 760 765
   Arg Cys Thr Leu Gly Gln Asn Ser Trp Gln Cys Val Cys Gln Thr Gly
   770 775 780
   Trp Arg Gly Pro Gly Cys Asn Val Ala Met Glu Thr Ser Cys Ala Asp
   785 790 795 800
   Asn Lys Asp Asn Glu Gly Asp Gly Leu Val Asp Cys Leu Asp Pro Asp
   805 810 815
   Cys Cys Leu Gln Ser Ala Cys Gln Asn Ser Leu Leu Cys Arg Gly Ser
   820 825 830
   Arg Asp Pro Leu Asp Ile Ile Gln Gln Gly Gln Thr Asp Trp Pro Ala
   835 840 845
   Val Lys Ser Phe Tyr Asp Arg Ile Lys Leu Leu Ala Gly Lys Asp Ser
   850 855 860
   Thr His Ile Ile Pro Gly Glu Asn Pro Phe Asn Ser Ser Leu Val Ser
   865 870 875 880
   Leu Ile Arg Gly Gln Val Val Thr Thr Asp Gly Thr Pro Leu Val Gly
   885 890 895
   Val Asn Val Ser Phe Val Lys Tyr Pro Lys Tyr Gly Tyr Thr Ile Thr
   900 905 910
   Arg Gln Asp Gly Thr Phe Asp Leu Ile Ala Asn Gly Gly Ala Ser Leu
   915 920 925
   Thr Leu His Phe Glu Arg Ala Pro Phe Met Ser Gln Glu Arg Thr Val
   930 935 940
   Trp Leu Pro Trp Asn Ser Phe Tyr Ala Met Asp Thr Leu Val Met Lys
   945 950 955 960
   Thr Glu Glu Asn Ser Ile Pro Ser Cys Asp Leu Ser Gly Phe Val Arg
   965 970 975
   Pro Asp Pro Ile Ile Ile Ser Ser Pro Leu Ser Thr Phe Phe Ser Ala
   980 985 990
   Ala Pro Gly Gln Asn Pro Ile Val Pro Glu Thr Gln Val Leu His Glu
   995 1000 1005
   Glu Ile Glu Leu Pro Gly Ser Asn Val Lys Leu Arg Tyr Leu Ser Ser
   1010 1015 1020
   Arg Thr Ala Gly Tyr Lys Ser Leu Leu Lys Ile Thr Met Thr Gln Ser
   1025 1030 1035 1040
   Thr Val Pro Leu Asn Leu Ile Arg Val His Leu Met Val Ala Val Glu
   1045 1050 1055
   Gly His Leu Phe Gln Lys Ser Phe Gln Ala Ser Pro Asn Leu Ala Ser
   1060 1065 1070
   Thr Phe Ile Trp Asp Lys Thr Asp Ala Tyr Gly Gln Arg Val Tyr Gly
   1075 1080 1085
   Leu Ser Asp Ala Val Val Ser Val Gly Phe Glu Tyr Glu Thr Cys Pro
   1090 1095 1100
   Ser Leu Ile Leu Trp Glu Lys Arg Thr Ala Leu Leu Gln Gly Phe Glu
   1105 1110 1115 1120
   Leu Asp Pro Ser Asn Leu Gly Gly Trp Ser Leu Asp Lys His His Ile
   1125 1130 1135
   Leu Asn Val Lys Ser Gly Ile Leu His Lys Gly Thr Gly Glu Asn Gln
   1140 1145 1150
   Phe Leu Thr Gln Gln Pro Ala Ile Ile Thr Ser Ile Met Gly Asn Gly
   1155 1160 1165
   Arg Arg Arg Ser Ile Ser Cys Pro Ser Cys Asn Gly Leu Ala Glu Gly
   1170 1175 1180
   Asn Lys Leu Leu Ala Pro Val Ala Leu Ala Val Gly Ile Asp Gly Ser
   1185 1190 1195 1200
   Leu Tyr Val Gly Asp Phe Asn Tyr Ile Arg Arg Ile Phe Pro Ser Arg
   1205 1210 1215
   Asn Val Thr Ser Ile Leu Glu Leu Arg Asn Lys Glu Phe Lys His Ser
   1220 1225 1230
   Asn Asn Pro Ala His Lys Tyr Tyr Leu Ala Val Asp Pro Val Ser Gly
   1235 1240 1245
   Ser Leu Tyr Val Ser Asp Thr Asn Ser Arg Arg Ile Tyr Arg Val Lys
   1250 1255 1260
   Ser Leu Ser Gly Thr Lys Asp Leu Ala Gly Asn Ser Glu Val Val Ala
   1265 1270 1275 1280
   Gly Thr Gly Glu Gln Cys Leu Pro Phe Asp Glu Ala Arg Cys Gly Asp
   1285 1290 1295
   Gly Gly Lys Ala Ile Asp Ala Thr Leu Met Ser Pro Arg Gly Ile Ala
   1300 1305 1310
   Val Asp Lys Asn Gly Leu Met Tyr Phe Val Asp Ala Thr Met Ile Arg
   1315 1320 1325
   Lys Val Asp Gln Asn Gly Ile Ile Ser Thr Leu Leu Gly Ser Asn Asp
   1330 1335 1340
   Leu Thr Ala Val Arg Pro Leu Ser Cys Asp Ser Ser Met Asp Val Ala
   1345 1350 1355 1360
   Gln Val Arg Leu Glu Trp Pro Thr Asp Leu Ala Val Asn Pro Met Asp
   1365 1370 1375
   Asn Ser Leu Tyr Val Leu Glu Asn Asn Val Ile Leu Arg Ile Thr Glu
   1380 1385 1390
   Asn His Gln Val Ser Ile Ile Ala Gly Arg Pro Met His Cys Gln Val
   1395 1400 1405
   Pro Gly Ile Asp Tyr Ser Leu Ser Lys Leu Ala Ile His Ser Ala Leu
   1410 1415 1420
   Glu Ser Ala Ser Ala Ile Ala Ile Ser His Thr Gly Val Leu Tyr Ile
   1425 1430 1435 1440
   Thr Glu Thr Asp Glu Lys Lys Ile Asn Arg Leu Arg Gln Val Thr Thr
   1445 1450 1455
   Asn Gly Glu Ile Cys Leu Leu Ala Gly Ala Ala Ser Asp Cys Asp Cys
   1460 1465 1470
   Lys Asn Asp Val Asn Cys Asn Cys Tyr Ser Gly Asp Asp Ala Tyr Ala
   1475 1480 1485
   Thr Asp Ala Ile Leu Asn Ser Pro Ser Ser Leu Ala Val Ala Pro Asp
   1490 1495 1500
   Gly Thr Ile Tyr Ile Ala Asp Leu Gly Asn Ile Arg Ile Arg Ala Val
   1505 1510 1515 1520
   Ser Lys Asn Lys Pro Val Leu Asn Ala Phe Asn Gln Tyr Glu Ala Ala
   1525 1530 1535
   Ser Pro Gly Glu Gln Glu Leu Tyr Val Phe Asn Ala Asp Gly Ile His
   1540 1545 1550
   Gln Tyr Thr Val Ser Leu Val Thr Gly Glu Tyr Leu Tyr Asn Phe Thr
   1555 1560 1565
   Tyr Ser Thr Asp Asn Asp Val Thr Glu Leu Ile Asp Asn Asn Gly Asn
   1570 1575 1580
   Ser Leu Lys Ile Arg Arg Asp Ser Ser Gly Met Pro Arg His Leu Leu
   1585 1590 1595 1600
   Met Pro Asp Asn Gln Ile Ile Thr Leu Thr Val Gly Thr Asn Gly Gly
   1605 1610 1615
   Leu Lys Val Val Ser Thr Gln Asn Leu Glu Leu Gly Leu Met Thr Tyr
   1620 1625 1630
   Asp Gly Asn Thr Gly Leu Leu Ala Thr Lys Ser Asp Glu Thr Gly Trp
   1635 1640 1645
   Thr Thr Phe Tyr Asp Tyr Asp His Glu Gly Arg Leu Thr Asn Val Thr
   1650 1655 1660
   Arg Pro Thr Gly Val Val Thr Ser Leu His Arg Glu Met Glu Lys Ser
   1665 1670 1675 1680
   Ile Thr Ile Asp Ile Glu Asn Ser Asn Arg Asp Asp Asp Val Thr Val
   1685 1690 1695
   Ile Thr Asn Leu Ser Ser Val Glu Ala Ser Tyr Thr Val Val Gln Asp
   1700 1705 1710
   Gln Val Arg Asn Ser Tyr Gln Leu Cys Asn Asn Gly Thr Leu Arg Val
   1715 1720 1725
   Met Tyr Ala Asn Gly Met Gly Ile Ser Phe His Ser Glu Pro His Val
   1730 1735 1740
   Leu Ala Gly Thr Ile Thr Pro Thr Ile Gly Arg Cys Asn Ile Ser Leu
   1745 1750 1755 1760
   Pro Met Glu Asn Gly Leu Asn Ser Ile Glu Trp Arg Leu Arg Lys Glu
   1765 1770 1775
   Gln Ile Lys Gly Lys Val Thr Ile Phe Gly Arg Lys Leu Arg Val His
   1780 1785 1790
   Gly Arg Asn Leu Leu Ser Ile Asp Tyr Asp Arg Asn Ile Arg Thr Glu
   1795 1800 1805
   Lys Ile Tyr Asp Asp His Arg Lys Phe Thr Leu Arg Ile Ile Tyr Asp
   1810 1815 1820
   Gln Val Gly Arg Pro Phe Leu Trp Leu Pro Ser Ser Gly Leu Ala Ala
   1825 1830 1835 1840
   Val Asn Val Ser Tyr Phe Phe Asn Gly Arg Leu Ala Gly Leu Gln Arg
   1845 1850 1855
   Gly Ala Met Ser Glu Arg Thr Asp Ile Asp Lys Gln Gly Arg Ile Val
   1860 1865 1870
   Ser Arg Met Phe Ala Asp Gly Lys Val Trp Ser Tyr Ser Tyr Leu Asp
   1875 1880 1885
   Lys Ser Met Val Leu Leu Leu Gln Ser Gln Arg Gln Tyr Ile Phe Glu
   1890 1895 1900
   Tyr Asp Ser Ser Asp Arg Leu Leu Ala Val Thr Met Pro Ser Val Ala
   1905 1910 1915 1920
   Arg His Ser Met Ser Thr His Thr Ser Ile Gly Tyr Ile Arg Asn Ile
   1925 1930 1935
   Tyr Asn Pro Pro Glu Ser Asn Ala Ser Val Ile Phe Asp Tyr Ser Asp
   1940 1945 1950
   Asp Gly Arg Ile Leu Lys Thr Ser Phe Leu Gly Thr Gly Arg Gln Val
   1955 1960 1965
   Phe Tyr Lys Tyr Gly Lys Leu Ser Lys Leu Ser Glu Ile Val Tyr Asp
   1970 1975 1980
   Ser Thr Ala Val Thr Phe Gly Tyr Asp Glu Thr Thr Gly Val Leu Lys
   1985 1990 1995 2000
   Met Val Asn Leu Gln Ser Gly Gly Phe Ser Cys Thr Ile Arg Tyr Arg
   2005 2010 2015
   Lys Ile Gly Pro Leu Val Asp Lys Gln Ile Tyr Arg Phe Ser Glu Glu
   2020 2025 2030
   Gly Met Val Asn Ala Arg Phe Asp Tyr Thr Tyr His Asp Asn Ser Phe
   2035 2040 2045
   Arg Ile Ala Ser Ile Lys Pro Val Ile Ser Glu Thr Pro Leu Pro Val
   2050 2055 2060
   Asp Leu Tyr Arg Tyr Asp Glu Ile Ser Gly Lys Val Glu His Phe Gly
   2065 2070 2075 2080
   Lys Phe Gly Val Ile Tyr Tyr Asp Ile Asn Gln Ile Ile Thr Thr Ala
   2085 2090 2095
   Val Met Thr Leu Ser Lys His Phe Asp Thr His Gly Arg Ile Lys Glu
   2100 2105 2110
   Val Gln Tyr Glu Met Phe Arg Ser Leu Met Tyr Trp Met Thr Val Gln
   2115 2120 2125
   Tyr Asp Ser Met Gly Arg Val Ile Lys Arg Glu Leu Lys Leu Gly Pro
   2130 2135 2140
   Tyr Ala Asn Thr Thr Lys Tyr Thr Tyr Asp Tyr Asp Gly Asp Gly Gln
   2145 2150 2155 2160
   Leu Gln Ser Val Ala Val Asn Asp Arg Pro Thr Trp Arg Tyr Ser Tyr
   2165 2170 2175
   Asp Leu Asn Gly Asn Leu His Leu Leu Asn Pro Gly Asn Ser Val Arg
   2180 2185 2190
   Leu Met Pro Leu Arg Tyr Asp Leu Arg Asp Arg Ile Thr Arg Leu Gly
   2195 2200 2205
   Asp Val Gln Tyr Lys Ile Asp Asp Asp Gly Tyr Leu Cys Gln Arg Gly
   2210 2215 2220
   Ser Asp Ile Phe Glu Tyr Asn Ser Lys Gly Leu Leu Thr Arg Ala Tyr
   2225 2230 2235 2240
   Asn Lys Ala Ser Gly Trp Ser Val Gln Tyr Arg Tyr Asp Gly Val Gly
   2245 2250 2255
   Arg Arg Ala Ser Tyr Lys Thr Asn Leu Gly His His Leu Gln Tyr Phe
   2260 2265 2270
   Tyr Ser Asp Leu His Asn Pro Thr Arg Ile Thr His Val Tyr Asn His
   2275 2280 2285
   Ser Asn Ser Glu Ile Thr Ser Leu Tyr Tyr Asp Leu Gln Gly His Leu
   2290 2295 2300
   Phe Ala Met Glu Ser Ser Ser Gly Glu Glu Tyr Tyr Val Ala Ser Asp
   2305 2310 2315 2320
   Asn Thr Gly Thr Pro Leu Ala Val Phe Ser Ile Asn Gly Leu Met Ile
   2325 2330 2335
   Lys Gln Leu Gln Tyr Thr Ala Tyr Gly Glu Ile Tyr Tyr Asp Ser Asn
   2340 2345 2350
   Pro Asp Phe Gln Met Val Ile Gly Phe His Gly Gly Leu Tyr Asp Pro
   2355 2360 2365
   Leu Thr Lys Leu Val His Phe Thr Gln Arg Asp Tyr Asp Val Leu Ala
   2370 2375 2380
   Gly Arg Trp Thr Ser Pro Asp Tyr Thr Met Trp Lys Asn Val Gly Lys
   2385 2390 2395 2400
   Glu Pro Ala Pro Phe Asn Leu Tyr Met Phe Lys Ser Asn Asn Pro Leu
   2405 2410 2415
   Ser Ser Glu Leu Asp Leu Lys Asn Tyr Val Thr Asp Val Lys Ser Trp
   2420 2425 2430
   Leu Val Met Phe Gly Phe Gln Leu Ser Asn Ile Ile Pro Gly Phe Pro
   2435 2440 2445
   Arg Ala Lys Met Tyr Phe Val Pro Pro Pro Tyr Glu Leu Ser Glu Ser
   2450 2455 2460
   Gln Ala Ser Glu Asn Gly Gln Leu Ile Thr Gly Val Gln Gln Thr Thr
   2465 2470 2475 2480
   Glu Arg His Asn Gln Ala Phe Met Ala Leu Glu Gly Gln Val Ile Thr
   2485 2490 2495
   Lys Lys Leu His Ala Ser Ile Arg Glu Lys Ala Gly His Trp Phe Ala
   2500 2505 2510
   Thr Thr Thr Pro Ile Ile Gly Lys Gly Ile Met Phe Ala Ile Lys Glu
   2515 2520 2525
   Gly Arg Val Thr Thr Gly Val Ser Ser Ile Ala Ser Glu Asp Ser Arg
   2530 2535 2540
   Lys Val Ala Ser Val Leu Asn Asn Ala Tyr Tyr Leu Asp Lys Met His
   2545 2550 2555 2560
   Tyr Ser Ile Glu Gly Lys Asp Thr His Tyr Phe Val Lys Ile Gly Ser
   2565 2570 2575
   Ala Asp Gly Asp Leu Val Thr Leu Gly Thr Thr Ile Gly Arg Lys Val
   2580 2585 2590
   Leu Glu Ser Gly Val Asn Val Thr Val Ser Gln Pro Thr Leu Leu Val
   2595 2600 2605
   Asn Gly Arg Thr Arg Arg Phe Thr Asn Ile Glu Phe Gln Tyr Ser Thr
   2610 2615 2620
   Leu Leu Leu Ser Ile Arg Tyr Gly Leu Thr Pro Asp Thr Leu Asp Glu
   2625 2630 2635 2640
   Glu Lys Ala Arg Val Leu Asp Gln Ala Arg Gln Arg Ala Leu Gly Thr
   2645 2650 2655
   Ala Trp Ala Lys Glu Gln Gln Lys Ala Arg Asp Gly Arg Glu Gly Ser
   2660 2665 2670
   Arg Leu Trp Thr Glu Gly Glu Lys Gln Gln Leu Leu Ser Thr Gly Arg
   2675 2680 2685
   Val Gln Gly Tyr Glu Gly Tyr Tyr Val Leu Pro Val Glu Gln Tyr Pro
   2690 2695 2700
   Glu Leu Ala Asp Ser Ser Ser Asn Ile Gln Phe Leu Arg Gln Asn Glu
   2705 2710 2715 2720
   Met Gly Lys Arg
<210> 14
   <211> 609
   <212> DNA
   <213> Homo sapiens
<220>
   <221> CDS
   <222> (99)..(521)
<400> 14
   ctgacatact atattagttg tttgttcact gtctccactc cagctagaat ataagttcca 60
   tagggcagag tttttgttca ctgctatatt ttataagc atg aat gaa tgc atg aac 116
   gaa tgg act gat aac cca caa gcc aaa gac ctc cat gac ctg cca ctg   164
   ccc tcc ttt cat ttt att ctc acc tct acc aat act aaa tca cct agt   212
   tat gta aat acg ata tgc act ttc atg gcc cct tgc ttt gtc ata tgc   260
   tgt tcc ctt tgc ctg gaa tat aaa ctc tca aaa tac cat cca cat ttt   308
   aaa atc ttc tcc aga aag ctt cct ctg tcc acc ccc acc ctc cca ccc   356
   cca tat aga gta agt cag tct ttc ctt tgt gct aca ttt gta cct gta   404
   tct aca gtg gct cta atc aaa ctg cac tgt gtc tct cac ttc cta gat   452
   tgt gaa ctc ttt gag gct gaa gac tac tta ttc atc tct tta cct cca     500
   atg cct agg aca gga cct tca taaagcaact actctataaa tgttgaaaca        551
   tatgcatgac tattctgtaa caggaatgaa aatatggcat ttcaagaagt cactactc     609
<210> 15
   <211> 141
   <212> PRT
   <213> Homo sapiens
<400> 15
<210> 16
   <211> 1667
   <212> DNA
   <213> Homo sapiens
<220>
   <221> CDS
   <222> (34)..(1494)
<400> 16
   gttctctcgc aggtcccaga tgtccagttc cag atg cct gga ccc aga gtg tgg   54
                                        Met Pro Gly Pro Arg Val Trp
                                        1               5
   ggg aaa tat ctc tgg aga agc cct cac tcc aaa ggc tgt cca ggc gca    102
   Gly Lys Tyr Leu Trp Arg Ser Pro His Ser Lys Gly Cys Pro Gly Ala
           10                  15                  20
   atg tgg tgg ctg ctt ctc tgg gga gtc ctc cag gct tgc cca acc cgg    150
   Met Trp Trp Leu Leu Leu Trp Gly Val Leu Gln Ala Cys Pro Thr Arg
       25                  30                  35
   ggc tcc gtc ctc ttg gcc caa gag cta ccc cag cag ctg aca tcc ccc    198
   Gly Ser Val Leu Leu Ala Gln Glu Leu Pro Gln Gln Leu Thr Ser Pro
   40                  45                  50                  55
   ggg tac cca gag ccg tat ggc aaa ggc caa gag agc agc acg gac atc    246
   Gly Tyr Pro Glu Pro Tyr Gly Lys Gly Gln Glu Ser Ser Thr Asp Ile
                   60                  65                  70
   aag gct cca gag ggc ttt gct gtg agg ctc gtc ttc cag gac ttc gac    294
   Lys Ala Pro Glu Gly Phe Ala Val Arg Leu Val Phe Gln Asp Phe Asp
               75                  80                  85
   ctg gag ccg tcc cag gac tgt gca ggg gac tct gtc aca atc tca ttc    342
   Leu Glu Pro Ser Gln Asp Cys Ala Gly Asp Ser Val Thr Ile Ser Phe
           90                  95                  100
   gtc ggt tcg gat cca agc cag ttc tgt ggt cag caa ggc tcc cct ctg    390
   Val Gly Ser Asp Pro Ser Gln Phe Cys Gly Gln Gln Gly Ser Pro Leu
       105                 110                 115
   ggc agg ccc cct ggt cag agg gag ttt gta tcc tca ggg agg agt ttg    438
   Gly Arg Pro Pro Gly Gln Arg Glu Phe Val Ser Ser Gly Arg Ser Leu
   120                 125                 130                 135
   cgg ctg acc ttc cgc aca cag cct tcc tcg gag aac aag act gcc cac    486
   Arg Leu Thr Phe Arg Thr Gln Pro Ser Ser Glu Asn Lys Thr Ala His
                   140                 145                 150
   ctc cac aag ggc ttc ctg gcc ctc tac caa acc gtg gct gtg aac tat    534
   Leu His Lys Gly Phe Leu Ala Leu Tyr Gln Thr Val Ala Val Asn Tyr
               155                 160                 165
   agt cag ccc atc agc gag gcc agc agg ggc tct gag gcc atc aac gca    582
   Ser Gln Pro Ile Ser Glu Ala Ser Arg Gly Ser Glu Ala Ile Asn Ala
           170                 175                 180
   cct gga gac aac cct gcc aag gtc cag aac cac tgc cag gag ccc tat    630
   Pro Gly Asp Asn Pro Ala Lys Val Gln Asn His Cys Gln Glu Pro Tyr
       185                 190                 195
   tat cag gcc gcg gca gca ggg gca ctc acc tgt gca acc cca ggg acc    678
   Tyr Gln Ala Ala Ala Ala Gly Ala Leu Thr Cys Ala Thr Pro Gly Thr
   200                 205                 210                 215
   tgg aaa gac aga cag gat ggg gag gag gtt ctt cag tgt atg cct gtc    726
   Trp Lys Asp Arg Gln Asp Gly Glu Glu Val Leu Gln Cys Met Pro Val
                   220                 225                 230
   tgc gga cgg cca gtc acc ccc att gcc cag aat cag acg acc ctc ggt    774
   Cys Gly Arg Pro Val Thr Pro Ile Ala Gln Asn Gln Thr Thr Leu Gly
               235                 240                 245
   tct tcc aga gcc aag ctg ggc aac ttc ccc tgg caa gcc ttc acc agt    822
   Ser Ser Arg Ala Lys Leu Gly Asn Phe Pro Trp Gln Ala Phe Thr Ser
           250                 255                 260
   atc cac ggc cgt ggg ggc ggg gcc ctg ctg ggg gac aga tgg atc ctc    870
   Ile His Gly Arg Gly Gly Gly Ala Leu Leu Gly Asp Arg Trp Ile Leu
       265                 270                 275
   act gct gcc cac acc atc tac ccc aag gac agt gtt tct ctc agg aag    918
   Thr Ala Ala His Thr Ile Tyr Pro Lys Asp Ser Val Ser Leu Arg Lys
   280                 285                 290                 295
   aac cag agt gtg aat gtg ttc ttg ggc cac aca gcc ata gat gag atg    966
   Asn Gln Ser Val Asn Val Phe Leu Gly His Thr Ala Ile Asp Glu Met
                   300                 305                 310
   ctg aaa ctg ggg aac cac cct gtc cac cgt gtc gtt gtg cac ccc gac    1014
   Leu Lys Leu Gly Asn His Pro Val His Arg Val Val Val His Pro Asp
               315                 320                 325
   tac cgt cag aat gag tcc cat aac ttt agc ggg gac atc gcc ctc ctg    1062
   Tyr Arg Gln Asn Glu Ser His Asn Phe Ser Gly Asp Ile Ala Leu Leu
               330                 335                 340
   gag ctg cag cac agc atc ccc ctg ggc ccc aac gtc ctc ccg gtc tgt    1110
   Glu Leu Gln His Ser Ile Pro Leu Gly Pro Asn Val Leu Pro Val Cys
       345                 350                 355
   ctg ccc gat aat gag acc ctc tac cgc agc ggc ttg ttg ggc tac gtc    1158
   Leu Pro Asp Asn Glu Thr Leu Tyr Arg Ser Gly Leu Leu Gly Tyr Val
   360                 365                 370                 375
   agt ggg ttt ggc atg gag atg ggc tgg cta act act gag ctg aag tac    1206
   Ser Gly Phe Gly Met Glu Met Gly Trp Leu Thr Thr Glu Leu Lys Tyr
                   380                 385                 390
   tcg agg ctg cct gta gct ccc agg gag gcc tgc aac gcc tgg ctc caa    1254
   Ser Arg Leu Pro Val Ala Pro Arg Glu Ala Cys Asn Ala Trp Leu Gln
               395                 400                 405
   aag aga cag aga ccc gag gtg ttt tct gac aat atg ttc tgt gtt ggg    1302
   Lys Arg Gln Arg Pro Glu Val Phe Ser Asp Asn Met Phe Cys Val Gly
           410                 415                 420
   gat gag acg caa agg cac agt gtc tgc cag ggg gac agt ggc agc ctc    1350
   Asp Glu Thr Gln Arg His Ser Val Cys Gln Gly Asp Ser Gly Ser Leu
       425                 430                 435
   tat gtg gta tgg gac aat cat gcc cat cac tgg gtg gcc acg ggc att    1398
   Tyr Val Val Trp Asp Asn His Ala His His Trp Val Ala Thr Gly Ile
   440                 445                 450                 455
   gtg tcc tgg ggc ata ggg tgt ggc gaa ggg tat gac ttc tac acc aag    1446
   Val Ser Trp Gly Ile Gly Cys Gly Glu Gly Tyr Asp Phe Tyr Thr Lys
                   460                 465                 470
   gtg ctc agc tat gtg gac tgg atc aag gga gtg atg aat ggc aag aat    1494
   Val Leu Ser Tyr Val Asp Trp Ile Lys Gly Val Met Asn Gly Lys Asn
               475                 480                 485
   tgaccctggg ggcttgaaca gggactgacc agcacagtgg aggccccagg caacagaggg 1554
   cctggagtga ggactgaaca ctggggtagg gggtgggggt ttctcttgca gtggcttggt 1614
   gcaacagtga tgtgaatagg atttcccttt tttttttttt ttttaaaaaa aaa        1667
<210> 17
   <211> 487
   <212> PRT
   <213> Homo sapiens
<400> 17
   Met Pro Gly Pro Arg Val Trp Gly Lys Tyr Leu Trp Arg Ser Pro His
   1 5 10 15
   Ser Lys Gly Cys Pro Gly Ala Met Trp Trp Leu Leu Leu Trp Gly Val
   20 25 30
   Leu Gln Ala Cys Pro Thr Arg Gly Ser Val Leu Leu Ala Gln Glu Leu
   35 40 45
   Pro Gln Gln Leu Thr Ser Pro Gly Tyr Pro Glu Pro Tyr Gly Lys Gly
   50 55 60
   Gln Glu Ser Ser Thr Asp Ile Lys Ala Pro Glu Gly Phe Ala Val Arg
   65 70 75 80
   Leu Val Phe Gln Asp Phe Asp Leu Glu Pro Ser Gln Asp Cys Ala Gly
   85 90 95
   Asp Ser Val Thr Ile Ser Phe Val Gly Ser Asp Pro Ser Gln Phe Cys
   100 105 110
   Gly Gln Gln Gly Ser Pro Leu Gly Arg Pro Pro Gly Gln Arg Glu Phe
   115 120 125
   Val Ser Ser Gly Arg Ser Leu Arg Leu Thr Phe Arg Thr Gln Pro Ser
   130 135 140
   Ser Glu Asn Lys Thr Ala His Leu His Lys Gly Phe Leu Ala Leu Tyr
   145 150 155 160
   Gln Thr Val Ala Val Asn Tyr Ser Gln Pro Ile Ser Glu Ala Ser Arg
   165 170 175
   Gly Ser Glu Ala Ile Asn Ala Pro Gly Asp Asn Pro Ala Lys Val Gln
   180 185 190
   Asn His Cys Gln Glu Pro Tyr Tyr Gln Ala Ala Ala Ala Gly Ala Leu
   195 200 205
   Thr Cys Ala Thr Pro Gly Thr Trp Lys Asp Arg Gln Asp Gly Glu Glu
   210 215 220
   Val Leu Gln Cys Met Pro Val Cys Gly Arg Pro Val Thr Pro Ile Ala
   225 230 235 240
   Gln Asn Gln Thr Thr Leu Gly Ser Ser Arg Ala Lys Leu Gly Asn Phe
                   245                 250                 255
   Pro Trp Gln Ala Phe Thr Ser Ile His Gly Arg Gly Gly Gly Ala Leu
               260                 265                 270
   Leu Gly Asp Arg Trp Ile Leu Thr Ala Ala His Thr Ile Tyr Pro Lys
           275                 280                 285
   Asp Ser Val Ser Leu Arg Lys Asn Gln Ser Val Asn Val Phe Leu Gly
       290                 295                 300
   His Thr Ala Ile Asp Glu Met Leu Lys Leu Gly Asn His Pro Val His
   305                 310                 315                 320
   Arg Val Val Val His Pro Asp Tyr Arg Gln Asn Glu Ser His Asn Phe
                   325                 330                 335
   Ser Gly Asp Ile Ala Leu Leu Glu Leu Gln His Ser Ile Pro Leu Gly
               340                 345                 350
   Pro Asn Val Leu Pro Val Cys Leu Pro Asp Asn Glu Thr Leu Tyr Arg
           355                 360                 365
   Ser Gly Leu Leu Gly Tyr Val Ser Gly Phe Gly Met Glu Met Gly Trp
       370                 375                 380
   Leu Thr Thr Glu Leu Lys Tyr Ser Arg Leu Pro Val Ala Pro Arg Glu
   385                 390                 395                 400
   Ala Cys Asn Ala Trp Leu Gln Lys Arg Gln Arg Pro Glu Val Phe Ser
                   405                 410                 415
   Asp Asn Met Phe Cys Val Gly Asp Glu Thr Gln Arg His Ser Val Cys
               420                 425                 430
   Gln Gly Asp Ser Gly Ser Leu Tyr Val Val Trp Asp Asn His Ala His
           435                 440                 445
   His Trp Val Ala Thr Gly Ile Val Ser Trp Gly Ile Gly Cys Gly Glu
       450                 455                 460
   Gly Tyr Asp Phe Tyr Thr Lys Val Leu Ser Tyr Val Asp Trp Ile Lys
   465                 470                 475                 480
   Gly Val Met Asn Gly Lys Asn
                   485
<210> 18
   <211> 1691
   <212> DNA
   <213> Homo sapiens
<400> 18
   ttttttttta aaaaaaaaaa aaaaaaggga aatcctattc acatcactgt tgcaccaagc 60
   cactgcaaga gaaaccccca ccccctaccc cagtgttcag tcctcactcc aggccctctg 120
   ttgcctgggg cctccactgt gctggtcagt ccctgttcaa gcccccaggg tcaattcttg 180
   ccattcatca ctcccttgat ccagtccaca tagctgagca ccttggtgta gaagtcatac 240
   ccttcgccac accctatgcc ccaggacaca atgcccgtgg ccacccagtg atgggcatga 300
   ttgtcccata ccacatagag gctgccactg tccccctggc agacactgtg cctttgcgtc 360
   tcatccccaa cacagaacat attgtcagaa aacacctcgg gtctctgtct cttttggagc 420
   caggcgttgc aggcctccct gggagctaca ggcagcctcg agtacttcag ctcagtagtt 480
   agccagccca tctccatgcc aaacccactg acgtagccca acaagccgct gcggtagagg 540
   gtctcattat cgggcagaca gaccgggagg acgttggggc ccagggggat gctgtgctgc 600
   agctccagga gggcgatgtc cccgctaaag ttatgggact cattctgacg gtagtcgggg 660
   tgcacaacga cacggtggac agggtggttc cccagtttca gcatctcatc tatggctgtg 720
   tggcccaaga acacattcac actctggttc ttcctgagag aaacactgtc cttggggtag 780
   atggtgtggg cagcagtgag gatccatctg tcccccagca gggccccgcc cccacggccg 840
   tggatactgg tgaaggcttg ccaggggaag ttgcccagct tggctctgga agaaccgagg 900
   gtcgtctgat tctgggcaat gggggtgact ggccgtccgc agacaggcat acactgaaga 960
   acctcctccc catcctgtct gtctttccag gtccctgggg ttgcacaggt gagtgcccct 1020
   gctgccgcgg cctgataata gggctcctgg cagtggttct ggaccttggc agggttgtct 1080
   ccaggtgcgt tgatggcctc agagcccctg ctggcctcgc tgatgggctg actatagttc 1140
   acagccacgg tttggtagag ggccaggaag cccttgtgga ggtgggcagt cttgttctcc 1200
   gaggaaggct gtgtgcggaa ggtcagccgc aaactcctcc ctgaggatac aaactccctc 1260
   tgaccagggg gcctgcccag aggggagcct tgctgaccac agaactggct tggatccgaa 1320
   ccgacgaatg agattgtgac agagtcccct gcacagtcct gggacggctc caggtcgaag 1380
   tcctggaaga cgagcctcac agcaaagccc tctggagcct tgatgtccgt gctgctctct 1440
   tggcctttgc catacggctc tgggtacccg ggggatgtca gctgctgggg tagctcttgg 1500
   gccaagagga cggagccccg ggttgggcaa gcctggagga ctccccagag aagcagccac 1560
   cacattgcgc ctggacagcc tttggagtga gggcttctcc agagatattt cccccacact 1620
   ctgggtccag gcatctggaa ctggacatct gggacctgcg agagaactgg cccaggatag 1680
   ggaacaaaag g                                                      1691
<210> 19
   <211> 487
   <212> PRT
   <213> Homo sapiens
<400> 19
   Met Pro Gly Pro Arg Val Trp Gly Lys Tyr Leu Trp Arg Ser Pro His
   1               5                   10                  15
   Ser Lys Gly Cys Pro Gly Ala Met Trp Trp Leu Leu Leu Trp Gly Val
               20                  25                  30
   Leu Gln Ala Cys Pro Thr Arg Gly Ser Val Leu Leu Ala Gln Gln Leu
           35                  40                  45
   Pro Gln Gln Leu Thr Ser Pro Gly Tyr Pro Glu Pro Tyr Gly Lys Gly
       50                  55                  60
   Gln Glu Ser Ser Thr Asp Ile Lys Ala Pro Glu Gly Phe Ala Val Arg
   65                  70                  75                  80
   Leu Val Phe Gln Asp Phe Asp Leu Glu Pro Ser Gln Asp Cys Ala Gly
                   85                  90                  95
   Asp Ser Val Thr Ile Ser Phe Val Gly Ser Asp Pro Ser Gln Phe Cys
               100                 105                 110
   Gly Gln Gln Gly Ser Pro Leu Gly Arg Pro Pro Gly Gln Arg Glu Phe
           115                 120                 125
   Val Ser Ser Gly Arg Ser Leu Arg Leu Thr Phe Arg Thr Gln Pro Ser
       130                 135                 140
   Ser Glu Asn Lys Thr Ala His Leu His Lys Gly Phe Leu Ala Leu Tyr
   145                 150                 155                 160
   Gln Thr Val Ala Val Asn Tyr Ser Gln Pro Ile Ser Glu Ala Ser Arg
                   165                 170                 175
   Gly Ser Glu Ala Ile Asn Ala Pro Gly Asp Asn Pro Ala Lys Val Gln
               180                 185                 190
   Asn His Cys Gln Glu Pro Tyr Tyr Gln Ala Ala Ala Ala Gly Ala Leu
           195                 200                 205
   Thr Cys Ala Thr Pro Gly Thr Trp Lys Asp Arg Gln Asp Gly Glu Glu
       210                 215                 220
   Val Leu Gln Cys Met Pro Val Cys Gly Arg Pro Val Thr Pro Ile Ala
   225                 230                 235                 240
   Gln Asn Gln Thr Thr Leu Gly Ser Ser Arg Ala Lys Leu Gly Asn Phe
                   245                 250                 255
   Pro Trp Gln Ala Phe Thr Ser Ile His Gly Arg Gly Gly Gly Ala Leu
               260                 265                 270
   Leu Gly Asp Arg Trp Ile Leu Thr Ala Ala His Thr Ile Tyr Pro Lys
   275 280 285
   Asp Ser Val Ser Leu Arg Lys Asn Gln Ser Val Asn Val Phe Leu Gly
   290 295 300
   His Thr Ala Ile Asp Glu Met Leu Lys Leu Gly Asn His Pro Val His
   305 310 315 320
   Arg Val Val Val His Pro Asp Tyr Arg Gln Asn Glu Ser His Asn Phe
   325 330 335
   Ser Gly Asp Ile Ala Leu Leu Glu Leu Gln His Ser Ile Pro Leu Gly
   340 345 350
   Pro Asn Val Leu Pro Val Cys Leu Pro Asp Asn Glu Thr Leu Tyr Arg
   355 360 365
   Ser Gly Leu Leu Gly Tyr Val Ser Gly Phe Gly Met Glu Met Gly Trp
   370 375 380
   Leu Thr Thr Glu Leu Lys Tyr Ser Arg Leu Pro Val Ala Pro Arg Glu
   385 390 395 400
   Ala Cys Asn Ala Trp Leu Gln Lys Arg Gln Arg Pro Glu Val Phe Ser
   405 410 415
   Asp Asn Met Phe Cys Val Gly Asp Glu Thr Gln Arg His Ser Val Cys
   420 425 430
   Gln Gly Asp Ser Gly Ser Leu Tyr Val Val Trp Asp Asn His Ala His
   435 440 445
   His Trp Val Ala Thr Gly Ile Val Ser Trp Gly Ile Gly Cys Gly Glu
   450 455 460
   Gly Tyr Asp Phe Tyr Thr Lys Val Leu Ser Tyr Val Asp Trp Ile Lys
   465 470 475 480
   Gly Val Met Asn Gly Lys Asn
   485
<210> 20
   <211> 1078
   <212> DNA
   <213> Homo sapiens
<220>
   <221> CDS
   <222> (293) .. (1043)
<400> 20
   ttgatccgtg ccaagtggct ttttgtgggc tctgtagagt gctctaaacc cagctcggcc 60
   tttgctgtat tagacagaag cacctcattc atatccctgg ggcccctgat ggtgcagtgg 120
   tctggctgtg gtctgcacac cagctattct gttttgtttt gttttgtttt tttcctacct 180
   ttttccaatc ctcacacctt ctgatcaaca gccccagtag ggtttaaagg tcctagagct 240
   ac atg gga ttt agg ttt ctg ggc aca gcc aat tct gcc act ttt gag     287
      Met Gly Phe Arg Phe Leu Gly Thr Ala Asn Ser Ala Thr Phe Glu
      1               5                   10                  15
   act tcc ctt ccc ctt cca ctt gcc cct ctc tgg ttc tct gcc acc agt    335
   Thr Ser Leu Pro Leu Pro Leu Ala Pro Leu Trp Phe Ser Ala Thr Ser
                   20                  25                  30
   cca gaa gaa ctg agt gtc gtg ctg ggg acc aac gac tta act agc cca    383
   Pro Glu Glu Leu Ser Val Val Leu Gly Thr Asn Asp Leu Thr Ser Pro
               35                  40                  45
   tcc atg gaa ata aag gag gtc gcc agc atc att ctt cac aaa gac ttt    431
   Ser Met Glu Ile Lys Glu Val Ala Ser Ile Ile Leu His Lys Asp Phe
           50                  55                  60
   aag aga gcc aac atg gac aat gac att gcc ttg ctg ctg ctg gct tcg    479
   Lys Arg Ala Asn Met Asp Asn Asp Ile Ala Leu Leu Leu Leu Ala Ser
       65                  70                  75
   ccc atc aag ctc gat gac ctg aag gtg ccc atc tgc ctc ccc acg cag    527
   Pro Ile Lys Leu Asp Asp Leu Lys Val Pro Ile Cys Leu Pro Thr Gln
   80                  85                  90                  95
   ccc ggc cct gcc aca tgg cgc gaa tgc tgg gtg gca ggt tgg ggc cag    575
   Pro Gly Pro Ala Thr Trp Arg Glu Cys Trp Val Ala Gly Trp Gly Gln
                   100                 105                 110
   acc aat gct gct gac aaa aac tct gtg aaa acg gat ctg atg aaa gtg    623
   Thr Asn Ala Ala Asp Lys Asn Ser Val Lys Thr Asp Leu Met Lys Val
               115                 120                 125
   cca atg gtc atc atg gac tgg gag gag tgt tca aag atg ttt cca aaa    671
   Pro Met Val Ile Met Asp Trp Glu Glu Cys Ser Lys Met Phe Pro Lys
           130                 135                 140
   ctt acc aaa aat atg ctg tgt gcc gga tac aag aat gag agc tat gat    719
   Leu Thr Lys Asn Met Leu Cys Ala Gly Tyr Lys Asn Glu Ser Tyr Asp
       145                 150                 155
   gcc tgc aag ggt gac agt ggg ggg cct ctg gtc tgc acc cca gag cct    767
   Ala Cys Lys Gly Asp Ser Gly Gly Pro Leu Val Cys Thr Pro Glu Pro
   160                 165                 170                 175
   ggt gag aag tgg tac cag gtg ggc atc atc agc tgg gga aag agc tgt    815
   Gly Glu Lys Trp Tyr Gln Val Gly Ile Ile Ser Trp Gly Lys Ser Cys
                   180                 185                 190
   gga gat aag aac acc cca ggg ata tac acc tcg ttg gtg aac tac aac    863
   Gly Asp Lys Asn Thr Pro Gly Ile Tyr Thr Ser Leu Val Asn Tyr Asn
               195                 200                 205
   ctc tgg atc gag aaa gtg acc cag cta gga ggc agg ccc ttc aat gca    911
   Leu Trp Ile Glu Lys Val Thr Gln Leu Gly Gly Arg Pro Phe Asn Ala
           210                 215                 220
   gag aaa agg agg act tct gtc aaa cag aaa cct atg ggc tcc cca gtc    959
   Glu Lys Arg Arg Thr Ser Val Lys Gln Lys Pro Met Gly Ser Pro Val
       225                 230                 235
   tcg gga gtc cca gag cca ggc agc ccc aga tcc tgg ctc ctg ctc tgt    1007
   Ser Gly Val Pro Glu Pro Gly Ser Pro Arg Ser Trp Leu Leu Leu Cys
   240                 245                 250                 255
   ccc ctg tcc cat gtg ttg ttc aga gct att ttg tac tgataataaa         1053
   Pro Leu Ser His Val Leu Phe Arg Ala Ile Leu Tyr
                   260                 265
   atagaggcta ttctttcaac cgaaa                                        1078
<210> 21
   <211> 267
   <212> PRT
   <213> Homo sapiens
<400> 21
   Met Gly Phe Arg Phe Leu Gly Thr Ala Asn Ser Ala Thr Phe Glu Thr
   1               5                   10                  15
   Ser Leu Pro Leu Pro Leu Ala Pro Leu Trp Phe Ser Ala Thr Ser Pro
               20                  25                  30
   Glu Glu Leu Ser Val Val Leu Gly Thr Asn Asp Leu Thr Ser Pro Ser
           35                  40                  45
   Met Glu Ile Lys Glu Val Ala Ser Ile Ile Leu His Lys Asp Phe Lys
       50                  55                  60
   Arg Ala Asn Met Asp Asn Asp Ile Ala Leu Leu Leu Leu Ala Ser Pro
   65                  70                  75                  80
   Ile Lys Leu Asp Asp Leu Lys Val Pro Ile Cys Leu Pro Thr Gln Pro
                   85                  90                  95
   Gly Pro Ala Thr Trp Arg Glu Cys Trp Val Ala Gly Trp Gly Gln Thr
               100                 105                 110
   Asn Ala Ala Asp Lys Asn Ser Val Lys Thr Asp Leu Met Lys Val Pro
           115                 120                 125
   Met Val Ile Met Asp Trp Glu Glu Cys Ser Lys Met Phe Pro Lys Leu
       130                 135                 140
   Thr Lys Asn Met Leu Cys Ala Gly Tyr Lys Asn Glu Ser Tyr Asp Ala
   145                 150                 155                 160
   Cys Lys Gly Asp Ser Gly Gly Pro Leu Val Cys Thr Pro Glu Pro Gly
                   165                 170                 175
   Glu Lys Trp Tyr Gln Val Gly Ile Ile Ser Trp Gly Lys Ser Cys Gly
               180                 185                 190
   Asp Lys Asn Thr Pro Gly Ile Tyr Thr Ser Leu Val Asn Tyr Asn Leu
           195                 200                 205
   Trp Ile Glu Lys Val Thr Gln Leu Gly Gly Arg Pro Phe Asn Ala Glu
       210                 215                 220
   Lys Arg Arg Thr Ser Val Lys Gln Lys Pro Met Gly Ser Pro Val Ser
   225                 230                 235                 240
   Gly Val Pro Glu Pro Gly Ser Pro Arg Ser Trp Leu Leu Leu Cys Pro
                   245                 250                 255
   Leu Ser His Val Leu Phe Arg Ala Ile Leu Tyr
               260                 265
<210> 22
   <211> 1334
   <212> DNA
   <213> Homo sapiens
<220>
   <221> CDS
   <222> (499)..(1299)
<400> 22
   gattttagaa ggttaatcaa aaacccgggg acagtttctt catggcataa ccacagacct 60
   ttgtggcacc cgctgtcgtg ggatatcaaa tatcctctgg ggttcggaat gtgggcttat 120
   tactgaagat cctgtctgct tggtcagtgg caggtctaga ctaacttctg gtcctgagtt 180
   tctaaagtgc tggtagacca gttgatacaa aacagatata ataatgaatg ccttatctat 240
   ctgaaggtca gtttgatccg tgccaagtgg ctttttgtgg gctgtgtaga gtgctctaaa 300
   cccagctcgg cctttgctgt attagacaga agcacctcat tcatatccct ggggcccctg 360
   atggtgcagt ggtctggctg tggtctgcac accagctatt ctgttttgtt ttgttttgtt 420
   ttgttttttc ctaccttttt ccaatcctca caccttctga tcaacagccc cagtagggtt 480
   taaaggtcct agagctac atg gga ttt agg ttt ctg ggc aca gcc aat tct    531
                       Met Gly Phe Arg Phe Leu Gly Thr Ala Asn Ser
                       1               5                   10
   gcc act ttt gag act tcc ctt ccc ctt cca ctt gcc cct ctc tgg ttc    579
   Ala Thr Phe Glu Thr Ser Leu Pro Leu Pro Leu Ala Pro Leu Trp Phe
               15                  20                  25
   tct gcc acc agt cca gaa gaa ctg agt gtc gtg ctg ggg acc aac gac    627
   Ser Ala Thr Ser Pro Glu Glu Leu Ser Val Val Leu Gly Thr Asn Asp
           30                  35                  40
   tta act agc cca tcc atg gaa ata aag gag gtc gcc agc atc att ctt    675
   Leu Thr Ser Pro Ser Met Glu Ile Lys Glu Val Ala Ser Ile Ile Leu
       45                  50                  55
   cac aaa gac ttt aag aga gcc aac atg gac aat gac att gcc ttg ctg    723
   His Lys Asp Phe Lys Arg Ala Asn Met Asp Asn Asp Ile Ala Leu Leu
   60                  65                  70                  75
   ctg ctg gct tcg ccc atc aag ctc gat gac ctg aag gtg ccc atc tgc    771
   Leu Leu Ala Ser Pro Ile Lys Leu Asp Asp Leu Lys Val Pro Ile Cys
                   80                  85                  90
   ctc ccc acg cag ccc ggc cct gcc aca tgg cgc gaa tgc tgg gtg gca     819
   Leu Pro Thr Gln Pro Gly Pro Ala Thr Trp Arg Glu Cys Trp Val Ala
                95                 100                 105
   ggt tgg ggc cag acc aat gct gct gac aaa aac tct gtg aaa acg gat     867
   Gly Trp Gly Gln Thr Asn Ala Ala Asp Lys Asn Ser Val Lys Thr Asp
           110                 115                 120
   ctg atg aaa gtg cca atg gtc atc atg gac tgg gag gag tgt tca aag     915
   Leu Met Lys Val Pro Met Val Ile Met Asp Trp Glu Glu Cys Ser Lys
       125                 130                 135
   atg ttt cca aaa ctt acc aaa aat atg ctg tgt gcc gga tac aag aat     963
   Met Phe Pro Lys Leu Thr Lys Asn Met Leu Cys Ala Gly Tyr Lys Asn
   140                 145                 150                 155
   gag agc tat gat gcc tgc aag ggt gac agt ggg ggg cct ctg gtc tgc     1011
   Glu Ser Tyr Asp Ala Cys Lys Gly Asp Ser Gly Gly Pro Leu Val Cys
                   160                 165                 170
   acc cca gag cct ggt gag aag tgg tac cag gtg ggc atc atc agc tgg     1059
   Thr Pro Glu Pro Gly Glu Lys Trp Tyr Gln Val Gly Ile Ile Ser Trp
               175                 180                 185
   gga aag agc tgt gga gag aag aac acc cca ggg ata tac acc tcg ttg     1107
   Gly Lys Ser Cys Gly Glu Lys Asn Thr Pro Gly Ile Tyr Thr Ser Leu
           190                 195                 200
   gtg aac tac aac ctc tgg atc gag aaa gtg acc cag cta gag ggc agg     1155
   Val Asn Tyr Asn Leu Trp Ile Glu Lys Val Thr Gln Leu Glu Gly Arg
       205                 210                 215
   ccc ttc aat gca gag aaa agg agg act tct gtc aaa cag aaa cct atg     1203
   Pro Phe Asn Ala Glu Lys Arg Arg Thr Ser Val Lys Gln Lys Pro Met
   220                 225                 230                 235
   ggc tcc cca gtc tcg gga gtc cca gag cca ggc agc ccc aga tcc tgg     1251
   Gly Ser Pro Val Ser Gly Val Pro Glu Pro Gly Ser Pro Arg Ser Trp
                   240                 245                 250
   ctc ctg ctc tgt ccc ctg tcc cat gtg ttg ttc aga gct att ttg tac     1299
   Leu Leu Leu Cys Pro Leu Ser His Val Leu Phe Arg Ala Ile Leu Tyr
               255                 260                 265
   tgataataaa atagaggcta ttctttcaac cgaaa                              1334
<210> 23
   <211> 267
   <212> PRT
   <213> Homo sapiens
<400> 23
   Met Gly Phe Arg Phe Leu Gly Thr Ala Asn Ser Ala Thr Phe Glu Thr
   1               5                   10                  15
   Ser Leu Pro Leu Pro Leu Ala Pro Leu Trp Phe Ser Ala Thr Ser Pro
               20                  25                  30
   Glu Glu Leu Ser Val Val Leu Gly Thr Asn Asp Leu Thr Ser Pro Ser
           35                  40                  45
   Met Glu Ile Lys Glu Val Ala Ser Ile Ile Leu His Lys Asp Phe Lys
       50                  55                  60
   Arg Ala Asn Met Asp Asn Asp Ile Ala Leu Leu Leu Leu Ala Ser Pro
   65                  70                  75                  80
   Ile Lys Leu Asp Asp Leu Lys Val Pro Ile Cys Leu Pro Thr Gln Pro
                   85                  90                  95
   Gly Pro Ala Thr Trp Arg Glu Cys Trp Val Ala Gly Trp Gly Gln Thr
               100                 105                 110
   Asn Ala Ala Asp Lys Asn Ser Val Lys Thr Asp Leu Met Lys Val Pro
           115                 120                 125
   Met Val Ile Met Asp Trp Glu Glu Cys Ser Lys Met Phe Pro Lys Leu
       130                 135                 140
   Thr Lys Asn Met Leu Cys Ala Gly Tyr Lys Asn Glu Ser Tyr Asp Ala
   145                 150                 155                 160
   Cys Lys Gly Asp Ser Gly Gly Pro Leu Val Cys Thr Pro Glu Pro Gly
                   165                 170                 175
   Glu Lys Trp Tyr Gln Val Gly Ile Ile Ser Trp Gly Lys Ser Cys Gly
               180                 185                 190
   Glu Lys Asn Thr Pro Gly Ile Tyr Thr Ser Leu Val Asn Tyr Asn Leu
           195                 200                 205
   Trp Ile Glu Lys Val Thr Gln Leu Glu Gly Arg Pro Phe Asn Ala Glu
       210                 215                 220
   Lys Arg Arg Thr Ser Val Lys Gln Lys Pro Met Gly Ser Pro Val Ser
   225 230 235 240
   Gly Val Pro Glu Pro Gly Ser Pro Arg Ser Trp Leu Leu Leu Cys Pro
   245 250 255
   Leu Ser His Val Leu Phe Arg Ala Ile Leu Tyr
   260 265
<210> 24
   <211> 1498
   <212> DNA
   <213> Homo sapiens
<400> 24
   aggcgcctgg ttctgcgcgt actggctgta cggagcagga gcaagaggtc gccgccagcc 60
   tccgccgccg agcctcgttc gtgtccccgc ccctcgctcc tgcagctact gctcagaaac 120
   gctggggcgc ccaccctggc agactaacga agcagctccc ttcccacccc aactgcaggt 180
   ctaattttgg acgctttgcc tgccatttct tccaggttga gggagccgca gaggcggagg 240
   ctcgcgtatt cctgcagtca gcacccacgt cgcccccgga cgctcggtgc tcaggccctt 300
   cgcgagcggg gctctccgtc tgcggtccct tgtgaaggct ctgggcggct gcagaggccg 360
   gccgtccggt ttggctcacc tctcccagga aacttcacac tggagagcca aaaggagtgg 420
   aagagcctgt cttggagatt ttcctgggga aatcctgagg tcattcatta tgaagtgtac 480
   cgcgcgggag tggctcagag taaccacagt gctgttcatg gctagagcaa ttccagccat 540
   ggtggttccc aatgccactt tattggagaa acttttggaa aaatacatgg atgaggatgg 600
   tgagtggtgg atagccaaac aacgagggaa aagggccatc acagacaatg acatgcagag 660
   tattttggac cttcataata aattacgaag tcaggtgtat ccaacagcct ctaatatgga 720
   gtatatgaca tgggatgtag agctggaaag atctgcagaa tccagggctg aaattgcttg 780
   tgggaacatg gacctgcaag cttgcttcca tcaattggac agaatttggg agcacactgg 840
   ggaagatata ggcccccgac gtttcatgta caatcgtggt atgatgaagt gaaagacttt 900
   agctacccat atgaacatga atgcaaccca tattgtccat tcaggtgttc tggccctgta 960
   tgtacacatt atacacaggt cgtgtgggca actagtaaca gaatcggttg tgccattaat 1020
   ttgtgtcata acatgaacat ctgggggcag atatggccca aagctgtcta cctggtgtgc 1080
   aattactccc caaagggaaa ctggtggggc catgcccctt acaaacatgg gcggccctgt 1140
   tctgcttgcc cacctagttt tggagggggc tgtagagaaa atctgtgcta caaagaaggg 1200
   tcagacaggt attatccccc tcgagaagag gaaacaaatg aaatagaacg gcagcagtca 1260
   caagtccatg acacccatgt ccggacaaga tcagatgata gtagcagaaa tgaagtcatt 1320
   agctttggga aaagtaatga aaatataatg gttttagaaa tcctgtgtta aatattgcta 1380
   tattttctta gcagttattt ctacagttaa ttacatagtc atgattgttc tacgtttcat 1440
   atattatatg gtgctttgta tatgccccta ataaaatgaa tctaaacatt gaaaaaaa 1498
<210> 25
   <211> 300
   <212> PRT
   <213> Homo sapiens
<400> 25
   Met Lys Cys Thr Ala Arg Glu Trp Leu Arg Val Thr Thr Val Leu Phe
   1               5                   10                  15
   Met Ala Arg Ala Ile Pro Ala Met Val Val Pro Asn Ala Thr Leu Leu
               20                  25                  30
   Glu Lys Leu Leu Glu Lys Tyr Met Asp Glu Asp Gly Glu Trp Trp Ile
           35                  40                  45
   Ala Lys Gln Arg Gly Lys Arg Ala Ile Thr Asp Asn Asp Met Gln Ser
       50                  55                  60
   Ile Leu Asp Leu His Asn Lys Leu Arg Ser Gln Val Tyr Pro Thr Ala
   65                  70                  75                  80
   Ser Asn Met Glu Tyr Met Thr Trp Asp Val Glu Leu Glu Arg Ser Ala
                   85                  90                  95
   Glu Ser Arg Ala Glu Ser Cys Leu Trp Glu His Gly Pro Ala Ser Leu
               100                 105                 110
   Leu Pro Ser Ile Gly Gln Asn Leu Gly Ala His Trp Gly Arg Tyr Arg
           115                 120                 125
   Pro Pro Thr Phe His Val Gln Ser Trp Tyr Asp Glu Val Lys Asp Phe
       130                 135                 140
   Ser Tyr Pro Tyr Glu His Glu Cys Asn Pro Tyr Cys Pro Phe Arg Cys
   145                 150                 155                 160
   Ser Gly Pro Val Cys Thr His Tyr Thr Gln Val Val Trp Ala Thr Ser
                   165                 170                 175
   Asn Arg Ile Gly Cys Ala Ile Asn Leu Cys His Asn Met Asn Ile Trp
               180                 185                 190
   Gly Gln Ile Trp Pro Lys Ala Val Tyr Leu Val Cys Asn Tyr Ser Pro
           195                 200                 205
   Lys Gly Asn Trp Trp Gly His Ala Pro Tyr Lys His Gly Arg Pro Cys
       210                 215                 220
   Ser Ala Cys Pro Pro Ser Phe Gly Gly Gly Cys Arg Glu Asn Leu Cys
   225                 230                 235                 240
   Tyr Lys Glu Gly Ser Asp Arg Tyr Tyr Pro Pro Arg Glu Glu Glu Thr
   245 250 255
   Asn Glu Ile Glu Arg Gln Gln Ser Gln Val His Asp Thr His Val Arg
   260 265 270
   Thr Arg Ser Asp Asp Ser Ser Arg Asn Glu Val Ile Ser Phe Gly Lys
   275 280 285
   Ser Asn Glu Asn Ile Met Val Leu Glu Ile Leu Cys
   290 295 300
<210> 26
   <211> 22
   <212> DNA
   <213> Artificial Sequence
<220>
   <223> Description of Artificial Sequence:Ag809 Forward Primer
<400> 26
   atgtgatctt tggctgtgaa gt 22
<210> 27
   <211> 23
   <212> DNA
   <213> Artificial Sequence
<220>
   <223> Description of Artificial Sequence:Ag809 Probe Primer
<400> 27
   ctaccccatg gcctccatcg agt 23
<210> 28
   <211> 19
   <212> DNA
   <213> Artificial Sequence
<220>
   <223> Description of Artificial Sequence:Ag809 Reverse Primer
<400> 28
   ggatgtccaa gccatcctt 19
<210> 29
   <211> 22
   <212> DNA
   <213> Artificial Sequence
<220>
   <223> Description of Artificial Sequence:Ag2773 Forward Primer
<400> 29
   ccttgctttg tcatatgctg tt 22
<210> 30
   <211> 26
   <212> DNA
   <213> Artificial Sequence
<220>
   <223> Description of Artificial Sequence:Ag2773 Probe Primer
<400> 30
   ccctttgcct ggaatataaa ctctca 26
<210> 31
   <211> 22
   <212> DNA
   <213> Artificial Sequence
<220>
   <223> Description of Artificial Sequence:Ag2773 Reverse Primer
<400> 31
   agaggaagct ttctggagaa ga 22
<210> 32
   <211> 21
   <212> DNA
   <213> Artificial Sequence
<220>
   <223> Description of Artificial Sequence:Ag427 Forward Primer
<400> 32
   gagctacagg cagcctcqag t 21
<210> 33
   <211> 21
   <212> DNA
   <213> Artificial Sequence
<220>
   <223> Description of Artificial Sequence:Ag427 Probe Primer
<400> 33
   tggcccagct gaccctgctc a 21
<210> 34
   <211> 20
   <212> DNA
   <213> Artificial Sequence
<220>
   <223> Description of Artificial Sequence:Ag427 Reverse Primer
<400> 34
   ggctacgtca gtgggtttgg 20
<210> 35
   <211> 22
   <212> DNA
   <213> Artificial Sequence
<220>
   <223> Description of Artificial Sequence:Agl541 Forward Primer
<400> 35
   agaagaacac cccagggata ta 22
<210> 36
   <211> 26
   <212> DNA
   <213> Artificial Sequence
<220>
   <223> Description of Artificial Sequence:Ag1541 Probe Primer
<400> 36
   cctcgttggt gaactacaac ctctgg 26
<210> 37
   <211> 22
   <212> DNA
   <213> Artificial Sequence
<220>
   <223> Description of Artificial Sequence:Ag1541 Reverse Primer
<400> 37
   cctctagctg ggtcactttc tc 22
<210> 38
   <211> 270
   <212> PRT
   <213> Mus musculus
<400> 38
   Met Pro Arg Leu Pro Leu Leu Leu Leu Leu Leu Pro Ser Leu Ala Arg
   1               5                   10                  15
   Gly Leu Gly Leu Arg Asp Ala Gly Arg Arg His Pro Glu Cys Ser Pro
               20                  25                  30
   Cys Gln Gln Asp Arg Cys Pro Ala Pro Ser Pro Cys Pro Ala Pro Trp
           35                  40                  45
   Ile Ser Ala Arg Asp Glu Cys Gly Cys Cys Ala Arg Cys Leu Gly Ala
       50                  55                  60
   Glu Gly Ala Ser Cys Gly Gly Pro Val Gly Ser Arg Cys Gly Pro Gly
   65                  70                  75                  80
   Leu Val Cys Ala Ser Arg Ala Ser Gly Thr Ala Pro Glu Gly Thr Gly
                   85                  90                  95
   Leu Cys Val Cys Ala Gln Arg Gly Ala Val Cys Gly Ser Asp Gly Arg
   100 105 110
   Ser Tyr Ser Ser Ile Cys Ala Leu Arg Leu Arg Ala Arg His Ala Pro
   115 120 125
   Arg Ala His His Gly His Leu His Lys Ala Arg Asp Gly Pro Cys Glu
   130 135 140
   Phe Ala Pro Val Val Leu Met Pro Pro Arg Asp Ile His Asn Val Thr
   145 150 155 160
   Gly Thr Gln Val Phe Leu Ser Cys Glu Val Lys Ala Val Pro Thr Pro
   165 170 175
   Val Ile Thr Trp Lys Lys Val Lys His Ser Pro Glu Gly Thr Glu Gly
   180 185 190
   Leu Glu Glu Leu Pro Gly Asp His Val Asn Ile Ala Val Gln Val Arg
   195 200 205
   Gly Gly Pro Ser Asp His Glu Thr Thr Ser Trp Ile Leu Ile Asn Pro
   210 215 220
   Leu Arg Lys Glu Asp Glu Gly Val Tyr His Cys His Ala Ala Asn Ala
   225 230 235 240
   Ile Gly Glu Ala Gln Ser His Gly Thr Val Thr Val Leu Asp Leu Asn
   245 250 255
   Arg Tyr Lys Ser Leu Tyr Ser Ser Val Pro Gly Asp Leu Leu
   260 265 270
<210> 39
   <211> 281
   <212> PRT
   <213> Mus musculus
<400> 39
   Met Glu Arg Pro Pro Arg Ala Leu Leu Leu Gly Ala Ala Gly Leu Leu
   1 5 10 15
   Leu Leu Leu Leu Pro Leu Ser Ser Ser Ser Ser Ser Asp Ala Cys Gly
   20 25 30
   Pro Cys Val Pro Ala Ser Cys Pro Ala Leu Pro Arg Leu Gly Cys Pro
   35 40 45
   Leu Gly Glu Thr Arg Asp Ala Cys Gly Cys Cys Pro Val Cys Ala Arg
   50 55 60
   Gly Glu Gly Glu Pro Cys Gly Gly Gly Ala Ala Gly Arg Gly His Cys
   65 70 75 80
   Ala Pro Gly Met Glu Cys Val Lys Ser Arg Lys Arg Arg Arg Gly Lys
   85 90 95
   Ala Gly Ala Ala Ala Gly Gly Pro Ala Thr Leu Ala Val Cys Val Cys
   100 105 110
   Lys Ser Arg Tyr Pro Val Cys Gly Ser Asn Gly Ile Thr Tyr Pro Ser
   115 120 125
   Gly Cys Gln Leu Arg Ala Ala Ser Leu Arg Ala Glu Ser Arg Gly Glu
   130 135 140
   Lys Pro Ile Thr Gln Val Ser Lys Gly Thr Cys Glu Gln Gly Pro Ser
   145 150 155 160
   Ile Val Thr Pro Pro Lys Asp Ile Trp Asn Val Thr Gly Ala Lys Val
   165 170 175
   Phe Leu Ser Cys Glu Val Ile Gly Ile Pro Thr Pro Val Leu Ile Trp
   180 185 190
   Asn Lys Val Lys Arg Asp His Ser Gly Val Gln Arg Thr Glu Leu Leu
   195 200 205
   Pro Gly Asp Arg Glu Asn Leu Ala Ile Gln Thr Arg Gly Gly Pro Glu
   210 215 220
   Lys His Glu Val Thr Gly Trp Val Leu Val Ser Pro Leu Ser Lys Glu
   225 230 235 240
   Asp Ala Gly Glu Tyr Glu Cys His Ala Ser Asn Ser Gln Gly Gln Ala
   245 250 255
   Ser Ala Ala Ala Lys Ile Thr Val Val Asp Ala Leu His Glu Ile Pro
   260 265 270
   Leu Lys Lys Gly Glu Gly Ala Gln Leu
   275 280
<210> 40
   <211> 277
   <212> PRT
   <213> Homo sapiens
<400> 40
   Met Glu Arg Ala Ser Leu Arg Ala Leu Leu Phe Gly Pro Ala Gly Leu
   1 5 10 15
   Leu Leu Leu Leu Leu Pro Leu Ser Ser Ser Ser Ser Ser Asp Thr Cys
   20 25 30
   Gly Pro Cys Glu Pro Ala Ser Cys Pro Pro Leu Pro Pro Leu Gly Cys
   35 40 45
   Leu Leu Gly Glu Thr Arg Asp Ala Cys Gly Cys Cys Pro Met Cys Ala
   50 55 60
   Arg Gly Glu Gly Glu Pro Cys Gly Gly Gly Gly Ala Gly Arg Gly Tyr
   65 70 75 80
   Cys Ala Pro Gly Met Glu Cys Val Lys Ser Arg Lys Arg Arg Arg Gly
   85 90 95
   Lys Ala Gly Ala Ala Ala Gly Gly Pro Gly Val Ser Gly Val Cys Val
   100 105 110
   Cys Lys Ser Arg Val Pro Val Cys Gly Ser Asp Gly Thr Thr Tyr Pro
   115 120 125
   Ser Gly Cys Gln Leu Arg Ala Ala Ser Gln Arg Ala Glu Ser Arg Gly
   130 135 140
   Glu Lys Ala Ile Thr Gln Val Ser Lys Gly Thr Cys Glu Gln Gly Pro
   145 150 155 160
   Ser Ile Val Thr Pro Pro Lys Asp Ile Trp Asn Val Thr Gly Ala Gln
   165 170 175
   Val Tyr Leu Ser Cys Glu Val Ile Gly Ile Pro Thr Pro Val Leu Ile
   180 185 190
   Trp Asn Lys Val Lys Arg Gly His Tyr Gly Val Gln Arg Thr Glu Leu
   195 200 205
   Leu Pro Gly Asp Arg Asp Asn Leu Ala Ile Gln Thr Arg Gly Gly Pro
   210 215 220
   Glu Lys His Glu Val Thr Gly Trp Val Leu Val Ser Pro Leu Ser Lys
   225 230 235 240
   Glu Asp Ala Gly Glu Tyr Glu Cys His Ala Ser Asn Ser Gln Gly Gln
   245 250 255
   Ala Ser Ala Ser Ala Lys Ile Thr Val Val Asp Ala Leu His Glu Ile
   260 265 270
   Ala Ser Glu Lys Arg
   275
<210> 41
   <211> 281
   <212> PRT
   <213> Mus musculus
<400> 41
   Met Glu Arg Pro Pro Arg Ala Leu Leu Leu Gly Ala Ala Gly Leu Leu
   1 5 10 15
   Leu Leu Leu Leu Pro Leu Ser Ser Ser Ser Ser Ser Asp Ala Cys Gly
   20 25 30
   Pro Cys Val Pro Ala Ser Cys Pro Ala Leu Pro Arg Leu Gly Cys Pro
   35 40 45
   Leu Gly Glu Thr Arg Asp Ala Cys Gly Cys Cys Pro Val Cys Ala Arg
   50 55 60
   Gly Glu Gly Glu Pro Cys Gly Gly Gly Ala Ala Gly Arg Gly His Cys
   65 70 75 80
   Ala Pro Gly Met Glu Cys Val Lys Ser Arg Lys Arg Arg Lys Gly Lys
   85 90 95
   Ala Gly Ala Ala Ala Gly Gly Pro Ala Thr Leu Ala Val Cys Val Cys
   100 105 110
   Lys Ser Arg Tyr Pro Val Cys Gly Ser Asn Gly Ile Thr Tyr Pro Ser
   115 120 125
   Gly Cys Gln Leu Arg Ala Ala Ser Leu Arg Ala Glu Ser Arg Gly Glu
   130 135 140
   Lys Ala Ile Thr Gln Val Ser Lys Gly Thr Cys Glu Gln Gly Pro Ser
   145 150 155 160
   Ile Val Thr Pro Pro Lys Asp Ile Trp Asn Val Thr Gly Ala Lys Val
   165 170 175
   Phe Leu Ser Cys Glu Val Ile Gly Ile Pro Thr Pro Val Leu Ile Trp
   180 185 190
   Asn Lys Val Lys Arg Asp His Ser Gly Val Gln Arg Thr Glu Leu Leu
   195 200 205
   Pro Gly Asp Arg Glu Asn Leu Ala Ile Gln Thr Arg Gly Gly Pro Glu
   210 215 220
   Lys His Glu Val Thr Gly Trp Val Leu Val Ser Pro Leu Ser Lys Glu
   225 230 235 240
   Asp Ala Gly Glu Tyr Glu Cys His Ala Ser Asn Ser Gln Gly Gln Ala
   245 250 255
   Ser Ala Ala Ala Lys Ile Thr Val Val Asp Ala Leu His Glu Ile Pro
   260 265 270
   Leu Lys Lys Gly Glu Gly Ala Gln Leu
   275 280
<210> 42
   <211> 282
   <212> PRT
   <213> Homo sapiens
<400> 42
   Met Glu Arg Pro Ser Leu Arg Ala Leu Leu Leu Gly Ala Ala Gly Leu
   1 5 10 15
   Leu Leu Leu Leu Leu Pro Leu Ser Ser Ser Ser Ser Ser Asp Thr Cys
   20 25 30
   Gly Pro Cys Glu Pro Ala Ser Cys Pro Pro Leu Pro Pro Leu Gly Cys
   35 40 45
   Leu Leu Gly Glu Thr Arg Asp Ala Cys Gly Cys Cys Pro Met Cys Ala
   50 55 60
   Arg Gly Glu Gly Glu Pro Cys Gly Gly Gly Gly Ala Gly Arg Gly Tyr
   65 70 75 80
   Cys Ala Pro Gly Met Glu Cys Val Lys Ser Arg Lys Arg Arg Lys Gly
   85 90 95
   Lys Ala Gly Ala Ala Ala Gly Gly Pro Gly Val Ser Gly Val Cys Val
   100 105 110
   Cys Lys Ser Arg Tyr Pro Val Cys Gly Ser Asp Gly Thr Thr Tyr Pro
   115 120 125
   Ser Gly Cys Gln Leu Arg Ala Ala Ser Gln Arg Ala Glu Ser Arg Gly
   130 135 140
   Glu Lys Ala Ile Thr Gln Val Ser Lys Gly Thr Cys Glu Gln Gly Pro
   145 150 155 160
   Ser Ile Val Thr Pro Pro Lys Asp Ile Trp Asn Val Thr Gly Ala Gln
   165 170 175
   Val Tyr Leu Ser Cys Glu Val Ile Gly Ile Pro Thr Pro Val Leu Ile
   180 185 190
   Trp Asn Lys Val Lys Arg Gly His Tyr Gly Val Gln Arg Thr Glu Leu
   195 200 205
   Leu Pro Gly Asp Arg Asp Asn Leu Ala Ile Gln Thr Arg Gly Gly Pro
   210 215 220
   Glu Lys His Glu Val Thr Gly Trp Val Leu Val Ser Pro Leu Ser Lys
   225 230 235 240
   Glu Asp Ala Gly Glu Tyr Glu Cys His Ala Ser Asn Ser Gln Gly Gln
   245 250 255
   Ala Ser Ala Ser Ala Lys Ile Thr Val Val Asp Ala Leu His Glu Ile
   260 265 270
   Pro Val Lys Lys Gly Glu Gly Ala Glu Leu
   275 280
<210> 43
   <211> 144
   <212> PRT
   <213> Rattus norvegicus
<400> 43
   Pro Leu Arg Phe Leu Ser Gln Thr Glu Ser Ile Thr Ala Phe Met Gly
   1 5 10 15
   Asp Thr Val Leu Leu Lys Cys Glu Val Ile Gly Asp Pro Met Pro Thr
   20 25 30
   Ile His Trp Gln Lys Asn Gln Gln Asp Leu Thr Pro Asn Pro Gly Asp
   35 40 45
   Ser Arg Val Val Val Pro Pro Trp Phe Leu Asn His Pro Ser Asn Leu
   50 55 60
   Tyr Ala Tyr Glu Ser Met Asp Ile Glu Phe Glu Cys Ala Val Ser Gly
   65 70 75 80
   Lys Pro Val Pro Thr Val Asn Trp Met Lys Asn Gly Asp Val Val Val
   85 90 95
   Ile Ser Asp Tyr Phe Gln Ile Val Gly Gly Ser Asn Leu Arg Ile Leu
   100 105 110
   Gly Val Val Lys Ser Asp Glu Gly Phe Tyr Gln Cys Val Ala Glu Asn
   115 120 125
   Glu Ala Gly Asn Ala Gln Ser Ser Ala Gln Leu Ile Val Pro Lys Pro
   130 135 140
<210> 44
   <211> 1502
   <212> PRT
   <213> Homo sapiens
<400> 44
<210> 46
   <211> 4719
   <212> DNA
   <213> Homo sapiens
<400> 46
   agaatgtcct tctggcactc cagacccgtc tgcagccact ccaagaagga gacagcagac 60
   aagaccctgc ctcccagaag cgcctcctgg tggaatctct gttcagggac ttagatgcag 120
   atggcaatgg ccacctcagc agctccgaac tggctcagca tgtgctgaag aagcaggacc 180
   tggatgaaga cttacttggt tgctcaccag gtgacctcct ccgatttgac gattacaaca 240
   gtgacagctc cctgaccctc cgcgagttct acatggcctt ccaagtggtt cagctcagcc 300
   tcgcccccga ggacagggtc agtgtgacca cagtgaccgt ggggctgagc acagtgctga 360
   cctgcgccgt ccatggagac ctgaggccac caatcatctg gaagcgcaac gggctcaccc 420
   tgaacttcct ggacttggaa gacatcaatg actttggaga ggatgattcc ctgtacatca 480
   ccaaggtgac caccatccac atgggcaatt acacctgcca tgcttccggc cacgagcagc 540
   tgttccagac ccacgtcctg caggtgaatg tgccgccagt catccgtgtc tatccagaga 600
   gccaggcaca ggagcctgga gtggcagcca gcctaagatg ccatgctgag ggcattccca 660
   tgcccagaat cacttggctg aaaaacggcg tggatgtctc aactcagatg tccaaacagc 720
   tctccctttt agccaatggg agcgaactcc acatcagcag tgttcggtat gaagacacag 780
   gggcatacac ctgcattgcc aaaaatgaag tgggtgtgga tgaagatatc tcctcgctct 840
   tcattgaaga ctcagctaga aagacccttg caaacatcct gtggcgagag gaaggcctca 900
   gcgtgggaaa catgttctat gtcttctccg acgacggtat catcgtcatc catcctgtgg 960
   actgtgagat ccagaggcac ctcaaaccca cggaaaagat tttcatgagc tatgaagaaa 1020
   tctgtcctca aagagaaaaa aatgcaaccc agccctgcca gtgggtatct gcagtcaatg 1080
   tccggaaccg gtacatctat gtggcccagc cagcactgag cagagtcctt gtggtcgaca 1140
   tccaagccca gaaagtccta cagtccatag gtgtggaccc tctgccggct aagctgtcct 1200
   atgacaagtc acatgaccaa gtgtgggtcc tgagctgggg ggacgtgcac aagtcccgac 1260
   caagtctcca ggtgatcaca gaagccagca ccggccagag ccagcacctc atccgcacac 1320
   cctttgcagg agtggatgat ttcttcattc ccccaacaaa cctcatcatc aaccacatca 1380
   ggtttggctt catcttcaac aagtctgatc ctgcagtcca caaggtggac ctggaaacaa 1440
   tgatgcccct caagaccatc ggcctgcacc accatggctg cgtgccccag gccatggcac 1500
   acacccacct gggcggctac ttcttcatcc agtgccgaca ggacagcccc gcctctgctg 1560
   cccgacagct gctcgttgac agtgtcacag actctgtgct tggccccaat ggtgatgtaa 1620
   caggcacccc acacacatcc cccgacgggc gcttcatagt cagtgctgca gctgacagcc 1680
   cctggctgca cgtgcaggag atcacagtgc ggggcgagat ccagaccctg tatgacctgc 1740
   aaataaactc gggcatctca gacttggcct tccagcgctc cttcactgaa agcaatcaat 1800
   acaacatcta cgcggctctg cacacggagc cggacctgct gttcctggag ctgtccacgg 1860
   ggaaggtggg catgctgaag aacttaaagg agccacccgc agggccagct cagccctggg 1920
   ggggtaccca cagaatcatg agggacagtg ggctgtttgg acagtacctc ctcacaccag 1980
   cccgagagtc actgttcctc atcaatggga gacaaaacac gctgcggtgt gaggtgtcag 2040
   gtataaaggg ggggaccaca gtggtgtggg tgggtgaggt atgaagggcc cagagcagag 2100
   ccctgggcca aggaacaccc cctagtcctg acactgcagc ctcaagcagg tacgctgtac 2160
   atttttacag acaaaagcaa aaacctgtac tcgctttgtg gttcaacact ggtctccttg 2220
   caagtttcct agtataaggt atgcgctgct accaagattg gggttttttc gttaggaagt 2280
   atgatttatg ccttgagcta cgatgagaac atatgctgct gtgtaaaggg atcatttctg 2340
   tgccaagctg cacaccgagt gacctgggga catcatggaa ccaagggatc ctgctctcca 2400
   agcagacacc tctgtcagtt gccttcacat agtcattgtc ccttactgcc agacccagcc 2460
   agactttgcc ctgacggagt ggcccggaag cagaggccga ccaggagcag gggcctccct 2520
   cccgaactga aagcccatcc gtcctcgcgt gggaccgcat cttctccctc gcagctgctt 2580
   cttgcttttc tttccatttg acttgctgta agcctgaggg agagccaaca agacttactg 2640
   catcttgggg gatggggaaa tcactcactt tattttggaa atttttgatt aaaaaaaaat 2700
   tttataatct caaatgctag taagcagaaa gatgctctcc gaggtccaac tatatccttc 2760
   cctgccttag gccgagtctc gggggtggtc acaaccccac atcccacagc cagaaagaac 2820
   aatggtcatc tgagaatact ggccctgtcg actattgcca ccctgcttct ccaagagcag 2880
   accaggccac ctcatccgta aggactcggt tctgtgttgg gaccccaaaa aaccagaaca 2940
   agttctgtgt gcctcctttc agcacagaag ggagacatct cattagtcag gtctggtacc 3000
   ccagattcag ggcagactgg gcttgcctgg caaggtatgg gtggcctcca ggctcaatgc 3060
   agaaacccca aggacacgag tggggccagg tgagttcctg aagctatacc ttttcaaaac 3120
   agattttgtt ttcctacctg tggcccatcc actcctctct ggtaccccat ccccgcatca 3180
   gcactgcaga gagaacacat ttcggcgagg gtttcttacc ccacattccc caatcaatac 3240
   acacacactg cagaacccag aacagaaggc cacaggctgg cactactgca ttctccttat 3300
   gtgtctcagg ctgtggtgac tctcacatgg gcatcgaaga agtacaaccc acatagccct 3360
   ctggagaccg cctagatcag agactcagca aaaacaggct cgccttccct ctcccacata 3420
   tgagtggaac ttacatgtgt cctggtttga atgatcattt tgcaagccac acgggttggg 3480
   agaggtggtc tcaccacaga cgtctttgct aatttggcca ccttcaccta ctgacatgac 3540
   caggattttc ctttgccatt aaggaatgaa ctctttcaag gagaggaaac cctagactct 3600
   gtgtcactct caacacacac agctcctttc actcctgcct gactgccaag ccacctgcat 3660
   cccccgcccc agatctcatg agatcaatca cttgtatgtc tcacgcaact tggtccacca 3720
   aacgcctgtc ccctgtaact cctaggggtg cgcctagaca ggtacgtctg ttttttattt 3780
   taaaagatat gctatgtaga tataagttga ggaagctcac ctcaaaagcc tagaatgcag 3840
   tttcacagta gctgggatgc atggatgacc catctcaccc cttttttttt cctgcctcaa 3900
   tatcttgata tgttatgttt actcccaatc tcccattttt accactaaaa ttctccaact 3960
   ttcataaact tttttttgga aaaatttcca ttgtatcagc ccctgacaga aaaaggatct 4020
   ctgagcctaa aggaggaaaa gtcccaccaa ctaccagacc agaacacgag cccctctggg 4080
   cagcaggatt cctaagtcaa agaccagttt gacccaaact ggccttttaa aataatcagg 4140
   agtgacagag tcaacttctg cagcacctgc ttctccccca ctgtcccttc catcttggaa 4200
   tgtgtctaaa aaagcatagc tgccctttgc tgtcctcaga gtgcatttcc tggagacggc 4260
   aggcttaggt ctcactgaca gcatgccaga cacaactgaa tcgaagcagg cctgaagcct 4320
   aggtcagggt ttcaggagtc cagccccagg aggcaaagtc accaatgcag ggaggtaaat 4380
   gccttttggc aggaaaacca atagagttgg ttgggtgggg agtcaggggt gggaggagaa 4440
   ggaggaagag gaggaaggcc agactggcct gccctttctc ccatacttca ccccagcaga 4500
   ggttcatggg acacagttgg aaagccactg ggaggaaatg cctcactaca ggggggcctc 4560
   ctgtagcaag cccagccggt aatcctccta atgaacccac aaggtcaatt cacaactgat 4620
   atcttagcta ttaaagaagt actgacttta ccaaaagaat catcaagaaa gctatttata 4680
   taaaccccct cagtcatttt gaaataaaat taattttac                        4719
<210> 47
   <211> 850
   <212> PRT
   <213> Homo sapiens
<400> 47
   Lys Ala Ile Arg Met Phe Lys Cys Trp Ser Val Val Leu Val Leu Gly
   1               5                   10                  15
   Phe Ile Phe Leu Glu Ser Glu Gly Arg Pro Thr Lys Glu Gly Gly Tyr
               20                  25                  30
   Gly Leu Lys Ser Tyr Gln Pro Leu Met Arg Leu Arg His Lys Glu Lys
           35                  40                  45
   Asn Gln Glu Ser Ser Arg Val Lys Gly Phe Met Ile Gln Asp Gly Pro
       50                  55                  60
   Phe Gly Ser Cys Glu Asn Lys Tyr Cys Gly Leu Gly Arg His Cys Val
   65                  70                  75                  80
   Thr Ser Arg Glu Thr Gly Gln Ala Glu Cys Ala Cys Met Asp Leu Cys
                   85                  90                  95
   Lys Arg His Tyr Lys Pro Val Cys Gly Ser Asp Gly Glu Phe Tyr Glu
               100                 105                 110
   Asn His Cys Glu Val His Arg Ala Ala Cys Leu Lys Lys Gln Lys Ile
           115                 120                 125
   Thr Ile Val His Asn Glu Asp Cys Phe Phe Lys Gly Asp Lys Cys Lys
       130                 135                 140
   Thr Thr Glu Tyr Ser Lys Met Lys Asn Met Leu Leu Asp Leu Gln Asn
   145                 150                 155                 160
   Gln Lys Tyr Ile Met Gln Glu Asn Glu Asn Pro Asn Gly Asp Asp Ile
                   165                 170                 175
   Ser Arg Lys Lys Leu Leu Val Asp Gln Met Phe Lys Tyr Phe Asp Ala
   180 185 190
   Asp Ser Asn Gly Leu Val Asp Ile Asn Glu Leu Thr Gln Val Ile Lys
   195 200 205
   Gln Glu Glu Leu Gly Lys Asp Leu Phe Asp Cys Thr Leu Tyr Val Leu
   210 215 220
   Leu Lys Tyr Asp Asp Phe Asn Ala Asp Lys His Leu Ala Leu Glu Glu
   225 230 235 240
   Phe Tyr Arg Ala Phe Gln Val Ile Gln Leu Ser Leu Pro Glu Asp Gln
   245 250 255
   Lys Leu Ser Ile Thr Ala Ala Thr Val Gly Gln Ser Ala Val Leu Ser
   260 265 270
   Cys Ala Ile Gln Gly Thr Leu Arg Pro Pro Ile Ile Trp Lys Arg Asn
   275 280 285
   Asn Ile Ile Leu Asn Asn Leu Asp Leu Glu Asp Ile Asn Asp Phe Gly
   290 295 300
   Asp Asp Gly Ser Leu Tyr Ile Thr Lys Val Thr Thr Thr His Val Gly
   305 310 315 320
   Asn Tyr Thr Cys Tyr Ala Asp Gly Tyr Glu Gln Val Tyr Gln Thr His
   325 330 335
   Ile Phe Gln Val Asn Val Pro Pro Val Ile Arg Val Tyr Pro Glu Ser
   340 345 350
   Gln Ala Arg Glu Pro Gly Val Thr Ala Ser Leu Arg Cys His Ala Glu
   355 360 365
   Gly Ile Pro Lys Pro Gln Leu Gly Trp Leu Lys Asn Gly Ile Asp Ile
   370 375 380
   Thr Pro Lys Leu Ser Lys Gln Leu Thr Leu Gln Ala Asn Gly Ser Glu
   385 390 395 400
   Val His Ile Ser Asn Val Arg Tyr Glu Asp Thr Gly Ala Tyr Thr Cys
   405 410 415
   Ile Ala Lys Asn Glu Ala Gly Val Asp Glu Asp Ile Ser Ser Leu Phe
   420 425 430
   Val Glu Asp Ser Ala Arg Lys Thr Leu Ala Asn Ile Leu Trp Arg Glu
   435 440 445
   Glu Gly Leu Gly Ile Gly Asn Met Phe Tyr Val Phe Tyr Glu Asp Gly
   450 455 460
   Ile Lys Val Ile Gln Pro Ile Glu Cys Glu Phe Gln Arg His Ile Lys
   465 470 475 480
   Pro Ser Glu Lys Leu Leu Gly Phe Gln Asp Glu Val Cys Pro Lys Ala
   485 490 495
   Glu Gly Asp Glu Val Gln Arg Cys Val Trp Ala Ser Ala Val Asn Val
   500 505 510
   Lys Asp Lys Phe Ile Tyr Val Ala Gln Pro Thr Leu Asp Arg Val Leu
   515 520 525
   Ile Val Asp Val Gln Ser Gln Lys Val Val Gln Ala Val Ser Thr Asp
   530 535 540
   Pro Val Pro Val Lys Leu His Tyr Asp Lys Ser His Asp Gln Val Trp
   545 550 555 560
   Val Leu Ser Trp Gly Thr Leu Glu Lys Thr Ser Pro Thr Leu Gln Val
   565 570 575
   Ile Thr Leu Ala Ser Gly Asn Val Pro His His Thr Ile His Thr Gln
   580 585 590
   Pro Val Gly Lys Gln Phe Asp Arg Val Asp Asp Phe Phe Ile Pro Thr
   595 600 605
   Thr Thr Leu Ile Ile Thr His Met Arg Phe Gly Phe Ile Leu His Lys
   610 615 620
   Asp Glu Ala Ala Leu Gln Lys Ile Asp Leu Glu Thr Met Ser Tyr Ile
   625 630 635 640
   Lys Thr Ile Asn Leu Lys Asp Tyr Lys Cys Val Pro Gln Ser Leu Ala
   645 650 655
   Tyr Thr His Leu Gly Gly Tyr Tyr Phe Ile Gly Cys Lys Pro Asp Ser
   660 665 670
   Thr Gly Ala Val Ser Pro Gln Val Met Val Asp Gly Val Thr Asp Ser
   675 680 685
   Val Ile Gly Phe Asn Ser Asp Val Thr Gly Thr Pro Tyr Val Ser Pro
   690 695 700
   Asp Gly His Tyr Leu Val Ser Ile Asn Asp Val Lys Gly Leu Val Arg
   705 710 715 720
   Val Gln Tyr Ile Thr Ile Arg Gly Glu Ile Gln Glu Ala Phe Asp Ile
   725 730 735
   Tyr Thr Asn Leu His Ile Ser Asp Leu Ala Phe Gln Pro Ser Phe Thr
   740 745 750
   Glu Ala His Gln Tyr Asn Ile Tyr Gly Ser Ser Ser Thr Gln Thr Asp
   755 760 765
   Val Leu Phe Val Glu Leu Ser Ser Gly Lys Val Lys Met Ile Lys Ser
   770 775 780
   Leu Lys Glu Pro Leu Lys Ala Glu Glu Trp Pro Trp Asn Arg Lys Asn
   785 790 795 800
   Arg Gln Ile Gln Asp Ser Gly Leu Phe Gly Gln Tyr Leu Met Thr Pro
   805 810 815
   Ser Lys Asp Ser Leu Phe Ile Leu Asp Gly Arg Leu Asn Lys Leu Asn
   820 825 830
   Cys Glu Ile Thr Glu Val Glu Lys Gly Asn Thr Val Ile Trp Val Gly
   835 840 845
   Asp Ala
   850
<210> 48
   <211> 693
   <212> PRT
   <213> Homo sapiens
<400> 48
   Asn Val Leu Leu Ala Leu Gln Thr Arg Leu Gln Pro Leu Gln Glu Gly
   1 5 10 15
   Asp Ser Arg Gln Asp Pro Ala Ser Gln Lys Arg Leu Leu Val Glu Ser
   20 25 30
   Leu Phe Arg Asp Leu Asp Ala Asp Gly Asn Gly His Leu Ser Ser Ser
   35 40 45
   Glu Leu Ala Gln His Val Leu Lys Lys Gln Asp Leu Asp Glu Asp Leu
   50 55 60
   Leu Gly Cys Ser Pro Gly Asp Leu Leu Arg Phe Asp Asp Tyr Asn Ser
   65 70 75 80
   Asp Ser Ser Leu Thr Leu Arg Glu Phe Tyr Met Ala Phe Gln Val Val
   85 90 95
   Gln Leu Ser Leu Ala Pro Glu Asp Arg Val Ser Val Thr Thr Val Thr
   100 105 110
   Val Gly Leu Ser Thr Val Leu Thr Cys Ala Val His Gly Asp Leu Arg
   115 120 125
   Pro Pro Ile Ile Trp Lys Arg Asn Gly Leu Thr Leu Asn Phe Leu Asp
   130 135 140
   Leu Glu Asp Ile Asn Asp Phe Gly Glu Asp Asp Ser Leu Tyr Ile Thr
   145 150 155 160
   Lys Val Thr Thr Ile His Met Gly Asn Tyr Thr Cys His Ala Ser Gly
   165 170 175
   His Glu Gln Leu Phe Gln Thr His Val Leu Gln Val Asn Val Pro Pro
   180 185 190
   Val Ile Arg Val Tyr Pro Glu Ser Gln Ala Gln Glu Pro Gly Val Ala
   195 200 205
   Ala Ser Leu Arg Cys His Ala Glu Gly Ile Pro Met Pro Arg Ile Thr
   210 215 220
   Trp Leu Lys Asn Gly Val Asp Val Ser Thr Gln Met Ser Lys Gln Leu
   225 230 235 240
   Ser Leu Leu Ala Asn Gly Ser Glu Leu His Ile Ser Ser Val Arg Tyr
   245 250 255
   Glu Asp Thr Gly Ala Tyr Thr Cys Ile Ala Lys Asn Glu Val Gly Val
   260 265 270
   Asp Glu Asp Ile Ser Ser Leu Phe Ile Glu Asp Ser Ala Arg Lys Thr
   275 280 285
   Leu Ala Asn Ile Leu Trp Arg Glu Glu Gly Leu Ser Val Gly Asn Met
   290 295 300
   Phe Tyr Val Phe Ser Asp Asp Gly Ile Ile Val Ile His Pro Val Asp
   305 310 315 320
   Cys Glu Ile Gln Arg His Leu Lys Pro Thr Glu Lys Ile Phe Met Ser
   325 330 335
   Tyr Glu Glu Ile Cys Pro Gln Arg Glu Lys Asn Ala Thr Gln Pro Cys
   340 345 350
   Gln Trp Val Ser Ala Val Asn Val Arg Asn Arg Tyr Ile Tyr Val Ala
   355 360 365
   Gln Pro Ala Leu Ser Arg Val Leu Val Val Asp Ile Gln Ala Gln Lys
   370 375 380
   Val Leu Gln Ser Ile Gly Val Asp Pro Leu Pro Ala Lys Leu Ser Tyr
   385 390 395 400
   Asp Lys Ser His Asp Gln Val Trp Val Leu Ser Trp Gly Asp Val His
   405 410 415
   Lys Ser Arg Pro Ser Leu Gln Val Ile Thr Glu Ala Ser Thr Gly Gln
   420 425 430
   Ser Gln His Leu Ile Arg Thr Pro Phe Ala Gly Val Asp Asp Phe Phe
   435 440 445
   Ile Pro Pro Thr Asn Leu Ile Ile Asn His Ile Arg Phe Gly Phe Ile
   450 455 460
   Phe Asn Lys Ser Asp Pro Ala Val His Lys Val Asp Leu Glu Thr Met
   465 470 475 480
   Met Pro Leu Lys Thr Ile Gly Leu His His His Gly Cys Val Pro Gln
   485 490 495
   Ala Met Ala His Thr His Leu Gly Gly Tyr Phe Phe Ile Gln Cys Arg
   500 505 510
   Gln Asp Ser Pro Ala Ser Ala Ala Arg Gln Leu Leu Val Asp Ser Val
   515 520 525
   Thr Asp Ser Val Leu Gly Pro Asn Gly Asp Val Thr Gly Thr Pro His
   530 535 540
   Thr Ser Pro Asp Gly Arg Phe Ile Val Ser Ala Ala Ala Asp Ser Pro
   545 550 555 560
   Trp Leu His Val Gln Glu Ile Thr Val Arg Gly Glu Ile Gln Thr Leu
   565 570 575
   Tyr Asp Leu Gln Ile Asn Ser Gly Ile Ser Asp Leu Ala Phe Gln Arg
   580 585 590
   Ser Phe Thr Glu Ser Asn Gln Tyr Asn Ile Tyr Ala Ala Leu His Thr
   595 600 605
   Glu Pro Asp Leu Leu Phe Leu Glu Leu Ser Thr Gly Lys Val Gly Met
   610 615 620
   Leu Lys Asn Leu Lys Glu Pro Pro Ala Gly Pro Ala Gln Pro Trp Gly
   625 630 635 640
   Gly Thr His Arg Ile Met Arg Asp Ser Gly Leu Phe Gly Gln Tyr Leu
   645 650 655
   Leu Thr Pro Ala Arg Glu Ser Leu Phe Leu Ile Asn Gly Arg Gln Asn
   660 665 670
   Thr Leu Arg Cys Glu Val Ser Gly Ile Lys Gly Gly Thr Thr Val Val
   675 680 685
   Trp Val Gly Glu Val
   690
<210> 49
   <211> 773
   <212> PRT
   <213> Homo sapiens
<400> 49
   His Cys Val Thr Ser Arg Glu Thr Gly Gln Ala Glu Cys Ala Cys Met
   1 5 10 15
   Asp Leu Cys Lys Arg His Tyr Lys Pro Val Cys Gly Ser Asp Gly Glu
   20 25 30
   Phe Tyr Glu Asn His Cys Glu Val His Arg Ala Ala Cys Leu Lys Lys
   35 40 45
   Gln Lys Ile Thr Ile Val His Asn Glu Asp Cys Phe Phe Lys Gly Asp
   50 55 60
   Lys Cys Lys Thr Thr Glu Cys Ser Lys Met Lys Asn Met Leu Leu Asp
   65 70 75 80
   Leu Gln Asn Gln Arg Tyr Ile Met Gln Glu Asn Glu Asn Pro Asn Gly
   85 90 95
   Asp Asp Ile Ser Arg Lys Lys Leu Leu Val Asp Gln Met Phe Lys Tyr
   100 105 110
   Phe Asp Ala Asp Ser Asn Asp Leu Val Asp Ile Asn Glu Leu Thr Gln
   115 120 125
   Val Ile Lys Gln Glu Glu Leu Gly Lys Asp Leu Phe Asp Cys Thr Leu
   130 135 140
   Tyr Val Leu Leu Lys Tyr Asp Asp Phe Asn Ala Asp Lys His Leu Ala
   145 150 155 160
   Leu Glu Glu Phe Tyr Arg Ala Phe Gln Val Ile Gln Leu Ser Leu Pro
   165 170 175
   Glu Asp Gln Lys Leu Ser Ile Thr Ala Ala Thr Val Gly Gln Ser Ala
   180 185 190
   Val Leu Ser Cys Ala Ile Gln Gly Thr Leu Arg Pro Pro Ile Ile Trp
   195 200 205
   Lys Arg Asn Asn Ile Ile Leu Asn Asn Leu Gly Leu Glu Asp Ile Asn
   210 215 220
   Asp Phe Gly Asp Asp Gly Ser Leu Tyr Ile Thr Lys Val Thr Thr Thr
   225 230 235 240
   His Val Gly Asn Tyr Thr Cys Tyr Ala Asp Gly Tyr Glu Gln Val Tyr
   245 250 255
   Gln Thr His Ile Phe Gln Val Asn Val Pro Pro Val Ile Arg Val Tyr
   260 265 270
   Pro Glu Ser Gln Ala Arg Glu Pro Gly Val Thr Ala Ser Leu Arg Cys
   275 280 285
   His Ala Glu Gly Ile Pro Lys Pro Gln Leu Gly Trp Leu Lys Asn Gly
   290 295 300
   Ile Asp Ile Thr Pro Lys Leu Ser Lys Gln Leu Thr Leu Gln Ala Asn
   305 310 315 320
   Gly Ser Glu Val His Ile Ser Asn Val Arg Tyr Glu Asp Thr Gly Ala
   325 330 335
   Tyr Thr Cys Ile Ala Lys Asn Glu Ala Gly Val Asp Glu Asp Ile Ser
   340 345 350
   Ser Leu Phe Val Glu Asp Ser Ala Arg Lys Thr Leu Ala Asn Ile Leu
   355 360 365
   Trp Arg Glu Glu Gly Leu Gly Ile Gly Asn Met Phe Tyr Val Phe Tyr
   370 375 380
   Glu Asp Gly Ile Lys Val Ile Gln Pro Ile Glu Cys Glu Phe Gln Arg
   385 390 395 400
   His Ile Lys Pro Ser Glu Lys Leu Leu Gly Phe Gln Asp Glu Val Cys
   405 410 415
   Pro Ile Ala Glu Gly Asp Glu Val Gln Arg Cys Val Trp Ala Ser Ala
   420 425 430
   Val Asn Val Lys Asp Lys Phe Ile Tyr Val Ala Gln Pro Thr Leu Asp
   435 440 445
   Arg Val Leu Ile Val Asp Val Gln Ser Gln Lys Val Val Gln Ala Val
   450 455 460
   Ser Thr Asp Pro Val Pro Val Lys Leu His Tyr Asp Lys Ser His Asp
   465 470 475 480
   Gln Val Trp Val Leu Ser Trp Gly Thr Leu Glu Lys Thr Ser Pro Thr
   485 490 495
   Leu Gln Val Ile Thr Leu Ala Ser Gly Asn Val Pro His His Thr Ile
   500 505 510
   His Thr Gln Pro Val Gly Lys Gln Phe Asp Arg Val Asp Asp Phe Phe
   515 520 525
   Ile Pro Thr Thr Thr Leu Ile Ile Thr His Met Arg Phe Gly Phe Ile
   530 535 540
   Leu His Lys Asp Glu Ala Ala Leu Gln Lys Ile Asp Leu Glu Thr Met
   545 550 555 560
   Ser Tyr Ile Lys Thr Ile Asn Leu Lys Asp Tyr Lys Cys Val Pro Gln
   565 570 575
   Ser Leu Ala Tyr Thr His Leu Gly Gly Tyr Tyr Phe Ile Gly Cys Lys
   580 585 590
   Pro Asp Ser Thr Gly Ala Val Ser Pro Gln Val Met Val Asp Gly Val
   595 600 605
   Thr Asp Ser Val Ile Gly Phe Asn Ser Asp Val Thr Gly Thr Pro Tyr
   610 615 620
   Val Ser Pro Asp Gly His Tyr Leu Val Ser Ile Asn Asp Val Lys Gly
   625 630 635 640
   Leu Val Arg Val Gln Tyr Ile Thr Ile Arg Gly Glu Ile Gln Glu Ala
   645 650 655
   Phe Asp Ile Tyr Thr Asn Leu His Ile Ser Asp Leu Ala Phe Gln Pro
   660 665 670
   Ser Phe Thr Glu Ala His Gln Tyr Asn Ile Tyr Gly Ser Ser Ser Thr
   675 680 685
   Gln Thr Asp Val Leu Phe Val Glu Leu Ser Ser Gly Lys Val Lys Met
   690 695 700
   Ile Lys Ser Leu Lys Glu Pro Leu Lys Ala Glu Glu Trp Pro Trp Asn
   705 710 715 720
   Arg Lys Asn Arg Gln Ile Gln Asp Ser Gly Leu Phe Gly Gln Tyr Leu
   725 730 735
   Met Thr Pro Ser Lys Asp Ser Leu Phe Ile Leu Asp Gly Arg Leu Asn
   740 745 750
   Lys Leu Asn Cys Glu Ile Thr Glu Val Glu Lys Gly Asn Thr Val Ile
   755 760 765
   Trp Val Gly Asp Ala
   770
<210> 50
   <211> 306
   <212> PRT
   <213> Rattus norvegicus
<400> 50
   Met Trp Lys Arg Trp Leu Ala Leu Ala Leu Val Thr Ile Ala Leu Val
   1 5 10 15
   His Gly Glu Glu Glu Gln Arg Ser Lys Ser Lys Ile Cys Ala Asn Val
   20 25 30
   Phe Cys Gly Ala Gly Arg Glu Cys Ala Val Thr Glu Lys Gly Glu Pro
   35 40 45
   Thr Cys Leu Cys Ile Glu Gln Cys Lys Pro His Lys Arg Pro Val Cys
   50 55 60
   Gly Ser Asn Gly Lys Thr Tyr Leu Asn His Cys Glu Leu His Arg Asp
   65 70 75 80
   Ala Cys Leu Thr Gly Ser Lys Ile Gln Val Asp Tyr Asp Gly His Cys
   85 90 95
   Lys Glu Lys Lys Ser Val Ser Pro Ser Ala Ser Pro Val Val Cys Tyr
   100 105 110
   Gln Ala Asn Arg Asp Glu Leu Arg Arg Arg Ile Ile Gln Trp Leu Glu
   115 120 125
   Ala Glu Ile Ile Pro Asp Gly Trp Phe Ser Lys Gly Ser Asn Tyr Ser
   130 135 140
   Glu Ile Leu Asp Lys Tyr Phe Lys Ser Phe Asp Asn Gly Asp Ser His
   145 150 155 160
   Leu Asp Ser Ser Glu Phe Leu Lys Phe Val Glu Gln Asn Glu Thr Ala
   165 170 175
   Val Asn Ile Thr Ala Tyr Pro Asn Gln Glu Asn Asn Lys Leu Leu Arg
   180 185 190
   Gly Leu Cys Val Asp Ala Leu Ile Glu Leu Ser Asp Glu Asn Ala Asp
   195 200 205
   Trp Lys Leu Ser Phe Gln Glu Phe Leu Lys Cys Leu Asn Pro Ser Phe
   210 215 220
   Asn Pro Pro Glu Lys Lys Cys Ala Leu Glu Asp Glu Thr Tyr Ala Asp
   225 230 235 240
   Gly Ala Glu Thr Glu Val Asp Cys Asn Arg Cys Val Cys Ser Cys Gly
   245 250 255
   His Trp Val Cys Thr Ala Met Thr Cys Asp Gly Lys Asn Gln Lys Gly
   260 265 270
   Val Gln Thr His Thr Glu Glu Glu Met Thr Arg Tyr Ala Gln Glu Leu
   275 280 285
   Gln Lys His Gln Gly Thr Ala Glu Lys Thr Lys Lys Val Asn Thr Lys
   290 295 300
   Glu Ile
   305
<210> 51
   <211> 306
   <212> PRT
   <213> Mus musculus
<400> 51
   Met Trp Lys Arg Trp Leu Ala Leu Ser Leu Val Thr Ile Ala Leu Val
   1 5 10 15
   His Gly Glu Glu Glu Pro Arg Ser Lys Ser Lys Ile Cys Ala Asn Val
   20 25 30
   Phe Cys Gly Ala Gly Arg Glu Cys Ala Val Thr Glu Lys Gly Glu Pro
   35 40 45
   Thr Cys Leu Cys Ile Glu Gln Cys Lys Pro His Lys Arg Pro Val Cys
   50 55 60
   Gly Ser Asn Gly Lys Thr Tyr Leu Asn His Cys Glu Leu His Arg Asp
   65 70 75 80
   Ala Cys Leu Thr Gly Ser Lys Ile Gln Val Asp Tyr Asp Gly His Cys
   85 90 95
   Lys Glu Lys Lys Ser Ala Ser Pro Ser Ala Ser Pro Val Val Cys Tyr
   100 105 110
   Gln Ala Asn Arg Asp Glu Leu Arg Arg Arg Leu Ile Gln Trp Leu Glu
   115 120 125
   Ala Glu Ile Ile Pro Asp Gly Trp Phe Ser Lys Gly Ser Asn Tyr Ser
   130 135 140
   Glu Ile Leu Asp Lys Tyr Phe Lys Ser Phe Asp Asn Gly Asp Ser His
   145 150 155 160
   Leu Asp Ser Ser Glu Phe Leu Lys Phe Val Glu Gln Asn Glu Thr Ala
   165 170 175
   Ile Asn Ile Thr Thr Tyr Ala Asp Gln Glu Asn Asn Lys Leu Leu Arg
   180 185 190
   Ser Leu Cys Val Asp Ala Leu Ile Glu Leu Ser Asp Glu Asn Ala Asp
   195 200 205
   Trp Lys Leu Ser Phe Gln Glu Phe Leu Lys Cys Leu Asn Pro Ser Phe
   210 215 220
   Asn Pro Pro Glu Lys Lys Cys Ala Leu Glu Val Glu Thr Tyr Ala Asp
   225 230 235 240
   Gly Ala Glu Thr Glu Val Asp Cys Asn Arg Cys Val Cys Ser Cys Gly
   245 250 255
   His Trp Val Cys Thr Ala Met Thr Cys Asp Gly Lys Asn Gln Lys Gly
   260 265 270
   Val Gln Thr His Thr Glu Glu Glu Lys Thr Gly Tyr Val Gln Glu Leu
   275 280 285
   Gln Lys His Gln Gly Thr Ala Glu Lys Thr Lys Lys Val Asn Thr Lys
   290 295 300
   Glu Ile
   305
<210> 52
   <211> 299
   <212> PRT
   <213> Xenopus laevis
<400> 52
   Met Tyr Leu Arg Cys Val Pro Leu Leu Ala Leu Leu Val Leu Cys Ser
   1 5 10 15
   Ala Leu Glu Glu Pro Lys Ser Lys Ser Lys Val Cys Ala Asn Val Phe
   20 25 30
   Cys Gly Ala Gly Arg Glu Cys Ala Val Thr Glu Lys Gly Asp Pro Thr
   35 40 45
   Cys Asp Cys Ile Glu Lys Cys Lys Ser His Lys Arg Pro Val Cys Gly
   50 55 60
   Ser Asn Gly Lys Thr Tyr Leu Asn His Cys Glu Leu His Arg Asp Ala
   65 70 75 80
   Cys Leu Thr Gly Ser Lys Ile Gln Val Asp Tyr Asp Gly His Cys Lys
                   85                  90                  95
   Glu Lys Thr Ser Asp Thr Pro Ala Ala Val Pro Val Ala Cys Tyr Gln
               100                 105                 110
   Ser Asp Arg Asp Glu Met Arg Arg Arg Val Ile His Trp Leu Gln Thr
           115                 120                 125
   Glu Ile Thr Pro Asp Gly Trp Phe Ser Lys Gly Ser Asp Tyr Ser Glu
       130                 135                 140
   Ile Leu Asp Arg Tyr Phe Lys Lys Phe Asp Asp Gly Asp Ser His Leu
   145                 150                 155                 160
   Asp Ser Ala Glu Leu Gln Ser Phe Leu Glu Gln Ser Gln Ser Thr Asn
                   165                 170                 175
   Ile Thr Thr Tyr Lys Asp Glu Glu Thr Asn Arg Met Leu Lys Ser Leu
               180                 185                 190
   Cys Val Glu Ala Leu Ile Glu Leu Ser Asp Glu Asn Ala Asp Trp Lys
           195                 200                 205
   Leu Asn Lys Asn Glu Phe Leu Lys Cys Leu Asn Pro Asp Phe Gln Pro
       210                 215                 220
   Ser Glu Lys Lys Cys Ala Leu Glu Asp Glu Thr Tyr Glu Asp Gly Ala
   225                 230                 235                 240
   Glu Thr Gln Val Gln Cys Asn Arg Cys Val Cys Ala Cys Gly Asn Trp
                   245                 250                 255
   Val Cys Thr Ala Met Ala Cys Glu Gly Lys Asp Gly Asp His Gly Glu
               260                 265                 270
   Asp Met Gly Arg Tyr Val Glu Glu Ile Arg Lys Gln Gln Glu Thr Ile
           275                 280                 285
   Glu Asn Ser Lys Ser Ser Ser Asp Lys Asp Ala
       290                 295
<210> 53
   <211> 308
   <212> PRT
   <213> Homo sapiens
<400> 53
   Met Trp Lys Arg Trp Leu Ala Leu Ala Leu Ala Leu Val Ala Val Ala
   1               5                   10                  15
   Trp Val Arg Ala Glu Glu Glu Leu Arg Ser Lys Ser Lys Ile Cys Ala
               20                  25                  30
   Asn Val Phe Cys Gly Ala Gly Arg Glu Cys Ala Val Thr Glu Lys Gly
           35                  40                  45
   Glu Pro Thr Cys Leu Cys Ile Glu Gln Cys Lys Pro His Lys Arg Pro
       50                  55                  60
   Val Cys Gly Ser Asn Gly Lys Thr Tyr Leu Asn His Cys Glu Leu His
   65                  70                  75                  80
   Arg Asp Ala Cys Leu Thr Gly Ser Lys Ile Gln Val Asp Tyr Asp Gly
                   85                  90                  95
   His Cys Lys Glu Lys Lys Ser Val Ser Pro Ser Ala Ser Pro Val Val
               100                 105                 110
   Cys Tyr Gln Ser Asn Arg Asp Glu Leu Arg Arg Arg Ile Ile Gln Trp
           115                 120                 125
   Leu Glu Ala Glu Ile Ile Pro Asp Gly Trp Phe Ser Lys Gly Ser Asn
       130                 135                 140
   Tyr Ser Glu Ile Leu Asp Lys Tyr Phe Lys Asn Phe Asp Asn Gly Asp
   145                 150                 155                 160
   Ser Arg Leu Asp Ser Ser Glu Phe Leu Lys Phe Val Glu Gln Asn Glu
                   165                 170                 175
   Thr Ala Ile Asn Ile Thr Thr Tyr Pro Asp Gln Glu Asn Asn Lys Leu
               180                 185                 190
   Leu Arg Gly Leu Cys Val Asp Ala Leu Ile Glu Leu Ser Asp Glu Asn
           195                 200                 205
   Ala Asp Trp Lys Leu Ser Phe Gln Glu Phe Leu Lys Cys Leu Asn Pro
       210                 215                 220
   Ser Phe Asn Pro Pro Glu Lys Lys Cys Ala Leu Glu Asp Glu Thr Tyr
   225                 230                 235                 240
   Ala Asp Gly Ala Glu Thr Glu Val Asp Cys Asn Arg Cys Val Cys Ala
   245 250 255
   Cys Gly Asn Trp Val Cys Thr Ala Met Thr Cys Asp Gly Lys Asn Gln
   260 265 270
   Lys Gly Ala Gln Thr Gln Thr Glu Glu Glu Met Thr Arg Tyr Val Gln
   275 280 285
   Glu Leu Gln Lys His Gln Glu Thr Ala Glu Lys Thr Lys Arg Val Ser
   290 295 300
   Thr Lys Glu Ile
   305
<210> 54
   <211> 315
   <212> PRT
   <213> Gallus gallus
<400> 54
   Met Ile Trp Lys Thr Leu Pro Leu Leu Cys Ala Leu Leu Ala Val Ala
   1 5 10 15
   Arg Leu Arg Ala Glu Glu Glu Pro Arg Ser Lys Ser Lys Ile Cys Ala
   20 25 30
   Asn Val Phe Cys Gly Arg Gly Ala Glu Cys Ala Val Thr Glu Lys Gly
   35 40 45
   Glu Pro Thr Cys Leu Cys Ile Glu Gln Cys Lys Pro His Gly Arg Pro
   50 55 60
   Val Cys Gly Ser Asn Gly Lys Thr Tyr Leu Asn His Cys Glu Leu His
   65 70 75 80
   Arg Asp Ala Cys Leu Thr Gly Ser Lys Ile Gln Val Asp Tyr Asp Gly
   85 90 95
   His Cys Lys Glu Lys Lys Ser Glu Asn Pro Ala Ala Ser Pro Val Val
   100 105 110
   Cys Tyr Gln Ser Asp Arg Asp Glu Leu His Arg Arg Val Ile Arg Trp
   115 120 125
   Leu Glu Gly Glu Ile Ile Pro Asp Gly Trp Phe Ser Lys Gly Ser Asn
   130 135 140
   Tyr Ser Asp Val Leu Glu Lys Tyr Phe Lys Asn Phe Asp Asp Asp Ser
   145 150 155 160
   Arg Leu Asp Ser Thr Glu Phe Leu Lys Phe Val Glu Gln Asn Glu Thr
   165 170 175
   Ala Val Pro Thr Ile Thr Thr Tyr Val Asp Gln Glu Thr Asn Lys Leu
   180 185 190
   Leu Arg Gly Leu Cys Val Val Ala Leu Ile Glu Leu Ser Val Gln Asn
   195 200 205
   Ala Asp Trp Lys Leu Ser Phe Asn Glu Phe Leu Lys Cys Leu Ser Pro
   210 215 220
   Ser Phe Asn Pro Pro Glu Lys Lys Cys Ala Leu Glu Asp Glu Thr Tyr
   225 230 235 240
   Glu Asp Gly Ala Glu Thr Gln Val Glu Cys Asn Arg Cys Val Tyr Ala
   245 250 255
   Cys Gly Asn Trp Val Cys Thr Ala Met Thr Cys Glu Gly Lys Asn Glu
   260 265 270
   Lys Met Thr Ala His Arg Gln Gln Pro Gly Gln Asp Leu Thr Glu Glu
   275 280 285
   Glu Leu Ala Arg Tyr Val Gln Glu Leu Gln Lys His Gln Glu Gln Ala
   290 295 300
   Glu Lys Ile Lys Lys Met Ser Thr Lys Glu Met
   305 310 315
<210> 55
   <211> 1375
   <212> PRT
   <213> Drosophila melanogaster
<400> 55
   Met Ala Ile Thr Thr Asn Arg Ser Ser Arg Thr Leu Trp Asn Trp Leu
   1 5 10 15
   Leu Ser Ser Cys Leu Ile Phe Gln Leu Ile Gly Ser Ser Leu Ala Ser
   20 25 30
   Gln Ala Leu Ser Phe Thr Leu Glu Pro Gln Asp Ala Val Val Pro Glu
   35 40 45
   Gly His Ser Val Leu Leu Gln Cys Ala Gly Thr Ala Ser Ile Gly Arg
       50                  55                  60
   Gly Gly Lys Ser Lys Ser Asn Leu Pro Ser Ser Val Ser Ile Arg Trp
   65                  70                  75                  80
   Arg Gly Pro Asp Gly Gln Asp Leu Val Ile Val Gly Asp Thr Phe Arg
                   85                  90                  95
   Thr Gln Leu Lys Asn Gly Ser Leu Tyr Ile Ser Ser Val Glu Glu Asn
               100                 105                 110
   Arg Gly Leu Thr Gly Ala Tyr Gln Cys Leu Leu Thr Ala Glu Gly Val
           115                 120                 125
   Gly Ser Ile Leu Ser Arg Pro Ala Leu Val Ala Ile Val Arg Gln Pro
       130                 135                 140
   Asp Leu Asn Gln Asp Phe Leu Glu Thr Tyr Leu Leu Pro Gly Gln Thr
   145                 150                 155                 160
   Ala Tyr Phe Arg Cys Met Leu Gly Glu Ala Asn Trp Gln Glu Gly Val
                   165                 170                 175
   Lys His Ser Val Gln Trp Leu Lys Asp Asp Leu Pro Leu Pro Leu Asp
               180                 185                 190
   Lys Leu Arg Met Val Val Leu Pro Asn Gly Ala Leu Glu Ile Asp Glu
           195                 200                 205
   Val Gly Pro Ser Asp Arg Gly Ser Tyr Gln Cys Asn Val Thr Ser Gly
       210                 215                 220
   Ser Ser Ser Arg Leu Ser Ser Lys Thr Asn Leu Asn Ile Lys Lys Pro
   225                 230                 235                 240
   Ser Asp Pro Gly Val Glu Asn Ser Val Ala Pro Ser Phe Leu Val Gly
                   245                 250                 255
   Pro Ser Pro Lys Thr Val Arg Glu Gly Asp Thr Val Thr Leu Asp Cys
               260                 265                 270
   Val Ala Asn Gly Val Pro Lys Pro Gln Ile Lys Trp Leu Arg Asn Gly
           275                 280                 285
   Met Asp Leu Asp Phe Asn Asp Leu Asp Ser Arg Phe Ser Ile Val Gly
       290                 295                 300
   Thr Gly Ser Leu Gln Ile Ser Ser Ala Glu Asp Ile Asp Ser Gly Asn
   305 310 315 320
   Tyr Gln Cys Arg Ala Ser Asn Thr Val Asp Ser Leu Asp Ala Gln Ala
   325 330 335
   Thr Val Gln Val Gln Glu Pro Pro Lys Phe Ile Lys Ala Pro Lys Asp
   340 345 350
   Thr Thr Ala His Glu Lys Asp Glu Pro Glu Leu Lys Cys Asp Ile Trp
   355 360 365
   Gly Lys Pro Lys Pro Val Ile Arg Trp Leu Lys Asn Gly Asp Leu Ile
   370 375 380
   Thr Pro Asn Asp Tyr Met Gln Leu Val Asp Gly His Asn Leu Lys Ile
   385 390 395 400
   Leu Gly Leu Leu Asn Ser Asp Ala Gly Met Phe Gln Cys Val Gly Thr
   405 410 415
   Asn Ala Ala Gly Ser Val His Ala Ala Ala Arg Leu Arg Val Val Pro
   420 425 430
   Gln Gly Asp Ser Pro Glu Gln Asp Pro Ser Val Pro His Pro Gly Gly
   435 440 445
   Lys Pro Leu Asp Ser Gly Leu Gln Ala Arg Leu Pro Ser Gln Pro Arg
   450 455 460
   Asp Leu Val Ala Gln Ile Val Lys Ser Arg Phe Val Thr Leu Ser Trp
   465 470 475 480
   Val Glu Pro Leu Gln Asn Ala Gly Asp Val Val Tyr Tyr Thr Val Tyr
   485 490 495
   Tyr Lys Met Asn Asn Ser Glu Arg Glu Gln Lys Met Val Thr Lys Ser
   500 505 510
   His Asp Asp Gln Gln Val Asn Ile Gln Ser Leu Leu Pro Gly Arg Thr
   515 520 525
   Tyr Gln Phe Arg Val Glu Ala Asn Thr Asn Phe Gly Ser Gly Ala Ser
   530 535 540
   Ser Ala Pro Leu Glu Val Ser Thr Gln Pro Glu Val Asn Ile Ala Gly
   545 550 555 560
   Pro Pro Arg Asn Phe Glu Gly Tyr Ala Arg Ser His Lys Glu Ile Tyr
   565 570 575
   Val Lys Trp Glu Glu Pro Thr Val Thr Asn Gly Glu Ile Leu Lys Tyr
   580 585 590
   Arg Val Tyr Tyr Ser Glu Asn Asp Ser Gly Ala Asp Leu Tyr His Asp
   595 600 605
   Ser Thr Ala Leu Glu Ala Val Leu Thr Glu Leu Arg Pro His Thr Asp
   610 615 620
   Tyr Val Ile Ser Val Val Pro Phe Asn Arg Asn Gly Met Gly Asp Ser
   625 630 635 640
   Ser Ala Glu Ile Arg Val Lys Thr Phe Ser Ser Thr Pro Ser Glu Pro
   645 650 655
   Pro Asn Asn Val Thr Leu Glu Val Thr Ser Ser Ser Ser Ile Thr Val
   660 665 670
   His Trp Glu Pro Pro Ala Glu Glu Asp Arg Asn Gly Gln Ile Thr Gly
   675 680 685
   Tyr Lys Ile Arg Tyr Arg Lys Phe Lys Asp Ala Pro Gln Val Lys Ser
   690 695 700
   Thr Pro Ala Asn Ile Arg Tyr Phe Glu Leu Ser Asn Leu Asp Arg Asn
   705 710 715 720
   Ala Glu Tyr Gln Val Lys Ile Ala Ala Met Thr Val Asn Gly Ser Gly
   725 730 735
   Pro Phe Thr Glu Trp Asn Arg Ala Asn Thr Leu Glu Asn Asp Leu Asp
   740 745 750
   Glu Thr Gln Val Pro Gly Lys Pro Ile Trp Ile Ser Ile His Pro Gly
   755 760 765
   Ala Asn Asn Ile Ala Leu His Trp Gly Pro Pro Gln His Pro Glu Ile
   770 775 780
   Lys Ile Arg Asn Tyr Val Leu Gly Trp Gly Arg Gly Ile Pro Asp Glu
   785 790 795 800
   Asn Thr Ile Glu Leu Lys Glu Thr Glu Arg Tyr His Ile Leu Lys Asn
   805 810 815
   Leu Glu Ser Asn Met Asp Tyr Val Val Ser Leu Arg Ala Arg Asn Val
   820 825 830
   Lys Gly Asp Gly Pro Pro Ile Tyr Asp Asn Ile Lys Thr Arg Asp Glu
   835 840 845
   Glu Pro Val Asp Ala Pro Thr Pro Leu Glu Val Pro Val Gly Leu Arg
   850 855 860
   Ala Ile Thr Met Ser Ser Ser Ser Ile Val Val Tyr Trp Ile Asp Thr
   865 870 875 880
   Met Leu Asn Lys Asn Gln His Val Thr Asp Asn Arg His Tyr Thr Val
   885 890 895
   Ser Tyr Gly Ile Thr Gly Ser Asn Arg Tyr Arg Tyr His Asn Thr Thr
   900 905 910
   Asp Leu Asn Cys Met Ile Asn Asp Leu Arg Pro Asn Thr Gln Tyr Glu
   915 920 925
   Phe Ala Val Lys Val Val Lys Gly Arg Arg Glu Ser Ser Trp Ser Met
   930 935 940
   Ser Val Leu Asn Ser Thr Tyr Gln Asn Val Pro Val Thr Pro Pro Arg
   945 950 955 960
   Glu Val Thr Val Arg Leu Asp Glu Met Asn Pro Pro Thr Val Ile Val
   965 970 975
   Gln Trp Ile Pro Pro Lys His Thr Leu Gly Gln Ile Thr Gly Tyr Asn
   980 985 990
   Ile Tyr Tyr Thr Thr Asp Thr Thr Lys Arg Asp Arg Asp Trp Ser Val
   995 1000 1005
   Glu Ala Phe Ala Gly Glu Glu Thr Met Leu Met Leu Pro Asn Leu Lys
   1010 1015 1020
   Pro Tyr Thr Thr Tyr Tyr Phe Lys Val Gln Ala Arg Thr Thr Lys Gly
   1025 1030 1035 1040
   Ala Asn Asn Ala Pro Phe Ser Ala Leu Val Ser Tyr Thr Thr Ser Ala
   1045 1050 1055
   Ala Val Thr Met Gln Glu Pro Asp Thr Ile Ala Lys Gly Ile Asp Asn
   1060 1065 1070
   Glu Lys Leu Leu Tyr Ile Ile Ile Ala Ala Thr Ala Val Val Leu Leu
   1075 1080 1085
   Val Val Leu Leu Gly Val Leu Leu Leu Cys Arg Arg Lys Pro Gln Ser
   1090 1095 1100
   Ser Pro Glu His Thr Lys Lys Ser Tyr Gln Lys Asn Asn Val Gly Val
   1105 1110 1115 1120
   Pro Lys Pro Pro Asp Leu Trp Ile His His Asp Gln Met Glu Leu Lys
   1125 1130 1135
   Asn Ile Asp Lys Gly Leu His Thr Val Thr Pro Val Cys Ser Asp Gly
   1140 1145 1150
   Ala Ser Ser Ser Gly Ala Leu Thr Leu Pro Arg Ser Val Val His Ser
   1155 1160 1165
   Glu Tyr Glu Val Glu Thr Pro Val Pro Gly His Val Thr Asn Ser Leu
   1170 1175 1180
   Asp Lys Arg Ser Tyr Val Pro Gly Tyr Met Thr Thr Ser Met Asn Gly
   1185 1190 1195 1200
   Thr Met Glu Arg Pro Gln Tyr Pro Arg Thr Gln Tyr Ser His Gln Asn
   1205 1210 1215
   Arg Ser His Met Thr Met Glu Ala Gly Leu Ser Gln Gln Ser Leu Thr
   1220 1225 1230
   Gln Pro Gln Ser Asn Ser Met Ala Gln Thr Pro Glu His Pro Tyr Gly
   1235 1240 1245
   Gly Tyr Asp Ala Asn Phe Cys Asn Ala Gly Asn Ala Ala Ala Gly Asn
   1250 1255 1260
   Gly Cys Val Ser Thr Ile Glu Ser Ser Lys Arg Gly His Pro Leu Lys
   1265 1270 1275 1280
   Ser Phe Ser Val Pro Gly Pro Pro Pro Thr Gly Gly Ala Thr Pro Val
   1285 1290 1295
   Thr Lys His Thr Pro Ala Val Thr Ile Arg Pro Gln Asn Gln Ser Pro
   1300 1305 1310
   Tyr Lys Lys Pro Ser Phe Ser Ala Ala Thr Pro Asn Arg Leu Gln Gly
   1315 1320 1325
   Gly Gly Ser Val Val His Ser Thr Asp Glu Ile Gln Arg Leu Ala Pro
   1330 1335 1340
   Ser Thr Ser Thr Glu Glu Leu Asn Gln Glu Met Ala Asn Leu Glu Gly
   1345 1350 1355 1360
   Leu Met Lys Asp Leu Ser Ala Ile Thr Ala Asn Glu Phe Glu Cys
   1365 1370 1375
<210> 56
   <211> 1395
   <212> PRT
   <213> Drosophila melanogaster
<400> 56
   Met His Pro Met His Pro Glu Asn His Ala Ile Ala Arg Ser Thr Ser
   1 5 10 15
   Thr Thr Asn Asn Pro Ser Arg Ser Arg Ser Ser Arg Met Trp Leu Leu
   20 25 30
   Pro Ala Trp Leu Leu Leu Val Leu Val Ala Ser Asn Gly Leu Pro Ala
   35 40 45
   Val Arg Gly Gln Tyr Gln Ser Pro Arg Ile Ile Glu His Pro Thr Asp
   50 55 60
   Leu Val Val Lys Lys Asn Glu Pro Ala Thr Leu Asn Cys Lys Val Glu
   65 70 75 80
   Gly Lys Pro Glu Pro Thr Ile Glu Trp Phe Lys Asp Gly Glu Pro Val
   85 90 95
   Ser Thr Asn Glu Lys Lys Ser His Arg Val Gln Phe Lys Asp Gly Ala
   100 105 110
   Leu Phe Phe Tyr Arg Thr Met Gln Gly Lys Lys Glu Gln Asp Gly Gly
   115 120 125
   Glu Tyr Trp Cys Val Ala Lys Asn Arg Val Gly Gln Ala Val Ser Arg
   130 135 140
   His Ala Ser Leu Gln Ile Ala Val Leu Arg Asp Asp Phe Arg Val Glu
   145 150 155 160
   Pro Lys Asp Thr Arg Val Ala Lys Gly Glu Thr Ala Leu Leu Glu Cys
   165 170 175
   Gly Pro Pro Lys Gly Ile Pro Glu Pro Thr Leu Ile Trp Ile Lys Asp
   180 185 190
   Gly Val Pro Leu Asp Asp Leu Lys Ala Met Ser Phe Gly Ala Ser Ser
   195 200 205
   Arg Val Arg Ile Val Asp Gly Gly Asn Leu Leu Ile Ser Asn Val Glu
   210 215 220
   Pro Ile Asp Glu Gly Asn Tyr Lys Cys Ile Ala Gln Asn Leu Val Gly
   225 230 235 240
   Thr Arg Glu Ser Ser Tyr Ala Lys Leu Ile Val Gln Val Lys Pro Tyr
   245 250 255
   Phe Met Lys Glu Pro Lys Asp Gln Val Met Leu Tyr Gly Gln Thr Ala
   260 265 270
   Thr Phe His Cys Ser Val Gly Gly Asp Pro Pro Pro Lys Val Leu Trp
   275 280 285
   Lys Lys Glu Glu Gly Asn Ile Pro Val Ser Arg Ala Arg Ile Leu His
   290 295 300
   Asp Glu Lys Ser Leu Glu Ile Ser Asn Ile Thr Pro Thr Asp Glu Gly
   305 310 315 320
   Thr Tyr Val Cys Glu Ala His Asn Asn Val Gly Gln Ile Ser Ala Arg
   325 330 335
   Ala Ser Leu Ile Val His Ala Pro Pro Asn Phe Thr Lys Arg Pro Ser
   340 345 350
   Asn Lys Lys Val Gly Leu Asn Gly Val Val Gln Leu Pro Cys Met Ala
   355 360 365
   Ser Gly Asn Pro Pro Pro Ser Val Phe Trp Thr Lys Glu Gly Val Ser
   370 375 380
   Thr Leu Met Phe Pro Asn Ser Ser His Gly Arg Gln Tyr Val Ala Ala
   385 390 395 400
   Asp Gly Thr Leu Gln Ile Thr Asp Val Arg Gln Glu Asp Glu Gly Tyr
   405 410 415
   Tyr Val Cys Ser Ala Phe Ser Val Val Asp Ser Ser Thr Val Arg Val
               420                 425                 430
   Phe Leu Gln Val Ser Ser Val Asp Glu Arg Pro Pro Pro Ile Ile Gln
           435                 440                 445
   Ile Gly Pro Ala Asn Gln Thr Leu Pro Lys Gly Ser Val Ala Thr Leu
       450                 455                 460
   Pro Cys Arg Ala Thr Gly Asn Pro Ser Pro Arg Ile Lys Trp Phe His
   465                 470                 475                 480
   Asp Gly His Ala Val Gln Ala Gly Asn Arg Tyr Ser Ile Ile Gln Gly
                   485                 490                 495
   Ser Ser Leu Arg Val Asp Asp Leu Gln Leu Ser Asp Ser Gly Thr Tyr
               500                 505                 510
   Thr Cys Thr Ala Ser Gly Glu Arg Gly Glu Thr Ser Trp Ala Ala Thr
           515                 520                 525
   Leu Thr Val Glu Lys Pro Gly Ser Thr Ser Leu His Arg Ala Ala Asp
       530                 535                 540
   Pro Ser Thr Tyr Pro Ala Pro Pro Gly Thr Pro Lys Val Leu Asn Val
   545                 550                 555                 560
   Ser Arg Thr Ser Ile Ser Leu Arg Trp Ala Lys Ser Gln Glu Lys Pro
                   565                 570                 575
   Gly Ala Val Gly Pro Ile Ile Gly Tyr Thr Val Glu Tyr Phe Ser Pro
               580                 585                 590
   Asp Leu Gln Thr Gly Trp Ile Val Ala Ala His Arg Val Gly Asp Thr
           595                 600                 605
   Gln Val Thr Ile Ser Gly Leu Thr Pro Gly Thr Ser Tyr Val Phe Leu
       610                 615                 620
   Val Arg Ala Glu Asn Thr Gln Gly Ile Ser Val Pro Ser Gly Leu Ser
   625                 630                 635                 640
   Asn Val Ile Lys Thr Ile Glu Ala Asp Phe Asp Ala Ala Ser Ala Asn
                   645                 650                 655
   Asp Leu Ser Ala Ala Arg Thr Leu Leu Thr Gly Lys Ser Val Glu Leu
               660                 665                 670
   Ile Asp Ala Ser Ala Ile Asn Ala Ser Ala Val Arg Leu Glu Trp Met
   675 680 685
   Leu His Val Ser Ala Asp Glu Lys Tyr Val Glu Gly Leu Arg Ile His
   690 695 700
   Tyr Lys Asp Ala Ser Val Pro Ser Ala Gln Tyr His Ser Ile Thr Val
   705 710 715 720
   Met Asp Ala Ser Ala Glu Ser Phe Val Val Gly Asn Leu Lys Lys Tyr
   725 730 735
   Thr Lys Tyr Glu Phe Phe Leu Thr Pro Phe Phe Glu Thr Ile Glu Gly
   740 745 750
   Gln Pro Ser Asn Ser Lys Thr Ala Leu Thr Tyr Glu Asp Val Pro Ser
   755 760 765
   Ala Pro Pro Asp Asn Ile Gln Ile Gly Met Tyr Asn Gln Thr Ala Gly
   770 775 780
   Trp Val Arg Trp Thr Pro Pro Pro Ser Gln His His Asn Gly Asn Leu
   785 790 795 800
   Tyr Gly Tyr Lys Ile Glu Val Ser Ala Gly Asn Thr Met Lys Val Leu
   805 810 815
   Ala Asn Met Thr Leu Asn Ala Thr Thr Thr Ser Val Leu Leu Asn Asn
   820 825 830
   Leu Thr Thr Gly Ala Val Tyr Ser Val Arg Leu Asn Ser Phe Thr Lys
   835 840 845
   Ala Gly Asp Gly Pro Tyr Ser Lys Pro Ile Ser Leu Phe Met Asp Pro
   850 855 860
   Thr His His Val His Pro Pro Arg Ala His Pro Ser Gly Thr His Asp
   865 870 875 880
   Gly Arg His Glu Gly Gln Asp Leu Thr Tyr His Asn Asn Gly Asn Ile
   885 890 895
   Pro Pro Gly Asp Ile Asn Pro Thr Thr His Lys Lys Thr Thr Asp Tyr
   900 905 910
   Leu Ser Gly Pro Trp Leu Met Val Leu Val Cys Ile Val Leu Leu Val
   915 920 925
   Leu Val Ile Ser Ala Ala Ile Ser Met Val Tyr Phe Lys Arg Lys His
   930 935 940
   Gln Met Thr Lys Glu Leu Gly His Leu Ser Val Val Ser Asp Asn Glu
   945 950 955 960
   Ile Thr Ala Leu Asn Ile Asn Ser Lys Glu Ser Leu Trp Ile Asp His
   965 970 975
   His Arg Gly Trp Arg Thr Ala Asp Thr Asp Lys Asp Ser Gly Leu Ser
   980 985 990
   Glu Ser Lys Leu Leu Ser His Val Asn Ser Ser Gln Ser Asn Tyr Asn
   995 1000 1005
   Asn Ser Asp Gly Gly Thr Asp Tyr Ala Glu Val Asp Thr Arg Asn Leu
   1010 1015 1020
   Thr Thr Phe Tyr Asn Cys Arg Lys Ser Pro Asp Asn Pro Thr Pro Tyr
   1025 1030 1035 1040
   Ala Thr Thr Met Ile Ile Gly Thr Ser Ser Ser Glu Thr Cys Thr Lys
   1045 1050 1055
   Thr Thr Ser Ile Ser Ala Asp Lys Asp Ser Gly Thr His Ser Pro Tyr
   1060 1065 1070
   Ser Asp Ala Phe Ala Gly Gln Val Pro Ala Val Pro Val Val Lys Ser
   1075 1080 1085
   Asn Tyr Leu Gln Tyr Pro Val Glu Pro Ile Asn Trp Ser Glu Phe Leu
   1090 1095 1100
   Pro Pro Pro Pro Glu His Pro Pro Pro Ser Ser Thr Tyr Gly Tyr Ala
   1105 1110 1115 1120
   Gln Gly Ser Pro Glu Ser Ser Arg Lys Ser Ser Lys Ser Ala Gly Ser
   1125 1130 1135
   Gly Ile Ser Thr Asn Gln Ser Ile Leu Asn Ala Ser Ile His Ser Ser
   1140 1145 1150
   Ser Ser Gly Gly Phe Ser Ala Trp Gly Val Ser Pro Gln Tyr Ala Val
   1155 1160 1165
   Ala Cys Pro Pro Glu Asn Val Tyr Ser Asn Pro Leu Ser Ala Val Ala
   1170 1175 1180
   Gly Gly Thr Gln Asn Arg Tyr Gln Ile Thr Pro Thr Asn Gln His Pro
   1185 1190 1195 1200
   Pro Gln Leu Pro Ala Tyr Phe Ala Thr Thr Gly Pro Gly Gly Ala Val
   1205 1210 1215
   Pro Pro Asn His Leu Pro Phe Ala Thr Gln Arg His Ala Ala Ser Glu
   1220 1225 1230
   Tyr Gln Ala Gly Leu Asn Ala Ala Arg Cys Ala Gln Ser Arg Ala Cys
   1235 1240 1245
   Asn Ser Cys Asp Ala Leu Ala Thr Pro Ser Pro Met Gln Pro Pro Pro
   1250 1255 1260
   Pro Val Pro Val Pro Glu Gly Trp Tyr Gln Pro Val His Pro Asn Ser
   1265 1270 1275 1280
   His Pro Met His Pro Thr Ser Ser Asn His Gln Ile Tyr Gln Cys Ser
   1285 1290 1295
   Ser Glu Cys Ser Asp His Ser Arg Ser Ser Gln Ser His Lys Arg Gln
   1300 1305 1310
   Leu Gln Leu Glu Glu His Gly Ser Ser Ala Lys Gln Arg Gly Gly His
   1315 1320 1325
   His Arg Arg Arg Ala Pro Val Val Gln Pro Cys Met Glu Ser Glu Asn
   1330 1335 1340
   Glu Asn Met Leu Ala Glu Tyr Glu Gln Arg Gln Tyr Thr Ser Asp Cys
   1345 1350 1355 1360
   Cys Asn Ser Ser Arg Glu Gly Asp Thr Cys Ser Cys Ser Glu Gly Ser
   1365 1370 1375
   Cys Leu Tyr Ala Glu Ala Gly Glu Pro Ala Pro Arg Gln Met Thr Ala
   1380 1385 1390
   Lys Asn Thr
   1395
<210> 57
   <211> 2012
   <212> PRT
   <213> Homo sapiens
<400> 57
<210> 58
   <211> 338
   <212> PRT
   <213> Homo sapiens
<400> 58
   Met Val Gly Arg Val Gln Pro Asp Arg Lys Gln Leu Pro Leu Val Leu
   1               5                   10                  15
   Leu Arg Leu Leu Cys Leu Leu Pro Thr Gly Leu Pro Val Arg Ser Val
               20                  25                  30
   Asp Phe Asn Arg Gly Thr Asp Asn Ile Thr Val Arg Gln Gly Asp Thr
           35                  40                  45
   Ala Ile Leu Arg Cys Val Leu Glu Asp Lys Asn Ser Lys Val Ala Trp
       50                  55                  60
   Leu Asn Arg Ser Gly Ile Ile Phe Ala Gly His Asp Lys Trp Ser Leu
   65                  70                  75                  80
   Asp Pro Arg Val Glu Leu Glu Lys Arg His Ser Leu Glu Tyr Ser Leu
                   85                  90                  95
   Arg Ile Gln Lys Val Asp Val Tyr Asp Glu Gly Ser Tyr Thr Cys Ser
               100                 105                 110
   Val Gln Thr Gln His Glu Pro Lys Thr Ser Gln Val Tyr Leu Ile Val
           115                 120                 125
   Gln Val Pro Pro Lys Ile Ser Asn Ile Ser Ser Asp Val Thr Val Asn
       130                 135                 140
   Glu Gly Ser Asn Val Thr Leu Val Cys Met Ala Asn Gly Arg Pro Glu
   145                 150                 155                 160
   Pro Val Ile Thr Trp Arg His Leu Thr Pro Thr Gly Arg Glu Phe Glu
                   165                 170                 175
   Gly Glu Glu Glu Tyr Leu Glu Ile Leu Gly Ile Thr Arg Glu Gln Ser
               180                 185                 190
   Gly Lys Tyr Glu Cys Lys Ala Ala Asn Glu Val Ser Ser Ala Asp Val
           195                 200                 205
   Lys Gln Val Lys Val Thr Val Asn Tyr Pro Pro Thr Ile Thr Glu Ser
       210                 215                 220
   Lys Ser Asn Glu Ala Thr Thr Gly Arg Gln Ala Ser Leu Lys Cys Glu
   225                 230                 235                 240
   Ala Ser Ala Val Pro Ala Pro Asp Phe Glu Trp Tyr Arg Asp Asp Thr
                   245                 250                 255
   Arg Ile Asn Ser Ala Asn Gly Leu Glu Ile Lys Ser Thr Glu Gly Gln
   260 265 270
   Ser Ser Leu Thr Val Thr Asn Val Thr Glu Glu His Tyr Gly Asn Tyr
   275 280 285
   Thr Cys Val Ala Ala Asn Lys Leu Gly Val Thr Asn Ala Ser Leu Val
   290 295 300
   Leu Phe Arg Pro Gly Ser Val Arg Gly Ile Asn Gly Ser Ile Ser Leu
   305 310 315 320
   Ala Val Pro Leu Trp Leu Leu Ala Ala Ser Leu Leu Cys Leu Leu Ser
   325 330 335
   Lys Cys
<210> 59
   <211> 793
   <212> PRT
   <213> Mus musculus
<400> 59
   Met Ala Glu Pro Arg Thr Ala Ser Pro Arg Arg Leu Pro Ala Leu Arg
   1 5 10 15
   Arg Pro Gly Phe Leu Pro Pro Leu Leu Pro Pro Pro Pro Pro Pro Leu
   20 25 30
   Leu Leu Leu Leu Leu Leu Leu Pro Leu Pro Ala Pro Ser Leu Gly Leu
   35 40 45
   Gly His Ser Ala Glu Leu Ala Phe Ser Val Glu Pro Asn Asp Asp Ile
   50 55 60
   Ala Asn Pro Gly Gln Pro Ile Val Leu Gly Cys Lys Val Glu Gly Thr
   65 70 75 80
   Pro Pro Val Gln Val Ser Trp Arg Lys Asn Gly Ala Glu Leu Pro Glu
   85 90 95
   Gly Thr His Thr Thr Leu Leu Ala Asn Gly Ser Leu Leu Ile His His
   100 105 110
   Phe Arg Leu Glu Gln Gly Gly Ser Pro Ser Asp Glu Gly Asp Tyr Glu
   115 120 125
   Cys Val Ala Gln Asn Arg Phe Gly Leu Leu Val Ser Arg Lys Ala Arg
   130 135 140
   Leu Gln Ala Ala Thr Met Ser Asp Phe His Val His Pro Gln Ala Val
   145 150 155 160
   Thr Gly Glu Glu Gly Gly Val Ala Arg Phe Gln Cys Gln Ile His Gly
   165 170 175
   Leu Pro Lys Pro Leu Ile Thr Trp Glu Lys Asn Arg Val Pro Ile Asp
   180 185 190
   Thr Asp Asp Glu Arg Tyr Thr Leu Leu Pro Lys Gly Val Leu Gln Ile
   195 200 205
   Thr Gly Leu Arg Ala Glu Asp Ser Gly Ile Phe His Cys Val Ala Ser
   210 215 220
   Asn Ile Ala Ser Val Arg Val Ser His Gly Ala Arg Leu Thr Val Ser
   225 230 235 240
   Gly Ser Gly Ser Gly Thr Tyr Lys Glu Pro Thr Ile Leu Val Gly Pro
   245 250 255
   Glu Asn Leu Thr Leu Thr Val His Gln Thr Ala Val Leu Glu Cys Val
   260 265 270
   Ala Thr Gly Asn Pro Arg Pro Ile Val Ser Trp Ser Arg Leu Asp Gly
   275 280 285
   Arg Pro Ile Gly Val Glu Gly Ile Gln Val Leu Gly Thr Gly Asn Leu
   290 295 300
   Ile Ile Ser Asp Val Thr Val Gln His Ser Gly Val Tyr Val Cys Ala
   305 310 315 320
   Ala Asn Arg Pro Gly Thr Arg Val Arg Arg Thr Ala Gln Gly Arg Leu
   325 330 335
   Val Val Gln Ala Pro Ala Glu Phe Val Gln His Pro Gln Ser Ile Ser
   340 345 350
   Arg Pro Ala Gly Thr Thr Ala Met Phe Thr Cys Gln Ala Gln Gly Glu
   355 360 365
   Pro Pro Pro His Val Thr Trp Leu Lys Asn Gly Gln Val Leu Gly Ala
   370 375 380
   Gly Gly His Val Arg Leu Lys Asn Asn Asn Ser Thr Leu Ser Ile Ser
   385 390 395 400
   Gly Val Gly Pro Glu Asp Glu Ala Ile Tyr Gln Cys Val Ala Glu Asn
   405 410 415
   Ile Ala Gly Ser Ser Gln Ala Ser Ala Arg Leu Thr Val Leu Trp Ala
   420 425 430
   Glu Gly Leu Pro Gly Pro Pro Arg Asn Val Arg Ala Val Ser Val Ser
   435 440 445
   Ser Thr Glu Val Arg Val Ser Trp Ser Glu Pro Leu Ala His Thr Lys
   450 455 460
   Glu Ile Ile Gly Tyr Val Leu His Ile Arg Lys Ala Ala Asp Ser Pro
   465 470 475 480
   Lys Leu Glu Tyr Gln Glu Ala Val Ser Lys Ser Thr Phe Gln His Leu
   485 490 495
   Val Arg Asp Leu Glu Pro Ser Thr Ala Tyr Ser Phe Tyr Ile Lys Ala
   500 505 510
   Tyr Thr Pro Arg Gly Ala Ser Leu Ala Ser Val Pro Thr Leu Ala Ser
   515 520 525
   Thr Leu Gly Glu Ala Pro Val Pro Pro Pro Leu Ser Val Arg Leu Leu
   530 535 540
   Gly Ser Ser Ser Leu Gln Leu Leu Trp Lys Pro Trp Pro Arg Leu Ala
   545 550 555 560
   Gln His Asn Gly Gly Phe Lys Leu Phe Tyr Arg Pro Val Ser Ala Thr
   565 570 575
   Ser Phe Thr Gly Pro Ile Leu Leu Pro Gly Thr Val Ser Ser Tyr Asn
   580 585 590
   Leu Ser Gln Leu Asp Pro Ser Thr Val Tyr Glu Val Lys Leu Leu Ala
   595 600 605
   Tyr Asn Gln His Gly Asp Gly Asn Ala Thr Val Arg Phe Val Ser Leu
   610 615 620
   Lys Gly Ala Ser Glu Arg Thr Gly Ile Val Ile Gly Ile His Ile Gly
   625 630 635 640
   Val Thr Cys Ile Ile Phe Cys Val Leu Phe Leu Leu Phe Gly Gln Arg
   645 650 655
   Gly Arg Val Leu Leu Cys Lys Asp Val Glu Asn Gln Leu Ser Pro Pro
   660 665 670
   Gln Gly Pro Arg Ser Gln Arg Asp Pro Gly Ile Leu Ala Leu Asn Gly
   675 680 685
   Leu Ser Arg Gly Glu Gly Gly Gln Leu Ser Arg Asp Glu Lys Pro Val
   690 695 700
   Asp Ala Lys Glu Leu Glu Gln Leu Phe Pro Thr Ala Gly Ser Ala Ala
   705 710 715 720
   Gln Pro Gly Ser Thr Pro Thr Asp Pro Ala Ala Pro Ala Pro Cys Glu
   725 730 735
   Glu Thr Gln Leu Ser Met Val Gln Leu Gln Gly Phe Asn Leu Val Ala
   740 745 750
   Gly Arg Thr Thr Glu Ala Thr Ser Pro Cys Ala Gly Pro Gly Pro Val
   755 760 765
   Pro Ala Pro Gln Asp Ile Gly Pro Val Pro Leu Ser Glu Gly Gln Thr
   770 775 780
   Gln Pro Pro Ala Val Ala Ala Pro Gln
   785 790
<210> 60
   <211> 350
   <212> PRT
   <213> Gallus gallus
<400> 60
   Met Val Ala Arg Ala Gln Pro Asp Arg Lys Gln Leu Pro Leu Val Leu
   1 5 10 15
   Leu Arg Leu Leu Cys Leu Leu Pro Thr Gly Leu Pro Val Arg Ser Val
   20 25 30
   Asp Phe Thr Arg Gly Thr Asp Asn Ile Thr Val Arg Gln Gly Asp Thr
   35 40 45
   Ala Ile Leu Arg Cys Phe Val Glu Asp Arg Ser Ser Lys Val Ala Trp
   50 55 60
   Leu Asn Arg Ser Gly Ile Ile Phe Ala Gly Glu Asp Lys Trp Ser Leu
   65 70 75 80
   Asp Pro Arg Val Glu Leu Glu Lys Arg Ser Pro Leu Glu Tyr Ser Leu
   85 90 95
   Arg Ile Gln Lys Val Asp Val Tyr Asp Glu Gly Ser Tyr Thr Cys Ser
   100 105 110
   Val Gln Thr Gln His His Pro Lys Thr Ser Gln Val Tyr Leu Ile Val
   115 120 125
   Gln Val Pro Pro Lys Ile Ser Asn Ile Ser Ser Asp Ile Thr Val Asn
   130 135 140
   Glu Gly Ser Asn Val Thr Leu Val Cys Met Ala Asn Gly Arg Pro Glu
   145 150 155 160
   Pro Val Ile Thr Trp Arg His Leu Thr Pro Thr Gly Lys Glu Phe Glu
   165 170 175
   Gly Glu Glu Glu Tyr Leu Glu Ile Leu Gly Ile Thr Arg Glu Gln Ser
   180 185 190
   Gly Lys Tyr Glu Cys Lys Ala Ala Asn Glu Val Ala Ser Ala Asp Val
   195 200 205
   Lys Gln Val Arg Val Thr Val Asn Tyr Pro Pro Thr Ile Thr Glu Ser
   210 215 220
   Lys Ser Asn Glu Ala Ala Thr Gly Arg Gln Ala Leu Leu Arg Cys Glu
   225 230 235 240
   Ala Ser Ala Val Pro Thr Pro Asp Phe Glu Trp Tyr Arg Asp Asp Thr
   245 250 255
   Arg Ile Asn Ser Ala Asn Gly Leu Glu Ile Lys Ser Thr Gly Ser Gln
   260 265 270
   Ser Leu Leu Met Val Ala Asn Val Thr Glu Glu His Tyr Gly Asn Tyr
   275 280 285
   Thr Cys Val Ala Ala Asn Lys Leu Gly Val Thr Asn Ala Ser Leu Tyr
   290 295 300
   Leu Tyr Lys Arg Val Leu Pro Thr Leu Pro Asn Pro Phe Pro Gly Pro
   305 310 315 320
   Gly Thr Gly Arg Val Asp Asn Gly Ser Val Ser Leu Ala Val Pro Leu
   325 330 335
   Trp Leu Leu Ala Ala Ser Leu Leu Cys Leu Leu Ser Lys Cys
   340 345 350
<210> 61
   <211> 338
   <212> PRT
   <213> Rattus norvegicus
<400> 61
   Met Val Gly Arg Val Gln Pro Asp Arg Lys Gln Leu Pro Leu Val Leu
   1 5 10 15
   Leu Arg Leu Leu Cys Leu Leu Pro Thr Gly Leu Pro Val Arg Ser Val
   20 25 30
   Asp Phe Asn Arg Gly Thr Asp Asn Ile Thr Val Arg Gln Gly Asp Thr
   35 40 45
   Ala Ile Leu Arg Cys Val Val Glu Asp Lys Asn Ser Lys Val Ala Trp
   50 55 60
   Leu Asn Arg Ser Gly Ile Ile Phe Ala Gly His Asp Lys Trp Ser Leu
   65 70 75 80
   Asp Pro Arg Val Glu Leu Glu Lys Arg His Ala Leu Glu Tyr Ser Leu
   85 90 95
   Arg Ile Gln Lys Val Asp Val Tyr Asp Glu Gly Ser Tyr Thr Cys Ser
   100 105 110
   Val Gln Thr Gln His Glu Pro Lys Thr Ser Gln Val Tyr Leu Ile Val
   115 120 125
   Gln Val Pro Pro Lys Ile Ser Asn Ile Ser Ser Asp Val Thr Val Asn
   130 135 140
   Glu Gly Ser Asn Val Thr Leu Val Cys Met Ala Asn Gly Arg Pro Glu
   145 150 155 160
   Pro Val Ile Thr Trp Arg His Leu Thr Pro Leu Gly Arg Glu Phe Glu
   165 170 175
   Gly Glu Glu Glu Tyr Leu Glu Ile Leu Gly Ile Thr Arg Glu Gln Ser
               180                 185                 190
   Gly Lys Tyr Glu Cys Lys Ala Ala Asn Glu Val Ser Ser Ala Asp Val
           195                 200                 205
   Lys Gln Val Lys Val Thr Val Asn Tyr Pro Pro Thr Ile Thr Glu Ser
       210                 215                 220
   Lys Ser Asn Glu Ala Thr Thr Gly Arg Gln Ala Ser Leu Lys Cys Glu
   225                 230                 235                 240
   Ala Ser Ala Val Pro Ala Pro Asp Phe Glu Trp Tyr Arg Asp Asp Thr
                   245                 250                 255
   Arg Ile Asn Ser Ala Asn Gly Leu Glu Ile Lys Ser Thr Glu Gly Gln
               260                 265                 270
   Ser Ser Leu Thr Val Thr Asn Val Thr Glu Glu His Tyr Gly Asn Tyr
           275                 280                 285
   Thr Cys Val Ala Ala Asn Lys Leu Gly Val Thr Asn Ala Ser Leu Val
       290                 295                 300
   Leu Phe Arg Pro Gly Ser Val Arg Gly Ile Asn Gly Ser Ile Ser Leu
   305                 310                 315                 320
   Ala Val Pro Leu Trp Leu Leu Ala Ala Ser Leu Phe Cys Leu Leu Ser
                   325                 330                 335
   Lys Cys
<210> 62
   <211> 8797
   <212> DNA
   <213> Mus musculus
<400> 62
<210> 63
   <211> 2496
   <212> DNA
   <213> Gallus gallus
<400> 63
   atggatataa aagatcgaag acaccgctct ttgacgagag gccggtgcgg gaaggagtgt 60
   cgctatacta gttcttcact cgacagtgaa gactgcagag taccaactca gaagtcctac 120
   agctccagtg agactctgaa agcatatgac catgacacga ggatgcacta cggaaatcga 180
   gtttcagacc tggttcacag ggagtcggat gagtttccaa ggcaaggaac gaacttcacc 240
   cttgcagaac tgggaatctg tgagccctct ccccatcgaa gtggctactg ctcggacata 300
   ggaatactcc atcaaggcta ttccttgagc actggctctg atgctgactc agacacggag 360
   ggcgggatgt ctccagagca cgcgatcagg ctgtggggaa gagggatcaa atccaggcga 420
   agttctggcc tgtcaagtcg tgaaaactcg gctctcacgc tcactgactc cgacaatgag 480
   aacaagtcag atgaggaaaa cggtcgtccc attccaccta catcctcgtc tagccttctc 540
   ccatctgctc agctgcccag ttctcataat cctccaccag ttagctgcca gatgccattg 600
   ctagacagca atacgtccca tcaaatcatg gacaccaatc ctgacgagga gttctctcct 660
   aattcatacc tactaagagc atgttcaggg ccacagcagg catccagcag tggcccttca 720
   aaccatcaca gccagtcaac gctgaggcca cctctccccc ctcctcacaa ccactcgctg 780
   tcccatcatc actcgtctgc caactccctc aacaggaact cgctcaccaa ccgccgcaac 840
   cagatccacg cgcctgctcc cgctcccaat gacctggcga ccacgcctga gtctgtgcag 900
   ctgcaggaca gctgggtgct caacagcaac gtgccgctgg agaccaggca tttcttgttt 960
   aagacatctt ctggaacgac tccgctgttc agtagctctt cccctggcta cccactgacc 1020
   tcaggaacag tttatactcc acctcccagg ctgttaccta gaaatacatt ttccaggaat 1080
   gcattcaagc tgaaaaagcc ctccaagtat tgtagctgga aatgtgctgc tttatctgca 1140
   attgctgctg cagtcctgct tgccatcctg ctagcatatt tcatagcgat gcacctcctg 1200
   gggctgaact ggcagctgca gcccgcggac ggacacacct tcagcaacgg gctgcggccg 1260
   ggcgcggcgg gcgcggagga cggagcggcg gcgccacctg caggcagagg accgtgggtc 1320
   actaggaata gcagcataga tagtggagaa acagaagttg gccgcaaggt cacccaagag 1380
   gtgccccctg gagtgttctg gcggtctcag atccatatca gccagccaca gttcctgaag 1440
   ttcaacatat ccctagggaa ggatgctctt ttcggtgttt atataagaag aggactccca 1500
   ccatcacatg cacagtatga tttcatggaa cgcttggatg ggaaagagaa atggagtgtg 1560
   gtggaatccc cacgggaacg gcgaagtatt cagactcttg ttcagaatga ggctgtgttt 1620
   gttcagtact tggatgtggg tttgtggcac ctggcgtttt acaatgatgg caaggacaaa 1680
   gaagtggtct ccttcagtac agttattttg gattcagtgc aagactgtcc acgtaattgt 1740
   catggcaatg gcgagtgtgt ttctggtgtc tgccactgtt ttcccggatt tcatggagca 1800
   gattgtgcta aagctgcctg cccggtgctg tgcagtggca atggtcagta ctccaaagga 1860
   acctgcttgt gctacagtgg ctggaaaggt ccggaatgtg atgtacccat cagccagtgt 1920
   attgatccct cgtgtggagg tcatggttcc tgcatcgaag ggaactgtgt ctgttccatt 1980
   ggctataaag gagaaaactg tgaggaagtt gattgcttag atccaacatg ctccaatcac 2040
   ggggtctgtg tgaacggaga atgtctctgc agcccaggct ggggtggaat aaactgtgag 2100
   cttcccagag cccagtgccc agaccagtgc agtgggcatg gcacatacct gtctgacacc 2160
   ggtctctgta gctgcgatcc caactggatg ggtcccgact gctccgttga agtgtgctct 2220
   gtagactgtg gcacccatgg ggtgtgcatt ggcggagcgt gtcgctgtga agaagggtgg 2280
   acaggagtgg cgtgtgacca gcgtgtgtgt catccccggt gtacagagca cggaacttgt 2340
   aaagatggga aatgtgaatg cagagagggc tggaatgggg agcactgcac cattggtagg 2400
   caaacgactg gcaccgaaac aggctcatat tgctttcttt tcataaatcg tagagacata 2460
   gttactgtgg tgtactgtga taggctgttc ctttaa                           2496
<210> 64
   <211> 6560
   <212> DNA
   <213> Homo sapiens
<400> 64
   caccttcttt agtgctgccc ctgggcagaa tcccatcgtg cctgagaccc aggttcttca 60
   tgaagaaatc gagctccctg gttccaatgt gaaacttcgc tatctgagct ctagaactgc 120
   agggtacaag tcactgctga agatcaccat gacccagtcc acagtgcccc tgaacctcat 180
   tagggttcac ctgatggtgg ctgtcgaggg gcatctcttc cagaagtcat tccaggcttc 240
   tcccaacctg gcctacacct tcatctggga caagacagat gcgtatggcc aaagggtgta 300
   tggactctca gatgctgttg tgtctgtcgg gtttgaatat gagacctgtc ccagtctaat 360
   tctctgggag aaaaggacag ccctccttca gggattcgag ctggacccct ccaacctcgg 420
   tggctggtcc ctagacaaac accacatcct caatgttaaa agtggaatcc tacacaaagg 480
   cactggggaa aaccagttcc tgacccagca gcctgccatc atcaccagca tcatgggcaa 540
   tggtcgccgc cggagcattt cctgtcccag ctgcaacggc cttgctgaag gcaacaagct 600
   gctggcccca gtggctctgg ctgttggaat cgatgggagc ctctatgtgg gtgacttcaa 660
   ttacatccga cgcatctttc cctctcgaaa tgtgaccagc atcttggagt tacgaaataa 720
   agagtttaaa catagcaaca acccagcaca caagtactac ttggcagtgg accccgtgtc 780
   cggctcgctc tacgtgtccg acaccaacag caggagaatc taccgcgtca agtctctgag 840
   tggaaccaaa gacctggctg ggaattcgga agttgtggca gggacgggag agcagtgtct 900
   accctttgat gaagcccgct gcggggatgg agggaaggcc atagatgcaa ccctgatgag 960
   cccgagaggt attgcagtag acaagaatgg gctcatgtac tttgtcgatg ccaccatgat 1020
   ccggaaggtt gaccagaatg gaatcatctc caccctgctg ggctccaatg acctcactgc 1080
   cgtccggccg ctgagctgtg attccagcat ggatgtagcc caggttcgtc tggagtggcc 1140
   aacagacctt gctgtcaatc ccatggataa ctccttgtat gttctagaga acaatgtcat 1200
   ccttcgaatc accgagaacc accaagtcag catcattgcg ggacgcccca tgcactgcca 1260
   agttcctggc attgactact cactcagcaa actagccatt cactctgccc tggagtcagc 1320
   cagtgccatt gccatttctc acactggggt cctctacatc actgagacag atgagaagaa 1380
   gattaaccgt ctacgccagg taacaaccaa cggggagatc tgccttttag ctggggcagc 1440
   ctcggactgc gactgcaaaa acgatgtcaa ttgcaactgc tattcaggag atgatgccta 1500
   cgcgactgat gccatcttga attccccatc atccttagct gtagctccag atggtaccat 1560
   ttacattgca gaccttggaa atattcggat cagggcggtc agcaagaaca agcctgttct 1620
   taatgccttc aaccagtatg aggctgcatc ccccggagag caggagttat atgttttcaa 1680
   cgctgatggc atccaccaat acactgtgag cctggtgaca ggggagtact tgtacaattt 1740
   cacatatagt actgacaatg atgtcactga attgattgac aataatggga attccctgaa 1800
   gatccgtcgg gacagcagtg gcatgccccg tcacctgctc atgcctgaca accagatcat 1860
   caccctcacc gtgggcacca atggaggcct caaagtcgtg tccacacaga acctggagct 1920
   tggtctcatg acctatgatg gcaacactgg gctcctggcc accaagagcg atgaaacagg 1980
   atggacgact ttctatgact atgaccacga aggccgcctg accaacgtga cgcgccccac 2040
   gggggtggta accagtctgc accgggaaat ggagaaatct attaccattg acattgagaa 2100
   ctccaaccgt gatgatgacg tcactgtcat caccaacctc tcttcagtag aggcctccta 2160
   cacagtggta caagatcaag ttcggaacag ctaccagctc tgtaataatg gtaccctgag 2220
   ggtgatgtat gctaatggga tgggtatcag cttccacagc gagccccatg tcctagcggg 2280
   caccatcacc cccaccattg gacgctgcaa catctccctg cctatggaga atggcttaaa 2340
   ctccattgag tggcgcctaa gaaaggaaca gattaaaggc aaagtcacca tctttggcag 2400
   gaagctccgg gtccatggaa gaaatctctt gtccattgac tatgatcgaa atattcggac 2460
   tgaaaagatc tatgatgacc accggaagtt caccctgagg atcatttatg accaggtggg 2520
   ccgccccttc ctctggctgc ccagcagcgg gctggcagct gtcaacgtgt catacttctt 2580
   caatgggcgc ctggctgggc ttcagcgtgg ggccatgagc gagaggacag acatcgacaa 2640
   gcaaggccgc atcgtgtccc gcatgttcgc tgacgggaaa gtgtggagct actcctacct 2700
   tgacaagtcc atggtcctcc tgcttcagag ccaacgtcag tatatatttg agtatgactc 2760
   ctctgaccgc ctccttgccg tcaccatgcc cagcgtggcc cggcacagca tgtccacaca 2820
   cacctccatc ggctacatcc gtaatattta caacccgcct gaaagcaatg cttcggtcat 2880
   ctttgactac agtgatgacg gccgcatcct gaagacctcc tttttgggca ccggacgcca 2940
   ggtgttctac aagtatggga aactctccaa gttatcagag attgtctacg acagtaccgc 3000
   cgtcaccttc gggtatgacg agaccactgg tgtcttgaag atggtcaacc tccaaagtgg 3060
   gggcttctcc tgcaccatca ggtaccggaa gattggcccc ctggtggaca agcagatcta 3120
   caggttctcc gaggaaggca tggtcaatgc caggtttgac tacacctatc atgacaacag 3180
   cttccgcatc gcaagcatca agcccgtcat aagtgagact cccctccccg ttgacctcta 3240
   ccgctatgat gagatttctg gcaaggtgga acactttggt aagtttggag tcatctatta 3300
   tgacatcaac cagatcatca ccactgccgt gatgaccctc agcaaacact tcgacaccca 3360
   tgggcggatc aaggaggtcc agtatgagat gttccggtcc ctcatgtact ggatgacggt 3420
   gcaatatgac agcatgggca gggtgatcaa gagggagcta aaactggggc cctatgccaa 3480
   taccacgaag tacacctatg actacgatgg ggacgggcag ctccagagcg tggccgtcaa 3540
   tgaccgcccg acctggcgct acagctatga ccttaatggg aatctccact tactgaaccc 3600
   aggcaacagt gtgcgcctca tgcccttgcg ctatgacctc cgggatcgga taaccagact 3660
   cggggatgtg cagtacaaaa ttgacgacga tggctatctg tgccagagag ggtctgacat 3720
   cttcgaatac aattccaagg gcctcctaac aagagcctac aacaaggcca gcgggtggag 3780
   tgtccagtac cgctatgatg gcgtaggacg gcgggcttcc tacaagacca acctgggcca 3840
   ccacctgcag tacttctact ctgacctcca caacccgacg cgcatcaccc atgtctacaa 3900
   tcactccaac tcggagatta cctcactgta ctacgacctc cagggccacc tctttgccat 3960
   ggagagcagc agtggggagg agtactatgt tgcctctgat aacacaggga ctcctctggc 4020
   tgtgttcagc atcaacggcc tcatgatcaa acagctgcag tacacggcct atggggagat 4080
   ttattatgac tccaaccccg acttccagat ggtcattggc ttccatgggg gactctatga 4140
   ccccctgacc aagctggtcc acttcactca gcgtgattat gatgtgctgg caggacgatg 4200
   gacctcccca gactatacca tgtggaaaaa cgtgggcaag gagccggccc cctttaacct 4260
   gtatatgttc aagagcaaca atcctctcag cagtgagcta gatttgaaga actacgtgac 4320
   agatgtgaaa agctggcttg tgatgtttgg atttcagctt agcaacatca ttcctggctt 4380
   cccgagagcc aaaatgtatt tcgtgcctcc tccctatgaa ttgtcagaga gtcaagcaag 4440
   tgagaatgga cagctcatta caggtgtcca acagacaaca gagagacata accaggcctt 4500
   catggctctg gaaggacagg tcattactaa aaagctccac gccagcatcc gagagaaagc 4560
   aggtcactgg tttgccacca ccacgcccat cattggcaaa ggcatcatgt ttgccatcaa 4620
   agaagggcgg gtgaccacgg gcgtgtccag catcgccagc gaagatagcc gcaaggtggc 4680
   atctgtgctg aacaacgcct actacctgga caagatgcac tacagcatcg agggcaagga 4740
   cacccactac tttgtgaaga ttggctcagc cgatggcgac ctggtcacac taggcaccac 4800
   catcggccgc aaggtgctag agagcggggt gaacgtgacc gtgtcccagc ccacgctgct 4860
   ggtcaacggc aggactcgaa ggttcacgaa cattgagttc cagtactcca cgctgctgct 4920
   cagcatccgc tatggcctca cccccgacac cctggacgaa gagaaggccc gcgtcctgga 4980
   ccaggcgaga cagagggccc tgggcacggc ctgggccaag gagcagcaga aagccaggga 5040
   cgggagagag gggagccgcc tgtggactga gggcgagaag cagcagcttc tgagcaccgg 5100
   gcgcgtgcaa gggtacgagg gatattacgt gcttcccgtg gagcaatacc cagagcttgc 5160
   agacagtagc agcaacatcc agtttttaag acagaatgag atgggaaaga ggtaacaaaa 5220
   taatctgctg ccattccttg tctgaatggc tcagcaggag taactgttat ctcctctcct 5280
   aaggagatga agacctaaca ggggcactgc ggctgggctg ctttaggaga ccaagtggca 5340
   agaaagctca cattttttga gttcaaatgc tactgtccaa gcgagaagtc cctcatcctg 5400
   aagtagacta aagcccggct gaaaattccg aggaaaacaa aacaaacgaa tgaatgaaca 5460
   gacacacaca atgttccaag ttcccctaaa atatgaccca cttgttctgg gtctacgcag 5520
   aaaagagacg caaagtgtcc aaaaggaaca aaagaacaaa aacgaataag caaagaagaa 5580
   aacaaacaaa aacaaaacaa aacaaacaca cggaccgata aacaaagaag cgaagataag 5640
   aaagaaggcc tcatatccaa ttacctcact cattcacatg tgagcgacac gcagacatcc 5700
   gcgagggcca gcgtcaccag accagctgcg ggacaaacca ctcagactgc ttgtaggaca 5760
   aatacttctg acattttcgt ttaagcaaat acaggtgcat ttaaaacacg actttggggg 5820
   tgatttgtgt gtagcgcctg gggagggggg ataaaagagg aggagtgagc actggaaata 5880
   ctttttaaag aaaaaaaaac atgagggaat aaaagaaatt cctatcaaaa atcaaagtga 5940
   aataatacca tccagcactt aactctcagg tcccaactaa gtctggcctg agctaattta 6000
   tttgagcgca gagtgtaaaa tttaattcaa aatggtggct ataatcacta cagataaatt 6060
   tcatactctt ttgtctttgg agattccatt gtggacagta atacgcagtt acagggtgta 6120
   gtctgtttag attccgtagt tcgtgggtat cagtttcggt agaggtgcag catcgtgaca 6180
   cttttgctaa caggtaccac ttctgatcac cctgtacata catgagccga aaggcacaat 6240
   cactgtttca gatttaaaat tattagtgtg tttgtttggt ccagaaactg agacaatcac 6300
   atgacagtca ccacgaggag agaaaattta aaaaataaaa ataaaaacaa aaaaaatttt 6360
   aaaaattaaa aaaacaaaaa taaagtctaa taagaacttt ggtacaggaa cttttttgta 6420
   atatacatgt atgaattgtt catcgagttt ttatattaat tttaatttgc tgctaagcaa 6480
   agactaggga caggcaaaga taatttatgg caaagtgttt aaattgttta tacataaata 6540
   aagtctctaa aactcctgtg 6560
<210> 65
   <211> 8797
   <212> DNA
   <213> Mus musculus
<400> 65
   ctggtgcacc gggagtccga tgagttttct agacaaggtg ggtgagtgac aggtgccaca 60
   actccagcct ccccttttct gaaagtcgcg gagttctgtt tcatctggaa taatggatgt 120
   aaaggaccgg cgacatcgct ctttgaccag gggacggtgt ggcaaagagt gtcgctacac 180
   cagctcctct ctggacagtg aggactgccg tgtgcccact cagaagtcct acagttccag 240
   tgagaccttg aaggcttatg accatgacag cagaatgcac tatggaaacc gagtcacaga 300
   cctggtgcac cgggagtccg atgagttttc tagacaaggg acaaacttca ccctggcaga 360
   attgggaatc tgcgagccct ccccacaccg aagtggttac tgttccgaca tgggtatcct 420
   ccaccagggc tactccctga gcactgggtc tgatgcagac tcggacaccg agggagggat 480
   gtctccagaa catgccatca gactgtgggg acgagggata aaatccaggc gcagctctgg 540
   cttgtccagc cgcgagaact cggcccttac tctgactgac tctgacaatg aaaataaatc 600
   ggatgacgac aatggtcgtc ccattccacc tacatcctcg tctagcctcc tcccatctgc 660
   tcagctgcct agctcccata atcctccacc agttagctgc cagatgccat tgctagacag 720
   caacacctcc catcagatca tggacaccaa ccctgatgag gaattctccc ccaattcata 780
   cctgctcaga gcatgctcag ggccccagca agcctccagc agtggccctc caaaccacca 840
   cagccagtca acactgaggc cccctctgcc accccctcat aaccacaccc tgtcccacca 900
   ccactcctcg gccaactccc tcaacaggaa ctcactgacc aatcggcgga gtcaaatcca 960
   cgccccagct cctgcgccca acgacctggc caccacccca gagtctgttc agctccagga 1020
   tagctgggtg ctgaacagta acgtcccact ggagactcgg cacttccttt tcaaaacgtc 1080
   gtctggaagc acacccctgt tcagcagctc ttctccggga taccctttga cctcagggac 1140
   cgtttataca ccaccacccc gcctgctgcc acggaataca ttctccagga aggccttcaa 1200
   gctgaagaaa ccctccaaat actgcagttg gaaatgtgct gccctgtctg ccatcgccgc 1260
   cgccctcctc ttggccattt tgctggcata tttcatagca atgcatctgc tcggactcaa 1320
   ttggcaactc cagccggcag atggacacac ctttaacaat ggcgtaagga ccggcttacc 1380
   aggaaacgat gatgtggcaa cagtgccatc tggaggcaaa gtgccctggt cattgaaaaa 1440
   cagcagcata gacagtggcg aagcagaagt tggtcggcgg gtgacacagg aagtcccacc 1500
   aggggtgttt tggaggtccc agattcacat cagtcagcct caattcttaa agttcaacat 1560
   ctccctgggc aaggatgccc tcttcggtgt ctatataagg agaggactac caccgtctca 1620
   tgcccagtat gacttcatgg aacgcctgga tggaaaggag aaatggagcg tggtcgagtc 1680
   gcccagggaa cgccggagca tccagactct ggtgcagaac gaggctgtgt ttgtgcagta 1740
   cttggatgtg ggcctgtggc acctggcctt ctacaatgac ggcaaggaca aggagatggt 1800
   ctccttcaac actgttgtct tagattcagt gcaggactgt ccacggaact gtcacgggaa 1860
   cggtgaatgc gtgtctggac tgtgtcactg tttcccagga ttcctaggtg cagactgtgc 1920
   taaagctgcc tgccctgtac tgtgcagcgg aaatggacag tattctaaag gaacgtgcca 1980
   gtgctacagc ggctggaaag gtgcagagtg tgatgtgcct atgaaccaat gtatcgatcc 2040
   ttcctgtggg ggccatggct cctgcattga tgggaactgc gtgtgtgctg ctggctacaa 2100
   gggcgagcac tgtgaggaag ttgattgctt ggatcctacc tgctccagcc atggtgtctg 2160
   tgtgaatgga gagtgtctat gcagccccgg ctggggtggt ctcaactgtg agctggcgag 2220
   ggtccagtgc ccagaccagt gtagtgggca tggcacttac ctccctgact ccggcctctg 2280
   cagctgtgat ccgaactgga tgggtcccga ctgctctgtt gtgtgctcag tagactgtgg 2340
   cactcacggc gtctgcatcg ggggagcctg ccgctgtgaa gagggctgga caggcgcagc 2400
   ttgtgaccag cgcgtgtgcc acccccgctg cattgagcac gggacctgta aagatggcaa 2460
   atgtgaatgc cgagagggct ggaatggtga acactgcacc attgatggct gccctgattt 2520
   gtgcaacggt aacgggagat gcacactggg tcagaacagc tggcagtgtg tctgccagac 2580
   cggctggaga gggcctggat gcaacgttgc catggaaacc tcctgcgctg ataacaagga 2640
   taatgaggga gatggcctgg tggactgcct ggaccctgac tgctgcctac agtcagcctg 2700
   tcagaacagc ctgctctgcc gggggtctcg ggaccccttg gacatcattc agcaaggtca 2760
   gacagactgg cctgcagtga agtccttcta tgaccgcatc aagctcttgg caggcaagga 2820
   cagcacccac atcattcctg gagacaaccc cttcaatagc agcctggtgt ctctgatccg 2880
   aggccaagta gtaaccatgg atgggactcc cttggtgggt gtgaatgtgt cttttgtcaa 2940
   gtacccaaaa tatggctaca ccatcactcg ccaggatggc acgtttgacc tgattgccaa 3000
   tgggggttct gccttgactc ttcactttga gcgagcccct ttcatgagcc aggagcgcac 3060
   agtgtggctg ccatggaaca gcttctatgc catggacacc ctggtaatga agaccgagga 3120
   aaactccatc cccagctgtg acctcagtgg ctttgtccgg ccagatccaa tcatcatctc 3180
   ctctcctctg tccaccttct tcagcgcttc ccctgcctcg aaccccattg tgcctgagac 3240
   ccaggttctt catgaagaaa ttgagctccc tggtaccaat gtgaagctcc gttatctcag 3300
   ctctagaact gcagggtata agtcgctgct gaagatcacc atgacgcagt ccacagtgcc 3360
   cttgaacctc atcagggttc acttgatggt tgctgtagag gggcatctct tccagaagtc 3420
   attccaggct tctcccaacc tagcctacac attcatctgg gacaagacag atgcttatgg 3480
   ccaaagggtt tatggcctat cggatgctgt tgtgtctgtt gggtttgaat atgagacctg 3540
   ccccagtctc atcctgtggg agaaaaggac agccctgctt cagggattcg agctggaccc 3600
   ttccaacctt ggaggctggt ccctggacaa acaccacacc ctcaatgtga aaagcggaat 3660
   actacacaaa gggacagggg agaaccagtt cctgacccag cagcctgcca tcatcacgag 3720
   catcatgggc aacggtcgcc gcagaagcat ctcctgtccc agctgcaatg gccttgctga 3780
   aggcaacaaa ctgttagccc ctgtggccct ggctgtgggg atcgatggga gcctctttgt 3840
   tggtgacttc aactatatcc ggcgcatctt tccctctcga aatgtgacca gtatcttgga 3900
   gttacgaaat aaagagttta aacatagcaa cagcccagga cacaagtact acttggctgt 3960
   ggaccccgtg actggctcac tctacgtctc tgacaccaac agtcgccgaa tctaccgagt 4020
   caagtctctg agcggagcca aagacctggc tggaaattcg gaagttgtgg cagggactgg 4080
   cgaacaatgt ctaccctttg atgaagcccg ctgtggggat ggagggaagg ctgtggacgc 4140
   caccctgatg agccccagag gtattgcagt agacaagaat gggcttatgt actttgttga 4200
   tgccaccatg atccggaagg tggaccaaaa cggaatcatc tccaccctgc tgggctccaa 4260
   tgacctcaca gctgtccgac cactgagctg tgactcgagc atggacgtgg cccaggtccg 4320
   tctagaatgg ccgacagacc tcgccgtcaa ccccatggac aactccctgt acgttctgga 4380
   gaacaacgtc atcctgcgga tcacggagaa ccaccaggtc agcatcatcg cgggacggcc 4440
   tatgcactgc caggttcccg gcatcgacta ctcgctcagc aaactcgcca tccactctgc 4500
   gctggaatca gccagcgcca ttgccatttc tcacactggg gtgctctaca tcactgagac 4560
   ggacgagaag aagatcaacc gcctacgcca agtcaccacc aatggagaga tctgcctctt 4620
   agccggggcg gcctcagact gtgactgcaa aaacgatgtc aactgcatct gctactcggg 4680
   agatgacgct tacgccacgg acgccatcct gaactcgccg tcctccttag ccgtggctcc 4740
   ggatggcacc atctacattg cagaccttgg gaatatccgg atcagggcgg tcagcaaaaa 4800
   taaacccgtt cttaacgcat tcaaccagta tgaggctgca tctccgggag aacaggaatt 4860
   gtacgtgttc aacgctgatg gtatccatca gtacactgtg agtctggtga ctggggagta 4920
   cttgtacaat ttcacataca gcgctgacaa tgacgtcacc gagttgattg acaacaacgg 4980
   gaattcccta aagatccgcc gggacagcag tggcatgccc cgccacctgc tcatgccgga 5040
   taatcagatt atcaccctta ctgtgggcac caatggaggc ctcaaagccg tgtccactca 5100
   gaacctggag ctgggcctca tgacttatga tgggaacact ggactcctag ccaccaagag 5160
   tgatgaaacc ggatggacaa ctttttatga ctatgaccac gagggccgtc tgaccaatgt 5220
   gacccgcccc acgggcgtgg tgaccagtct gcaccgggaa atggagaaat ctatcaccat 5280
   tgacattgag aactccaacc gggatgatga cgtcactgtg atcaccaacc tctcctccgt 5340
   ggaggcctcc tatacagtgg tacaagatca agtgcgaaac agctaccagc tctgcaataa 5400
   tggaaccctg cgggtgatgt acgccaacgg catggctgtc agcttccaca gtgagcccca 5460
   cgtcctcgca ggcaccatca cccccaccat cggccgctgc aacatctctc tgcccatgga 5520
   gaatggcctg aactccatcg agtggcgcct gaggaaggaa cagatcaaag gcaaagtcac 5580
   catctttggg aggaagcttc gggtccacgg aaggaatctc ctgtccattg attatgaccg 5640
   aaatatccgt acggagaaga tctacgatga ccaccggaaa ttcaccctga ggatcatcta 5700
   tgaccaggtg ggccgcccct tcctgtggct cccgagcagt gggctggcag ccgtcaatgt 5760
   ctcctacttc ttcaatgggc gcttggccgg cctccagcga ggggccatga gcgagaggac 5820
   agacattgac aagcaaggcc ggatcgtgtc ccgcatgttc gccgacggga aagtctggag 5880
   ttattcctat cttgacaagt ccatggtcct tctgctacag agccaacgtc agtacatatt 5940
   tgaatatgac tcctccgatc gcctccacgc agtcactatg cccagtgtcg cccggcacag 6000
   catgtccacg cacacctcca ttggttacat ccgaaacatt tacaacccac ccgaaagcaa 6060
   tgcatcggtc atctttgact acagtgatga cggccgcatc ctaaagacat ctttcttggg 6120
   cactgggcgc caggtgttct acaagtatgg aaaactctcc aagttatcag agatagtcta 6180
   cgacagcaca gccgtcacct ttgggtatga cgagaccacc ggtgtcctga agatggtcaa 6240
   tctccaaagt gggggcttct cctgtaccat caggtaccga aaggttgggc cccttgtgga 6300
   caagcagatt tacaggttct ctgaggaagg aatgatcaac gccaggtttg attataccta 6360
   tcacgacaat agcttccgca ttgccagcat caaacccgtc attagcgaga ctccccttcc 6420
   tgttgacctc taccgctatg acgagatttc cggcaaggtg gaacacttcg gcaagtttgg 6480
   ggtcatctac tacgacatca accagatcat caccactgcc gtcatgacgc ttagcaagca 6540
   ctttgacacc catgggcgca tcaaggaagt gcaatatgag atgttccggt ccctcatgta 6600
   ctggatgact gtgcaatatg acagtatggg tagggtcatc aagagggaac tgaaactagg 6660
   gccctatgcc aacaccacaa agtacaccta tgactatgac ggggacggcc agctccagag 6720
   tgtggccgtc aatgaccggc ctacctggcg ctatagctat gacctcaatg ggaacctgca 6780
   ccttctaaac ccaggaaaca gtgctcgcct catgccctta cgctatgacc tccgtgaccg 6840
   gataaccagg ctaggggacg tgcagtacaa aatcgatgac gatggctatt tgtgccagag 6900
   agggtcagac atctttgaat acaactccaa gggccttctg acgagagcat acaacaaggc 6960
   cagcggatgg agcgtgcagt accgctatga cggagtgggc cgccgggctt cctacaagac 7020
   caacctgggc caccacctac agtacttcta ctccgacctc cacaacccca cacgtatcac 7080
   ccatgtttac aaccactcca actctgagat cacctcgctc tactatgacc tccagggcca 7140
   cctatttgcc atggagagca gtagtggtga agaatactat gtcgcctcag acaacacggg 7200
   gacccctctg gctgtgtaca gtatcaatgg cctcatgatc aagcaactgc agtacacagc 7260
   ctatggggag atctactatg actccaatcc agacttccag atggtcattg gcttccacgg 7320
   aggcctctat gaccccctca ccaagctcgt ccactttact caacgtgatt atgacgtgct 7380
   ggcaggacgg tggacgtccc ccgactacac catgtggagg aacgtgggca aggagccagc 7440
   ccccttcaac ctgtacatgt tcaagaacaa caatcctctg agcaatgagc tggacttaaa 7500
   gaactacgtg acagacgtga agagctggct tgtgatgttt ggatttcagc tcagcaacat 7560
   cattcctgga ttcccgagag ccaaaatgta ttttgtgcct cccccctatg aactgtcaga 7620
   gagtcaagca agcgagaacg gacagctcat tacaggtgtc cagcagacaa ctgagaggca 7680
   taaccaggcc ttcctggctc tggaaggaca ggtcatcact aaaaagctcc atgccagcat 7740
   ccgagagaaa gcaggccact ggtttgctac caccacaccc atcatcggca aaggcatcat 7800
   gtttgccatc aaagaagggc gggtgaccac aggagtgtct agcatcgcca gtgaggacag 7860
   ccgcaaggta gcatccgtgt tgaacaatgc ctactactta gacaagatgc actacagcat 7920
   cgagggcaag gacacacact actttgtgaa gatcggcgcc gcggatggtg acctggtcac 7980
   gctaggaacc accattgggc gcaaggtgct ggagagtggg gtgaacgtga cggtgtcaca 8040
   gcccacgctg ctggtgaatg gcaggactcg aaggttcacc aacattgagt tccagtactc 8100
   cacgctgctg ctcagtatcc gctacggcct cacccccgac acgctggacg aagaaaaggc 8160
   ccgcgtcctg gaccaagcgg gacagagagc cctgggtact gcctgggcca aggagcagca 8220
   gaaagccagg gacgggagag agggcagccg cctgtggacg gagggcgaga agcagcaact 8280
   cctgagcacg ggacgggtac aaggttatga gggctattac gtacttccgg tggaacagta 8340
   cccggagctg gcagacagta gcagcaacat ccagttctta agacagaatg agatggcaaa 8400
   gaggtaacaa aataacctgc tgccacctct tctctgggtg gctcagcagg agcaactgtg 8460
   acctcctctc ctaaggagac gaagacctaa cggggcactg aggccgggct gctttaggat 8520
   cccaagtggc aagaaagctc acattttttg agttcaaatg ctactgtcta agcgcaaagt 8580
   ccctcatcct gaagtagact agagcccggc cacaaatttc tgaggaaaaa caaaaactaa 8640
   aggatgaacg aacgaacgaa cgaatgaaaa cacacacaaa atgtttcaag ttcccctaaa 8700
   atatgaccca cttgttccgg gtctaaggca gaaaagagac gcagaatagc caaaaggaaa 8760
   ggaacagaaa agaaacaaat taaaaaaaaa aaaaaaa                        8797
<210> 66
   <211> 8689
   <212> DNA
   <213> Rattus norvegicus
<400> 66
   gtgagcattc ccggaaacgc agctgagttg ttggacaaca gacgtccacc cagtcggtgg 60
   gtaaagtgac aggtgccaca actccagcct ccccttttct gaaagtcgcg gagttccatt 120
   tcatctgcaa taatggatgt gaaggatcgg cgacatcgct ctttgaccag gggacggtgt 180
   ggcaaggagt gtcgctacac cagctcctct ctggacagtg aggactgccg tgtgcccacg 240
   cagaagtcct acagttccag tgagaccctg aaggcttatg accatgacag cagaatgcac 300
   tatggaaacc gagtcacaga cctggtgcac cgggagtccg atgagttttc tagacaaggg 360
   gctaatttca ccctggcaga attgggaatc tgcgagccct ccccacaccg aagtggttac 420
   tgttccgaca tggggatcct ccaccagggc tactccctga gcactgggtc tgatgcggac 480
   tcggacaccg agggagggat gtctccagaa catgccatca gactgtgggg acgagggata 540
   aaatcgaggc gcagctctgg cttgtccagc cgcgagaact cagcccttac tctgactgat 600
   tctgacaatg aaaataaatc ggatgacgac aatggtcgac ccattccacc tacatcctcg 660
   tctagcctcc tcccatctgc tcagctgcct agctcccata atcctccacc agttagctgc 720
   cagatgccat tgctagacag caacacctcc catcagatca tggacaccaa ccccgatgag 780
   gaattctccc ctaattcata cctgctcaga gcatgctcag ggccccagca agcctccagt 840
   agtggccctc cgaaccacca cagccagtca acgctgaggc cccctctgcc acctcctcat 900
   aaccacaccc tgtcccacca ccactcctct gccaactccc tcaacagaaa ctcactgacc 960
   aatcggcgga gtcaaatcca cgccccagct cctgcaccca atgacctggc caccacgccg 1020
   gagtccgttc agctccagga cagctgggtg ctgaacagta acgtgccgct ggagacgcgg 1080
   cacttcctct tcaagacgtc ctccggaagc acacccctgt tcagcagctc ttctccagga 1140
   taccccttga cctcagggac cgtttataca ccaccacccc gcctgctgcc acggaataca 1200
   ttctctagga aggccttcaa gctgaagaaa ccctccaaat actgcagttg gaaatgcgcc 1260
   gccctgtctg ccattgccgc tgccctcctt ctggccattt tgctggccta tttcatagca 1320
   atgcatctgc tcggactcaa ttggcaactc cagccggcag atggacacac ctttaacaat 1380
   ggcgtaagga ccggcttacc aggaaacgat gatgtggcaa cagtgccatc tggaggcaaa 1440
   gtgccctggt cgttgaaaaa cagcagcata gacagcggcg aggcagaagt cggtcgacgg 1500
   gtgacacagg aagtcccacc aggggtgttt tggaggtccc agattcacat cagtcagcct 1560
   cagttcttaa agttcaacat ctccctgggg aaggatgccc tcttcggcgt ctacataaga 1620
   agaggactgc caccatctca tgcacagtat gacttcatgg aacgcctgga cggaaaggag 1680
   aagtggagtg tggtcgagtc acccagggaa cgccggagca tccagaccct ggtgcagaac 1740
   gaggctgtgt tcgtgcagta cttggatgtg ggcctgtggc acctcgcctt ctacaatgac 1800
   ggcaaggaca aggagatggt ctccttcaat acggttgtct tagattcagt gcaggactgt 1860
   ccacgaaact gccacgggaa cggcgaatgc gtgtctggac tgtgtcactg tttcccagga 1920
   ttcctaggtg cagactgcgc taaagctgcc tgccctgttc tgtgcagtgg gaatggacag 1980
   tattccaaag ggacatgcca gtgctacagt ggctggaaag gagcagaatg cgatgtgccc 2040
   atgaaccagt gcatcgatcc ttcctgtggg ggccacggct cctgcattga tgggaactgc 2100
   gtgtgtgcag ctggctacaa gggcgagcac tgcgaagaag tggattgctt ggatccaacc 2160
   tgctccagcc atggtgtctg tgtgaacgga gagtgtctat gcagccccgg ctggggcggg 2220
   ctcaactgcg agctggcgag ggtccagtgc ccagaccagt gtagtgggca tggcacttac 2280
   ctccctgact ctggcctctg caactgtgat ccgaattgga tgggtcccga ctgctctgtt 2340
   gaagtgtgct cagtagactg tggcactcac ggcgtctgca tcgggggagc ctgccgctgt 2400
   gaagagggct ggacaggcgc ggcttgtgac cagcgcgtgt gccacccccg ctgcattgag 2460
   cacgggacct gtaaagatgg caaatgtgaa tgccgagagg gctggaatgg tgaacactgc 2520
   accattgatg gctgccctga tttgtgcaac ggtaacggga gatgcacact gggtcagaac 2580
   agctggcagt gtgtctgcca gaccggctgg agagggcccg gatgcaacgt tgccatggaa 2640
   acctcctgcg ctgataacaa ggataatgag ggagatggcc tggtggactg cctggacccc 2700
   gactgctgcc tccagtcagc ctgtcagaac agcctgctct gtcgggggtc tcgggacccc 2760
   ttggacatca ttcagcaagg ccagacagac tggcctgcgg tgaagtcctt ctatgatcgt 2820
   atcaagctct tggcaggcaa ggacagcacc cacatcattc ctggagacaa ccccttcaat 2880
   agcagcctgg tgtctctgat ccgaggccaa gtagtaacca cggatgggac ccccctggtg 2940
   ggtgtgaatg tgtcttttgt caagtaccca aaatatggct acaccatcac tcgccaggac 3000
   ggcacgtttg acctgattgc caatgggggc tctgccttga ctcttcactt tgagcgagcc 3060
   cctttcatga gccgggagcg cacagtatgg ccgccgtgga acagcttcta tgccatggac 3120
   accctggtaa tgaagacgga ggagaactcc atccccagct gtgacctcag tggctttgtc 3180
   cggcctgatc cgatcatcat ctcctctcct ctgtccacct tcttcagcgc ttcccctgcg 3240
   gcgaacccca ttgtgcctga gacccaggtt cttcatgagg agatcgagct ccctggcacc 3300
   aacgtgaagc tccgttacct cagctccaga acagcagggt acaagtcact gctgaagatc 3360
   accatgaccc agtccacggt gcccttgaac ctcatccggg ttcacttgat ggttgccgtg 3420
   gaggggcatc tcttccagaa gtcgttccag gcttctccca acctggccta cacattcatc 3480
   tgggacaaga cagacgctta tggccaaagg gtttatggcc tatcggatgc tgttgtgtct 3540
   gttggatttg aatatgagac ctgccccagt ctcatcctgt gggaaaaaag gacagcccta 3600
   cttcaaggat tcgagctgga cccttccaac cttggtggct ggtccctgga taagcaccac 3660
   accctcaatg tgaaaagcgg aatactactc aaaggcacag gggagaacca gttcctgacc 3720
   cagcagcccg ccatcatcac cagcatcatg ggtaacggtc gccgcagaag catctcctgt 3780
   cccagctgca atggccttgc tgaaggcaac aaactgttgg cccccgtggc cctggctgtg 3840
   gggatcgatg ggagcctctt tgtcggtgac ttcaattata tccggcgcat cttcccttct 3900
   cgaaacgtga ccagtatctt ggagttacga aataaagagt ttaaacatag caacagccca 3960
   ggacacaagt actacttggc tgtggaccct gtgactggct cgctctatgt ctctgacacc 4020
   aacagtcgcc ggatctaccg agtcaagtct ctaagcggag ccaaagacct ggctgggaat 4080
   tcggaagttg tggccgggac tggcgaacaa tgtctaccct ttgatgaagc ccgctgtggg 4140
   gatggcggga aggctgtgga tgccaccctg atgagcccta gaggtattgc agtagacaag 4200
   aacgggctta tgtattttgt tgatgccacc atgatccgga aggtcgacca aaatggaatc 4260
   atctccaccc tgctgggctc caatgacctc acagctgtcc gaccactgag ctgtgactct 4320
   agcatggacg tggcccaggt ccgtctagaa tggccgacag accttgcggt caaccccatg 4380
   gacaattccc tgtacgtcct ggagaacaac gtcatcctgc ggatcaccga gaatcaccag 4440
   gtcagcatca tcgcgggacg gcccatgcac tgccaggttc ccggcatcga ctactcgctc 4500
   agcaagctcg ccatccactc tgctctggag tcagccagcg ccatcgccat ttctcacacc 4560
   ggggtgctct acatcaccga gacggacgag aagaagatca accgcctacg ccaggtcacc 4620
   accaacggag agatctgcct cttagccggg gcagcctcag actgtgactg caaaaatgac 4680
   gtcaactgca tctgctattc gggagatgac gcatacgcca cggatgccat cttgaactcc 4740
   ccgtcctcct tagctgtggc tccggatggc accatctaca tcgcagacct cgggaatatc 4800
   cggatcaggg cggtcagcaa aaacaaacct gttcttaacg cgttcaacca gtatgaggct 4860
   gcgtctccgg gagaacagga actgtacgtg ttcaacgccg atggtatcca tcagtacacc 4920
   gtgagcctgg tgaccgggga gtacttatac aatttcacct acagcgctga caatgatgtc 4980
   accgagttga ttgacaacaa cgggaattcc ctaaagatcc gccgggacag cagtggcatg 5040
   ccccgacacc tgctcatgcc tgataatcag atcatcaccc ttacggtggg caccaacgga 5100
   ggcctcaaag ccgtgtcaac gcagaacctg gagctgggcc tcatgactta tgatgggaac 5160
   actggactcc tagccaccaa gagcgatgaa accggatgga caacttttta tgactatgac 5220
   cacgagggcc gtctgaccaa tgtgactcgc cccacggggg tggtgaccag cctgcaccgg 5280
   gaaatggaga aatccatcac cgttgacatt gagaactcca accgtgataa cgatgtcact 5340
   gtgattacca acctctcttc agtggaggcc tcctacaccg tggtacaaga tcaagtgcgg 5400
   aacagctacc agctctgcag caacgggacc ctgcgcgtca tgtacgccaa cggcatgggc 5460
   gtcagcttcc acagcgagcc ccacgtcctc gcaggcaccc tcacccccac catcgggcgc 5520
   tgtaacatct ccctgcccat ggagaacggc ctgaactcca tcgagtggcg cctgaggaag 5580
   gaacagatta aaggcaaagt caccatcttt gggaggaagc ttcgggtcca cggaaggaac 5640
   ctcctgtcca ttgattatga ccgaaatatc cgcactgaga agatctatga cgaccaccgg 5700
   aagttcaccc tgaggatcat ttatgaccag gtgggccgcc ccttcctgtg gctccccagc 5760
   agtggactgg cggccgtcaa tgtctcctac ttcttcaacg ggcgcctggc cggcctccag 5820
   cgcggggcca tgagcgagag gacagacatt gacaagcaag gccggattgt gtcccgaatg 5880
   ttcgccgacg ggaaagtctg gagctattcc taccttgaca agtccatggt cctcctgctg 5940
   cagagccagc gtcagtacat atttgaatat gactcctctg accgcctcca cgcagtcacc 6000
   atgcccagtg tcgcccggca cagcatgtcc acgcacacct ccattggcta catccggaac 6060
   atttacaacc caccggaaag caacgcctcg gtcatctttg actacagtga tgacggccgc 6120
   atcctgaaga cgtctttcct gggcaccggg cgccaggtgt tctataagta cggaaaactg 6180
   tccaagttat cggagatcgt ctacgacagc actgccgtca ccttcggcta tgacgagacc 6240
   actggcgtcc tgaagatggt gaatctccaa agcgggggct tctcctgtac catcaggtac 6300
   cgaaaggtcg ggcccctcgt ggacaagcag atttacaggt tctctgagga aggcatgatc 6360
   aacgccaggt tcgattacac ctaccacgac aacagcttcc gcatcgccag catcaagccc 6420
   gtcatcagtg agactcccct tcccgttgac ctctaccgct acgatgagat ttctggcaag 6480
   gtggaacact tcggcaagtt cggggtcatc tactacgaca tcaaccagat catcaccact 6540
   gccgtcatga cactcagcaa gcactttgac acccatgggc gcatcaagga agtgcagtat 6600
   gagatgttcc ggtccctcat gtactggatg acggtgcaat atgacagtat gggcagggtc 6660
   atcaagaggg aactgaaact ggggccctat gccaacacca caaagtacac ctatgactac 6720
   gacggggacg gccagctcca gagtgtggcc gtcaatgacc ggcctacctg gcgttatagc 6780
   tatgacctca atgggaacct gcacctgcta aacccaggaa acagtgctcg cctcatgccg 6840
   ttacgctatg acctccgtga ccggataacc aggctagggg acgtgcagta caaaatcgat 6900
   gatgatggct atttatgcca gagaggatct gacatctttg aatacaactc caagggcctt 6960
   ctaacgagag cgtacaacaa ggccagcggg tggagtgtgc agtaccgcta tgatggcgtg 7020
   agccgccggg cttcctacaa gaccaacctg ggccaccacc tacagtactt ctattccgac 7080
   ctccaccacc ccacacgtat cacccatgtt tacaaccact ccaactctga gatcacctca 7140
   ctctactatg acctccaggg ccacctcttt gccatggaga gcagtagtgg ggaagagtac 7200
   tatgttgcct cagataacac cgggactcct ctggctgttt ttagtatcaa tggcctcatg 7260
   atcaagcaac tccaatacac agcctatggg gagatttact atgactccaa tccagacttt 7320
   cagatggtca tcggcttcca cggaggcctc tacgaccccc tcaccaagct cgttcacttt 7380
   acgcagcgtg attatgacgt gctggcagga cggtggacgt cccccgacta caccatgtgg 7440
   aggaatgtgg gcaaggagcc agcccccttc aacctgtaca tgttcaagaa caacaatcca 7500
   ctcagtaatg agctggattt aaagaactac gtgacagacg tgaagagctg gctcgtgatg 7560
   tttggatttc agctcagcaa catcattcct ggattcccaa gagccaaaat gtattttgtg 7620
   cctcccccct atgaactgtc agagagccaa gcaagtgaga atggacagct cattacaggt 7680
   gtccagcaga caacagagag gcataaccag gcctttctgg ctctagaagg acaggtcatc 7740
   tctaaaaagc tccatgcagg catccgagag aaagcaggcc actggtttgc tacgaccacg 7800
   cccatcatcg gcaaaggcat catgttcgcc atcaaagaag ggcgggtgac cacaggcgtg 7860
   tctagcatcg ccagtgagga cagccgcaag gtagcatccg tgttgaacaa cgcctactac 7920
   ttggacaaga tgcactacag catcgagggc aaggacacac actacttcgt gaagatcggt 7980
   gcagcggacg gtgacctggt tacgctgggg accaccattg ggcgcaaggt gctggagagc 8040
   ggggtgaacg tgaccgtgtc acagcccacg ctgctggtga acggcaggac tcgaaggttc 8100
   accaacattg aattccagta ctccacgctg ctgctcagca tacgctacgg cctcaccccc 8160
   gacacactgg atgaagagaa ggcccgcgtc ctggaccaag cgcgacagag ggccctgggt 8220
   actgcctggg ccaaggagca gcagaaagcc agggacggga gagagggcag ccgtctgtgg 8280
   acggagggcg agaagcagca actcctgagc acgggacggg tgcaaggtta tgagggctat 8340
   tacgtgcttc cggtggaaca gtacccagag ctggcagaca gtagcagcaa catccagttc 8400
   ttaagacaga atgagatggg aaagaggtaa caaaataacc tgctgccacc tcttctctgg 8460
   gtggctcagc aggagcaact gtgacctcct ctcctaagga gacgaagacc taacaggggc 8520
   actgaggccg ggctgcttta ggaccccaag tggcaagaaa gctcacattt tttgagttca 8580
   aatgctactg tccaagcgca aagtccctca tcctgaagta gactagagct cggccacaaa 8640
   ttctgaggaa aacaaaacta aaggatgaac gaatgaacca acgaacgaa            8689
<210> 67
   <211> 8409
   <212> DNA
   <213> Gallus gallus
<400> 67
   atggatataa aagatcgaag acaccgctct ttgacgagag gccggtgcgg gaaggagtgt 60
   cgctatacta gttcttcact cgacagtgaa gactgcagag taccagctca gaagtcctac 120
   agctccagtg agaccctgaa agcatatggc catgacacga ggatgcacta cggaaatcga 180
   gtttcagacc tggttcacag ggagtcggat gagtttccaa ggcaaggaac gaacttcacc 240
   cttgcagaac tgggaatctg tgagccctct ccccatcgaa gtggctactg ctcggacata 300
   ggaatactcc atcaaggcta ttccttgagc actggctctg atgctgactc agacacggag 360
   ggcgggatgt ctccagagca cgcgatcagg ctgtggggaa gagggatcaa atccagccga 420
   agttctggcc tgtcaagtcg tgaaaactcg gctctcacgc tcactgactc cgacaatgag 480
   aacaagtcag atgaggaaaa cgattttcac acacaccttt ctgagaaatt gaaagacaga 540
   cagacaagct ggcagcagct ggctgagaca aagaactctc taatacgtcg tcccattcca 600
   cctacatcct cgtctagcct tctcccatct gctcagctgc ccagttctca taatcctcca 660
   ccagttagct gccagatgcc attgctagac agcaatacgt cccatcaaat catggacacc 720
   aatcctgacg aggagttctc tcctaattca tacctactaa gagcatgttc agggccacag 780
   caggcatcca gcagtggccc ttcaaaccat cacagccagt caacgctgag gccacctctc 840
   ccccctcctc acaaccactc gctgtcccat catcactcgt ctgccaactc cctcaacagg 900
   aactcgctca ccaaccgccg caaccagatc cacgcgcctg ctcccgctcc caatgacctg 960
   gcgaccacgc ctgagtctgt gcagctgcag gacagctggg tgctcaacag caacgtgccg 1020
   ctggagacca ggcatttctt gtttaagaca tcttctggaa cgactccgct gttcagtagc 1080
   tcttcccctg gctacccact gacctcagga acagtttata ctccacctcc caggctgtta 1140
   cctagaaata cattttccag gaatgcattc aagctgaaaa agccctccaa gtattgtagc 1200
   tggaaatgtg ctgctttatc tgcaattgct gctgcagtcc tgcttgccat cctgctagca 1260
   tatttcatag cgatgcacct cctggggctg aactggcagc tgcagcccgc ggacggacac 1320
   accttcagca acgggctgcg gccgggcgcg gcgggcgcgg aggacggagc ggcggcgcca 1380
   cctgcaggca gaggaccgtg ggtcactagg aatagcagca tagatagtgg agaaacagaa 1440
   gttggccgca aggtcaccca agaggtgccc cctggagtgt tctggcggtc tcagatccat 1500
   atcagccagc cacagttcct gaagttcaac atatccctag ggaaggatgc tcttttcggt 1560
   gtttatataa gaagaggact cccaccatca catgcacagt atgatttcat ggaacgcttg 1620
   gatgggaaag agaaatggag tgtggtggaa tccccacggg aacggcgaag tattcagact 1680
   cttgttcaga atgaggctgt gtttgttcag tacttggatg tgggtttgtg gcacctggcg 1740
   ttttacaatg atggcaagga caaagaagtg gtctccttca gtacagttat tttggattca 1800
   gtgcaagact gtccacgtaa ttgtcatggc aatggcgagt gtgtttctgg tgtctgccac 1860
   tgttttcccg gatttcatgg agcagattgt gctaaagctg cctgcccggt gctgtgcagt 1920
   ggcaatggtc agtactccaa aggaacctgc ttgtgctaca gtggctggaa aggtccggaa 1980
   tgtgatgtac ccatcagcca gtgtattgat ccctcgtgtg gaggtcatgg ttcctgcatc 2040
   gaagggaact gtgtctgttc cattggctat aaaggagaaa actgtgagga agttgattgc 2100
   ttagatccaa catgctccaa tcacggggtc tgtgtgaacg gagaatgtct ctgcagccca 2160
   ggctggggtg gaataaactg tgagcttccc agagcccagt gcccagacca gtgcagtggg 2220
   catggcacat acctgtctga caccggtctc tgtagctgcg atcccaactg gatgggtccc 2280
   gactgctccg ttgaagtgtg ctctgtagac tgtggcaccc atggggtgtg cattggcgga 2340
   gcgtgtcgct gtgaagaagg gtggacagga gtggcgtgtg accagcgtgt gtgtcatccc 2400
   cggtgtacag agcacggaac ttgtaaagat gggaaatgtg aatgcagaga gggctggaat 2460
   ggggagcact gcaccattgg taggcaaacg acaggcaccg aaacagatgg ctgccctgac 2520
   ttgtgcaatg gcaacgggag gtgcacgctg ggccagaaca gctggcagtg tgtctgccag 2580
   accggctgga gagggcctgg atgcaacgtt gccatggaaa cctcctgtgc cgataacaag 2640
   gataacgagg gagatggctt ggttgactgc ctagtcccag attgctgcct ccagtccact 2700
   tgtcaaaaca gcctgctgtg ccggggttcc cgcgatcctc ttgacatcat acaacagagc 2760
   cattctggtt caccagctgt gaagtcattc tatgatcgaa tcaagctctt agtggggaag 2820
   gacagcactc atatcattcc aggagaaaat cccttcaaca gcagccttgt gtctcttata 2880
   agaggccaag tggtgactac agatggaacg cctctagttg gggtcaacgt gtcatttgtc 2940
   aagtatccaa agtatggcta taccatcact cgtcaggatg gcatgtttga cttggttgct 3000
   aacggtggat catccctaac tttgcacttt gaacgggccc catttatgag tcaggaaagg 3060
   acagtatggc tgccgtggaa cagcttctat gccatggaca cgcttgtaat gaaaacagag 3120
   gagaactcca ttcccagctg tgatctcagt ggctttgtca gacctgatcc agtcatcatt 3180
   tcatcaccac tgtcaacttt cttcagtgat gctcctggcc gaaatcctat tgtaccagaa 3240
   acccaggttc ttcatgaaga aattgaggtc cctggctcaa gtataaagct gatctacctg 3300
   agttcccgta ctgctggata taagtcctta cttaagatca tcatgactca gtcacttgtg 3360
   ccactgaatc taatcaaagt tcatttgatg gtagcagtag aagggcatct atttcaaaaa 3420
   tcatttctgg catctcccaa cttggcttat acattcatct gggacaaaac agatgcatat 3480
   ggtcagaagg tttatgggtt gtcagatgct gtagtttctg tgggttttga atatgagact 3540
   tgtcccagtt tgattctgtg ggagaaaagg actgcgctgc tgcaaggatt tgagctagat 3600
   ccttccaatc taggaggatg gtctttggat aaacatcatg tactgaatgt caagagtggt 3660
   atattgcaca aaggcaatgg agaaaatcag tttctaactc agcagccagc tgtgataacc 3720
   agcattatgg ggaatgggcg ccgaagaagc atatcctgtc ctagctgcaa tggtcttgca 3780
   gaaggaaata agcttttggc ccctgtagca ctggcagtgg gaattgatgg aagcctcttt 3840
   gttggagatt ttaattacat tcggcgtatc ttcccatcca ggaatgtgac tagcatattg 3900
   gagctgagaa ataaagagtt taaacatagc aacaatcctg ctcacaaata ctatctggcc 3960
   gtggaccccg tttcgggctc cctgtacgta tcagacacca acagccgacg gatatacaaa 4020
   gtcaaatctc ttactggcac gaaagacctg gctggtaatt ctgaagtggt agcggggact 4080
   ggagagcaat gcctgccctt tgatgaagcc agatgtggag atggagggaa agcagtggac 4140
   gcaaccctaa tgagtcctcg aggaattgca gtggataagt atggactcat gtattttgtt 4200
   gatgccacta tgattcgaaa agtggatcag aatggaatta tatcaactct gctgggctcc 4260
   aatgacctaa ctgccgtccg acctctaagc tgtgattcca gcatggatgt cagccaggta 4320
   cggctggagt ggcctactga tctcgctgtc gatcccatgg acaactcact ttatgtccta 4380
   gagaacaatg ttattttacg gatcacagaa aaccatcaag ttagcattat tgctggacgc 4440
   cccatgcact gccaggttcc tggtatagac tactctctta gcaaactggc tattcattcc 4500
   gcacttgaat cagccagtgc cattgccatc tcacacacag gagttcttta catcagtgag 4560
   acagatgaaa aaaaaattaa tcggctacgc caggtaacta ccaatggaga aatatgcctt 4620
   cttgcagggg cagcttcaga ctgtgattgc aaaaatgatg tcaactgtaa ttgctattct 4680
   ggggatgatg ggtatgccac tgatgccatc ttaaattcac catcttcctt agctgtggcc 4740
   ccagatggta ccatctacat agctgatctc ggaaatatcc gcattagggc tgtcagtaaa 4800
   aacaggccca ttcttaattc ttttaaccaa tatgaagctg catctccagg agaacaggag 4860
   ctgtatgtct tcaatgctga tgggattcac cagtacactc tcagccttgt taccggggag 4920
   tacttgtaca atttcaccta tagcagtgat aacgatgtca ccgaggtgat ggacagcaat 4980
   ggcaactcct tgaaggtccg tcgggatgcc agcggaatgc cccgccattt actgatgcct 5040
   gataatcaga ttgtcacgct ggccgttggc actaatggtg gactcaaact agtctcaacg 5100
   cagaccctgg aacttggatt aatgacttat aacggaaaca gtggtctctt agcaacgaag 5160
   agtgatgaaa caggatggac aacattttat gactatgatc atgaagggcg cctgaccaat 5220
   gtaacacgtc ccactggagt ggtaactagc cttcatcgag aaatggaaaa gtctattacc 5280
   atcgacattg agaattctaa tcgggatgat gatgtcacgg tcatcacaaa tctctcctct 5340
   gtggaggctt cctatacagt tgttcaagat caagtgagga acagctacca gctctgtaat 5400
   aatggtactt tgagagtgat gtatgccaat ggcatgagta ttagctttca cagcgaacct 5460
   catgtcctgg ctgggacagt aactcccacc ataggacgat gtaatatttc tctaccaatg 5520
   gagaatggtt tgaactcaat tgaatggcgt ctgaggaaag aacagattaa aggcaaagtg 5580
   actgtgtttg gaagaaagct cagggttcat ggaaggaatt tgctgtccat tgattacgac 5640
   cggaatatac gcacagaaaa aatctacgat gatcaccgca agttcaccct gaggataatt 5700
   tacgatcagc tgggacggcc cttcctctgg ctgcccagca gcggcctggc tgccgtcaac 5760
   gtgtcctatt tcttcaacgg gcgcctggct gggcttcagc gcggagccat gagcgaaagg 5820
   acagacatcg acaagcaagg caggatcata tcgcgcatgt ttgcagatgg gaaggtttgg 5880
   agttacacct acctagaaaa atccatggta ctactgcttc agagccagcg gcagtacatc 5940
   tttgagtatg attcttcaga ccggctccat gctgttacta tgcctagtgt tgctcggcat 6000
   agcatgtcaa ctcacacgtc tgttggctac attaggaata tttataatcc tcctgaaagc 6060
   aacgcatcag tgatttttga ttacagtgat gatgggagga ttttgaaaac atcattttta 6120
   ggtactggtc gacaagtctt ttacaagtat ggaaagctat ccaaattatc tgaaattgtt 6180
   tatgacagta ctgcggttac ttttggatat gatgaaacta caggtgtcct aaaaatggtg 6240
   aatttgcaaa gtggaggatt ttcttgtaca atccgctatc gtaaaattgg ccctcttgtt 6300
   gacaaacaaa tctacagatt ctctgaagaa ggtatggtca atgcaaggtt tgattataca 6360
   tatcacgaca atagttttcg cattgcaagc atcaaaccca tcataagtga gactcctctt 6420
   ccagttgatc tttaccgtta tgatgagatt tctggcaaag ttgagcattt tggcaaattt 6480
   ggagttattt attatgatat aaatcaaatt attactacag cagttatgac actgagtaag 6540
   cactttgata cccacggacg cattaaagaa gttcaatatg agatgttccg atccctgatg 6600
   tactggatga ctgtgcaata tgacagcatg ggaagagtaa ctaaaagaga actgaaactt 6660
   gggccgtatg ccaacacaac caagtatacc tatgattatg atggagatgg gcaattgcaa 6720
   agcgtagcag taaatgatag gcctacctgg cgttacagtt atgacctgaa tggaaatctt 6780
   cacctcctga atcctggaaa cagtgttcga ttgatgcccc tgcgctacga cctcagagac 6840
   aggattacgc gcttaggtga cataccgtac aaaatcgatg atgacggatt cctgtgtcaa 6900
   cgaggctcag atgtatttga gtacaattcc aaaggacttt taacaagagc ttacaacaaa 6960
   gcaaatggat ggaacgttca gtaccgttac gacggacttg gccgaagggc ttcctgtaag 7020
   actaacctag gacatcatct acagtacttt tatgctgatc ttcacaatcc aacaagagta 7080
   acacatgtct acaatcattc caattcagaa attacctctc tgtattatga tctgcaaggc 7140
   cacctctttg caatggagag tagcagtggg gaagaatatt atgtcgcctc cgataacacg 7200
   ggcactccgc tagccgtatt cagcatcaat ggcctcatga tcaaacagct tcagtacact 7260
   gcatacggag agatttatta tgactcaaac cctgatttcc agctggttat tgggttccat 7320
   ggagggctgt atgatccttt aaccaaactc gtccatttta cccaaaggga ctacgatgtc 7380
   cttgctggac gctggacatc tcctgattac acaatgtgga aaaacattgg tagagaacct 7440
   gctcccttca atctgtacat gttcaagagt aacaaccctc tcagcaatga actggatcta 7500
   aagaattatg taacagatgt caaaagctgg ctggtgatgt tcggatttca gcttagcaac 7560
   attattcctg gcttccctag agcaaaaatg tactttgtgt cacctccata cgagctgact 7620
   gagagtcaag cgtgtgaaaa tggacagcta attacaggag tccagcagac aacagaaaga 7680
   cacaatcaag ctttcatggc tcttgaggga caggtcatat ctaaaagatt acatgccagt 7740
   attagagaaa aagcaggcca ctggtttgca acaagcactc ctattattgg gaaaggaatc 7800
   atgtttgctg tgaagaaagg ccgtgtaacc actggcattt ccagtatagc cacagacgat 7860
   agcagaaaaa ttgcctctgt ccttaacagt gctcactacc tggaaaaaat gcactacagc 7920
   atcgagggga aggatactca ctactttgtc aagataggct cagccgatag cgacctcgtc 7980
   accctcgcga tgaccagcgg gaggaaggtc ctggacagcg gagtaaacgt gaccgtctcc 8040
   cagccaaccc tccttatcaa cggaaggact cgacggttca caaacatcga gtttcagtat 8100
   tccaccctgc tgatcaacat ccgctacggg ctcaccgccg acacgctgga tgaggagaag 8160
   gcacgagtgc tagaccaggc tcggcagcga gccctggggt cggcctgggc caaagagcag 8220
   cagaaggcac gggatggccg cgagggcagc cgcgtatgga cagacggaga gaagcaacag 8280
   cttctgaaca cgggaagggt tcaaggttac gagggatatt atgtcttgcc tgtggagcag 8340
   tacccagagc tagcagacag tagcagcaac atccagtttt taagacagaa tgaaatggga 8400
   aagaggtaa                                                         8409
<210> 68
   <211> 2764
   <212> PRT
   <213> Drosophila melanogaster
<400> 68
   Met Asp Val Lys Asp Arg Arg His Arg Ser Leu Thr Arg Gly Arg Cys
   1 5 10 15
   Gly Lys Glu Cys Arg Tyr Thr Ser Ser Ser Leu Asp Ser Glu Asp Cys
   20 25 30
   Arg Val Pro Thr Gln Lys Ser Tyr Ser Ser Ser Glu Thr Leu Lys Ala
   35 40 45
   Tyr Asp His Asp Ser Arg Met His Tyr Gly Asn Arg Val Thr Asp Leu
   50 55 60
   Val His Arg Glu Ser Asp Glu Phe Ser Arg Gln Gly Thr Asn Phe Thr
   65 70 75 80
   Leu Ala Glu Leu Gly Ile Cys Glu Pro Ser Pro His Arg Ser Gly Tyr
   85 90 95
   Cys Ser Asp Met Gly Ile Leu His Gln Gly Tyr Ser Leu Ser Thr Gly
   100 105 110
   Ser Asp Ala Asp Ser Asp Thr Glu Gly Gly Met Ser Pro Glu His Ala
   115 120 125
   Ile Arg Leu Trp Gly Arg Gly Ile Lys Ser Arg Arg Ser Ser Gly Leu
   130 135 140
   Ser Ser Arg Glu Asn Ser Ala Leu Thr Leu Thr Asp Ser Asp Asn Glu
   145 150 155 160
   Asn Lys Ser Asp Asp Asp Asn Gly Arg Pro Ile Pro Pro Thr Ser Ser
   165 170 175
   Ser Ser Leu Leu Pro Ser Ala Gln Leu Pro Ser Ser His Asn Pro Pro
   180 185 190
   Pro Val Ser Cys Gln Met Pro Leu Leu Asp Ser Asn Thr Ser His Gln
   195 200 205
   Ile Met Asp Thr Asn Pro Asp Glu Glu Phe Ser Pro Asn Ser Tyr Leu
   210 215 220
   Leu Arg Ala Cys Ser Gly Pro Gln Gln Ala Ser Ser Ser Gly Pro Pro
   225 230 235 240
   Asn His His Ser Gln Ser Thr Leu Arg Pro Pro Leu Pro Pro Pro His
   245 250 255
   Asn His Thr Leu Ser His His His Ser Ser Ala Asn Ser Leu Asn Arg
   260 265 270
   Asn Ser Leu Thr Asn Arg Arg Ser Gln Ile His Ala Pro Ala Pro Ala
   275 280 285
   Pro Asn Asp Leu Ala Thr Thr Pro Glu Ser Val Gln Leu Gln Asp Ser
   290 295 300
   Trp Val Leu Asn Ser Asn Val Pro Leu Glu Thr Arg His Phe Leu Phe
   305 310 315 320
   Lys Thr Ser Ser Gly Ser Thr Pro Leu Phe Ser Ser Ser Ser Pro Gly
   325 330 335
   Tyr Pro Leu Thr Ser Gly Thr Val Tyr Thr Pro Pro Pro Arg Leu Leu
   340 345 350
   Pro Arg Asn Thr Phe Ser Arg Lys Ala Phe Lys Leu Lys Lys Pro Ser
   355 360 365
   Lys Tyr Cys Ser Trp Lys Cys Ala Ala Leu Ser Ala Ile Ala Ala Ala
   370 375 380
   Leu Leu Leu Ala Ile Leu Leu Ala Tyr Phe Ile Ala Met His Leu Leu
   385 390 395 400
   Gly Leu Asn Trp Gln Leu Gln Pro Ala Asp Gly His Thr Phe Asn Asn
   405 410 415
   Gly Val Arg Thr Gly Leu Pro Gly Asn Asp Asp Val Ala Thr Val Pro
   420 425 430
   Ser Gly Gly Lys Val Pro Trp Ser Leu Lys Asn Ser Ser Ile Asp Ser
   435 440 445
   Gly Glu Ala Glu Val Gly Arg Arg Val Thr Gln Glu Val Pro Pro Gly
   450 455 460
   Val Phe Trp Arg Ser Gln Ile His Ile Ser Gln Pro Gln Phe Leu Lys
   465 470 475 480
   Phe Asn Ile Ser Leu Gly Lys Asp Ala Leu Phe Gly Val Tyr Ile Arg
   485 490 495
   Arg Gly Leu Pro Pro Ser His Ala Gln Tyr Asp Phe Met Glu Arg Leu
   500 505 510
   Asp Gly Lys Glu Lys Trp Ser Val Val Glu Ser Pro Arg Glu Arg Arg
   515 520 525
   Ser Ile Gln Thr Leu Val Gln Asn Glu Ala Val Phe Val Gln Tyr Leu
   530 535 540
   Asp Val Gly Leu Trp His Leu Ala Phe Tyr Asn Asp Gly Lys Asp Lys
   545 550 555 560
   Glu Met Val Ser Phe Asn Thr Val Val Leu Asp Ser Val Gln Asp Cys
   565 570 575
   Pro Arg Asn Cys His Gly Asn Gly Glu Cys Val Ser Gly Leu Cys His
   580 585 590
   Cys Phe Pro Gly Phe Leu Gly Ala Asp Cys Ala Lys Ala Ala Cys Pro
   595 600 605
   Val Leu Cys Ser Gly Asn Gly Gln Tyr Ser Lys Gly Thr Cys Gln Cys
   610 615 620
   Tyr Ser Gly Trp Lys Gly Ala Glu Cys Asp Val Pro Met Asn Gln Cys
   625 630 635 640
   Ile Asp Pro Ser Cys Gly Gly His Gly Ser Cys Ile Asp Gly Asn Cys
   645 650 655
   Val Cys Ala Ala Gly Tyr Lys Gly Glu His Cys Glu Glu Val Asp Cys
   660 665 670
   Leu Asp Pro Thr Cys Ser Ser His Gly Val Cys Val Asn Gly Glu Cys
   675 680 685
   Leu Cys Ser Pro Gly Trp Gly Gly Leu Asn Cys Glu Leu Ala Arg Val
   690 695 700
   Gln Cys Pro Asp Gln Cys Ser Gly His Gly Thr Tyr Leu Pro Asp Ser
   705 710 715 720
   Gly Leu Cys Ser Cys Asp Pro Asn Trp Met Gly Pro Asp Cys Ser Val
   725 730 735
   Val Cys Ser Val Asp Cys Gly Thr His Gly Val Cys Ile Gly Gly Ala
   740 745 750
   Cys Arg Cys Glu Glu Gly Trp Thr Gly Ala Ala Cys Asp Gln Arg Val
   755 760 765
   Cys His Pro Arg Cys Ile Glu His Gly Thr Cys Lys Asp Gly Lys Cys
   770 775 780
   Glu Cys Arg Glu Gly Trp Asn Gly Glu His Cys Thr Ile Asp Gly Cys
   785 790 795 800
   Pro Asp Leu Cys Asn Gly Asn Gly Arg Cys Thr Leu Gly Gln Asn Ser
   805 810 815
   Trp Gln Cys Val Cys Gln Thr Gly Trp Arg Gly Pro Gly Cys Asn Val
   820 825 830
   Ala Met Glu Thr Ser Cys Ala Asp Asn Lys Asp Asn Glu Gly Asp Gly
   835 840 845
   Leu Val Asp Cys Leu Asp Pro Asp Cys Cys Leu Gln Ser Ala Cys Gln
   850 855 860
   Asn Ser Leu Leu Cys Arg Gly Ser Arg Asp Pro Leu Asp Ile Ile Gln
   865 870 875 880
   Gln Gly Gln Thr Asp Trp Pro Ala Val Lys Ser Phe Tyr Asp Arg Ile
   885 890 895
   Lys Leu Leu Ala Gly Lys Asp Ser Thr His Ile Ile Pro Gly Asp Asn
   900 905 910
   Pro Phe Asn Ser Ser Leu Val Ser Leu Ile Arg Gly Gln Val Val Thr
   915 920 925
   Met Asp Gly Thr Pro Leu Val Gly Val Asn Val Ser Phe Val Lys Tyr
   930 935 940
   Pro Lys Tyr Gly Tyr Thr Ile Thr Arg Gln Asp Gly Thr Phe Asp Leu
   945 950 955 960
   Ile Ala Asn Gly Gly Ser Ala Leu Thr Leu His Phe Glu Arg Ala Pro
   965 970 975
   Phe Met Ser Gln Glu Arg Thr Val Trp Leu Pro Trp Asn Ser Phe Tyr
   980 985 990
   Ala Met Asp Thr Leu Val Met Lys Thr Glu Glu Asn Ser Ile Pro Ser
   995 1000 1005
   Cys Asp Leu Ser Gly Phe Val Arg Pro Asp Pro Ile Ile Ile Ser Ser
   1010 1015 1020
   Pro Leu Ser Thr Phe Phe Ser Ala Ser Pro Ala Ser Asn Pro Ile Val
   1025 1030 1035 1040
   Pro Glu Thr Gln Val Leu His Glu Glu Ile Glu Leu Pro Gly Thr Asn
   1045 1050 1055
   Val Lys Leu Arg Tyr Leu Ser Ser Arg Thr Ala Gly Tyr Lys Ser Leu
   1060 1065 1070
   Leu Lys Ile Thr Met Thr Gln Ser Thr Val Pro Leu Asn Leu Ile Arg
   1075 1080 1085
   Val His Leu Met Val Ala Val Glu Gly His Leu Phe Gln Lys Ser Phe
   1090 1095 1100
   Gln Ala Ser Pro Asn Leu Ala Tyr Thr Phe Ile Trp Asp Lys Thr Asp
   1105 1110 1115 1120
   Ala Tyr Gly Gln Arg Val Tyr Gly Leu Ser Asp Ala Val Val Ser Val
   1125 1130 1135
   Gly Phe Glu Tyr Glu Thr Cys Pro Ser Leu Ile Leu Trp Glu Lys Arg
   1140 1145 1150
   Thr Ala Leu Leu Gln Gly Phe Glu Leu Asp Pro Ser Asn Leu Gly Gly
   1155 1160 1165
   Trp Ser Leu Asp Lys His His Thr Leu Asn Val Lys Ser Gly Ile Leu
   1170 1175 1180
   His Lys Gly Thr Gly Glu Asn Gln Phe Leu Thr Gln Gln Pro Ala Ile
   1185 1190 1195 1200
   Ile Thr Ser Ile Met Gly Asn Gly Arg Arg Arg Ser Ile Ser Cys Pro
   1205 1210 1215
   Ser Cys Asn Gly Leu Ala Glu Gly Asn Lys Leu Leu Ala Pro Val Ala
   1220 1225 1230
   Leu Ala Val Gly Ile Asp Gly Ser Leu Phe Val Gly Asp Phe Asn Tyr
   1235 1240 1245
   Ile Arg Arg Ile Phe Pro Ser Arg Asn Val Thr Ser Ile Leu Glu Leu
   1250 1255 1260
   Arg Asn Lys Glu Phe Lys His Ser Asn Ser Pro Gly His Lys Tyr Tyr
   1265 1270 1275 1280
   Leu Ala Val Asp Pro Val Thr Gly Ser Leu Tyr Val Ser Asp Thr Asn
   1285 1290 1295
   Ser Arg Arg Ile Tyr Arg Val Lys Ser Leu Ser Gly Ala Lys Asp Leu
   1300 1305 1310
   Ala Gly Asn Ser Glu Val Val Ala Gly Thr Gly Glu Gln Cys Leu Pro
   1315 1320 1325
   Phe Asp Glu Ala Arg Cys Gly Asp Gly Gly Lys Ala Val Asp Ala Thr
   1330 1335 1340
   Leu Met Ser Pro Arg Gly Ile Ala Val Asp Lys Asn Gly Leu Met Tyr
   1345 1350 1355 1360
   Phe Val Asp Ala Thr Met Ile Arg Lys Val Asp Gln Asn Gly Ile Ile
   1365 1370 1375
   Ser Thr Leu Leu Gly Ser Asn Asp Leu Thr Ala Val Arg Pro Leu Ser
   1380 1385 1390
   Cys Asp Ser Ser Met Asp Val Ala Gln Val Arg Leu Glu Trp Pro Thr
   1395 1400 1405
   Asp Leu Ala Val Asn Pro Met Asp Asn Ser Leu Tyr Val Leu Glu Asn
   1410 1415 1420
   Asn Val Ile Leu Arg Ile Thr Glu Asn His Gln Val Ser Ile Ile Ala
   1425 1430 1435 1440
   Gly Arg Pro Met His Cys Gln Val Pro Gly Ile Asp Tyr Ser Leu Ser
   1445 1450 1455
   Lys Leu Ala Ile His Ser Ala Leu Glu Ser Ala Ser Ala Ile Ala Ile
   1460 1465 1470
   Ser His Thr Gly Val Leu Tyr Ile Thr Glu Thr Asp Glu Lys Lys Ile
   1475 1480 1485
   Asn Arg Leu Arg Gln Val Thr Thr Asn Gly Glu Ile Cys Leu Leu Ala
   1490 1495 1500
   Gly Ala Ala Ser Asp Cys Asp Cys Lys Asn Asp Val Asn Cys Ile Cys
   1505 1510 1515 1520
   Tyr Ser Gly Asp Asp Ala Tyr Ala Thr Asp Ala Ile Leu Asn Ser Pro
   1525 1530 1535
   Ser Ser Leu Ala Val Ala Pro Asp Gly Thr Ile Tyr Ile Ala Asp Leu
   1540 1545 1550
   Gly Asn Ile Arg Ile Arg Ala Val Ser Lys Asn Lys Pro Val Leu Asn
   1555 1560 1565
   Ala Phe Asn Gln Tyr Glu Ala Ala Ser Pro Gly Glu Gln Glu Leu Tyr
   1570 1575 1580
   Val Phe Asn Ala Asp Gly Ile His Gln Tyr Thr Val Ser Leu Val Thr
   1585 1590 1595 1600
   Gly Glu Tyr Leu Tyr Asn Phe Thr Tyr Ser Ala Asp Asn Asp Val Thr
   1605 1610 1615
   Glu Leu Ile Asp Asn Asn Gly Asn Ser Leu Lys Ile Arg Arg Asp Ser
   1620 1625 1630
   Ser Gly Met Pro Arg His Leu Leu Met Pro Asp Asn Gln Ile Ile Thr
   1635 1640 1645
   Leu Thr Val Gly Thr Asn Gly Gly Leu Lys Ala Val Ser Thr Gln Asn
   1650 1655 1660
   Leu Glu Leu Gly Leu Met Thr Tyr Asp Gly Asn Thr Gly Leu Leu Ala
   1665 1670 1675 1680
   Thr Lys Ser Asp Glu Thr Gly Trp Thr Thr Phe Tyr Asp Tyr Asp His
   1685 1690 1695
   Glu Gly Arg Leu Thr Asn Val Thr Arg Pro Thr Gly Val Val Thr Ser
   1700 1705 1710
   Leu His Arg Glu Met Glu Lys Ser Ile Thr Ile Asp Ile Glu Asn Ser
   1715 1720 1725
   Asn Arg Asp Asp Asp Val Thr Val Ile Thr Asn Leu Ser Ser Val Glu
   1730 1735 1740
   Ala Ser Tyr Thr Val Val Gln Asp Gln Val Arg Asn Ser Tyr Gln Leu
   1745 1750 1755 1760
   Cys Asn Asn Gly Thr Leu Arg Val Met Tyr Ala Asn Gly Met Ala Val
   1765 1770 1775
   Ser Phe His Ser Glu Pro His Val Leu Ala Gly Thr Ile Thr Pro Thr
   1780 1785 1790
   Ile Gly Arg Cys Asn Ile Ser Leu Pro Met Glu Asn Gly Leu Asn Ser
   1795 1800 1805
   Ile Glu Trp Arg Leu Arg Lys Glu Gln Ile Lys Gly Lys Val Thr Ile
   1810 1815 1820
   Phe Gly Arg Lys Leu Arg Val His Gly Arg Asn Leu Leu Ser Ile Asp
   1825 1830 1835 1840
   Tyr Asp Arg Asn Ile Arg Thr Glu Lys Ile Tyr Asp Asp His Arg Lys
   1845 1850 1855
   Phe Thr Leu Arg Ile Ile Tyr Asp Gln Val Gly Arg Pro Phe Leu Trp
   1860 1865 1870
   Leu Pro Ser Ser Gly Leu Ala Ala Val Asn Val Ser Tyr Phe Phe Asn
   1875 1880 1885
   Gly Arg Leu Ala Gly Leu Gln Arg Gly Ala Met Ser Glu Arg Thr Asp
   1890 1895 1900
   Ile Asp Lys Gln Gly Arg Ile Val Ser Arg Met Phe Ala Asp Gly Lys
   1905 1910 1915 1920
   Val Trp Ser Tyr Ser Tyr Leu Asp Lys Ser Met Val Leu Leu Leu Gln
   1925 1930 1935
   Ser Gln Arg Gln Tyr Ile Phe Glu Tyr Asp Ser Ser Asp Arg Leu His
   1940 1945 1950
   Ala Val Thr Met Pro Ser Val Ala Arg His Ser Met Ser Thr His Thr
   1955 1960 1965
   Ser Ile Gly Tyr Ile Arg Asn Ile Tyr Asn Pro Pro Glu Ser Asn Ala
   1970 1975 1980
   Ser Val Ile Phe Asp Tyr Ser Asp Asp Gly Arg Ile Leu Lys Thr Ser
   1985 1990 1995 2000
   Phe Leu Gly Thr Gly Arg Gln Val Phe Tyr Lys Tyr Gly Lys Leu Ser
   2005 2010 2015
   Lys Leu Ser Glu Ile Val Tyr Asp Ser Thr Ala Val Thr Phe Gly Tyr
   2020 2025 2030
   Asp Glu Thr Thr Gly Val Leu Lys Met Val Asn Leu Gln Ser Gly Gly
   2035 2040 2045
   Phe Ser Cys Thr Ile Arg Tyr Arg Lys Val Gly Pro Leu Val Asp Lys
   2050 2055 2060
   Gln Ile Tyr Arg Phe Ser Glu Glu Gly Met Ile Asn Ala Arg Phe Asp
   2065 2070 2075 2080
   Tyr Thr Tyr His Asp Asn Ser Phe Arg Ile Ala Ser Ile Lys Pro Val
   2085 2090 2095
   Ile Ser Glu Thr Pro Leu Pro Val Asp Leu Tyr Arg Tyr Asp Glu Ile
   2100 2105 2110
   Ser Gly Lys Val Glu His Phe Gly Lys Phe Gly Val Ile Tyr Tyr Asp
   2115 2120 2125
   Ile Asn Gln Ile Ile Thr Thr Ala Val Met Thr Leu Ser Lys His Phe
   2130 2135 2140
   Asp Thr His Gly Arg Ile Lys Glu Val Gln Tyr Glu Met Phe Arg Ser
   2145 2150 2155 2160
   Leu Met Tyr Trp Met Thr Val Gln Tyr Asp Ser Met Gly Arg Val Ile
   2165 2170 2175
   Lys Arg Glu Leu Lys Leu Gly Pro Tyr Ala Asn Thr Thr Lys Tyr Thr
   2180 2185 2190
   Tyr Asp Tyr Asp Gly Asp Gly Gln Leu Gln Ser Val Ala Val Asn Asp
   2195 2200 2205
   Arg Pro Thr Trp Arg Tyr Ser Tyr Asp Leu Asn Gly Asn Leu His Leu
   2210 2215 2220
   Leu Asn Pro Gly Asn Ser Ala Arg Leu Met Pro Leu Arg Tyr Asp Leu
   2225 2230 2235 2240
   Arg Asp Arg Ile Thr Arg Leu Gly Asp Val Gln Tyr Lys Ile Asp Asp
   2245 2250 2255
   Asp Gly Tyr Leu Cys Gln Arg Gly Ser Asp Ile Phe Glu Tyr Asn Ser
   2260 2265 2270
   Lys Gly Leu Leu Thr Arg Ala Tyr Asn Lys Ala Ser Gly Trp Ser Val
   2275 2280 2285
   Gln Tyr Arg Tyr Asp Gly Val Gly Arg Arg Ala Ser Tyr Lys Thr Asn
   2290 2295 2300
   Leu Gly His His Leu Gln Tyr Phe Tyr Ser Asp Leu His Asn Pro Thr
   2305 2310 2315 2320
   Arg Ile Thr His Val Tyr Asn His Ser Asn Ser Glu Ile Thr Ser Leu
   2325 2330 2335
   Tyr Tyr Asp Leu Gln Gly His Leu Phe Ala Met Glu Ser Ser Ser Gly
   2340 2345 2350
   Glu Glu Tyr Tyr Val Ala Ser Asp Asn Thr Gly Thr Pro Leu Ala Val
   2355 2360 2365
   Tyr Ser Ile Asn Gly Leu Met Ile Lys Gln Leu Gln Tyr Thr Ala Tyr
   2370 2375 2380
   Gly Glu Ile Tyr Tyr Asp Ser Asn Pro Asp Phe Gln Met Val Ile Gly
   2385 2390 2395 2400
   Phe His Gly Gly Leu Tyr Asp Pro Leu Thr Lys Leu Val His Phe Thr
   2405 2410 2415
   Gln Arg Asp Tyr Asp Val Leu Ala Gly Arg Trp Thr Ser Pro Asp Tyr
   2420 2425 2430
   Thr Met Trp Arg Asn Val Gly Lys Glu Pro Ala Pro Phe Asn Leu Tyr
   2435 2440 2445
   Met Phe Lys Asn Asn Asn Pro Leu Ser Asn Glu Leu Asp Leu Lys Asn
   2450 2455 2460
   Tyr Val Thr Asp Val Lys Ser Trp Leu Val Met Phe Gly Phe Gln Leu
   2465 2470 2475 2480
   Ser Asn Ile Ile Pro Gly Phe Pro Arg Ala Lys Met Tyr Phe Val Pro
   2485 2490 2495
   Pro Pro Tyr Glu Leu Ser Glu Ser Gln Ala Ser Glu Asn Gly Gln Leu
   2500 2505 2510
   Ile Thr Gly Val Gln Gln Thr Thr Glu Arg His Asn Gln Ala Phe Leu
   2515 2520 2525
   Ala Leu Glu Gly Gln Val Ile Thr Lys Lys Leu His Ala Ser Ile Arg
   2530 2535 2540
   Glu Lys Ala Gly His Trp Phe Ala Thr Thr Thr Pro Ile Ile Gly Lys
   2545 2550 2555 2560
   Gly Ile Met Phe Ala Ile Lys Glu Gly Arg Val Thr Thr Gly Val Ser
   2565 2570 2575
   Ser Ile Ala Ser Glu Asp Ser Arg Lys Val Ala Ser Val Leu Asn Asn
   2580 2585 2590
   Ala Tyr Tyr Leu Asp Lys Met His Tyr Ser Ile Glu Gly Lys Asp Thr
   2595 2600 2605
   His Tyr Phe Val Lys Ile Gly Ala Ala Asp Gly Asp Leu Val Thr Leu
   2610 2615 2620
   Gly Thr Thr Ile Gly Arg Lys Val Leu Glu Ser Gly Val Asn Val Thr
   2625 2630 2635 2640
   Val Ser Gln Pro Thr Leu Leu Val Asn Gly Arg Thr Arg Arg Phe Thr
   2645 2650 2655
   Asn Ile Glu Phe Gln Tyr Ser Thr Leu Leu Leu Ser Ile Arg Tyr Gly
   2660 2665 2670
   Leu Thr Pro Asp Thr Leu Asp Glu Glu Lys Ala Arg Val Leu Asp Gln
   2675 2680 2685
   Ala Gly Gln Arg Ala Leu Gly Thr Ala Trp Ala Lys Glu Gln Gln Lys
   2690 2695 2700
   Ala Arg Asp Gly Arg Glu Gly Ser Arg Leu Trp Thr Glu Gly Glu Lys
   2705 2710 2715 2720
   Gln Gln Leu Leu Ser Thr Gly Arg Val Gln Gly Tyr Glu Gly Tyr Tyr
   2725 2730 2735
   Val Leu Pro Val Glu Gln Tyr Pro Glu Leu Ala Asp Ser Ser Ser Asn
   2740 2745 2750
   Ile Gln Phe Leu Arg Gln Asn Glu Met Gly Lys Arg
   2755 2760
<210> 69
   <211> 2802
   <212> PRT
   <213> Gallus gallus
<400> 69
   Met Asp Ile Lys Asp Arg Arg His Arg Ser Leu Thr Arg Gly Arg Cys
    1              5                  10                  15
   Gly Lys Glu Cys Arg Tyr Thr Ser Ser Ser Leu Asp Ser Glu Asp Cys
               20                  25                  30
   Arg Val Pro Ala Gln Lys Ser Tyr Ser Ser Ser Glu Thr Leu Lys Ala
           35                  40                  45
   Tyr Gly His Asp Thr Arg Met His Tyr Gly Asn Arg Val Ser Asp Leu
       50                  55                  60
   Val His Arg Glu Ser Asp Glu Phe Pro Arg Gln Gly Thr Asn Phe Thr
   65                  70                  75                  80
   Leu Ala Glu Leu Gly Ile Cys Glu Pro Ser Pro His Arg Ser Gly Tyr
                   85                  90                  95
   Cys Ser Asp Ile Gly Ile Leu His Gln Gly Tyr Ser Leu Ser Thr Gly
               100                 105                 110
   Ser Asp Ala Asp Ser Asp Thr Glu Gly Gly Met Ser Pro Glu His Ala
           115                 120                 125
   Ile Arg Leu Trp Gly Arg Gly Ile Lys Ser Ser Arg Ser Ser Gly Leu
       130                 135                 140
   Ser Ser Arg Glu Asn Ser Ala Leu Thr Leu Thr Asp Ser Asp Asn Glu
   145                 150                 155                 160
   Asn Lys Ser Asp Glu Glu Asn Asp Phe His Thr His Leu Ser Glu Lys
                   165                 170                 175
   Leu Lys Asp Arg Gln Thr Ser Trp Gln Gln Leu Ala Glu Thr Lys Asn
               180                 185                 190
   Ser Leu Ile Arg Arg Pro Ile Pro Pro Thr Ser Ser Ser Ser Leu Leu
           195                 200                 205
   Pro Ser Ala Gln Leu Pro Ser Ser His Asn Pro Pro Pro Val Ser Cys
       210                 215                 220
   Gln Met Pro Leu Leu Asp Ser Asn Thr Ser His Gln Ile Met Asp Thr
   225                 230                 235                 240
   Asn Pro Asp Glu Glu Phe Ser Pro Asn Ser Tyr Leu Leu Arg Ala Cys
   245 250 255
   Ser Gly Pro Gln Gln Ala Ser Ser Ser Gly Pro Ser Asn His His Ser
   260 265 270
   Gln Ser Thr Leu Arg Pro Pro Leu Pro Pro Pro His Asn His Ser Leu
   275 280 285
   Ser His His His Ser Ser Ala Asn Ser Leu Asn Arg Asn Ser Leu Thr
   290 295 300
   Asn Arg Arg Asn Gln Ile His Ala Pro Ala Pro Ala Pro Asn Asp Leu
   305 310 315 320
   Ala Thr Thr Pro Glu Ser Val Gln Leu Gln Asp Ser Trp Val Leu Asn
   325 330 335
   Ser Asn Val Pro Leu Glu Thr Arg His Phe Leu Phe Lys Thr Ser Ser
   340 345 350
   Gly Thr Thr Pro Leu Phe Ser Ser Ser Ser Pro Gly Tyr Pro Leu Thr
   355 360 365
   Ser Gly Thr Val Tyr Thr Pro Pro Pro Arg Leu Leu Pro Arg Asn Thr
   370 375 380
   Phe Ser Arg Asn Ala Phe Lys Leu Lys Lys Pro Ser Lys Tyr Cys Ser
   385 390 395 400
   Trp Lys Cys Ala Ala Leu Ser Ala Ile Ala Ala Ala Val Leu Leu Ala
   405 410 415
   Ile Leu Leu Ala Tyr Phe Ile Ala Met His Leu Leu Gly Leu Asn Trp
   420 425 430
   Gln Leu Gln Pro Ala Asp Gly His Thr Phe Ser Asn Gly Leu Arg Pro
   435 440 445
   Gly Ala Ala Gly Ala Glu Asp Gly Ala Ala Ala Pro Pro Ala Gly Arg
   450 455 460
   Gly Pro Trp Val Thr Arg Asn Ser Ser Ile Asp Ser Gly Glu Thr Glu
   465 470 475 480
   Val Gly Arg Lys Val Thr Gln Glu Val Pro Pro Gly Val Phe Trp Arg
   485 490 495
   Ser Gln Ile His Ile Ser Gln Pro Gln Phe Leu Lys Phe Asn Ile Ser
               500                 505                 510
   Leu Gly Lys Asp Ala Leu Phe Gly Val Tyr Ile Arg Arg Gly Leu Pro
           515                 520                 525
   Pro Ser His Ala Gln Tyr Asp Phe Met Glu Arg Leu Asp Gly Lys Glu
       530                 535                 540
   Lys Trp Ser Val Val Glu Ser Pro Arg Glu Arg Arg Ser Ile Gln Thr
   545                 550                 555                 560
   Leu Val Gln Asn Glu Ala Val Phe Val Gln Tyr Leu Asp Val Gly Leu
                   565                 570                 575
   Trp His Leu Ala Phe Tyr Asn Asp Gly Lys Asp Lys Glu Val Val Ser
               580                 585                 590
   Phe Ser Thr Val Ile Leu Asp Ser Val Gln Asp Cys Pro Arg Asn Cys
           595                 600                 605
   His Gly Asn Gly Glu Cys Val Ser Gly Val Cys His Cys Phe Pro Gly
       610                 615                 620
   Phe His Gly Ala Asp Cys Ala Lys Ala Ala Cys Pro Val Leu Cys Ser
   625                 630                 635                 640
   Gly Asn Gly Gln Tyr Ser Lys Gly Thr Cys Leu Cys Tyr Ser Gly Trp
                   645                 650                 655
   Lys Gly Pro Glu Cys Asp Val Pro Ile Ser Gln Cys Ile Asp Pro Ser
               660                 665                 670
   Cys Gly Gly His Gly Ser Cys Ile Glu Gly Asn Cys Val Cys Ser Ile
           675                 680                 685
   Gly Tyr Lys Gly Glu Asn Cys Glu Glu Val Asp Cys Leu Asp Pro Thr
       690                 695                 700
   Cys Ser Asn His Gly Val Cys Val Asn Gly Glu Cys Leu Cys Ser Pro
   705                 710                 715                 720
   Gly Trp Gly Gly Ile Asn Cys Glu Leu Pro Arg Ala Gln Cys Pro Asp
                   725                 730                 735
   Gln Cys Ser Gly His Gly Thr Tyr Leu Ser Asp Thr Gly Leu Cys Ser
               740                 745                 750
   Cys Asp Pro Asn Trp Met Gly Pro Asp Cys Ser Val Glu Val Cys Ser
   755 760 765
   Val Asp Cys Gly Thr His Gly Val Cys Ile Gly Gly Ala Cys Arg Cys
   770 775 780
   Glu Glu Gly Trp Thr Gly Val Ala Cys Asp Gln Arg Val Cys His Pro
   785 790 795 800
   Arg Cys Thr Glu His Gly Thr Cys Lys Asp Gly Lys Cys Glu Cys Arg
   805 810 815
   Glu Gly Trp Asn Gly Glu His Cys Thr Ile Gly Arg Gln Thr Thr Gly
   820 825 830
   Thr Glu Thr Asp Gly Cys Pro Asp Leu Cys Asn Gly Asn Gly Arg Cys
   835 840 845
   Thr Leu Gly Gln Asn Ser Trp Gln Cys Val Cys Gln Thr Gly Trp Arg
   850 855 860
   Gly Pro Gly Cys Asn Val Ala Met Glu Thr Ser Cys Ala Asp Asn Lys
   865 870 875 880
   Asp Asn Glu Gly Asp Gly Leu Val Asp Cys Leu Val Pro Asp Cys Cys
   885 890 895
   Leu Gln Ser Thr Cys Gln Asn Ser Leu Leu Cys Arg Gly Ser Arg Asp
   900 905 910
   Pro Leu Asp Ile Ile Gln Gln Ser His Ser Gly Ser Pro Ala Val Lys
   915 920 925
   Ser Phe Tyr Asp Arg Ile Lys Leu Leu Val Gly Lys Asp Ser Thr His
   930 935 940
   Ile Ile Pro Gly Glu Asn Pro Phe Asn Ser Ser Leu Val Ser Leu Ile
   945 950 955 960
   Arg Gly Gln Val Val Thr Thr Asp Gly Thr Pro Leu Val Gly Val Asn
   965 970 975
   Val Ser Phe Val Lys Tyr Pro Lys Tyr Gly Tyr Thr Ile Thr Arg Gln
   980 985 990
   Asp Gly Met Phe Asp Leu Val Ala Asn Gly Gly Ser Ser Leu Thr Leu
   995 1000 1005
   His Phe Glu Arg Ala Pro Phe Met Ser Gln Glu Arg Thr Val Trp Leu
   1010 1015 1020
   Pro Trp Asn Ser Phe Tyr Ala Met Asp Thr Leu Val Met Lys Thr Glu
   1025 1030 1035 1040
   Glu Asn Ser Ile Pro Ser Cys Asp Leu Ser Gly Phe Val Arg Pro Asp
   1045 1050 1055
   Pro Val Ile Ile Ser Ser Pro Leu Ser Thr Phe Phe Ser Asp Ala Pro
   1060 1065 1070
   Gly Arg Asn Pro Ile Val Pro Glu Thr Gln Val Leu His Glu Glu Ile
   1075 1080 1085
   Glu Val Pro Gly Ser Ser Ile Lys Leu Ile Tyr Leu Ser Ser Arg Thr
   1090 1095 1100
   Ala Gly Tyr Lys Ser Leu Leu Lys Ile Ile Met Thr Gln Ser Leu Val
   1105 1110 1115 1120
   Pro Leu Asn Leu Ile Lys Val His Leu Met Val Ala Val Glu Gly His
   1125 1130 1135
   Leu Phe Gln Lys Ser Phe Leu Ala Ser Pro Asn Leu Ala Tyr Thr Phe
   1140 1145 1150
   Ile Trp Asp Lys Thr Asp Ala Tyr Gly Gln Lys Val Tyr Gly Leu Ser
   1155 1160 1165
   Asp Ala Val Val Ser Val Gly Phe Glu Tyr Glu Thr Cys Pro Ser Leu
   1170 1175 1180
   Ile Leu Trp Glu Lys Arg Thr Ala Leu Leu Gln Gly Phe Glu Leu Asp
   1185 1190 1195 1200
   Pro Ser Asn Leu Gly Gly Trp Ser Leu Asp Lys His His Val Leu Asn
   1205 1210 1215
   Val Lys Ser Gly Ile Leu His Lys Gly Asn Gly Glu Asn Gln Phe Leu
   1220 1225 1230
   Thr Gln Gln Pro Ala Val Ile Thr Ser Ile Met Gly Asn Gly Arg Arg
   1235 1240 1245
   Arg Ser Ile Ser Cys Pro Ser Cys Asn Gly Leu Ala Glu Gly Asn Lys
   1250 1255 1260
   Leu Leu Ala Pro Val Ala Leu Ala Val Gly Ile Asp Gly Ser Leu Phe
   1265 1270 1275 1280
   Val Gly Asp Phe Asn Tyr Ile Arg Arg Ile Phe Pro Ser Arg Asn Val
   1285 1290 1295
   Thr Ser Ile Leu Glu Leu Arg Asn Lys Glu Phe Lys His Ser Asn Asn
   1300 1305 1310
   Pro Ala His Lys Tyr Tyr Leu Ala Val Asp Pro Val Ser Gly Ser Leu
   1315 1320 1325
   Tyr Val Ser Asp Thr Asn Ser Arg Arg Ile Tyr Lys Val Lys Ser Leu
   1330 1335 1340
   Thr Gly Thr Lys Asp Leu Ala Gly Asn Ser Glu Val Val Ala Gly Thr
   1345 1350 1355 1360
   Gly Glu Gln Cys Leu Pro Phe Asp Glu Ala Arg Cys Gly Asp Gly Gly
   1365 1370 1375
   Lys Ala Val Asp Ala Thr Leu Met Ser Pro Arg Gly Ile Ala Val Asp
   1380 1385 1390
   Lys Tyr Gly Leu Met Tyr Phe Val Asp Ala Thr Met Ile Arg Lys Val
   1395 1400 1405
   Asp Gln Asn Gly Ile Ile Ser Thr Leu Leu Gly Ser Asn Asp Leu Thr
   1410 1415 1420
   Ala Val Arg Pro Leu Ser Cys Asp Ser Ser Met Asp Val Ser Gln Val
   1425 1430 1435 1440
   Arg Leu Glu Trp Pro Thr Asp Leu Ala Val Asp Pro Met Asp Asn Ser
   1445 1450 1455
   Leu Tyr Val Leu Glu Asn Asn Val Ile Leu Arg Ile Thr Glu Asn His
   1460 1465 1470
   Gln Val Ser Ile Ile Ala Gly Arg Pro Met His Cys Gln Val Pro Gly
   1475 1480 1485
   Ile Asp Tyr Ser Leu Ser Lys Leu Ala Ile His Ser Ala Leu Glu Ser
   1490 1495 1500
   Ala Ser Ala Ile Ala Ile Ser His Thr Gly Val Leu Tyr Ile Ser Glu
   1505 1510 1515 1520
   Thr Asp Glu Lys Lys Ile Asn Arg Leu Arg Gln Val Thr Thr Asn Gly
   1525 1530 1535
   Glu Ile Cys Leu Leu Ala Gly Ala Ala Ser Asp Cys Asp Cys Lys Asn
   1540 1545 1550
   Asp Val Asn Cys Asn Cys Tyr Ser Gly Asp Asp Gly Tyr Ala Thr Asp
   1555 1560 1565
   Ala Ile Leu Asn Ser Pro Ser Ser Leu Ala Val Ala Pro Asp Gly Thr
   1570 1575 1580
   Ile Tyr Ile Ala Asp Leu Gly Asn Ile Arg Ile Arg Ala Val Ser Lys
   1585 1590 1595 1600
   Asn Arg Pro Ile Leu Asn Ser Phe Asn Gln Tyr Glu Ala Ala Ser Pro
   1605 1610 1615
   Gly Glu Gln Glu Leu Tyr Val Phe Asn Ala Asp Gly Ile His Gln Tyr
   1620 1625 1630
   Thr Leu Ser Leu Val Thr Gly Glu Tyr Leu Tyr Asn Phe Thr Tyr Ser
   1635 1640 1645
   Ser Asp Asn Asp Val Thr Glu Val Met Asp Ser Asn Gly Asn Ser Leu
   1650 1655 1660
   Lys Val Arg Arg Asp Ala Ser Gly Met Pro Arg His Leu Leu Met Pro
   1665 1670 1675 1680
   Asp Asn Gln Ile Val Thr Leu Ala Val Gly Thr Asn Gly Gly Leu Lys
   1685 1690 1695
   Leu Val Ser Thr Gln Thr Leu Glu Leu Gly Leu Met Thr Tyr Asn Gly
   1700 1705 1710
   Asn Ser Gly Leu Leu Ala Thr Lys Ser Asp Glu Thr Gly Trp Thr Thr
   1715 1720 1725
   Phe Tyr Asp Tyr Asp His Glu Gly Arg Leu Thr Asn Val Thr Arg Pro
   1730 1735 1740
   Thr Gly Val Val Thr Ser Leu His Arg Glu Met Glu Lys Ser Ile Thr
   1745 1750 1755 1760
   Ile Asp Ile Glu Asn Ser Asn Arg Asp Asp Asp Val Thr Val Ile Thr
   1765 1770 1775
   Asn Leu Ser Ser Val Glu Ala Ser Tyr Thr Val Val Gln Asp Gln Val
   1780 1785 1790
   Arg Asn Ser Tyr Gln Leu Cys Asn Asn Gly Thr Leu Arg Val Met Tyr
   1795 1800 1805
   Ala Asn Gly Met Ser Ile Ser Phe His Ser Glu Pro His Val Leu Ala
   1810 1815 1820
   Gly Thr Val Thr Pro Thr Ile Gly Arg Cys Asn Ile Ser Leu Pro Met
   1825 1830 1835 1840
   Glu Asn Gly Leu Asn Ser Ile Glu Trp Arg Leu Arg Lys Glu Gln Ile
   1845 1850 1855
   Lys Gly Lys Val Thr Val Phe Gly Arg Lys Leu Arg Val His Gly Arg
   1860 1865 1870
   Asn Leu Leu Ser Ile Asp Tyr Asp Arg Asn Ile Arg Thr Glu Lys Ile
   1875 1880 1885
   Tyr Asp Asp His Arg Lys Phe Thr Leu Arg Ile Ile Tyr Asp Gln Leu
   1890 1895 1900
   Gly Arg Pro Phe Leu Trp Leu Pro Ser Ser Gly Leu Ala Ala Val Asn
   1905 1910 1915 1920
   Val Ser Tyr Phe Phe Asn Gly Arg Leu Ala Gly Leu Gln Arg Gly Ala
   1925 1930 1935
   Met Ser Glu Arg Thr Asp Ile Asp Lys Gln Gly Arg Ile Ile Ser Arg
   1940 1945 1950
   Met Phe Ala Asp Gly Lys Val Trp Ser Tyr Thr Tyr Leu Glu Lys Ser
   1955 1960 1965
   Met Val Leu Leu Leu Gln Ser Gln Arg Gln Tyr Ile Phe Glu Tyr Asp
   1970 1975 1980
   Ser Ser Asp Arg Leu His Ala Val Thr Met Pro Ser Val Ala Arg His
   1985 1990 1995 2000
   Ser Met Ser Thr His Thr Ser Val Gly Tyr Ile Arg Asn Ile Tyr Asn
   2005 2010 2015
   Pro Pro Glu Ser Asn Ala Ser Val Ile Phe Asp Tyr Ser Asp Asp Gly
   2020 2025 2030
   Arg Ile Leu Lys Thr Ser Phe Leu Gly Thr Gly Arg Gln Val Phe Tyr
   2035 2040 2045
   Lys Tyr Gly Lys Leu Ser Lys Leu Ser Glu Ile Val Tyr Asp Ser Thr
   2050 2055 2060
   Ala Val Thr Phe Gly Tyr Asp Glu Thr Thr Gly Val Leu Lys Met Val
   2065 2070 2075 2080
   Asn Leu Gln Ser Gly Gly Phe Ser Cys Thr Ile Arg Tyr Arg Lys Ile
   2085 2090 2095
   Gly Pro Leu Val Asp Lys Gln Ile Tyr Arg Phe Ser Glu Glu Gly Met
   2100 2105 2110
   Val Asn Ala Arg Phe Asp Tyr Thr Tyr His Asp Asn Ser Phe Arg Ile
   2115 2120 2125
   Ala Ser Ile Lys Pro Ile Ile Ser Glu Thr Pro Leu Pro Val Asp Leu
   2130 2135 2140
   Tyr Arg Tyr Asp Glu Ile Ser Gly Lys Val Glu His Phe Gly Lys Phe
   2145 2150 2155 2160
   Gly Val Ile Tyr Tyr Asp Ile Asn Gln Ile Ile Thr Thr Ala Val Met
   2165 2170 2175
   Thr Leu Ser Lys His Phe Asp Thr His Gly Arg Ile Lys Glu Val Gln
   2180 2185 2190
   Tyr Glu Met Phe Arg Ser Leu Met Tyr Trp Met Thr Val Gln Tyr Asp
   2195 2200 2205
   Ser Met Gly Arg Val Thr Lys Arg Glu Leu Lys Leu Gly Pro Tyr Ala
   2210 2215 2220
   Asn Thr Thr Lys Tyr Thr Tyr Asp Tyr Asp Gly Asp Gly Gln Leu Gln
   2225 2230 2235 2240
   Ser Val Ala Val Asn Asp Arg Pro Thr Trp Arg Tyr Ser Tyr Asp Leu
   2245 2250 2255
   Asn Gly Asn Leu His Leu Leu Asn Pro Gly Asn Ser Val Arg Leu Met
   2260 2265 2270
   Pro Leu Arg Tyr Asp Leu Arg Asp Arg Ile Thr Arg Leu Gly Asp Ile
   2275 2280 2285
   Pro Tyr Lys Ile Asp Asp Asp Gly Phe Leu Cys Gln Arg Gly Ser Asp
   2290 2295 2300
   Val Phe Glu Tyr Asn Ser Lys Gly Leu Leu Thr Arg Ala Tyr Asn Lys
   2305 2310 2315 2320
   Ala Asn Gly Trp Asn Val Gln Tyr Arg Tyr Asp Gly Leu Gly Arg Arg
   2325 2330 2335
   Ala Ser Cys Lys Thr Asn Leu Gly His His Leu Gln Tyr Phe Tyr Ala
   2340 2345 2350
   Asp Leu His Asn Pro Thr Arg Val Thr His Val Tyr Asn His Ser Asn
   2355 2360 2365
   Ser Glu Ile Thr Ser Leu Tyr Tyr Asp Leu Gln Gly His Leu Phe Ala
   2370 2375 2380
   Met Glu Ser Ser Ser Gly Glu Glu Tyr Tyr Val Ala Ser Asp Asn Thr
   2385 2390 2395 2400
   Gly Thr Pro Leu Ala Val Phe Ser Ile Asn Gly Leu Met Ile Lys Gln
   2405 2410 2415
   Leu Gln Tyr Thr Ala Tyr Gly Glu Ile Tyr Tyr Asp Ser Asn Pro Asp
   2420 2425 2430
   Phe Gln Leu Val Ile Gly Phe His Gly Gly Leu Tyr Asp Pro Leu Thr
   2435 2440 2445
   Lys Leu Val His Phe Thr Gln Arg Asp Tyr Asp Val Leu Ala Gly Arg
   2450 2455 2460
   Trp Thr Ser Pro Asp Tyr Thr Met Trp Lys Asn Ile Gly Arg Glu Pro
   2465 2470 2475 2480
   Ala Pro Phe Asn Leu Tyr Met Phe Lys Ser Asn Asn Pro Leu Ser Asn
   2485 2490 2495
   Glu Leu Asp Leu Lys Asn Tyr Val Thr Asp Val Lys Ser Trp Leu Val
   2500 2505 2510
   Met Phe Gly Phe Gln Leu Ser Asn Ile Ile Pro Gly Phe Pro Arg Ala
   2515 2520 2525
   Lys Met Tyr Phe Val Ser Pro Pro Tyr Glu Leu Thr Glu Ser Gln Ala
   2530 2535 2540
   Cys Glu Asn Gly Gln Leu Ile Thr Gly Val Gln Gln Thr Thr Glu Arg
   2545 2550 2555 2560
   His Asn Gln Ala Phe Met Ala Leu Glu Gly Gln Val Ile Ser Lys Arg
   2565 2570 2575
   Leu His Ala Ser Ile Arg Glu Lys Ala Gly His Trp Phe Ala Thr Ser
   2580 2585 2590
   Thr Pro Ile Ile Gly Lys Gly Ile Met Phe Ala Val Lys Lys Gly Arg
   2595 2600 2605
   Val Thr Thr Gly Ile Ser Ser Ile Ala Thr Asp Asp Ser Arg Lys Ile
   2610 2615 2620
   Ala Ser Val Leu Asn Ser Ala His Tyr Leu Glu Lys Met His Tyr Ser
   2625 2630 2635 2640
   Ile Glu Gly Lys Asp Thr His Tyr Phe Val Lys Ile Gly Ser Ala Asp
   2645 2650 2655
   Ser Asp Leu Val Thr Leu Ala Met Thr Ser Gly Arg Lys Val Leu Asp
   2660 2665 2670
   Ser Gly Val Asn Val Thr Val Ser Gln Pro Thr Leu Leu Ile Asn Gly
   2675 2680 2685
   Arg Thr Arg Arg Phe Thr Asn Ile Glu Phe Gln Tyr Ser Thr Leu Leu
   2690 2695 2700
   Ile Asn Ile Arg Tyr Gly Leu Thr Ala Asp Thr Leu Asp Glu Glu Lys
   2705 2710 2715 2720
   Ala Arg Val Leu Asp Gln Ala Arg Gln Arg Ala Leu Gly Ser Ala Trp
   2725 2730 2735
   Ala Lys Glu Gln Gln Lys Ala Arg Asp Gly Arg Glu Gly Ser Arg Val
   2740 2745 2750
   Trp Thr Asp Gly Glu Lys Gln Gln Leu Leu Asn Thr Gly Arg Val Gln
   2755 2760 2765
   Gly Tyr Glu Gly Tyr Tyr Val Leu Pro Val Glu Gln Tyr Pro Glu Leu
   2770 2775 2780
   Ala Asp Ser Ser Ser Asn Ile Gln Phe Leu Arg Gln Asn Glu Met Gly
   2785 2790 2795 2800
   Lys Arg
<210> 70
   <211> 2771
   <212> PFT
   <213> Mus musculus
<400> 70
   Met Asp Val Lys Glu Arg Lys Pro Tyr Arg Ser Leu Thr Arg Arg Arg
   1               5                   10                  15
   Asp Ala Glu Arg Arg Tyr Thr Ser Ser Ser Ala Asp Ser Glu Glu Gly
               20                  25                  30
   Lys Gly Pro Gln Lys Ser Tyr Ser Ser Ser Glu Thr Leu Lys Ala Tyr
           35                  40                  45
   Asp Gln Asp Ala Arg Leu Ala Tyr Gly Ser Arg Val Lys Asp Met Val
       50                  55                  60
   Pro Gln Glu Ala Glu Glu Phe Cys Arg Thr Gly Thr Asn Phe Thr Leu
   65                  70                  75                  80
   Arg Glu Leu Gly Leu Gly Glu Met Thr Pro Pro His Gly Thr Leu Tyr
                   85                  90                  95
   Arg Thr Asp Ile Gly Leu Pro His Cys Gly Tyr Ser Met Gly Ala Ser
               100                 105                 110
   Ser Asp Ala Asp Leu Glu Ala Asp Thr Val Leu Ser Pro Glu His Pro
           115                 120                 125
   Val Arg Leu Trp Gly Arg Ser Thr Arg Ser Gly Arg Ser Ser Cys Leu
       130                 135                 140
   Ser Ser Arg Ala Asn Ser Asn Leu Thr Leu Thr Asp Thr Glu His Glu
   145                 150                 155                 160
   Asn Thr Glu Thr Asp His Pro Ser Ser Leu Gln Asn His Pro Arg Leu
                   165                 170                 175
   Arg Thr Pro Pro Pro Pro Leu Pro His Ala His Thr Pro Asn Gln His
               180                 185                 190
   His Ala Ala Ser Ile Asn Ser Leu Asn Arg Gly Asn Phe Thr Pro Arg
           195                 200                 205
   Ser Asn Pro Ser Pro Ala Pro Thr Asp His Ser Leu Ser Gly Glu Pro
       210                 215                 220
   Pro Ala Gly Ser Ala Gln Glu Pro Thr His Ala Gln Asp Asn Trp Leu
   225                 230                 235                 240
   Leu Asn Ser Asn Ile Pro Leu Glu Thr Arg Asn Leu Gly Lys Gln Pro
                   245                 250                 255
   Phe Leu Gly Thr Leu Gln Asp Asn Leu Ile Glu Met Asp Ile Leu Ser
               260                 265                 270
   Ala Ser Arg His Asp Gly Ala Tyr Ser Asp Gly His Phe Leu Phe Lys
           275                 280                 285
   Pro Gly Gly Thr Ser Pro Leu Phe Cys Thr Thr Ser Pro Gly Tyr Pro
       290                 295                 300
   Leu Thr Ser Ser Thr Val Tyr Ser Pro Pro Pro Arg Pro Leu Pro Arg
   305                 310                 315                 320
   Ser Thr Phe Ser Arg Pro Ala Phe Asn Leu Lys Lys Pro Ser Lys Tyr
                   325                 330                 335
   Cys Asn Trp Lys Cys Ala Ala Leu Ser Ala Ile Leu Ile Ser Ala Thr
               340                 345                 350
   Leu Val Ile Leu Leu Ala Tyr Phe Val Ala Met His Leu Phe Gly Leu
           355                 360                 365
   Asn Trp His Leu Gln Pro Met Glu Gly Gln Met Gln Met Tyr Glu Ile
       370                 375                 380
   Thr Glu Asp Thr Ala Ser Ser Trp Pro Val Pro Thr Asp Val Ser Leu
   385                 390                 395                 400
   Tyr Pro Ser Gly Gly Thr Gly Leu Glu Thr Pro Asp Arg Lys Gly Lys
                   405                 410                 415
   Gly Ala Ala Glu Gly Lys Pro Ser Ser Leu Phe Pro Glu Asp Ser Phe
               420                 425                 430
   Ile Asp Ser Gly Glu Ile Asp Val Gly Arg Arg Ala Ser Gln Lys Ile
           435                 440                 445
   Pro Pro Gly Thr Phe Trp Arg Ser Gln Val Phe Ile Asp His Pro Val
       450                 455                 460
   His Leu Lys Phe Asn Val Ser Leu Gly Lys Ala Ala Leu Val Gly Ile
   465 470 475 480
   Tyr Gly Arg Lys Gly Leu Pro Pro Ser His Thr Gln Phe Asp Phe Val
   485 490 495
   Glu Leu Leu Asp Gly Arg Arg Leu Leu Thr Gln Glu Ala Arg Ser Leu
   500 505 510
   Glu Gly Pro Gln Arg Gln Ser Arg Gly Pro Val Pro Pro Ser Ser His
   515 520 525
   Glu Thr Gly Phe Ile Gln Tyr Leu Asp Ser Gly Ile Trp His Leu Ala
   530 535 540
   Phe Tyr Asn Asp Gly Lys Glu Ser Glu Val Val Ser Phe Leu Thr Thr
   545 550 555 560
   Ala Ile Glu Ser Val Asp Asn Cys Pro Ser Asn Cys Tyr Gly Asn Gly
   565 570 575
   Asp Cys Ile Ser Gly Thr Cys His Cys Phe Leu Gly Phe Leu Gly Pro
   580 585 590
   Asp Cys Gly Arg Ala Ser Cys Pro Val Leu Cys Ser Gly Asn Gly Gln
   595 600 605
   Tyr Met Lys Gly Arg Cys Leu Cys His Ser Gly Trp Lys Gly Ala Glu
   610 615 620
   Cys Asp Val Pro Thr Asn Gln Cys Ile Asp Val Ala Cys Ser Ser His
   625 630 635 640
   Gly Thr Cys Ile Met Gly Thr Cys Ile Cys Asn Pro Gly Tyr Lys Gly
   645 650 655
   Glu Ser Cys Glu Glu Val Asp Cys Met Asp Pro Thr Cys Ser Ser Arg
   660 665 670
   Gly Val Cys Val Arg Gly Glu Cys His Cys Ser Val Gly Trp Gly Gly
   675 680 685
   Thr Asn Cys Glu Thr Pro Arg Ala Thr Cys Leu Asp Gln Cys Ser Gly
   690 695 700
   His Gly Thr Phe Leu Pro Asp Thr Gly Leu Cys Asn Cys Asp Pro Ser
   705 710 715 720
   Trp Thr Gly His Asp Cys Ser Ile Glu Ile Cys Ala Ala Asp Cys Gly
   725 730 735
   Gly His Gly Val Cys Val Gly Gly Thr Cys Arg Cys Glu Asp Gly Trp
   740 745 750
   Met Gly Ala Ala Cys Asp Gln Arg Ala Cys His Pro Arg Cys Ala Glu
   755 760 765
   His Gly Thr Cys Arg Asp Gly Lys Cys Glu Cys Ser Pro Gly Trp Asn
   770 775 780
   Gly Glu His Cys Thr Ile Ala His Tyr Leu Asp Arg Val Val Lys Glu
   785 790 795 800
   Gly Cys Pro Gly Leu Cys Asn Gly Asn Gly Arg Cys Thr Leu Asp Leu
   805 810 815
   Asn Gly Trp His Cys Val Cys Gln Leu Gly Trp Arg Gly Thr Gly Cys
   820 825 830
   Asp Thr Ser Met Glu Thr Gly Cys Gly Asp Gly Lys Asp Asn Asp Gly
   835 840 845
   Asp Gly Leu Val Asp Cys Met Asp Pro Asp Cys Cys Leu Gln Pro Leu
   850 855 860
   Cys His Val Asn Pro Leu Cys Leu Gly Ser Pro Asp Pro Leu Asp Ile
   865 870 875 880
   Ile Gln Glu Thr Gln Ala Pro Val Ser Gln Gln Asn Leu Asn Pro Phe
   885 890 895
   Tyr Asp Arg Ile Lys Phe Leu Val Gly Arg Asp Ser Thr His Ser Ile
   900 905 910
   Pro Gly Glu Asn Pro Phe Asp Gly Gly His Ala Cys Val Ile Arg Gly
   915 920 925
   Gln Val Met Thr Ser Asp Gly Thr Pro Leu Val Gly Val Asn Ile Ser
   930 935 940
   Phe Ile Asn Asn Pro Leu Phe Gly Tyr Thr Ile Ser Arg Gln Asp Gly
   945 950 955 960
   Ser Phe Asp Leu Val Thr Asn Gly Gly Ile Ser Ile Ile Leu Arg Phe
   965 970 975
   Glu Arg Ala Pro Phe Ile Thr Gln Glu His Thr Leu Trp Leu Pro Trp
   980 985 990
   Asp Arg Phe Phe Val Met Glu Thr Ile Val Met Arg His Glu Glu Asn
   995 1000 1005
   Glu Ile Pro Ser Cys Asp Leu Ser Asn Phe Ala Arg Pro Asn Pro Val
   1010 1015 1020
   Val Ser Pro Ser Pro Leu Thr Ser Phe Ala Ser Ser Cys Ala Glu Lys
   1025 1030 1035 1040
   Gly Pro Ile Val Pro Glu Ile Gln Ala Leu Gln Glu Glu Ile Val Ile
   1045 1050 1055
   Ala Gly Cys Lys Met Arg Leu Ser Tyr Leu Ser Ser Arg Thr Pro Gly
   1060 1065 1070
   Tyr Lys Ser Val Leu Arg Ile Ser Leu Thr His Pro Thr Ile Pro Phe
   1075 1080 1085
   Asn Leu Met Lys Val His Leu Met Val Ala Val Glu Gly Arg Leu Phe
   1090 1095 1100
   Arg Lys Trp Phe Ala Ala Ala Pro Asp Leu Ser Tyr Tyr Phe Ile Trp
   1105 1110 1115 1120
   Asp Lys Thr Asp Val Tyr Asn Gln Lys Val Phe Gly Phe Ser Glu Ala
   1125 1130 1135
   Phe Val Ser Val Gly Tyr Glu Tyr Glu Ser Cys Pro Asp Leu Ile Leu
   1140 1145 1150
   Trp Glu Lys Arg Thr Ala Val Leu Gln Gly Tyr Glu Ile Asp Ala Ser
   1155 1160 1165
   Lys Leu Gly Gly Trp Ser Leu Asp Lys His His Ala Leu Asn Ile Gln
   1170 1175 1180
   Ser Gly Ile Leu His Lys Gly Asn Gly Glu Asn Gln Phe Val Ser Gln
   1185 1190 1195 1200
   Gln Pro Pro Val Ile Gly Ser Ile Met Gly Asn Gly Arg Arg Arg Ser
   1205 1210 1215
   Ile Ser Cys Pro Ser Cys Asn Gly Leu Ala Asp Gly Asn Lys Leu Leu
   1220 1225 1230
   Ala Pro Val Ala Leu Thr Cys Gly Ser Asp Gly Ser Leu Tyr Val Gly
   1235 1240 1245
   Asp Phe Asn Tyr Ile Arg Arg Ile Phe Pro Ser Gly Asn Val Thr Asn
   1250 1255 1260
   Ile Leu Glu Met Arg Asn Lys Asp Phe Arg His Ser His Ser Pro Ala
   1265 1270 1275 1280
   His Lys Tyr Tyr Leu Ala Thr Asp Pro Met Ser Gly Ala Val Phe Leu
   1285 1290 1295
   Ser Asp Thr Asn Ser Arg Arg Val Phe Lys Val Lys Ser Thr Thr Val
   1300 1305 1310
   Val Lys Asp Leu Val Lys Asn Ser Glu Val Val Ala Gly Thr Gly Asp
   1315 1320 1325
   Gln Cys Leu Pro Phe Asp Asp Thr Arg Cys Gly Asp Gly Gly Lys Ala
   1330 1335 1340
   Thr Glu Ala Thr Leu Thr Asn Pro Arg Gly Ile Thr Val Asp Lys Phe
   1345 1350 1355 1360
   Gly Leu Ile Tyr Phe Val Asp Gly Thr Met Ile Arg Arg Val Asp Gln
   1365 1370 1375
   Asn Gly Ile Ile Ser Thr Leu Leu Gly Ser Asn Asp Leu Thr Ser Ala
   1380 1385 1390
   Arg Pro Leu Ser Cys Asp Ser Val Met Glu Ile Ser Gln Val Arg Leu
   1395 1400 1405
   Glu Trp Pro Thr Asp Leu Ala Ile Asn Pro Met Asp Asn Ser Leu Tyr
   1410 1415 1420
   Val Leu Asp Asn Asn Val Val Leu Gln Ile Ser Glu Asn His Gln Val
   1425 1430 1435 1440
   Arg Ile Val Ala Gly Arg Pro Met His Cys Gln Val Pro Gly Ile Asp
   1445 1450 1455
   His Phe Leu Leu Ser Lys Val Ala Ile His Ala Thr Leu Glu Ser Ala
   1460 1465 1470
   Thr Ala Leu Ala Val Ser His Asn Gly Val Leu Tyr Ile Ala Glu Thr
   1475 1480 1485
   Asp Glu Lys Lys Ile Asn Arg Ile Arg Gln Val Thr Thr Ser Gly Glu
   1490 1495 1500
   Ile Ser Leu Val Ala Gly Ala Pro Ser Gly Cys Asp Cys Lys Asn Asp
   1505 1510 1515 1520
   Ala Asn Cys Asp Cys Phe Ser Gly Asp Asp Gly Tyr Ala Lys Asp Ala
   1525 1530 1535
   Lys Leu Asn Thr Pro Ser Ser Leu Ala Val Cys Ala Asp Gly Glu Leu
   1540 1545 1550
   Tyr Val Ala Asp Leu Gly Asn Ile Arg Ile Arg Phe Ile Arg Lys Asn
   1555 1560 1565
   Lys Pro Phe Leu Asn Thr Gln Asn Met Tyr Glu Leu Ser Ser Pro Ile
   1570 1575 1580
   Asp Gln Glu Leu Tyr Leu Phe Asp Thr Ser Gly Lys His Leu Tyr Thr
   1585 1590 1595 1600
   Gln Ser Leu Pro Thr Gly Asp Tyr Leu Tyr Asn Phe Thr Tyr Thr Gly
   1605 1610 1615
   Asp Gly Asp Ile Thr His Ile Thr Asp Asn Asn Gly Asn Met Val Asn
   1620 1625 1630
   Val Arg Arg Asp Ser Thr Gly Met Pro Leu Trp Leu Val Val Pro Asp
   1635 1640 1645
   Gly Gln Val Tyr Trp Val Thr Met Gly Thr Asn Ser Ala Leu Arg Ser
   1650 1655 1660
   Val Thr Thr Gln Gly His Glu Leu Ala Met Met Thr Tyr His Gly Asn
   1665 1670 1675 1680
   Ser Gly Leu Leu Ala Thr Lys Ser Asn Glu Asn Gly Trp Thr Thr Phe
   1685 1690 1695
   Tyr Glu Tyr Asp Ser Phe Gly Arg Leu Thr Asn Val Thr Phe Pro Thr
   1700 1705 1710
   Gly Gln Val Ser Ser Phe Arg Ser Asp Thr Asp Ser Ser Val His Val
   1715 1720 1725
   Gln Val Glu Thr Ser Ser Lys Asp Asp Val Thr Ile Thr Thr Asn Leu
   1730 1735 1740
   Ser Ala Ser Gly Ala Phe Tyr Thr Leu Leu Gln Asp Gln Val Arg Asn
   1745 1750 1755 1760
   Ser Tyr Tyr Ile Gly Ala Asp Gly Ser Leu Arg Leu Leu Leu Ala Asn
   1765 1770 1775
   Gly Met Glu Val Ala Leu Gln Thr Glu Pro His Leu Leu Ala Gly Thr
   1780 1785 1790
   Val Asn Pro Thr Val Gly Lys Arg Asn Val Thr Leu Pro Ile Asp Asn
   1795 1800 1805
   Gly Leu Asn Leu Val Glu Trp Arg Gln Arg Lys Glu Gln Ala Arg Gly
   1810 1815 1820
   Gln Val Thr Val Phe Gly Arg Arg Leu Arg Val His Asn Arg Asn Leu
   1825 1830 1835 1840
   Leu Ser Leu Asp Phe Asp Arg Val Thr Arg Thr Glu Lys Ile Tyr Asp
   1845 1850 1855
   Asp His Arg Lys Phe Thr Leu Arg Ile Leu Tyr Asp Gln Ala Gly Arg
   1860 1865 1870
   Pro Ser Leu Trp Ser Pro Ser Ser Arg Leu Asn Gly Val Asn Val Thr
   1875 1880 1885
   Tyr Ser Pro Gly Gly His Ile Ala Gly Ile Gln Arg Gly Ile Met Ser
   1890 1895 1900
   Glu Arg Met Glu Tyr Asp Gln Ala Gly Arg Ile Thr Ser Arg Ile Phe
   1905 1910 1915 1920
   Ala Asp Gly Lys Met Trp Ser Tyr Thr Tyr Leu Glu Lys Ser Met Val
   1925 1930 1935
   Leu His Leu His Ser Gln Arg Gln Tyr Ile Phe Glu Phe Asp Lys Asn
   1940 1945 1950
   Asp Arg Leu Ser Ser Val Thr Met Pro Asn Val Ala Arg Gln Thr Leu
   1955 1960 1965
   Glu Thr Ile Arg Ser Val Gly Tyr Tyr Arg Asn Ile Tyr Gln Pro Pro
   1970 1975 1980
   Glu Gly Asn Ala Ser Val Ile Gln Asp Phe Thr Glu Asp Gly His Leu
   1985 1990 1995 2000
   Leu His Thr Phe Tyr Leu Gly Thr Gly Arg Arg Val Ile Tyr Lys Tyr
   2005 2010 2015
   Gly Lys Leu Ser Lys Leu Ala Glu Thr Leu Tyr Asp Thr Thr Lys Val
   2020 2025 2030
   Ser Phe Thr Tyr Asp Glu Thr Ala Gly Met Leu Lys Thr Val Asn Leu
   2035 2040 2045
   Gln Asn Glu Gly Phe Thr Cys Thr Ile Arg Tyr Arg Gln Ile Gly Pro
   2050 2055 2060
   Leu Ile Asp Arg Gln Ile Phe Arg Phe Thr Glu Glu Gly Met Val Asn
   2065 2070 2075 2080
   Ala Arg Phe Asp Tyr Asn Tyr Asp Asn Ser Phe Arg Val Thr Ser Met
   2085 2090 2095
   Gln Ala Val Ile Asn Glu Thr Pro Leu Pro Ile Asp Leu Tyr Arg Tyr
   2100 2105 2110
   Asp Asp Val Ser Gly Lys Thr Glu Gln Phe Gly Lys Phe Gly Val Ile
   2115 2120 2125
   Tyr Tyr Asp Ile Asn Gln Ile Ile Thr Thr Ala Val Met Thr His Thr
   2130 2135 2140
   Lys His Phe Asp Ala Tyr Gly Arg Met Lys Glu Val Gln Tyr Glu Ile
   2145 2150 2155 2160
   Phe Arg Ser Leu Met Tyr Trp Met Thr Val Gln Tyr Asp Asn Met Gly
   2165 2170 2175
   Arg Val Val Lys Lys Glu Leu Lys Val Gly Pro Tyr Ala Asn Thr Thr
   2180 2185 2190
   Arg Tyr Ser Tyr Glu Tyr Asp Ala Asp Gly Gln Leu Gln Thr Val Ser
   2195 2200 2205
   Ile Asn Asp Lys Pro Leu Trp Arg Tyr Ser Tyr Asp Leu Asn Gly Asn
   2210 2215 2220
   Leu His Leu Leu Ser Pro Gly Asn Ser Ala Arg Leu Thr Pro Leu Arg
   2225 2230 2235 2240
   Tyr Asp Leu Arg Asp Arg Ile Thr Arg Leu Gly Asp Val Gln Tyr Lys
   2245 2250 2255
   Met Asp Glu Asp Gly Phe Leu Arg Gln Arg Gly Gly Asp Val Phe Glu
   2260 2265 2270
   Tyr Asn Ser Ala Gly Leu Leu Ile Lys Ala Tyr Asn Arg Ala Ser Gly
   2275 2280 2285
   Trp Ser Val Arg Tyr Arg Tyr Asp Gly Leu Gly Arg Arg Val Ser Ser
   2290 2295 2300
   Lys Ser Ser His Ser His His Leu Gln Phe Phe Tyr Ala Asp Leu Thr
   2305 2310 2315 2320
   Asn Pro Thr Lys Val Thr His Leu Tyr Asn His Ser Ser Ser Glu Ile
   2325 2330 2335
   Thr Ser Leu Tyr Tyr Asp Leu Gln Gly His Leu Phe Ala Met Glu Leu
   2340 2345 2350
   Ser Ser Gly Asp Glu Phe Tyr Ile Ala Cys Asp Asn Ile Gly Thr Pro
   2355 2360 2365
   Leu Ala Val Phe Ser Gly Thr Gly Leu Met Ile Lys Gln Ile Leu Tyr
   2370 2375 2380
   Thr Ala Tyr Gly Glu Ile Tyr Met Asp Thr Asn Pro Asn Phe Gln Ile
   2385 2390 2395 2400
   Ile Ile Gly Tyr His Gly Gly Leu Tyr Asp Pro Leu Thr Lys Leu Val
   2405 2410 2415
   His Met Gly Arg Arg Asp Tyr Asp Val Leu Ala Gly Arg Trp Thr Ser
   2420 2425 2430
   Pro Asp His Glu Leu Trp Lys Arg Leu Ser Ser Asn Ser Ile Val Pro
   2435 2440 2445
   Phe His Leu Tyr Met Phe Lys Asn Asn Asn Pro Ile Ser Asn Ser Gln
   2450 2455 2460
   Asp Ile Lys Cys Phe Met Thr Asp Val Asn Ser Trp Leu Leu Thr Phe
   2465 2470 2475 2480
   Gly Phe Gln Leu His Asn Val Ile Pro Gly Tyr Pro Lys Pro Asp Thr
   2485 2490 2495
   Asp Ala Met Glu Pro Ser Tyr Glu Leu Val His Thr Gln Met Lys Thr
   2500 2505 2510
   Gln Glu Trp Asp Asn Ser Lys Ser Ile Leu Gly Val Gln Cys Glu Val
   2515 2520 2525
   Gln Lys Gln Leu Lys Ala Phe Val Thr Leu Glu Arg Phe Asp Gln Leu
   2530 2535 2540
   Tyr Gly Ser Thr Ile Thr Ser Cys Gln Gln Ala Pro Glu Thr Lys Lys
   2545 2550 2555 2560
   Phe Ala Ser Ser Gly Ser Ile Phe Gly Lys Gly Val Lys Phe Ala Leu
   2565 2570 2575
   Lys Asp Gly Arg Val Thr Thr Asp Ile Ile Ser Val Ala Asn Glu Asp
   2580 2585 2590
   Gly Arg Arg Ile Ala Ala Ile Leu Asn Asn Ala His Tyr Leu Glu Asn
   2595 2600 2605
   Leu His Phe Thr Ile Asp Gly Val Asp Thr His Tyr Phe Val Lys Pro
   2610 2615 2620
   Gly Pro Ser Glu Gly Asp Leu Ala Ile Leu Gly Leu Ser Gly Gly Arg
   2625 2630 2635 2640
   Arg Thr Leu Glu Asn Gly Val Asn Val Thr Val Ser Gln Ile Asn Thr
   2645 2650 2655
   Met Leu Ser Gly Arg Thr Arg Arg Tyr Thr Asp Ile Gln Leu Gln Tyr
   2660 2665 2670
   Arg Ala Leu Cys Leu Asn Thr Arg Tyr Gly Thr Thr Val Asp Glu Glu
   2675 2680 2685
   Lys Val Arg Val Leu Glu Leu Ala Arg Gln Arg Ala Val Arg Gln Ala
   2690 2695 2700
   Trp Ala Arg Glu Gln Gln Arg Leu Arg Glu Gly Glu Glu Gly Leu Arg
   2705 2710 2715 2720
   Ala Trp Thr Asp Gly Glu Lys Gln Gln Val Leu Asn Thr Gly Arg Val
   2725 2730 2735
   Gln Gly Tyr Asp Gly Phe Phe Val Thr Ser Val Glu Gln Tyr Pro Glu
   2740 2745 2750
   Leu Ser Asp Ser Ala Asn Asn Ile His Phe Met Arg Gln Ser Glu Met
   2755 2760 2765
   Gly Arg Arg
   2770
<210> 71
   <211> 1737
   <212> PRT
   <213> Homo sapiens
<400> 71
   Thr Phe Phe Ser Ala Ala Pro Gly Gln Asn Pro Ile Val Pro Glu Thr
   1 5 10 15
   Gln Val Leu His Glu Glu Ile Glu Leu Pro Gly Ser Asn Val Lys Leu
   20 25 30
   Arg Tyr Leu Ser Ser Arg Thr Ala Gly Tyr Lys Ser Leu Leu Lys Ile
   35 40 45
   Thr Met Thr Gln Ser Thr Val Pro Leu Asn Leu Ile Arg Val His Leu
   50 55 60
   Met Val Ala Val Glu Gly His Leu Phe Gln Lys Ser Phe Gln Ala Ser
   65 70 75 80
   Pro Asn Leu Ala Tyr Thr Phe Ile Trp Asp Lys Thr Asp Ala Tyr Gly
   85 90 95
   Gln Arg Val Tyr Gly Leu Ser Asp Ala Val Val Ser Val Gly Phe Glu
   100 105 110
   Tyr Glu Thr Cys Pro Ser Leu Ile Leu Trp Glu Lys Arg Thr Ala Leu
   115 120 125
   Leu Gln Gly Phe Glu Leu Asp Pro Ser Asn Leu Gly Gly Trp Ser Leu
   130 135 140
   Asp Lys His His Ile Leu Asn Val Lys Ser Gly Ile Leu His Lys Gly
   145 150 155 160
   Thr Gly Glu Asn Gln Phe Leu Thr Gln Gln Pro Ala Ile Ile Thr Ser
   165 170 175
   Ile Met Gly Asn Gly Arg Arg Arg Ser Ile Ser Cys Pro Ser Cys Asn
   180 185 190
   Gly Leu Ala Glu Gly Asn Lys Leu Leu Ala Pro Val Ala Leu Ala Val
           195                 200                 205
   Gly Ile Asp Gly Ser Leu Tyr Val Gly Asp Phe Asn Tyr Ile Arg Arg
       210                 215                 220
   Ile Phe Pro Ser Arg Asn Val Thr Ser Ile Leu Glu Leu Arg Asn Lys
   225                 230                 235                 240
   Glu Phe Lys His Ser Asn Asn Pro Ala His Lys Tyr Tyr Leu Ala Val
                   245                 250                 255
   Asp Pro Val Ser Gly Ser Leu Tyr Val Ser Asp Thr Asn Ser Arg Arg
               260                 265                 270
   Ile Tyr Arg Val Lys Ser Leu Ser Gly Thr Lys Asp Leu Ala Gly Asn
           275                 280                 285
   Ser Glu Val Val Ala Gly Thr Gly Glu Gln Cys Leu Pro Phe Asp Glu
       290                 295                 300
   Ala Arg Cys Gly Asp Gly Gly Lys Ala Ile Asp Ala Thr Leu Met Ser
   305                 310                 315                 320
   Pro Arg Gly Ile Ala Val Asp Lys Asn Gly Leu Met Tyr Phe Val Asp
                   325                 330                 335
   Ala Thr Met Ile Arg Lys Val Asp Gln Asn Gly Ile Ile Ser Thr Leu
               340                 345                 350
   Leu Gly Ser Asn Asp Leu Thr Ala Val Arg Pro Leu Ser Cys Asp Ser
           355                 360                 365
   Ser Met Asp Val Ala Gln Val Arg Leu Glu Trp Pro Thr Asp Leu Ala
       370                 375                 380
   Val Asn Pro Met Asp Asn Ser Leu Tyr Val Leu Glu Asn Asn Val Ile
   385                 390                 395                 400
   Leu Arg Ile Thr Glu Asn His Gln Val Ser Ile Ile Ala Gly Arg Pro
                   405                 410                 415
   Met His Cys Gln Val Pro Gly Ile Asp Tyr Ser Leu Ser Lys Leu Ala
               420                 425                 430
   Ile His Ser Ala Leu Glu Ser Ala Ser Ala Ile Ala Ile Ser His Thr
           435                 440                 445
   Gly Val Leu Tyr Ile Thr Glu Thr Asp Glu Lys Lys Ile Asn Arg Leu
   450 455 460
   Arg Gln Val Thr Thr Asn Gly Glu Ile Cys Leu Leu Ala Gly Ala Ala
   465 470 475 480
   Ser Asp Cys Asp Cys Lys Asn Asp Val Asn Cys Asn Cys Tyr Ser Gly
   485 490 495
   Asp Asp Ala Tyr Ala Thr Asp Ala Ile Leu Asn Ser Pro Ser Ser Leu
   500 505 510
   Ala Val Ala Pro Asp Gly Thr Ile Tyr Ile Ala Asp Leu Gly Asn Ile
   515 520 525
   Arg Ile Arg Ala Val Ser Lys Asn Lys Pro Val Leu Asn Ala Phe Asn
   530 535 540
   Gln Tyr Glu Ala Ala Ser Pro Gly Glu Gln Glu Leu Tyr Val Phe Asn
   545 550 555 560
   Ala Asp Gly Ile His Gln Tyr Thr Val Ser Leu Val Thr Gly Glu Tyr
   565 570 575
   Leu Tyr Asn Phe Thr Tyr Ser Thr Asp Asn Asp Val Thr Glu Leu Ile
   580 585 590
   Asp Asn Asn Gly Asn Ser Leu Lys Ile Arg Arg Asp Ser Ser Gly Met
   595 600 605
   Pro Arg His Leu Leu Met Pro Asp Asn Gln Ile Ile Thr Leu Thr Val
   610 615 620
   Gly Thr Asn Gly Gly Leu Lys Val Val Ser Thr Gln Asn Leu Glu Leu
   625 630 635 640
   Gly Leu Met Thr Tyr Asp Gly Asn Thr Gly Leu Leu Ala Thr Lys Ser
   645 650 655
   Asp Glu Thr Gly Trp Thr Thr Phe Tyr Asp Tyr Asp His Glu Gly Arg
   660 665 670
   Leu Thr Asn Val Thr Arg Pro Thr Gly Val Val Thr Ser Leu His Arg
   675 680 685
   Glu Met Glu Lys Ser Ile Thr Ile Asp Ile Glu Asn Ser Asn Arg Asp
   690 695 700
   Asp Asp Val Thr Val Ile Thr Asn Leu Ser Ser Val Glu Ala Ser Tyr
   705 710 715 720
   Thr Val Val Gln Asp Gln Val Arg Asn Ser Tyr Gln Leu Cys Asn Asn
   725 730 735
   Gly Thr Leu Arg Val Met Tyr Ala Asn Gly Met Gly Ile Ser Phe His
   740 745 750
   Ser Glu Pro His Val Leu Ala Gly Thr Ile Thr Pro Thr Ile Gly Arg
   755 760 765
   Cys Asn Ile Ser Leu Pro Met Glu Asn Gly Leu Asn Ser Ile Glu Trp
   770 775 780
   Arg Leu Arg Lys Glu Gln Ile Lys Gly Lys Val Thr Ile Phe Gly Arg
   785 790 795 800
   Lys Leu Arg Val His Gly Arg Asn Leu Leu Ser Ile Asp Tyr Asp Arg
   805 810 815
   Asn Ile Arg Thr Glu Lys Ile Tyr Asp Asp His Arg Lys Phe Thr Leu
   820 825 830
   Arg Ile Ile Tyr Asp Gln Val Gly Arg Pro Phe Leu Trp Leu Pro Ser
   835 840 845
   Ser Gly Leu Ala Ala Val Asn Val Ser Tyr Phe Phe Asn Gly Arg Leu
   850 855 860
   Ala Gly Leu Gln Arg Gly Ala Met Ser Glu Arg Thr Asp Ile Asp Lys
   865 870 875 880
   Gln Gly Arg Ile Val Ser Arg Met Phe Ala Asp Gly Lys Val Trp Ser
   885 890 895
   Tyr Ser Tyr Leu Asp Lys Ser Met Val Leu Leu Leu Gln Ser Gln Arg
   900 905 910
   Gln Tyr Ile Phe Glu Tyr Asp Ser Ser Asp Arg Leu Leu Ala Val Thr
   915 920 925
   Met Pro Ser Val Ala Arg His Ser Met Ser Thr His Thr Ser Ile Gly
   930 935 940
   Tyr Ile Arg Asn Ile Tyr Asn Pro Pro Glu Ser Asn Ala Ser Val Ile
   945 950 955 960
   Phe Asp Tyr Ser Asp Asp Gly Arg Ile Leu Lys Thr Ser Phe Leu Gly
   965 970 975
   Thr Gly Arg Gln Val Phe Tyr Lys Tyr Gly Lys Leu Ser Lys Leu Ser
   980 985 990
   Glu Ile Val Tyr Asp Ser Thr Ala Val Thr Phe Gly Tyr Asp Glu Thr
   995 1000 1005
   Thr Gly Val Leu Lys Met Val Asn Leu Gln Ser Gly Gly Phe Ser Cys
   1010 1015 1020
   Thr Ile Arg Tyr Arg Lys Ile Gly Pro Leu Val Asp Lys Gln Ile Tyr
   1025 1030 1035 1040
   Arg Phe Ser Glu Glu Gly Met Val Asn Ala Arg Phe Asp Tyr Thr Tyr
   1045 1050 1055
   His Asp Asn Ser Phe Arg Ile Ala Ser Ile Lys Pro Val Ile Ser Glu
   1060 1065 1070
   Thr Pro Leu Pro Val Asp Leu Tyr Arg Tyr Asp Glu Ile Ser Gly Lys
   1075 1080 1085
   Val Glu His Phe Gly Lys Phe Gly Val Ile Tyr Tyr Asp Ile Asn Gln
   1090 1095 1100
   Ile Ile Thr Thr Ala Val Met Thr Leu Ser Lys His Phe Asp Thr His
   1105 1110 1115 1120
   Gly Arg Ile Lys Glu Val Gln Tyr Glu Met Phe Arg Ser Leu Met Tyr
   1125 1130 1135
   Trp Met Thr Val Gln Tyr Asp Ser Met Gly Arg Val Ile Lys Arg Glu
   1140 1145 1150
   Leu Lys Leu Gly Pro Tyr Ala Asn Thr Thr Lys Tyr Thr Tyr Asp Tyr
   1155 1160 1165
   Asp Gly Asp Gly Gln Leu Gln Ser Val Ala Val Asn Asp Arg Pro Thr
   1170 1175 1180
   Trp Arg Tyr Ser Tyr Asp Leu Asn Gly Asn Leu His Leu Leu Asn Pro
   1185 1190 1195 1200
   Gly Asn Ser Val Arg Leu Met Pro Leu Arg Tyr Asp Leu Arg Asp Arg
   1205 1210 1215
   Ile Thr Arg Leu Gly Asp Val Gln Tyr Lys Ile Asp Asp Asp Gly Tyr
   1220 1225 1230
   Leu Cys Gln Arg Gly Ser Asp Ile Phe Glu Tyr Asn Ser Lys Gly Leu
   1235 1240 1245
   Leu Thr Arg Ala Tyr Asn Lys Ala Ser Gly Trp Ser Val Gln Tyr Arg
   1250 1255 1260
   Tyr Asp Gly Val Gly Arg Arg Ala Ser Tyr Lys Thr Asn Leu Gly His
   1265 1270 1275 1280
   His Leu Gln Tyr Phe Tyr Ser Asp Leu His Asn Pro Thr Arg Ile Thr
   1285 1290 1295
   His Val Tyr Asn His Ser Asn Ser Glu Ile Thr Ser Leu Tyr Tyr Asp
   1300 1305 1310
   Leu Gln Gly His Leu Phe Ala Met Glu Ser Ser Ser Gly Glu Glu Tyr
   1315 1320 1325
   Tyr Val Ala Ser Asp Asn Thr Gly Thr Pro Leu Ala Val Phe Ser Ile
   1330 1335 1340
   Asn Gly Leu Met Ile Lys Gln Leu Gln Tyr Thr Ala Tyr Gly Glu Ile
   1345 1350 1355 1360
   Tyr Tyr Asp Ser Asn Pro Asp Phe Gln Met Val Ile Gly Phe His Gly
   1365 1370 1375
   Gly Leu Tyr Asp Pro Leu Thr Lys Leu Val His Phe Thr Gln Arg Asp
   1380 1385 1390
   Tyr Asp Val Leu Ala Gly Arg Trp Thr Ser Pro Asp Tyr Thr Met Trp
   1395 1400 1405
   Lys Asn Val Gly Lys Glu Pro Ala Pro Phe Asn Leu Tyr Met Phe Lys
   1410 1415 1420
   Ser Asn Asn Pro Leu Ser Ser Glu Leu Asp Leu Lys Asn Tyr Val Thr
   1425 1430 1435 1440
   Asp Val Lys Ser Trp Leu Val Met Phe Gly Phe Gln Leu Ser Asn Ile
   1445 1450 1455
   Ile Pro Gly Phe Pro Arg Ala Lys Met Tyr Phe Val Pro Pro Pro Tyr
   1460 1465 1470
   Glu Leu Ser Glu Ser Gln Ala Ser Glu Asn Gly Gln Leu Ile Thr Gly
   1475 1480 1485
   Val Gln Gln Thr Thr Glu Arg His Asn Gln Ala Phe Met Ala Leu Glu
   1490 1495 1500
   Gly Gln Val Ile Thr Lys Lys Leu His Ala Ser Ile Arg Glu Lys Ala
   1505 1510 1515 1520
   Gly His Trp Phe Ala Thr Thr Thr Pro Ile Ile Gly Lys Gly Ile Met
   1525 1530 1535
   Phe Ala Ile Lys Glu Gly Arg Val Thr Thr Gly Val Ser Ser Ile Ala
   1540 1545 1550
   Ser Glu Asp Ser Arg Lys Val Ala Ser Val Leu Asn Asn Ala Tyr Tyr
   1555 1560 1565
   Leu Asp Lys Met His Tyr Ser Ile Glu Gly Lys Asp Thr His Tyr Phe
   1570 1575 1580
   Val Lys Ile Gly Ser Ala Asp Gly Asp Leu Val Thr Leu Gly Thr Thr
   1585 1590 1595 1600
   Ile Gly Arg Lys Val Leu Glu Ser Gly Val Asn Val Thr Val Ser Gln
   1605 1610 1615
   Pro Thr Leu Leu Val Asn Gly Arg Thr Arg Arg Phe Thr Asn Ile Glu
   1620 1625 1630
   Phe Gln Tyr Ser Thr Leu Leu Leu Ser Ile Arg Tyr Gly Leu Thr Pro
   1635 1640 1645
   Asp Thr Leu Asp Glu Glu Lys Ala Arg Val Leu Asp Gln Ala Arg Gln
   1650 1655 1660
   Arg Ala Leu Gly Thr Ala Trp Ala Lys Glu Gln Gln Lys Ala Arg Asp
   1665 1670 1675 1680
   Gly Arg Glu Gly Ser Arg Leu Trp Thr Glu Gly Glu Lys Gln Gln Leu
   1685 1690 1695
   Leu Ser Thr Gly Arg Val Gln Gly Tyr Glu Gly Tyr Tyr Val Leu Pro
   1700 1705 1710
   Val Glu Gln Tyr Pro Glu Leu Ala Asp Ser Ser Ser Asn Ile Gln Phe
   1715 1720 1725
   Leu Arg Gln Asn Glu Met Gly Lys Arg
   1730 1735
<210> 72
   <211> 2765
   <212> PRT
   <213> Rattus norvegicus
<400> 72
   Met Asp Val Lys Asp Arg Arg His Arg Ser Leu Thr Arg Gly Arg Cys
    1               5                  10                  15
   Gly Lys Glu Cys Arg Tyr Thr Ser Ser Ser Leu Asp Ser Glu Asp Cys
               20                  25                  30
   Arg Val Pro Thr Gln Lys Ser Tyr Ser Ser Ser Glu Thr Leu Lys Ala
           35                  40                  45
   Tyr Asp His Asp Ser Arg Met His Tyr Gly Asn Arg Val Thr Asp Leu
       50                  55                  60
   Val His Arg Glu Ser Asp Glu Phe Ser Arg Gln Gly Ala Asn Phe Thr
   65                  70                  75                  80
   Leu Ala Glu Leu Gly Ile Cys Glu Pro Ser Pro His Arg Ser Gly Tyr
                   85                  90                  95
   Cys Ser Asp Met Gly Ile Leu His Gln Gly Tyr Ser Leu Ser Thr Gly
               100                 105                 110
   Ser Asp Ala Asp Ser Asp Thr Glu Gly Gly Met Ser Pro Glu His Ala
           115                 120                 125
   Ile Arg Leu Trp Gly Arg Gly Ile Lys Ser Arg Arg Ser Ser Gly Leu
       130                 135                 140
   Ser Ser Arg Glu Asn Ser Ala Leu Thr Leu Thr Asp Ser Asp Asn Glu
   145                 150                 155                 160
   Asn Lys Ser Asp Asp Asp Asn Gly Arg Pro Ile Pro Pro Thr Ser Ser
                   165                 170                 175
   Ser Ser Leu Leu Pro Ser Ala Gln Leu Pro Ser Ser His Asn Pro Pro
               180                 185                 190
   Pro Val Ser Cys Gln Met Pro Leu Leu Asp Ser Asn Thr Ser His Gln
           195                 200                 205
   Ile Met Asp Thr Asn Pro Asp Glu Glu Phe Ser Pro Asn Ser Tyr Leu
   210 215 220
   Leu Arg Ala Cys Ser Gly Pro Gln Gln Ala Ser Ser Ser Gly Pro Pro
   225 230 235 240
   Asn His His Ser Gln Ser Thr Leu Arg Pro Pro Leu Pro Pro Pro His
   245 250 255
   Asn His Thr Leu Ser His His His Ser Ser Ala Asn Ser Leu Asn Arg
   260 265 270
   Asn Ser Leu Thr Asn Arg Arg Ser Gln Ile His Ala Pro Ala Pro Ala
   275 280 285
   Pro Asn Asp Leu Ala Thr Thr Pro Glu Ser Val Gln Leu Gln Asp Ser
   290 295 300
   Trp Val Leu Asn Ser Asn Val Pro Leu Glu Thr Arg His Phe Leu Phe
   305 310 315 320
   Lys Thr Ser Ser Gly Ser Thr Pro Leu Phe Ser Ser Ser Ser Pro Gly
   325 330 335
   Tyr Pro Leu Thr Ser Gly Thr Val Tyr Thr Pro Pro Pro Arg Leu Leu
   340 345 350
   Pro Arg Asn Thr Phe Ser Arg Lys Ala Phe Lys Leu Lys Lys Pro Ser
   355 360 365
   Lys Tyr Cys Ser Trp Lys Cys Ala Ala Leu Ser Ala Ile Ala Ala Ala
   370 375 380
   Leu Leu Leu Ala Ile Leu Leu Ala Tyr Phe Ile Ala Met His Leu Leu
   385 390 395 400
   Gly Leu Asn Trp Gln Leu Gln Pro Ala Asp Gly His Thr Phe Asn Asn
   405 410 415
   Gly Val Arg Thr Gly Leu Pro Gly Asn Asp Asp Val Ala Thr Val Pro
   420 425 430
   Ser Gly Gly Lys Val Pro Trp Ser Leu Lys Asn Ser Ser Ile Asp Ser
   435 440 445
   Gly Glu Ala Glu Val Gly Arg Arg Val Thr Gln Glu Val Pro Pro Gly
   450 455 460
   Val Phe Trp Arg Ser Gln Ile His Ile Ser Gln Pro Gln Phe Leu Lys
   465 470 475 480
   Phe Asn Ile Ser Leu Gly Lys Asp Ala Leu Phe Gly Val Tyr Ile Arg
   485 490 495
   Arg Gly Leu Pro Pro Ser His Ala Gln Tyr Asp Phe Met Glu Arg Leu
   500 505 510
   Asp Gly Lys Glu Lys Trp Ser Val Val Glu Ser Pro Arg Glu Arg Arg
   515 520 525
   Ser Ile Gln Thr Leu Val Gln Asn Glu Ala Val Phe Val Gln Tyr Leu
   530 535 540
   Asp Val Gly Leu Trp His Leu Ala Phe Tyr Asn Asp Gly Lys Asp Lys
   545 550 555 560
   Glu Met Val Ser Phe Asn Thr Val Val Leu Asp Ser Val Gln Asp Cys
   565 570 575
   Pro Arg Asn Cys His Gly Asn Gly Glu Cys Val Ser Gly Leu Cys His
   580 585 590
   Cys Phe Pro Gly Phe Leu Gly Ala Asp Cys Ala Lys Ala Ala Cys Pro
   595 600 605
   Val Leu Cys Ser Gly Asn Gly Gln Tyr Ser Lys Gly Thr Cys Gln Cys
   610 615 620
   Tyr Ser Gly Trp Lys Gly Ala Glu Cys Asp Val Pro Met Asn Gln Cys
   625 630 635 640
   Ile Asp Pro Ser Cys Gly Gly His Gly Ser Cys Ile Asp Gly Asn Cys
   645 650 655
   Val Cys Ala Ala Gly Tyr Lys Gly Glu His Cys Glu Glu Val Asp Cys
   660 665 670
   Leu Asp Pro Thr Cys Ser Ser His Gly Val Cys Val Asn Gly Glu Cys
   675 680 685
   Leu Cys Ser Pro Gly Trp Gly Gly Leu Asn Cys Glu Leu Ala Arg Val
   690 695 700
   Gln Cys Pro Asp Gln Cys Ser Gly His Gly Thr Tyr Leu Pro Asp Ser
   705 710 715 720
   Gly Leu Cys Asn Cys Asp Pro Asn Trp Met Gly Pro Asp Cys Ser Val
   725 730 735
   Glu Val Cys Ser Val Asp Cys Gly Thr His Gly Val Cys Ile Gly Gly
   740 745 750
   Ala Cys Arg Cys Glu Glu Gly Trp Thr Gly Ala Ala Cys Asp Gln Arg
   755 760 765
   Val Cys His Pro Arg Cys Ile Glu His Gly Thr Cys Lys Asp Gly Lys
   770 775 780
   Cys Glu Cys Arg Glu Gly Trp Asn Gly Glu His Cys Thr Ile Asp Gly
   785 790 795 800
   Cys Pro Asp Leu Cys Asn Gly Asn Gly Arg Cys Thr Leu Gly Gln Asn
   805 810 815
   Ser Trp Gln Cys Val Cys Gln Thr Gly Trp Arg Gly Pro Gly Cys Asn
   820 825 830
   Val Ala Met Glu Thr Ser Cys Ala Asp Asn Lys Asp Asn Glu Gly Asp
   835 840 845
   Gly Leu Val Asp Cys Leu Asp Pro Asp Cys Cys Leu Gln Ser Ala Cys
   850 855 860
   Gln Asn Ser Leu Leu Cys Arg Gly Ser Arg Asp Pro Leu Asp Ile Ile
   865 870 875 880
   Gln Gln Gly Gln Thr Asp Trp Pro Ala Val Lys Ser Phe Tyr Asp Arg
   885 890 895
   Ile Lys Leu Leu Ala Gly Lys Asp Ser Thr His Ile Ile Pro Gly Asp
   900 905 910
   Asn Pro Phe Asn Ser Ser Leu Val Ser Leu Ile Arg Gly Gln Val Val
   915 920 925
   Thr Thr Asp Gly Thr Pro Leu Val Gly Val Asn Val Ser Phe Val Lys
   930 935 940
   Tyr Pro Lys Tyr Gly Tyr Thr Ile Thr Arg Gln Asp Gly Thr Phe Asp
   945 950 955 960
   Leu Ile Ala Asn Gly Gly Ser Ala Leu Thr Leu His Phe Glu Arg Ala
   965 970 975
   Pro Phe Met Ser Arg Glu Arg Thr Val Trp Pro Pro Trp Asn Ser Phe
   980 985 990
   Tyr Ala Met Asp Thr Leu Val Met Lys Thr Glu Glu Asn Ser Ile Pro
   995 1000 1005
   Ser Cys Asp Leu Ser Gly Phe Val Arg Pro Asp Pro Ile Ile Ile Ser
   1010 1015 1020
   Ser Pro Leu Ser Thr Phe Phe Ser Ala Ser Pro Ala Ala Asn Pro Ile
   1025 1030 1035 1040
   Val Pro Glu Thr Gln Val Leu His Glu Glu Ile Glu Leu Pro Gly Thr
   1045 1050 1055
   Asn Val Lys Leu Arg Tyr Leu Ser Ser Arg Thr Ala Gly Tyr Lys Ser
   1060 1065 1070
   Leu Leu Lys Ile Thr Met Thr Gln Ser Thr Val Pro Leu Asn Leu Ile
   1075 1080 1085
   Arg Val His Leu Met Val Ala Val Glu Gly His Leu Phe Gln Lys Ser
   1090 1095 1100
   Phe Gln Ala Ser Pro Asn Leu Ala Tyr Thr Phe Ile Trp Asp Lys Thr
   1105 1110 1115 1120
   Asp Ala Tyr Gly Gln Arg Val Tyr Gly Leu Ser Asp Ala Val Val Ser
   1125 1130 1135
   Val Gly Phe Glu Tyr Glu Thr Cys Pro Ser Leu Ile Leu Trp Glu Lys
   1140 1145 1150
   Arg Thr Ala Leu Leu Gln Gly Phe Glu Leu Asp Pro Ser Asn Leu Gly
   1155 1160 1165
   Gly Trp Ser Leu Asp Lys His His Thr Leu Asn Val Lys Ser Gly Ile
   1170 1175 1180
   Leu Leu Lys Gly Thr Gly Glu Asn Gln Phe Leu Thr Gln Gln Pro Ala
   1185 1190 1195 1200
   Ile Ile Thr Ser Ile Met Gly Asn Gly Arg Arg Arg Ser Ile Ser Cys
   1205 1210 1215
   Pro Ser Cys Asn Gly Leu Ala Glu Gly Asn Lys Leu Leu Ala Pro Val
   1220 1225 1230
   Ala Leu Ala Val Gly Ile Asp Gly Ser Leu Phe Val Gly Asp Phe Asn
   1235 1240 1245
   Tyr Ile Arg Arg Ile Phe Pro Ser Arg Asn Val Thr Ser Ile Leu Glu
   1250 1255 1260
   Leu Arg Asn Lys Glu Phe Lys His Ser Asn Ser Pro Gly His Lys Tyr
   1265 1270 1275 1280
   Tyr Leu Ala Val Asp Pro Val Thr Gly Ser Leu Tyr Val Ser Asp Thr
   1285 1290 1295
   Asn Ser Arg Arg Ile Tyr Arg Val Lys Ser Leu Ser Gly Ala Lys Asp
   1300 1305 1310
   Leu Ala Gly Asn Ser Glu Val Val Ala Gly Thr Gly Glu Gln Cys Leu
   1315 1320 1325
   Pro Phe Asp Glu Ala Arg Cys Gly Asp Gly Gly Lys Ala Val Asp Ala
   1330 1335 1340
   Thr Leu Met Ser Pro Arg Gly Ile Ala Val Asp Lys Asn Gly Leu Met
   1345 1350 1355 1360
   Tyr Phe Val Asp Ala Thr Met Ile Arg Lys Val Asp Gln Asn Gly Ile
   1365 1370 1375
   Ile Ser Thr Leu Leu Gly Ser Asn Asp Leu Thr Ala Val Arg Pro Leu
   1380 1385 1390
   Ser Cys Asp Ser Ser Met Asp Val Ala Gln Val Arg Leu Glu Trp Pro
   1395 1400 1405
   Thr Asp Leu Ala Val Asn Pro Met Asp Asn Ser Leu Tyr Val Leu Glu
   1410 1415 1420
   Asn Asn Val Ile Leu Arg Ile Thr Glu Asn His Gln Val Ser Ile Ile
   1425 1430 1435 1440
   Ala Gly Arg Pro Met His Cys Gln Val Pro Gly Ile Asp Tyr Ser Leu
   1445 1450 1455
   Ser Lys Leu Ala Ile His Ser Ala Leu Glu Ser Ala Ser Ala Ile Ala
   1460 1465 1470
   Ile Ser His Thr Gly Val Leu Tyr Ile Thr Glu Thr Asp Glu Lys Lys
   1475 1480 1485
   Ile Asn Arg Leu Arg Gln Val Thr Thr Asn Gly Glu Ile Cys Leu Leu
   1490 1495 1500
   Ala Gly Ala Ala Ser Asp Cys Asp Cys Lys Asn Asp Val Asn Cys Ile
   1505 1510 1515 1520
   Cys Tyr Ser Gly Asp Asp Ala Tyr Ala Thr Asp Ala Ile Leu Asn Ser
   1525 1530 1535
   Pro Ser Ser Leu Ala Val Ala Pro Asp Gly Thr Ile Tyr Ile Ala Asp
   1540 1545 1550
   Leu Gly Asn Ile Arg Ile Arg Ala Val Ser Lys Asn Lys Pro Val Leu
   1555 1560 1565
   Asn Ala Phe Asn Gln Tyr Glu Ala Ala Ser Pro Gly Glu Gln Glu Leu
   1570 1575 1580
   Tyr Val Phe Asn Ala Asp Gly Ile His Gln Tyr Thr Val Ser Leu Val
   1585 1590 1595 1600
   Thr Gly Glu Tyr Leu Tyr Asn Phe Thr Tyr Ser Ala Asp Asn Asp Val
   1605 1610 1615
   Thr Glu Leu Ile Asp Asn Asn Gly Asn Ser Leu Lys Ile Arg Arg Asp
   1620 1625 1630
   Ser Ser Gly Met Pro Arg His Leu Leu Met Pro Asp Asn Gln Ile Ile
   1635 1640 1645
   Thr Leu Thr Val Gly Thr Asn Gly Gly Leu Lys Ala Val Ser Thr Gln
   1650 1655 1660
   Asn Leu Glu Leu Gly Leu Met Thr Tyr Asp Gly Asn Thr Gly Leu Leu
   1665 1670 1675 1680
   Ala Thr Lys Ser Asp Glu Thr Gly Trp Thr Thr Phe Tyr Asp Tyr Asp
   1685 1690 1695
   His Glu Gly Arg Leu Thr Asn Val Thr Arg Pro Thr Gly Val Val Thr
   1700 1705 1710
   Ser Leu His Arg Glu Met Glu Lys Ser Ile Thr Val Asp Ile Glu Asn
   1715 1720 1725
   Ser Asn Arg Asp Asn Asp Val Thr Val Ile Thr Asn Leu Ser Ser Val
   1730 1735 1740
   Glu Ala Ser Tyr Thr Val Val Gln Asp Gln Val Arg Asn Ser Tyr Gln
   1745 1750 1755 1760
   Leu Cys Ser Asn Gly Thr Leu Arg Val Met Tyr Ala Asn Gly Met Gly
   1765 1770 1775
   Val Ser Phe His Ser Glu Pro His Val Leu Ala Gly Thr Leu Thr Pro
   1780 1785 1790
   Thr Ile Gly Arg Cys Asn Ile Ser Leu Pro Met Glu Asn Gly Leu Asn
   1795 1800 1805
   Ser Ile Glu Trp Arg Leu Arg Lys Glu Gln Ile Lys Gly Lys Val Thr
   1810 1815 1820
   Ile Phe Gly Arg Lys Leu Arg Val His Gly Arg Asn Leu Leu Ser Ile
   1825 1830 1835 1840
   Asp Tyr Asp Arg Asn Ile Arg Thr Glu Lys Ile Tyr Asp Asp His Arg
   1845 1850 1855
   Lys Phe Thr Leu Arg Ile Ile Tyr Asp Gln Val Gly Arg Pro Phe Leu
   1860 1865 1870
   Trp Leu Pro Ser Ser Gly Leu Ala Ala Val Asn Val Ser Tyr Phe Phe
   1875 1880 1885
   Asn Gly Arg Leu Ala Gly Leu Gln Arg Gly Ala Met Ser Glu Arg Thr
   1890 1895 1900
   Asp Ile Asp Lys Gln Gly Arg Ile Val Ser Arg Met Phe Ala Asp Gly
   1905 1910 1915 1920
   Lys Val Trp Ser Tyr Ser Tyr Leu Asp Lys Ser Met Val Leu Leu Leu
   1925 1930 1935
   Gln Ser Gln Arg Gln Tyr Ile Phe Glu Tyr Asp Ser Ser Asp Arg Leu
   1940 1945 1950
   His Ala Val Thr Met Pro Ser Val Ala Arg His Ser Met Ser Thr His
   1955 1960 1965
   Thr Ser Ile Gly Tyr Ile Arg Asn Ile Tyr Asn Pro Pro Glu Ser Asn
   1970 1975 1980
   Ala Ser Val Ile Phe Asp Tyr Ser Asp Asp Gly Arg Ile Leu Lys Thr
   1985 1990 1995 2000
   Ser Phe Leu Gly Thr Gly Arg Gln Val Phe Tyr Lys Tyr Gly Lys Leu
   2005 2010 2015
   Ser Lys Leu Ser Glu Ile Val Tyr Asp Ser Thr Ala Val Thr Phe Gly
   2020 2025 2030
   Tyr Asp Glu Thr Thr Gly Val Leu Lys Met Val Asn Leu Gln Ser Gly
   2035 2040 2045
   Gly Phe Ser Cys Thr Ile Arg Tyr Arg Lys Val Gly Pro Leu Val Asp
   2050 2055 2060
   Lys Gln Ile Tyr Arg Phe Ser Glu Glu Gly Met Ile Asn Ala Arg Phe
   2065 2070 2075 2080
   Asp Tyr Thr Tyr His Asp Asn Ser Phe Arg Ile Ala Ser Ile Lys Pro
   2085 2090 2095
   Val Ile Ser Glu Thr Pro Leu Pro Val Asp Leu Tyr Arg Tyr Asp Glu
   2100 2105 2110
   Ile Ser Gly Lys Val Glu His Phe Gly Lys Phe Gly Val Ile Tyr Tyr
   2115 2120 2125
   Asp Ile Asn Gln Ile Ile Thr Thr Ala Val Met Thr Leu Ser Lys His
   2130 2135 2140
   Phe Asp Thr His Gly Arg Ile Lys Glu Val Gln Tyr Glu Met Phe Arg
   2145 2150 2155 2160
   Ser Leu Met Tyr Trp Met Thr Val Gln Tyr Asp Ser Met Gly Arg Val
   2165 2170 2175
   Ile Lys Arg Glu Leu Lys Leu Gly Pro Tyr Ala Asn Thr Thr Lys Tyr
   2180 2185 2190
   Thr Tyr Asp Tyr Asp Gly Asp Gly Gln Leu Gln Ser Val Ala Val Asn
   2195 2200 2205
   Asp Arg Pro Thr Trp Arg Tyr Ser Tyr Asp Leu Asn Gly Asn Leu His
   2210 2215 2220
   Leu Leu Asn Pro Gly Asn Ser Ala Arg Leu Met Pro Leu Arg Tyr Asp
   2225 2230 2235 2240
   Leu Arg Asp Arg Ile Thr Arg Leu Gly Asp Val Gln Tyr Lys Ile Asp
   2245 2250 2255
   Asp Asp Gly Tyr Leu Cys Gln Arg Gly Ser Asp Ile Phe Glu Tyr Asn
   2260 2265 2270
   Ser Lys Gly Leu Leu Thr Arg Ala Tyr Asn Lys Ala Ser Gly Trp Ser
   2275 2280 2285
   Val Gln Tyr Arg Tyr Asp Gly Val Ser Arg Arg Ala Ser Tyr Lys Thr
   2290 2295 2300
   Asn Leu Gly His His Leu Gln Tyr Phe Tyr Ser Asp Leu His His Pro
   2305 2310 2315 2320
   Thr Arg Ile Thr His Val Tyr Asn His Ser Asn Ser Glu Ile Thr Ser
   2325 2330 2335
   Leu Tyr Tyr Asp Leu Gln Gly His Leu Phe Ala Met Glu Ser Ser Ser
   2340 2345 2350
   Gly Glu Glu Tyr Tyr Val Ala Ser Asp Asn Thr Gly Thr Pro Leu Ala
   2355 2360 2365
   Val Phe Ser Ile Asn Gly Leu Met Ile Lys Gln Leu Gln Tyr Thr Ala
   2370 2375 2380
   Tyr Gly Glu Ile Tyr Tyr Asp Ser Asn Pro Asp Phe Gln Met Val Ile
   2385 2390 2395 2400
   Gly Phe His Gly Gly Leu Tyr Asp Pro Leu Thr Lys Leu Val His Phe
   2405 2410 2415
   Thr Gln Arg Asp Tyr Asp Val Leu Ala Gly Arg Trp Thr Ser Pro Asp
   2420 2425 2430
   Tyr Thr Met Trp Arg Asn Val Gly Lys Glu Pro Ala Pro Phe Asn Leu
   2435 2440 2445
   Tyr Met Phe Lys Asn Asn Asn Pro Leu Ser Asn Glu Leu Asp Leu Lys
   2450 2455 2460
   Asn Tyr Val Thr Asp Val Lys Ser Trp Leu Val Met Phe Gly Phe Gln
   2465 2470 2475 2480
   Leu Ser Asn Ile Ile Pro Gly Phe Pro Arg Ala Lys Met Tyr Phe Val
   2485 2490 2495
   Pro Pro Pro Tyr Glu Leu Ser Glu Ser Gln Ala Ser Glu Asn Gly Gln
   2500 2505 2510
   Leu Ile Thr Gly Val Gln Gln Thr Thr Glu Arg His Asn Gln Ala Phe
   2515 2520 2525
   Leu Ala Leu Glu Gly Gln Val Ile Ser Lys Lys Leu His Ala Gly Ile
   2530 2535 2540
   Arg Glu Lys Ala Gly His Trp Phe Ala Thr Thr Thr Pro Ile Ile Gly
   2545 2550 2555 2560
   Lys Gly Ile Met Phe Ala Ile Lys Glu Gly Arg Val Thr Thr Gly Val
   2565 2570 2575
   Ser Ser Ile Ala Ser Glu Asp Ser Arg Lys Val Ala Ser Val Leu Asn
   2580 2585 2590
   Asn Ala Tyr Tyr Leu Asp Lys Met His Tyr Ser Ile Glu Gly Lys Asp
   2595 2600 2605
   Thr His Tyr Phe Val Lys Ile Gly Ala Ala Asp Gly Asp Leu Val Thr
   2610 2615 2620
   Leu Gly Thr Thr Ile Gly Arg Lys Val Leu Glu Ser Gly Val Asn Val
   2625 2630 2635 2640
   Thr Val Ser Gln Pro Thr Leu Leu Val Asn Gly Arg Thr Arg Arg Phe
   2645 2650 2655
   Thr Asn Ile Glu Phe Gln Tyr Ser Thr Leu Leu Leu Ser Ile Arg Tyr
   2660 2665 2670
   Gly Leu Thr Pro Asp Thr Leu Asp Glu Glu Lys Ala Arg Val Leu Asp
   2675 2680 2685
   Gln Ala Arg Gln Arg Ala Leu Gly Thr Ala Trp Ala Lys Glu Gln Gln
   2690 2695 2700
   Lys Ala Arg Asp Gly Arg Glu Gly Ser Arg Leu Trp Thr Glu Gly Glu
   2705 2710 2715 2720
   Lys Gln Gln Leu Leu Ser Thr Gly Arg Val Gln Gly Tyr Glu Gly Tyr
   2725 2730 2735
   Tyr Val Leu Pro Val Glu Gln Tyr Pro Glu Leu Ala Asp Ser Ser Ser
   2740 2745 2750
   Asn Ile Gln Phe Leu Arg Gln Asn Glu Met Gly Lys Arg
   2755 2760 2765
<210> 73
   <211> 86
   <212> PRT
   <213> Trypanosoma cruzi
<400> 73
   Met Ile Asn Asn Lys His Gln Thr Ile Pro Thr Thr His Ser Tyr Thr
   1 5 10 15
   Leu Pro Leu Ile His Tyr Ile Lys Thr Tyr Thr Thr Asp Asn Lys His
   20 25 30
   Asn His Tyr Asn Ser Ile Ser His Tyr Tyr Thr Asn Thr Gln Leu Ser
   35 40 45
   Asn Ser Arg Cys Thr Ser Tyr Pro Ile Gln Leu His Lys Phe Ile Asn
   50 55 60
   Thr Tyr Ser Ser Ile Leu His Gln Pro Gln Ser Asn Pro Thr Ser Pro
   65 70 75 80
   Lys Ile Pro Pro Asn Pro
   85
<210> 74
   <211> 533
   <212> PRT
   <213> Aedes aegypti
<400> 74
   Met Ser Leu Glu Ile Glu Val Pro His Val Arg Cys Pro Ser Leu Gly
   1 5 10 15
   Val Leu Ile Leu Thr Leu Asn Leu Ala Leu Phe Leu Pro Gln Thr Ile
   20 25 30
   Asn Arg Thr Pro Pro Tyr Val Leu Ala Gly Thr Gly Gly Gly Ser Met
   35 40 45
   Leu Gly Asp Val Asn Ile Ser Ala Ile Leu Asp Ser Phe Ser Val Gly
   50 55 60
   Tyr Asp Lys Arg Val Arg Pro Asn Tyr Gly Gly Pro Pro Val Glu Val
   65 70 75 80
   Gly Val Thr Met Tyr Val Leu Ser Ile Ser Ser Val Ser Glu Val Leu
   85 90 95
   Met Asp Phe Thr Leu Asp Phe Tyr Phe Arg Gln Phe Trp Thr Asp Pro
   100 105 110
   Arg Leu Ala Tyr Arg Lys Arg Pro Gly Val Glu Thr Leu Ser Val Gly
   115 120 125
   Ser Glu Phe Ile Lys Asn Ile Trp Val Pro Asp Thr Phe Phe Val Asn
   130 135 140
   Glu Lys Gln Ser Tyr Phe His Ile Ala Thr Thr Ser Asn Glu Phe Ile
   145 150 155 160
   Arg Val His His Ser Gly Ser Ile Thr Arg Ser Ile Arg Leu Thr Ile
   165 170 175
   Thr Ala Ser Cys Pro Met Gly Leu Gln Tyr Phe Pro Met Asp Arg Gln
   180 185 190
   Leu Cys His Ile Glu Ile Glu Ser Phe Gly Tyr Thr Met Arg Asp Ile
   195 200 205
   Arg Tyr Phe Trp Lys Asp Gly Leu Ser Ser Val Gly Met Ser Ser Glu
   210 215 220
   Val Glu Leu Pro Gln Phe Arg Val Leu Gly His Arg Gln Arg Ala Thr
   225 230 235 240
   Glu Ile Asn Leu Thr Thr Gly Asn Tyr Ser Arg Leu Ala Cys Glu Ile
   245 250 255
   Gln Phe Val Arg Ser Met Gly Tyr Tyr Leu Ile Gln Ile Tyr Ile Pro
   260 265 270
   Ser Gly Leu Ile Val Ile Ile Ser Trp Val Ser Phe Trp Leu Asn Arg
   275 280 285
   Asp Ala Thr Pro Ala Arg Val Ala Leu Gly Val Thr Thr Val Leu Thr
   290 295 300
   Met Thr Thr Leu Met Ser Ser Thr Asn Ala Ala Leu Pro Lys Ile Ser
   305 310 315 320
   Tyr Val Lys Ser Ile Asp Val Tyr Leu Gly Thr Cys Phe Val Met Val
   325 330 335
   Phe Ala Ser Leu Leu Glu Tyr Ala Thr Val Gly Tyr Met Ala Lys Arg
   340 345 350
   Ile Gln Ile Gly Lys Gln Arg Phe Met Ala Ile Gln Lys Ile Ala Glu
   355 360 365
   Gln Lys Lys Gln Gln Ala Ala Asp Ala Asn His Pro Pro Pro Pro Pro
   370 375 380
   Pro Val Ser Asp His Ser His Gly His Gly His Gly His Ser His Gly
   385 390 395 400
   His Gln His Thr Pro Lys Gln Gln Met Gly Ser Arg Ser Gly Pro Leu
   405 410 415
   Phe Gln Glu Val Arg Phe Lys Val His Asp Pro Lys Ala His Ser Lys
   420 425 430
   Gly Gly Thr Leu Glu Asn Thr Ile Asn Gly Gly Arg Gly Gly Gly Gly
   435 440 445
   Pro Pro Gly Gly Gly Gly Gly Pro Pro Gly Gly Gly Gly Gly Gly Pro
   450 455 460
   Asp Glu Glu Ser Gly Ala Pro Gln His Leu Ile His Pro Gly Lys Asp
   465 470 475 480
   Ile Asn Lys Leu Leu Gly Ile Thr Pro Ser Asp Ile Asp Lys Tyr Ser
   485 490 495
   Arg Ile Val Phe Pro Val Cys Phe Val Cys Phe Asn Leu Met Tyr Trp
   500 505 510
   Ile Ile Tyr Leu His Val Ser Asp Val Val Ala Asp Asp Leu Val Leu
   515 520 525
   Leu Gly Glu Glu Lys
   530
<210> 75
   <211> 176
   <212> PRT
   <213> Leptinotarsa decemlineata
<400> 75
   Thr Thr Val Leu Thr Met Thr Thr Leu Met Ser Ser Thr Asn Ala Ala
   1 5 10 15
   Leu Pro Lys Ile Ser Tyr Val Lys Ser Ile Asp Val Tyr Leu Gly Thr
   20 25 30
   Cys Phe Val Met Val Phe Ala Ser Leu Leu Glu Tyr Ala Thr Val Gly
   35 40 45
   Tyr Met Ala Lys Arg Ile Gln Met Arg Lys Asn Arg Phe Leu Ala Ile
   50 55 60
   Gln Lys Ile Ala Glu Gln Lys Lys Leu Asn Val Asp Gly Gly Pro Asp
   65 70 75 80
   Gly Asp His Ala Pro Lys Gln Thr Glu Val Arg Phe Lys Val His Asp
   85 90 95
   Pro Lys Ala His Ser Lys Gly Gly Thr Leu Glu Ser Thr Val Asn Gly
   100 105 110
   Gly Arg Gly Gly Asp Arg Gly Gly Gly Gly Pro Asp Glu Glu Ala Ala
   115 120 125
   Gly Pro Thr Pro Gln His Ile Ile His Pro Asn Lys Asp Val Asn Lys
   130 135 140
   Leu Tyr Gly Met Thr Pro Ser Asp Ile Asp Lys Tyr Ser Arg Ile Val
   145 150 155 160
   Phe Pro Val Cys Phe Val Cys Phe Asn Leu Met Tyr Trp Ile Ile Tyr
   165 170 175
<210> 76
   <211> 83
   <212> PRT
   <213> Arabidopsis thaliana
<400> 76
   Met Tyr Ser Lys Ala Gly Met Leu Leu Leu Leu Leu His Val Leu Gly
   1 5 10 15
   Phe Met Leu Leu Ala Ile Leu Arg Ile Lys Leu Leu Val Cys Met Phe
   20 25 30
   Leu Ser Leu Cys Leu Leu Phe Cys Ser Leu Cys Trp Phe Cys Leu Asn
   35 40 45
   Glu Trp Phe Asn Asn Pro Phe Gly Asn Leu Leu Phe Asp Val Cys Leu
   50 55 60
   Val Thr Leu Gly Met Gln Asn Tyr Leu Glu Ser Trp Phe Gln Asn Leu
   65 70 75 80
   Val Ser Phe
<210> 77
   <211> 71
   <212> PRT
   <213> Homo sapiens
<400> 77
   Gln Val Thr Val Arg Asp Pro Ser Gly Arg Pro Leu Arg Leu Pro Pro
   1 5 10 15
   Val Leu Pro His Pro Ile Phe Asp Asn His Asp Arg His Arg Ile Glu
   20 25 30
   Glu Lys Arg Lys Arg Thr Tyr Glu Thr Phe Lys Ser Ile Met Lys Lys
   35 40 45
   Ser Pro Phe Ser Gly Pro Thr Asp Pro Arg Pro Pro Pro Arg Arg Ile
   50 55 60
   Ala Val Pro Ser Arg Ser Ser
   65 70
<210>78
   <211> 3318
   <212> DNA
   <213> Homo sapiens
<400> 78
   cagatgtcca gttccagatg cctggaccca gagtgtgggg gaaatatctc tggagaagcc 60
   ctcactccaa aggctgtcca ggcgcaatgt ggtggctgct tctctgggga gtcctccagg 120
   cttgcccaac ccggggctcc gtcctcttgg cccaagagct accccagcag ctgacatccc 180
   ccgggtaccc agagccgtat ggcaaaggcc aagagagcag cacggacatc aaggctccag 240
   agggctttgc tgtgaggctc gtcttccagg acttcgacct ggagccgtcc caggactgtg 300
   caggggactc tgtcacaatc tcattcgtcg gttcggatcc aagccagttc tgtggtcagc 360
   aaggctcccc tctgggcagg ccccctggtc agagggagtt tgtatcctca gggaggagtt 420
   tgcggctgac cttccgcaca cagccttcct cggagaacaa gactgcccac ctccacaagg 480
   gcttcctggc cctctaccaa accgtggctg tgaactatag tcagcccatc agcgaggcca 540
   gcaggggctc tgaggccatc aacgcacctg gagacaaccc tgccaaggtc cagaaccact 600
   gccaggagcc ctattatcag gccgcggcag caggggcact cacctgtgca accccaggga 660
   cctggaaaga cagacaggat ggggaggagg ttcttcagtg tatgcctgtc tgcggacggc 720
   cagtcacccc cattgcccag aatcagacga ccctcggttc ttccagagcc aagctgggca 780
   acttcccctg gcaagccttc accagtatcc acggccgtgg gggcggggcc ctgctggggg 840
   acagatggat cctcactgct gcccacaccg tctaccccaa ggacagtgtt tctctcagga 900
   agaaccagag tgtgaatgtg ttcttgggcc acacagccat agatgagatg ctgaaactgg 960
   ggaaccaccc tgtccaccgt gtcgttgtgc accccgacta ccgtcagaat gagtcccata 1020
   actttagcgg ggacatcgcc ctcctggagc tgcagcacag catccccctg ggccccaacg 1080
   tcctcccggt ctgtctgccc gataatgaga ccctctaccg cagcggcttg ttgggctacg 1140
   tcagtgggtt tggcatggag atgggctggc taactactga gctgaagtac tcgaggctgc 1200
   ctgtagctcc cagggaggcc tgcaacgcct ggctccaaaa gagacagaga cccgaggtgt 1260
   tttctgacaa tatgttctgt gttggggatg agacgcaaag gcacagtgtc tgccaggggg 1320
   acagtggcag cgtctatgtg gtatgggaca atcatgccca tcactgggtg gccacgggca 1380
   ttgtgtcctg ggcatagggg tgtggcgaag ggtatgactt ctacaccaag gtgctcagct 1440
   atgtggactg gatcaaggga gtgatgaatg gcaagaattg accctggggg cttgaacagg 1500
   gactgaccag cacagtggag gccccaggca acagagggcc tggagtgagg actgaacact 1560
   ggggtagggg ttgggggtgg ggggttgggg gaggcagggg aaatcctatt cacatcactg 1620
   ttgcaccaag ccactgcaag agaaaccccc acccggcaag cccgccccat cccagacagg 1680
   aagcagagtc ccacagaccg ctcctcctca ccctctacct ccctgtgctc atgcactagg 1740
   ccccgggaag cctgtacatc tcaacaactt tcgccttgaa tgtccttaga accgccttcc 1800
   cctacttcat ctgttgacac agcttttata ctcacctgtg gaagagtcag ctactcaccc 1860
   gctattagag tatggaggaa ggggttttca ttgcattgca tttctgaaac attcctaaga 1920
   ccctttagtt gaccttcaaa tattcaagct attctgcagc tccaagatgc aattatagaa 1980
   acagctcctt ttttatttta tgtcctctat atgccaggtg cttcacctgt tatttcactt 2040
   aatcctcata ccatatttgc aaaggatgtg ttattatcta tgtgtgacaa atgaggaaac 2100
   tgaggctcag gggataaagg gacttgccca agtcccacag ctggtgtgtg actgcagaga 2160
   ctgtgctctt cccagtgtgc tgcaatactt ctcaaccctc ctctaacctg ctgtgtcacc 2220
   cgctttccct cccagccccc acatccttac cattttccct ccctgggaat tcctgcttct 2280
   gcgaaaatgg tatcctctag ctcacacttt cctaatggcc ccatctcctg cagaagccag 2340
   gtgagcccag cactggactg aagttcttgc agacacccca cctgtgcccc tatcatcagg 2400
   ggaactgctc cacctgagag gaccaactct ttaattttta gtaaaacctg gaggtgatgg 2460
   gccgggcgca gtggctcacg cctgtaatcc caacacctta ggagtccgag gtgggtggat 2520
   cacgaggtca ggagatccag cccatcctgg ccaacatggt gaaaccccat ctctactaaa 2580
   aatacaaaaa ttagccgggc gtggtgacac gtgcctgtag tcccagctac tcgggaggct 2640
   gaggcaggag aatcacttga acctgggagg cggaggttgc agtgagctaa gatcacgcca 2700
   ctgcactcca gcctgcggac agaccaagac ttcatccccc ccaaaaaaaa aagattggag 2760
   gtgatttaca gtgaaagaca caaataaaat acaactgttc aatggaaata gaaaataaac 2820
   accataaaag agagaagaga ggtaatttgt tagcatcaag agtcaagttg ctatatggtc 2880
   aaaggttaaa tttatctcta aaaaatggca ggattcaaag ttgtacatac atgtgattac 2940
   ttctgttttt tacacccaca tacagtacaa aagattatta aaaatattcc caaaaggcag 3000
   gtgcaatgat gcacacttat acccccagcc actcaggagg ctgatgcaag aggatcgctt 3060
   gagcccagga gttgaagtcc agcctaagca acatagtgaa accccatcgc caaaaatata 3120
   ataataattc tctcaaaata ctaaacagag gtggttttat tgataagatt ttggctgttt 3180
   ggttttccac tattctctat tggctaaaat ttgtttaatg agcatgaaat gtttttattt 3240
   tattttgctt atttttatga ttgcaaaaaa tgatatgagt ttctccctgc caaggcaaaa 3300
   aaatatatat atacctat                                               3318
<210> 79
   <211> 2386
   <212> DNA
   <213> Homo sapiens
<400> 79
   tgcacgaaga cgctgtcggg agagcccagg attcaacacg ggccttgaga aatgtggctc 60
   ttgtacctcc tggtgccggc cctgttctgc agggcaggag gctccattcc catccctcag 120
   aagttatttg gggaggtgac ttcccctctg ttccccaagc cttaccccaa caactttgaa 180
   acaaccactg tgatcacagt ccccacggga tacagggtga agctcgtctt ccagcagttt 240
   gacctggagc cttctgaagg ctgcttctat gattatgtca agatctctgc tgataagaaa 300
   agcctgggga ggttctgtgg gcaactgggt tctccactgg gcaacccccc gggaaagaag 360
   gaatttatgt cccaagggaa caagatgctg ctgaccttcc acacagactt ctccaacgag 420
   gagaatggga ccatcatgtt ctacaagggc ttcctggcct actaccaagc tgtggacctt 480
   gatgaatgtg cttcccggag caaattaggg gaggaggatc cccagcccca gtgccagcac 540
   ctgtgtcaca actacgttgg aggctacttc tgttcctgcc gtccaggcta tgagcttcag 600
   gaagacaggc attcctgcca ggctgagtgc agcagcgagc tgtacacgga ggcatcaggc 660
   tacatctcca gcctggagta ccctcggtcc tacccccctg acctgcgctg caactacagc 720
   atccgggtgg agcggggcct caccctgcac ctcaagttcc tggagccttt tgatattgat 780
   gaccaccagc aagtacactg cccctatgac cagctacaga tctatgccaa cgggaagaac 840
   attggcgagt tctgtgggaa gcaaaggccc cccgacctcg acaccagcag caatgctgtg 900
   gatctgctgt tcttcacaga tgagtcgggg gacagccggg gctggaagct gcgctacacc 960
   accgagatca tcaagtgccc ccagcccaag accctagacg agttcaccat catccagaac 1020
   ctgcagcctc agtaccagtt ccgtgactac ttcattgcta cctgcaagca aggctaccag 1080
   ctcatagagg ggaaccaggt gctgcattcc ttcacagctg tctgccagga tgatggcacg 1140
   tggcatcgtg ccatgcccag atgcaagatc aaggactgtg ggcagccccg aaacctgcct 1200
   aatggtgact tccgttacac caccacaatg ggagtgaaca cctacaaggc ccgtatccag 1260
   tactactgcc atgagccata ttacaagatg cagaccagag ctggcagcag ggagtctgag 1320
   caaggggtgt acacctgcac agcacagggc atttggaaga atgaacagaa gggagagaag 1380
   attcctcggt gcttgccagt gtgtgggaag cccgtgaacc ccgtggaaca gaggcagcgc 1440
   atcatcggag ggcaaaaagc caagatgggc aacttcccct ggcaggtgtt caccaacatc 1500
   cacgggcgcg ggggcggggc cctgctgggc gaccgctgga tcctcacagc tgcccacacc 1560
   ctgtatccca aggaacacga agcgcaaagc aacgcctctt tggatgtgtt cctgggccac 1620
   acaaatgtgg aagagctcat gaagctagga aatcacccca tccgcagggt cagcgtccac 1680
   ccggactacc gtcaggatga gtcctacaat tttgaggggg acatcgccct gctggagctg 1740
   gaaaatagtg tcaccctggg tcccaacctc ctccccatct gcctccctga caacgatacc 1800
   ttctacgacc tgggcttgat gggctatgtc agtggcttcg gggtcatgga ggagaagatt 1860
   gctcatgacc tcaggtttgt ccgtctgccc gtagctaatc cacaggcctg tgagaactgg 1920
   ctccggggaa agaataggat ggatgtgttc tctcaaaaca tgttctgtgc tggacaccca 1980
   tctctaaagc aggacgcctg ccagggggat agtgggggcg tttttgcagt aagggacccg 2040
   aacactgatc gctgggtggc cacgggcatc gtgtcctggg gcatcgggtg cagcaggggc 2100
   tatggcttct acaccaaagt gctcaactac gtggactgga tcaagaaaga gatggaggag 2160
   gaggactgag cccagaattc actaggttcg aatccagaga gcagtgtgga aaaaaaaaaa 2220
   caaaaaacaa ctgaccagtt gttgataacc actaagagtc tctattaaaa ttactgatgc 2280
   agaaagaccg tgtgtgaaat tctctttcct gtagtcccat tgatgtactt tacctgaaac 2340
   aaccaaaggg cccctttctt tcttctgagg attgcagagg atatag                2386
<210> 80
   <211> 487
   <212> PRT
   <213> Homo sapiens
<400> 80
   Met Pro Gly Pro Arg Val Trp Gly Lys Tyr Leu Trp Arg Ser Pro His
   1 5 10 15
   Ser Lys Gly Cys Pro Gly Ala Met Trp Trp Leu Leu Leu Trp Gly Val
   20 25 30
   Leu Gln Ala Cys Pro Thr Arg Gly Ser Val Leu Leu Ala Gln Glu Leu
   35 40 45
   Pro Gln Gln Leu Thr Ser Pro Gly Tyr Pro Glu Pro Tyr Gly Lys Gly
   50 55 60
   Gln Glu Ser Ser Thr Asp Ile Lys Ala Pro Glu Gly Phe Ala Val Arg
   65 70 75 80
   Leu Val Phe Gln Asp Phe Asp Leu Glu Pro Ser Gln Asp Cys Ala Gly
   85 90 95
   Asp Ser Val Thr Ile Ser Phe Val Gly Ser Asp Pro Ser Gln Phe Cys
   100 105 110
   Gly Gln Gln Gly Ser Pro Leu Gly Arg Pro Pro Gly Gln Arg Glu Phe
   115 120 125
   Val Ser Ser Gly Arg Ser Leu Arg Leu Thr Phe Arg Thr Gln Pro Ser
   130 135 140
   Ser Glu Asn Lys Thr Ala His Leu His Lys Gly Phe Leu Ala Leu Tyr
   145 150 155 160
   Gln Thr Val Ala Val Asn Tyr Ser Gln Pro Ile Ser Glu Ala Ser Arg
   165 170 175
   Gly Ser Glu Ala Ile Asn Ala Pro Gly Asp Asn Pro Ala Lys Val Gln
   180 185 190
   Asn His Cys Gln Glu Pro Tyr Tyr Gln Ala Ala Ala Ala Gly Ala Leu
   195 200 205
   Thr Cys Ala Thr Pro Gly Thr Trp Lys Asp Arg Gln Asp Gly Glu Glu
   210 215 220
   Val Leu Gln Cys Met Pro Val Cys Gly Arg Pro Val Thr Pro Ile Ala
   225 230 235 240
   Gln Asn Gln Thr Thr Leu Gly Ser Ser Arg Ala Lys Leu Gly Asn Phe
   245 250 255
   Pro Trp Gln Ala Phe Thr Ser Ile His Gly Arg Gly Gly Gly Ala Leu
   260 265 270
   Leu Gly Asp Arg Trp Ile Leu Thr Ala Ala His Thr Val Tyr Pro Lys
   275 280 285
   Asp Ser Val Ser Leu Arg Lys Asn Gln Ser Val Asn Val Phe Leu Gly
   290 295 300
   His Thr Ala Ile Asp Glu Met Leu Lys Leu Gly Asn His Pro Val His
   305 310 315 320
   Arg Val Val Val His Pro Asp Tyr Arg Gln Asn Glu Ser His Asn Phe
   325 330 335
   Ser Gly Asp Ile Ala Leu Leu Glu Leu Gln His Ser Ile Pro Leu Gly
   340 345 350
   Pro Asn Val Leu Pro Val Cys Leu Pro Asp Asn Glu Thr Leu Tyr Arg
   355 360 365
   Ser Gly Leu Leu Gly Tyr Val Ser Gly Phe Gly Met Glu Met Gly Trp
   370 375 380
   Leu Thr Thr Glu Leu Lys Tyr Ser Arg Leu Pro Val Ala Pro Arg Glu
   385 390 395 400
   Ala Cys Asn Ala Trp Leu Gln Lys Arg Gln Arg Pro Glu Val Phe Ser
   405 410 415
   Asp Asn Met Phe Cys Val Gly Asp Glu Thr Gln Arg His Ser Val Cys
   420 425 430
   Gln Gly Asp Ser Gly Ser Val Tyr Val Val Trp Asp Asn His Ala His
   435 440 445
   His Trp Val Ala Thr Gly Ile Val Ser Trp Gly Ile Gly Cys Gly Glu
   450 455 460
   Gly Tyr Asp Phe Tyr Thr Lys Val Leu Ser Tyr Val Asp Trp Ile Lys
   465 470 475 480
   Gly Val Met Asn Gly Lys Asn
   485
<210> 81
   <211> 705
   <212> PRT
   <213> Homo sapiens
<400> 81
   Met Trp Leu Leu Tyr Leu Leu Val Pro Ala Leu Phe Cys Arg Ala Gly
   1 5 10 15
   Gly Ser Ile Pro Ile Pro Gln Lys Leu Phe Gly Glu Val Thr Ser Pro
   20 25 30
   Leu Phe Pro Lys Pro Tyr Pro Asn Asn Phe Glu Thr Thr Thr Val Ile
   35 40 45
   Thr Val Pro Thr Gly Tyr Arg Val Lys Leu Val Phe Gln Gln Phe Asp
   50 55 60
   Leu Glu Pro Ser Glu Gly Cys Phe Tyr Asp Tyr Val Lys Ile Ser Ala
   65 70 75 80
   Asp Lys Lys Ser Leu Gly Arg Phe Cys Gly Gln Leu Gly Ser Pro Leu
   85 90 95
   Gly Asn Pro Pro Gly Lys Lys Glu Phe Met Ser Gln Gly Asn Lys Met
   100 105 110
   Leu Leu Thr Phe His Thr Asp Phe Ser Asn Glu Glu Asn Gly Thr Ile
   115 120 125
   Met Phe Tyr Lys Gly Phe Leu Ala Tyr Tyr Gln Ala Val Asp Leu Asp
   130 135 140
   Glu Cys Ala Ser Arg Ser Lys Ser Gly Glu Glu Asp Pro Gln Pro Gln
   145 150 155 160
   Cys Gln His Leu Cys His Asn Tyr Val Gly Gly Tyr Phe Cys Ser Cys
   165 170 175
   Arg Pro Gly Tyr Glu Leu Gln Glu Asp Arg His Ser Cys Gln Ala Glu
   180 185 190
   Cys Ser Ser Glu Leu Tyr Thr Glu Ala Ser Gly Tyr Ile Ser Ser Leu
   195 200 205
   Glu Tyr Pro Arg Ser Tyr Pro Pro Asp Leu Arg Cys Asn Tyr Ser Ile
   210 215 220
   Arg Val Glu Arg Gly Leu Thr Leu His Leu Lys Phe Leu Glu Pro Phe
   225 230 235 240
   Asp Ile Asp Asp His Gln Gln Val His Cys Pro Tyr Asp Gln Leu Gln
   245 250 255
   Ile Tyr Ala Asn Gly Lys Asn Ile Gly Glu Phe Cys Gly Lys Gln Arg
   260 265 270
   Pro Pro Asp Leu Asp Thr Ser Ser Asn Ala Val Asp Leu Leu Phe Phe
   275 280 285
   Thr Asp Glu Ser Gly Asp Ser Arg Gly Trp Lys Leu Arg Tyr Thr Thr
   290 295 300
   Glu Ile Ile Lys Cys Pro Gln Pro Lys Thr Leu Asp Glu Phe Thr Ile
   305 310 315 320
   Ile Gln Asn Leu Gln Pro Gln Tyr Gln Phe Arg Asp Tyr Phe Ile Ala
   325 330 335
   Thr Cys Lys Gln Gly Tyr Gln Leu Ile Glu Gly Asn Gln Val Leu His
   340 345 350
   Ser Phe Thr Ala Val Cys Gln Asp Asp Gly Thr Trp His Arg Ala Met
   355 360 365
   Pro Arg Cys Lys Ile Lys Asp Cys Gly Gln Pro Arg Asn Leu Pro Asn
   370 375 380
   Gly Asp Phe Arg Tyr Thr Thr Thr Met Gly Val Asn Thr Tyr Lys Ala
   385 390 395 400
   Arg Ile Gln Tyr Tyr Cys His Glu Pro Tyr Tyr Lys Met Gln Thr Arg
   405 410 415
   Ala Gly Ser Arg Glu Ser Glu Gln Gly Val Tyr Thr Cys Thr Ala Gln
   420 425 430
   Gly Ile Trp Lys Asn Glu Gln Lys Gly Glu Lys Ile Pro Arg Cys Leu
   435 440 445
   Pro Val Cys Gly Lys Pro Val Asn Pro Val Glu Gln Arg Gln Arg Ile
   450 455 460
   Ile Gly Gly Gln Lys Ala Lys Met Gly Asn Phe Pro Trp Gln Val Phe
   465 470 475 480
   Thr Asn Ile His Gly Arg Gly Gly Gly Ala Leu Leu Gly Asp Arg Trp
   485 490 495
   Ile Leu Thr Ala Ala His Thr Leu Tyr Pro Lys Glu His Glu Ala Gln
   500 505 510
   Ser Asn Ala Ser Leu Asp Val Phe Leu Gly His Thr Asn Val Glu Glu
   515 520 525
   Leu Met Lys Leu Gly Asn His Pro Ile Arg Arg Val Ser Val His Pro
   530 535 540
   Asp Tyr Arg Gln Asp Glu Ser Tyr Asn Phe Glu Gly Asp Ile Ala Leu
   545 550 555 560
   Leu Glu Leu Glu Asn Ser Val Thr Leu Gly Pro Asn Leu Leu Pro Ile
   565 570 575
   Cys Leu Pro Asp Asn Asp Thr Phe Tyr Asp Leu Gly Leu Met Gly Tyr
   580 585 590
   Val Ser Gly Phe Gly Val Met Glu Glu Lys Ile Ala His Asp Leu Arg
   595 600 605
   Phe Val Arg Leu Pro Val Ala Asn Pro Gln Ala Cys Glu Asn Trp Leu
   610 615 620
   Arg Gly Lys Asn Arg Met Asp Val Phe Ser Gln Asn Met Phe Cys Ala
   625 630 635 640
   Gly His Pro Ser Leu Lys Gln Asp Ala Cys Gln Gly Asp Ser Gly Gly
   645 650 655
   Val Phe Ala Val Arg Asp Pro Asn Thr Asp Arg Trp Val Ala Thr Gly
   660 665 670
   Ile Val Ser Trp Gly Ile Gly Cys Ser Arg Gly Tyr Gly Phe Tyr Thr
   675 680 685
   Lys Val Leu Asn Tyr Val Asp Trp Ile Lys Lys Glu Met Glu Glu Glu
   690 695 700
   Asp
   705
<210> 82
   <211> 1746
   <212> DNA
   <213> Macaca fascicularis
<400> 82
   aataaattga ggccgctcac ccacggtacc cacctagtat atagagacag aattcaaact 60
   ctggctctag cacttgtgct ttctgctaca ccagctcaag gaagtttgaa gacctacaga 120
   agggctgatt ttagaaggtt aatcaaaaac ccaaggacag tttcatcatg tcataaccaa 180
   agacccttgt ggcacctgct gtcatgggat aacaaatatc ttgtggggtt ctgaatgtgg 240
   acttattact gaagctcctg tctgcttggt cagtggtggt ctagactaac ttctggtcct 300
   gagattctaa agtgttggta gaccggttga gataaaagat atataataat gaatgcctta 360
   cctatctgaa aaccagtttg atccgtgcca aggggctttt tgtgggctct gtagagtgcc 420
   ctaaacccag ctctgccttt gctgtgttag acagaagcac gccattcaca tctctggggc 480
   ccccaatggt gccatggtgt ggttgtggtc tgctcactgg ctcttctgtt ttttgttttt 540
   gtttttcctg cctttttcca atcctcacac cttctgagct acagccccag tagggtctaa 600
   atgtcctaga gctatatgag atttaggttt ctgagcacag ccaattctcc cacttttgag 660
   gcttcccttc ccctttcact cgcccctctc tggttctctg ccaccagtcc agaagaactg 720
   aatgtcgtgc tggggaccaa cgacttaact agctcatcca tggaaataaa ggaggtcgcc 780
   agcatcattc ttcacaagga ctttaagaga gccaacatgg acaatgacat tgccttgctg 840
   ctgctggcct cgcccatcac actcgatgac ctgaaggtgc ccatctgcct ccctacgcag 900
   cacggccccg ccacatggca cgaatgctgg gtggcaggtt ggggccagac caatgctgct 960
   gacaaaaact ctgtgaaaac ggatctgatg aaagcgccga tggtcatcat ggactgggag 1020
   gagtgttcaa aggcgtttcc aaaactcacc aaaaatatgc tgtgtgctgg atacaataat 1080
   gagagctatg acgcctgcca gggtgacagc gggggacctc tggtctgcac cccagagcct 1140
   ggtgagaagt ggtaccaggt gggtatcatc agctggggaa agagctgtgg agagaagaac 1200
   accccaggga tatacacctc gttggtgaac tacaacctct ggatcgagaa ggtgacccag 1260
   ctagagggca ggcccttcag tgcggagaaa atgaggacct ctgtcaaaca gaaacctatg 1320
   ggctcccgag tctcgggggt cccagagcca ggcggcctca gatcctggct cctgctctgt 1380
   cccctgtccc atgtgttgtt cagagctatt ttgtactgat aataaaatag aggctatttt 1440
   tttaaccaag ggagggtgca tgaaaatgtg tctccagcag aggctctggc tgcagctcag 1500
   ggctcaagga tggaaactga gactggaacc aggagaacca gaaagtcagg ctggggccct 1560
   ggtttgggga ctgcactttg ggtctgtgga ttagtcagga ctctctccat tctaggtgac 1620
   agtcacctaa gtctgactga attcagccaa aatgaggcat ttatggatac atataacagg 1680
   aaaataaaaa taaaaataaa aaaccgacac acaaataaaa aacaaaaaaa aaaaaaaaag 1740
   gccaca                                                            1746
<210> 83
   <211> 1047
   <212> DNA
   <213> Mus musculus
<400> 83
   gactattcct gtcagccgtg gcctccaaca caccagcaca gccgagagcc gatgatccgt 60
   gccctcgcat ccctgctact tgttggccca caccctgtga agcaaatgtt gtagtgtggt 120
   gtgagacccc tgctatgata gcagaattca atactccagg atcatagaag ggcaggaggc 180
   tgagctgggt gagtttccat ggcaggtgag cattcaggaa agtgaccacc atttctgcgg 240
   cggctccatt ctcagtgagt ggtggatcct caccgtggcc cactgcttct atgctcagga 300
   gctttcccca acagatctca gagtcagagt gggaaccaat gacttaacta cttcacccgt 360
   ggaactagag gtcaccacca taatccggca caaaggcttt aaacggctga acatggacaa 420
   cgacattgcc ttgttgctgc tagccaagcc cttgacgttc aatgagctga cggtgcccat 480
   ctgccttcct ctctggcccg cccctcccag ctggcacgaa tgctgggtgg caggatgggg 540
   cgtaaccaac tcaactgaca aggaatctat gtcaacggat ctgatgaagg tgcccatgcg 600
   tatcatagag tgggaggaat gcttacagat gtttcccagc ctcaccacaa acatgctgtg 660
   tgcctcatat ggtaatgaga gctacgatgc ttgccagtgg gggaccgctt gtctgcacca 720
   cagatcctgg cagtaggtgg taccaggtgg gcatcatcag ctggggcaag agctgtggaa 780
   aaaaaggctt cccagggata tatactgtat tggcaaagta taccctgtgg attgagaaaa 840
   tagcccagac agaggggaag cccctggatt ttagaggtca gagctcctct aacaagaaga 900
   aaaacagaca gaacaatcag ctctccaaat ccccagccct gaactgcccc caaagctggc 960
   tcctgccctg tctgctgtcc tttgcactgc ttagagcctt gtccaactgg aaataaaaca 1020
   atgcagtctc tgatccaccc taacccg                                     1047
<210> 84
   <211> 267
   <212> PRT
   <213> Macaca fascicularis
<400> 84
   Met Arg Phe Arg Phe Leu Ser Thr Ala Asn Ser Pro Thr Phe Glu Ala
   1               5                   10                  15
   Ser Leu Pro Leu Ser Leu Ala Pro Leu Trp Phe Ser Ala Thr Ser Pro
               20                  25                  30
   Glu Glu Leu Asn Val Val Leu Gly Thr Asn Asp Leu Thr Ser Ser Ser
           35                  40                  45
   Met Glu Ile Lys Glu Val Ala Ser Ile Ile Leu His Lys Asp Phe Lys
       50                  55                  60
   Arg Ala Asn Met Asp Asn Asp Ile Ala Leu Leu Leu Leu Ala Ser Pro
   65                  70                  75                  80
   Ile Thr Leu Asp Asp Leu Lys Val Pro Ile Cys Leu Pro Thr Gln His
                   85                  90                  95
   Gly Pro Ala Thr Trp His Glu Cys Trp Val Ala Gly Trp Gly Gln Thr
               100                 105                 110
   Asn Ala Ala Asp Lys Asn Ser Val Lys Thr Asp Leu Met Lys Ala Pro
           115                 120                 125
   Met Val Ile Met Asp Trp Glu Glu Cys Ser Lys Ala Phe Pro Lys Leu
   130 135 140
   Thr Lys Asn Met Leu Cys Ala Gly Tyr Asn Asn Glu Ser Tyr Asp Ala
   145 150 155 160
   Cys Gln Gly Asp Ser Gly Gly Pro Leu Val Cys Thr Pro Glu Pro Gly
   165 170 175
   Glu Lys Trp Tyr Gln Val Gly Ile Ile Ser Trp Gly Lys Ser Cys Gly
   180 185 190
   Glu Lys Asn Thr Pro Gly Ile Tyr Thr Ser Leu Val Asn Tyr Asn Leu
   195 200 205
   Trp Ile Glu Lys Val Thr Gln Leu Glu Gly Arg Pro Phe Ser Ala Glu
   210 215 220
   Lys Met Arg Thr Ser Val Lys Gln Lys Pro Met Gly Ser Arg Val Ser
   225 230 235 240
   Gly Val Pro Glu Pro Gly Gly Leu Arg Ser Trp Leu Leu Leu Cys Pro
   245 250 255
   Leu Ser His Val Leu Phe Arg Ala Ile Leu Tyr
   260 265
<210> 85
   <211> 638
   <212> PRT
   <213> Homo sapiens
<400> 85
   Met Ile Leu Phe Lys Gln Ala Thr Tyr Phe Ile Ser Leu Phe Ala Thr
   1 5 10 15
   Val Ser Cys Gly Cys Leu Thr Gln Leu Tyr Glu Asn Ala Phe Phe Arg
   20 25 30
   Gly Gly Asp Val Ala Ser Met Tyr Thr Pro Asn Ala Gln Tyr Cys Gln
   35 40 45
   Met Arg Cys Thr Phe His Pro Arg Cys Leu Leu Phe Ser Phe Leu Pro
   50 55 60
   Ala Ser Ser Ile Asn Asp Met Glu Lys Arg Phe Gly Cys Phe Leu Lys
   65                  70                  75                  80
   Asp Ser Val Thr Gly Thr Leu Pro Lys Val His Arg Thr Gly Ala Val
                   85                  90                  95
   Ser Gly His Ser Leu Lys Gln Cys Gly His Gln Ile Ser Ala Cys His
               100                 105                 110
   Arg Asp Ile Tyr Lys Gly Val Asp Met Arg Gly Val Asn Phe Asn Val
           115                 120                 125
   Ser Lys Val Ser Ser Val Glu Glu Cys Gln Lys Arg Cys Thr Asn Asn
       130                 135                 140
   Ile Arg Cys Gln Phe Phe Ser Tyr Ala Thr Gln Thr Phe His Lys Ala
   145                 150                 155                 160
   Glu Tyr Arg Asn Asn Cys Leu Leu Lys Tyr Ser Pro Gly Gly Thr Pro
                   165                 170                 175
   Thr Ala Ile Lys Val Leu Ser Asn Val Glu Ser Gly Phe Ser Leu Lys
               180                 185                 190
   Pro Cys Ala Leu Ser Glu Ile Gly Cys His Met Asn Ile Phe Gln His
           195                 200                 205
   Leu Ala Phe Ser Asp Val Asp Val Ala Arg Val Leu Thr Pro Asp Ala
       210                 215                 220
   Phe Val Cys Arg Thr Ile Cys Thr Tyr His Pro Asn Cys Leu Phe Phe
   225                 230                 235                 240
   Thr Phe Tyr Thr Asn Val Trp Lys Ile Glu Ser Gln Arg Asn Val Cys
                   245                 250                 255
   Leu Leu Lys Thr Ser Glu Ser Gly Thr Pro Ser Ser Ser Thr Pro Gln
               260                 265                 270
   Glu Asn Thr Ile Ser Gly Tyr Ser Leu Leu Thr Cys Lys Arg Thr Leu
           275                 280                 285
   Pro Glu Pro Cys His Ser Lys Ile Tyr Pro Gly Val Asp Phe Gly Gly
       290                 295                 300
   Glu Glu Leu Asn Val Thr Phe Val Lys Gly Val Asn Val Cys Gln Glu
   305                 310                 315                 320
   Thr Cys Thr Lys Met Ile Arg Cys Gln Phe Phe Thr Tyr Ser Leu Leu
   325 330 335
   Pro Glu Asp Cys Lys Glu Glu Lys Cys Lys Cys Phe Leu Arg Leu Ser
   340 345 350
   Met Asp Gly Ser Pro Thr Arg Ile Ala Tyr Gly Thr Gln Gly Ser Ser
   355 360 365
   Gly Tyr Ser Leu Arg Leu Cys Asn Thr Gly Asp Asn Ser Val Cys Thr
   370 375 380
   Thr Lys Thr Ser Thr Arg Ile Val Gly Gly Thr Asn Ser Ser Trp Gly
   385 390 395 400
   Glu Trp Pro Trp Gln Val Ser Leu Gln Val Lys Leu Thr Ala Gln Arg
   405 410 415
   His Leu Cys Gly Gly Ser Leu Ile Gly His Gln Trp Val Leu Thr Ala
   420 425 430
   Ala His Cys Phe Asp Gly Leu Pro Leu Gln Asp Val Trp Arg Ile Tyr
   435 440 445
   Ser Gly Ile Leu Asn Leu Ser Asp Ile Thr Lys Asp Thr Pro Phe Ser
   450 455 460
   Gln Ile Lys Glu Ile Ile Ile His Gln Asn Tyr Lys Val Ser Glu Gly
   465 470 475 480
   Asn His Asp Ile Ala Leu Ile Lys Leu Gln Ala Pro Leu Asn Tyr Thr
   485 490 495
   Glu Phe Gln Lys Pro Ile Cys Leu Pro Ser Lys Gly Asp Thr Ser Thr
   500 505 510
   Ile Tyr Thr Asn Cys Trp Val Thr Gly Trp Gly Phe Ser Lys Glu Lys
   515 520 525
   Gly Glu Ile Gln Asn Ile Leu Gln Lys Val Asn Ile Pro Leu Val Thr
   530 535 540
   Asn Glu Glu Cys Gln Lys Arg Tyr Gln Asp Tyr Lys Ile Thr Gln Arg
   545 550 555 560
   Met Val Cys Ala Gly Tyr Lys Glu Gly Gly Lys Asp Ala Cys Lys Gly
   565 570 575
   Asp Ser Gly Gly Pro Leu Val Cys Lys His Asn Gly Met Trp Arg Leu
   580 585 590
   Val Gly Ile Thr Ser Trp Gly Glu Gly Cys Ala Arg Arg Glu Gln Pro
   595 600 605
   Gly Val Tyr Thr Lys Val Ala Glu Tyr Met Asp Trp Ile Leu Glu Lys
   610 615 620
   Thr Gln Ser Ser Asp Gly Lys Ala Gln Met Gln Ser Pro Ala
   625 630 635
<210> 86
   <211> 643
   <212> PRT
   <213> Sus scrofa
<400> 86
   Met Glu Val Ile Val Leu Phe Arg Ile Ile Ser Phe Arg Gln Ala Val
   1 5 10 15
   Tyr Phe Met Cys Leu Phe Ala Ala Val Ser Cys Gly Cys Leu Pro Gln
   20 25 30
   Leu His Lys Asn Thr Phe Phe Arg Gly Gly Asp Val Ser Ala Met Tyr
   35 40 45
   Thr Pro Ser Ala Arg His Cys Gln Met Met Cys Thr Phe His Pro Arg
   50 55 60
   Cys Leu Leu Phe Ser Phe Leu Pro Ala Asp Ser Thr Ser Val Thr Asp
   65 70 75 80
   Lys Arg Phe Gly Cys Phe Leu Lys Asp Ser Val Thr Gly Met Leu Pro
   85 90 95
   Arg Val Leu Arg Glu Asn Ala Ile Ser Gly His Ser Leu Lys Gln Cys
   100 105 110
   Gly His Gln Ile Arg Ala Cys His Arg Asp Ile Tyr Lys Gly Ile Asp
   115 120 125
   Met Arg Gly Val Asn Phe Asn Val Ser Lys Val Lys Thr Val Glu Glu
   130 135 140
   Cys Gln Glu Arg Cys Thr Asn Ser Ile His Cys Leu Phe Phe Thr Tyr
   145 150 155 160
   Ala Thr Gln Ala Phe Asn Asn Ala Glu Tyr Arg Asn Asn Cys Leu Leu
                   165                 170                 175
   Lys His Ser Pro Gly Gly Thr Pro Thr Ser Ile Lys Val Leu Ala Asn
               180                 185                 190
   Val Glu Ser Gly Phe Ser Leu Lys Pro Cys Ala Asp Ser Glu Ile Gly
           195                 200                 205
   Cys His Met Asp Ile Phe Gln His Leu Ala Phe Ser Asp Val Asp Val
       210                 215                 220
   Ala Arg Val Ile Ala Pro Asp Ala Phe Val Cys Arg Thr Ile Cys Thr
   225                 230                 235                 240
   Tyr His Pro Asn Cys Leu Phe Phe Thr Phe Tyr Thr Asn Ala Trp Lys
                   245                 250                 255
   Ile Glu Ser Gln Arg Asn Val Cys Phe Leu Lys Thr Ser His Ser Gly
               260                 265                 270
   Thr Pro Ser Phe Pro Thr Pro Gln Glu Asn Ala Ile Ser Gly Tyr Ser
           275                 280                 285
   Leu Leu Thr Cys Lys Gln Thr Leu Pro Glu Pro Cys His Ser Lys Ile
       290                 295                 300
   Tyr Ser Glu Val Asp Phe Glu Gly Glu Glu Leu Asn Val Thr Phe Val
   305                 310                 315                 320
   Gln Gly Ala Asn Leu Cys Gln Glu Thr Cys Thr Lys Thr Ile Arg Cys
                   325                 330                 335
   Gln Phe Phe Thr Tyr Ser Leu His Pro Glu Asp Cys Arg Gly Glu Lys
               340                 345                 350
   Cys Lys Cys Ser Leu Arg Leu Ser Ser Asp Gly Ser Pro Thr Lys Ile
           355                 360                 365
   Thr His Gly Met Arg Ala Ser Ser Gly Tyr Ser Leu Arg Leu Cys Arg
       370                 375                 380
   Ser Gly Asp His Ser Ala Cys Ala Thr Lys Ala Asn Thr Arg Ile Val
   385                 390                 395                 400
   Gly Gly Thr Asp Ser Phe Leu Gly Glu Trp Pro Trp Gln Val Ser Leu
                   405                 410                 415
   Gln Ala Lys Leu Arg Ala Gln Asn His Leu Cys Gly Gly Ser Ile Ile
   420 425 430
   Gly His Gln Trp Val Leu Thr Ala Ala His Cys Phe Asp Gly Leu Ser
   435 440 445
   Leu Pro Asp Ile Trp Arg Ile Tyr Gly Gly Ile Leu Asn Ile Ser Glu
   450 455 460
   Ile Thr Lys Glu Thr Pro Phe Ser Gln Val Lys Glu Ile Ile Ile His
   465 470 475 480
   Gln Asn Tyr Lys Ile Leu Glu Ser Gly His Asp Ile Ala Leu Leu Lys
   485 490 495
   Leu Glu Thr Pro Leu Asn Tyr Thr Asp Phe Gln Lys Pro Ile Cys Leu
   500 505 510
   Pro Ser Arg Asp Asp Thr Asn Val Val Tyr Thr Asn Cys Trp Val Thr
   515 520 525
   Gly Trp Gly Phe Thr Glu Glu Lys Gly Glu Ile Gln Asn Ile Leu Gln
   530 535 540
   Lys Val Asn Ile Pro Leu Val Ser Asn Glu Glu Cys Gln Lys Ser Tyr
   545 550 555 560
   Arg Asp His Lys Ile Ser Lys Gln Met Ile Cys Ala Gly Tyr Lys Glu
   565 570 575
   Gly Gly Lys Asp Ala Cys Lys Gly Glu Ser Gly Gly Pro Leu Val Cys
   580 585 590
   Lys Tyr Asn Gly Ile Trp His Leu Val Gly Thr Thr Ser Trp Gly Glu
   595 600 605
   Gly Cys Ala Arg Arg Glu Gln Pro Gly Val Tyr Thr Lys Val Ile Glu
   610 615 620
   Tyr Met Asp Trp Ile Leu Glu Lys Thr Gln Asp Asp Asp Gly Gln Ser
   625 630 635 640
   Trp Met Lys
<210> 87
   <211> 625
   <212> PRT
   <213> Homo sapiens
<400> 87
<210> 88
   <211> 257
   <212> PRT
   <213> Homo sapiens
<400> 88
   Met Ile Ala Ile Ser Ala Val Ser Ser Ala Leu Leu Phe Ser Leu Leu
   1 5 10 15
   Cys Glu Ala Ser Thr Val Val Leu Leu Asn Ser Thr Asp Ser Ser Pro
   20 25 30
   Pro Thr Asn Asn Phe Thr Asp Ile Glu Ala Ala Leu Lys Ala Gln Leu
   35 40 45
   Asp Ser Ala Asp Ile Pro Lys Ala Arg Arg Lys Arg Tyr Ile Ser Gln
   50 55 60
   Asn Asp Met Ile Ala Ile Leu Asp Tyr His Asn Gln Val Arg Gly Lys
   65 70 75 80
   Val Phe Pro Pro Ala Ala Asn Met Glu Tyr Met Val Trp Asp Glu Asn
   85 90 95
   Leu Ala Lys Ser Ala Glu Ala Trp Ala Ala Thr Cys Ile Trp Asp His
   100 105 110
   Gly Pro Ser Tyr Leu Leu Arg Phe Leu Gly Gln Asn Leu Ser Val Arg
   115 120 125
   Thr Gly Arg Tyr Arg Ser Ile Leu Gln Leu Val Lys Pro Trp Tyr Asp
   130 135 140
   Glu Val Lys Asp Tyr Ala Phe Pro Tyr Pro Gln Asp Cys Asn Pro Arg
   145 150 155 160
   Cys Pro Met Arg Cys Phe Gly Pro Met Cys Thr His Tyr Thr Gln Met
   165 170 175
   Val Trp Ala Thr Ser Asn Arg Ile Gly Cys Ala Ile His Thr Cys Gln
   180 185 190
   Asn Met Asn Val Trp Gly Ser Val Trp Arg Arg Ala Val Tyr Leu Val
   195 200 205
   Cys Asn Tyr Ala Pro Lys Gly Asn Trp Ile Gly Glu Ala Pro Tyr Lys
   210 215 220
   Val Gly Val Pro Cys Ser Ser Cys Pro Pro Ser Tyr Gly Gly Ser Cys
   225 230 235 240
   Thr Asp Asn Leu Cys Phe Pro Gly Val Thr Ser Asn Tyr Leu Tyr Trp
   245 250 255
   Phe
<210> 89
   <211> 415
   <212> PRT
   <213> Halocynthia roretzi
<400> 89
   Met Leu Ile Val Gln Ile Asn Met Lys Leu Ser Val Phe Phe Leu Ala
   1 5 10 15
   Leu Leu Pro Leu Val Ala Arg Thr Ser Phe Ala Ser Asn Pro Asn Val
               20                  25                  30
   Leu Ser Ala Glu Glu Asn Trp Ser Asn Leu Val Gly Thr Glu Glu Ile
           35                  40                  45
   Glu Asn Val Asn Ser Glu Asn Glu Phe Ser Leu Ala Thr Glu Glu Glu
       50                  55                  60
   Arg Ser Asn Phe Glu Glu Asn Asn Val Ile Leu Ser Glu Glu Lys Val
   65                  70                  75                  80
   Val Glu Glu Ile Thr Ala Arg Trp Asp Ile Gly Leu Asp Pro Asp Ala
                   85                  90                  95
   Asn Glu Thr Phe Ser Val Lys Lys Ala Val Lys Ala Val Lys Ile Ile
               100                 105                 110
   Pro Gly Lys Ile Met Asp Lys Ile Val Leu Lys Lys Pro Phe Arg Met
           115                 120                 125
   Ala Leu Leu Arg Thr His Asn Ala Arg Arg Ala Ile Ala Gln Pro Lys
       130                 135                 140
   Ala Ala Asn Met Arg Arg Met Thr Trp Asp Met Glu Leu Glu Arg Leu
   145                 150                 155                 160
   Ala Val Ala Tyr Ser Arg Lys Cys Ile Tyr Glu His Asn Pro Arg Thr
                   165                 170                 175
   Lys His Ser Arg Phe Glu Tyr Val Gly Glu Asn Leu Phe Ile Ser Thr
               180                 185                 190
   Gly Tyr Ala Phe Thr Pro Ser Leu Met Lys His Ala Val Glu Ala Trp
           195                 200                 205
   Asp Asp Glu Lys Gln Tyr Tyr Asp Tyr Glu Thr Lys Lys Cys Gln Arg
       210                 215                 220
   Gly Lys Met Cys Gly His Tyr Thr Gln Val Val Trp Ala Asp Thr Phe
   225                 230                 235                 240
   Lys Met Gly Cys Gly Val Thr Arg Cys Ser Asp Ile Asp Val Arg Gly
                   245                 250                 255
   Arg Arg Trp Lys Asn Ala Ile Leu Leu Val Cys Asn Tyr Gly Pro Gly
               260                 265                 270
   Gly Asn Tyr Pro Thr His Pro Phe Val Thr Ala Pro Ser Cys Ser Lys
   275 280 285
   Cys Ala Pro Thr Asp Ile Cys Arg Arg Asn Leu Cys Asn Asn Val Ile
   290 295 300
   Arg Asp Arg Leu Lys Leu Asp Arg Lys Asp Ile Lys Trp Ser Glu Trp
   305 310 315 320
   Thr Thr Trp Ser Ser Cys Ser Lys Ser Cys Gly Val Gly Ser Thr Arg
   325 330 335
   Arg Glu Arg Gln Cys Asn Thr Phe Val Pro Gly Asp Cys Lys Asp Phe
   340 345 350
   Pro Ser Glu Val Lys Phe Cys Val Lys Lys Pro Cys Lys Ala Ala Met
   355 360 365
   Phe Gly Asn Gly Gly Ser Phe Ser Tyr Asn Ile Val Met Asn Gln Gly
   370 375 380
   Asp Lys Leu Leu Lys Gly Ser Leu Gln Gln Ala Leu Gln Lys His Leu
   385 390 395 400
   Ser Gly Phe Ser Phe Gly Asn Phe Val Lys Arg Arg Gly Arg Lys
   405 410 415
<210> 90
   <211> 266
   <212> PRT
   <213> Homo sapiens
<400> 90
   Met Arg Val Thr Leu Ala Thr Ile Ala Trp Met Val Ser Phe Val Ser
   1 5 10 15
   Asn Tyr Ser His Thr Ala Asn Ile Leu Pro Asp Ile Glu Asn Glu Asp
   20 25 30
   Phe Ile Lys Asp Cys Val Arg Ile His Asn Lys Phe Arg Ser Glu Val
   35 40 45
   Lys Pro Thr Ala Ser Asp Met Leu Tyr Met Thr Trp Asp Pro Ala Leu
   50 55 60
   Ala Gln Ile Ala Lys Ala Trp Ala Ser Asn Cys Gln Phe Ser His Asn
   65 70 75 80
   Thr Arg Leu Lys Pro Pro His Lys Leu His Pro Asn Phe Thr Ser Leu
   85 90 95
   Gly Glu Asn Ile Trp Thr Gly Ser Val Pro Ile Phe Ser Val Ser Ser
   100 105 110
   Ala Ile Thr Asn Trp Tyr Asp Glu Ile Gln Asp Tyr Asp Phe Lys Thr
   115 120 125
   Arg Ile Cys Lys Lys Val Cys Gly His Tyr Thr Gln Val Val Trp Ala
   130 135 140
   Asp Ser Tyr Lys Val Gly Cys Ala Val Gln Phe Cys Pro Lys Val Ser
   145 150 155 160
   Gly Phe Asp Ala Leu Ser Asn Gly Ala His Phe Ile Cys Asn Tyr Gly
   165 170 175
   Pro Gly Gly Asn Tyr Pro Thr Trp Pro Tyr Lys Arg Gly Ala Thr Cys
   180 185 190
   Ser Ala Cys Pro Asn Asn Asp Lys Cys Leu Asp Asn Leu Cys Val Asn
   195 200 205
   Arg Gln Arg Asp Gln Val Lys Arg Tyr Tyr Ser Val Val Tyr Pro Gly
   210 215 220
   Trp Pro Ile Tyr Pro Arg Asn Arg Tyr Thr Ser Leu Phe Leu Ile Val
   225 230 235 240
   Asn Ser Val Ile Leu Ile Leu Ser Val Ile Ile Thr Ile Leu Val Gln
   245 250 255
   Leu Lys Tyr Pro Asn Leu Val Leu Leu Asp
   260 265
<210> 91
   <211> 219
   <212> PRT
   <213> Homo sapiens
<400> 91
   Met Val Ser Phe Val Ser Asn Tyr Ser His Thr Ala Asn Ile Leu Pro
   1 5 10 15
   Asp Ile Glu Asn Glu Asp Phe Ile Lys Asp Cys Val Arg Ile His Asn
   20 25 30
   Lys Phe Arg Ser Glu Val Lys Pro Thr Ala Ser Asp Met Leu Tyr Met
   35 40 45
   Thr Trp Asp Pro Ala Leu Ala Gln Ile Ala Lys Ala Trp Ala Ser Asn
   50 55 60
   Cys Gln Phe Ser His Asn Thr Arg Leu Lys Pro Pro His Lys Leu His
   65 70 75 80
   Pro Asn Phe Thr Ser Leu Gly Glu Asn Ile Trp Thr Gly Ser Val Pro
   85 90 95
   Ile Phe Ser Val Ser Ser Ala Ile Thr Asn Trp Tyr Asp Glu Ile Gln
   100 105 110
   Asp Tyr Asn Phe Lys Thr Arg Ile Cys Lys Lys Val Cys Gly His Tyr
   115 120 125
   Thr Gln Val Val Trp Ala Asp Ser Tyr Lys Val Gly Cys Ala Val Gln
   130 135 140
   Phe Cys Pro Lys Val Ser Gly Phe Asp Ala Leu Ser Asn Gly Ala His
   145 150 155 160
   Phe Ile Cys Asn Tyr Gly Pro Gly Gly Asn Tyr Pro Thr Trp Pro Tyr
   165 170 175
   Lys Arg Gly Ala Thr Cys Ser Ala Cys Pro Asn Asn Asp Lys Cys Leu
   180 185 190
   Asp Asn Leu Cys Val Asn Asp Ser Glu Thr Lys Ser Asn Val Thr Thr
   195 200 205
   Met Leu Tyr Ile Arg Leu Ala His Ile Ser Thr
   210 215
<210> 92
   <211> 245
   <212> PRT
   <213> Equus caballus
<400> 92
   Met Ala Leu Leu Pro Val Leu Leu Phe Leu Ala Ala Val Leu Leu Pro
   1 5 10 15
   Phe Phe Pro Ala Ser Gly Gln Asp Pro Gly Phe Ala Ala Leu Ser Ile
               20                  25                  30
   Thr Lys Ser Glu Val Gln Lys Glu Ile Val Asn Lys His Asn Asp Leu
           35                  40                  45
   Arg Arg Thr Val Ser Pro Leu Ala Ser Asn Met Leu Lys Met Gln Trp
       50                  55                  60
   Asp Ser Lys Thr Ala Thr Asn Ala Gln Asn Trp Ala Asn Lys Cys Leu
   65                  70                  75                  80
   Leu Gln His Ser Lys Ala Glu Asp Arg Ala Val Gly Thr Met Lys Cys
                   85                  90                  95
   Gly Glu Asn Leu Phe Met Ser Ser Ile Pro Asn Ser Trp Ser Asp Ala
               100                 105                 110
   Ile Gln Asn Trp His Asp Glu Val His Asp Phe Lys Tyr Gly Val Gly
           115                 120                 125
   Pro Lys Thr Pro Asn Ala Val Val Gly His Tyr Thr Gln Val Val Trp
       130                 135                 140
   Tyr Ser Ser Tyr Arg Val Gly Cys Gly Ile Ala Tyr Cys Pro Lys Gln
   145                 150                 155                 160
   Gly Thr Leu Lys Tyr Tyr Tyr Val Cys Gln Tyr Cys Pro Ala Gly Asn
                   165                 170                 175
   Tyr Val Asn Lys Ile Asn Thr Pro Tyr Glu Gln Gly Thr Pro Cys Ala
               180                 185                 190
   Arg Cys Pro Gly Asn Cys Asp Asn Gly Leu Cys Thr Asn Ser Cys Glu
           195                 200                 205
   Tyr Glu Asp Leu Val Ser Asn Cys Asp Ser Leu Lys Lys Ile Ala Gly
       210                 215                 220
   Cys Glu His Glu Leu Leu Lys Glu Asn Cys Lys Thr Thr Cys Gln Cys
   225                 230                 235                 240
   Glu Asn Lys Ile Tyr
                   245
<210> 93
   <211> 2664
   <212> DNA
   <213> Homo sapiens
<400> 93
   gtccggtttg gctcacctct cccaggaaac ttcacactgg agagccaaaa ggagtggaag 60
   agcctgtctt ggagattttc ctggggaaat cctgaggtca ttcattatga agtgtaccgc 120
   gcgggagtgg ctcagagtaa ccacagtgct gttcatggct agagcaattc cagccatggt 180
   ggttcccaat gccactttat tggagaaact tttggaaaaa tacatggatg aggatggtga 240
   gtggtggata gccaaacaac gagggaaaag ggccatcaca gacaatgaca tgcagagtat 300
   tttggacctt cataataaat tacgaagtca ggtgtatcca acagcctcta atatggagta 360
   tatgacatgg gatgtagagc tggaaagatc tgcagaatcc tgggctgaaa gttgcttgtg 420
   ggaacatgga cctgcaagct tgcttccatc aattggacag aatttgggag cacactgggg 480
   aagatatagg cccccgacgt ttcatgtaca atcgtggtat gatgaagtga aagactttag 540
   ctacccatat gaacatgaat gcaacccata ttgtccattc aggtgttctg gccctgtatg 600
   tacacattat acacaggtcg tgtgggcaac tagtaacaga atcggttgtg ccattaattt 660
   gtgtcataac atgaacatct gggggcagat atggcccaaa gctgtctacc tggtgtgcaa 720
   ttactcccca aagggaaact ggtggggcca tgccccttac aaacatgggc ggccctgttc 780
   tgcttgccca cctagttttg gagggggctg tagagaaaat ctgtgctaca aagaagggtc 840
   agacaggtat tatccccctc gagaagagga aacaaatgaa atagaacgac agcagtcaca 900
   agtccatgac acccatgtcc ggacaagatc agatgatagt agcagaaatg aagtcataag 960
   cgcacagcaa atgtcccaaa ttgtttcttg tgaagtaaga ttaagagatc agtgcaaagg 1020
   aacaacctgc aataggtacg aatgtcctgc tggctgtttg gatagtaaag ctaaagttat 1080
   tggcagtgta cattatgaaa tgcaatccag catctgtaga gctgcaattc attatggtat 1140
   aatagacaat gatggtggct gggtagatat cactagacaa ggaagaaagc attatttcat 1200
   caagtccaat agaaatggta ttcaaacaat tggcaaatat cagtctgcta attccttcac 1260
   agtctctaaa gtaacagttc aggctgtgac ttgtgaaaca actgtggaac agctctgtcc 1320
   atttcataag cctgcttcac attgcccaag agtatactgt cctcgtaact gtatgcaagc 1380
   aaatccacat tatgctcgtg taattggaac tcgagtttat tctgatctgt ccagtatctg 1440
   cagagcagca gtacatgctg gagtggttcg aaatcacggt ggttatgttg atgtaatgcc 1500
   tgtggacaaa agaaagacct acattgcttc ttttcagaat ggaatcttct cagaaagttt 1560
   acagaatcct ccaggaggaa aggcattcag agtgtttgct gttgtgtgaa actgaatact 1620
   tggaagagga ccataaagac tattccaaat gcaatatttc tgaattttgt ataaaactgt 1680
   aacattactg tacagagtac atcaactatt ttcagcccaa aaaggtgcca aatgcatata 1740
   aatcttgata aacaaagtct ataaaataaa acatgggaca ttagctttgg gaaaagtaat 1800
   gaaaatataa tggttttaga aatcctgtgt taaatattgc tatattttct tagcagttat 1860
   ttctacagtt aattacatag tcatgattgt tctacgtttc atatattata tggtgctttg 1920
   tatatgccac taataaaatg aatctaaaca ttgaatgtga atggccctca gaaaatcatc 1980
   tagtgcattt aaaaataatc gactctaaaa ctgaaagaaa ccttatcaca ttttccccag 2040
   ttcaatgcta tgccattacc aactccaaat aatctcaaat aattttccac ttaataactg 2100
   taaagttttt ttctgttaat ttaggcatat agaatattaa attctgatat tgcacttctt 2160
   attttatata aaataatcct ttaatatcca aatgaatctg ttaaaatgtt tgattccttg 2220
   ggaatggcct taaaaataaa tgtaataaag tcagagtggt ggtatgaaaa cattcctagt 2280
   gatcatgtag taaatgtagg gttaagcatg gacagccaga gctttctatg tactgttaaa 2340
   attgaggtca catattttct tttgtatcct ggcaaatact cctgcaggcc aggaagtata 2400
   atagcaaaaa gttgaacaaa gatgaactaa tgtattacat taccattgcc actgattttt 2460
   ttttaaatgg taaatgacct tgtatataaa tattgccata tcatggtacc tataatggtg 2520
   atatatttgt ttctatgaaa aatgtattgt gctttgatac taaaaatctg taaaatgtta 2580
   gttttggtaa ttttttttct gctggtggat ttacatatta aattttttct gctggtggat 2640
   aaacattaaa attaatcatg tttc                                         2664
<210> 94
   <211> 500
   <212> PRT
   <213> Homo sapiens
<400> 94
   Met Lys Cys Thr Ala Arg Glu Trp Leu Arg Val Thr Thr Val Leu Phe
   1               5                   10                  15
   Met Ala Arg Ala Ile Pro Ala Met Val Val Pro Asn Ala Thr Leu Leu
               20                  25                  30
   Glu Lys Leu Leu Glu Lys Tyr Met Asp Glu Asp Gly Glu Trp Trp Ile
           35                  40                  45
   Ala Lys Gln Arg Gly Lys Arg Ala Ile Thr Asp Asn Asp Met Gln Ser
       50                  55                  60
   Ile Leu Asp Leu His Asn Lys Leu Arg Ser Gln Val Tyr Pro Thr Ala
   65                  70                  75                  80
   Ser Asn Met Glu Tyr Met Thr Trp Asp Val Glu Leu Glu Arg Ser Ala
                   85                  90                  95
   Glu Ser Trp Ala Glu Ser Cys Leu Trp Glu His Gly Pro Ala Ser Leu
               100                 105                 110
   Leu Pro Ser Ile Gly Gln Asn Leu Gly Ala His Trp Gly Arg Tyr Arg
           115                 120                 125
   Pro Pro Thr Phe His Val Gln Ser Trp Tyr Asp Glu Val Lys Asp Phe
       130                 135                 140
   Ser Tyr Pro Tyr Glu His Glu Cys Asn Pro Tyr Cys Pro Phe Arg Cys
   145                 150                 155                 160
   Ser Gly Pro Val Cys Thr His Tyr Thr Gln Val Val Trp Ala Thr Ser
                   165                 170                 175
   Asn Arg Ile Gly Cys Ala Ile Asn Leu Cys His Asn Met Asn Ile Trp
               180                 185                 190
   Gly Gln Ile Trp Pro Lys Ala Val Tyr Leu Val Cys Asn Tyr Ser Pro
           195                 200                 205
   Lys Gly Asn Trp Trp Gly His Ala Pro Tyr Lys His Gly Arg Pro Cys
       210                 215                 220
   Ser Ala Cys Pro Pro Ser Phe Gly Gly Gly Cys Arg Glu Asn Leu Cys
   225                 230                 235                 240
   Tyr Lys Glu Gly Ser Asp Arg Tyr Tyr Pro Pro Arg Glu Glu Glu Thr
                   245                 250                 255
   Asn Glu Ile Glu Arg Gln Gln Ser Gln Val His Asp Thr His Val Arg
               260                 265                 270
   Thr Arg Ser Asp Asp Ser Ser Arg Asn Glu Val Ile Ser Ala Gln Gln
           275                 280                 285
   Met Ser Gln Ile Val Ser Cys Glu Val Arg Leu Arg Asp Gln Cys Lys
       290                 295                 300
   Gly Thr Thr Cys Asn Arg Tyr Glu Cys Pro Ala Gly Cys Leu Asp Ser
   305                 310                 315                 320
   Lys Ala Lys Val Ile Gly Ser Val His Tyr Glu Met Gln Ser Ser Ile
                   325                 330                 335
   Cys Arg Ala Ala Ile His Tyr Gly Ile Ile Asp Asn Asp Gly Gly Trp
               340                 345                 350
   Val Asp Ile Thr Arg Gln Gly Arg Lys His Tyr Phe Ile Lys Ser Asn
           355                 360                 365
   Arg Asn Gly Ile Gln Thr Ile Gly Lys Tyr Gln Ser Ala Asn Ser Phe
       370                 375                 380
   Thr Val Ser Lys Val Thr Val Gln Ala Val Thr Cys Glu Thr Thr Val
   385                 390                 395                 400
   Glu Gln Leu Cys Pro Phe His Lys Pro Ala Ser His Cys Pro Arg Val
                   405                 410                 415
   Tyr Cys Pro Arg Asn Cys Met Gln Ala Asn Pro His Tyr Ala Arg Val
               420                 425                 430
   Ile Gly Thr Arg Val Tyr Ser Asp Leu Ser Ser Ile Cys Arg Ala Ala
           435                 440                 445
   Val His Ala Gly Val Val Arg Asn His Gly Gly Tyr Val Asp Val Met
       450                 455                 460
   Pro Val Asp Lys Arg Lys Thr Tyr Ile Ala Ser Phe Gln Asn Gly Ile
   465 470 475 480
   Phe Ser Glu Ser Leu Gln Asn Pro Pro Gly Gly Lys Ala Phe Arg Val
   485 490 495
   Phe Ala Val Val
   500
<210> 95
   <211> 188
   <212> PRT
   <213> Rattus norvegicus
<400> 95
   Met Leu His Asn Lys Leu Arg Gly Gln Val Tyr Pro Pro Ala Ser Asn
   1 5 10 15
   Met Glu Tyr Met Thr Trp Asp Glu Glu Leu Glu Arg Ser Ala Ala Ala
   20 25 30
   Trp Ala Gln Arg Cys Leu Trp Glu His Gly Pro Ala Ser Leu Leu Val
   35 40 45
   Ser Ile Gly Gln Asn Leu Ala Val His Trp Gly Arg Tyr Arg Ser Pro
   50 55 60
   Gly Phe His Val Gln Ser Trp Tyr Asp Glu Val Lys Asp Tyr Thr Tyr
   65 70 75 80
   Pro Tyr Pro His Glu Cys Asn Pro Trp Cys Pro Glu Arg Cys Ser Gly
   85 90 95
   Ala Met Cys Thr His Tyr Thr Gln Met Val Trp Ala Thr Thr Asn Lys
   100 105 110
   Ile Gly Cys Ala Val His Thr Cys Arg Ser Met Ser Val Trp Gly Asp
   115 120 125
   Ile Trp Glu Asn Ala Val Tyr Leu Val Cys Asn Tyr Ser Pro Lys Gly
   130 135 140
   Asn Trp Ile Gly Glu Ala Pro Tyr Lys His Gly Arg Pro Cys Ser Glu
   145 150 155 160
   Cys Pro Ser Ser Tyr Gly Gly Gly Cys Arg Asn Asn Leu Cys Tyr Arg
   165 170 175
   Glu Glu His Tyr His Gln Lys Pro Glu Trp Met Arg
   180 185
<210> 96
   <211> 258
   <212> PRT
   <213> Homo sapiens
<400> 96
   Met Ile Ala Ile Ser Ala Val Ser Ser Ala Leu Leu Phe Ser Leu Leu
   1 5 10 15
   Cys Glu Ala Ser Thr Val Val Leu Leu Asn Ser Thr Asp Ser Ser Pro
   20 25 30
   Pro Thr Asn Asn Phe Thr Asp Ile Glu Ala Ala Leu Lys Ala Gln Leu
   35 40 45
   Asp Ser Ala Asp Ile Pro Lys Ala Arg Arg Lys Arg Tyr Ile Ser Gln
   50 55 60
   Asn Asp Met Ile Ala Ile Leu Asp Tyr His Asn Gln Val Arg Gly Lys
   65 70 75 80
   Val Phe Pro Pro Ala Ala Asn Met Glu Tyr Met Val Trp Asp Glu Asn
   85 90 95
   Leu Ala Lys Ser Ala Glu Ala Trp Ala Ala Thr Cys Ile Trp Asp His
   100 105 110
   Gly Pro Ser Tyr Leu Leu Arg Phe Leu Gly Gln Asn Leu Ser Val Arg
   115 120 125
   Thr Gly Arg Tyr Arg Ser Ile Leu Gln Leu Val Lys Pro Trp Tyr Asp
   130 135 140
   Glu Val Lys Asp Tyr Ala Phe Pro Tyr Pro Gln Asp Cys Asn Pro Arg
   145 150 155 160
   Cys Pro Met Arg Cys Phe Gly Pro Met Cys Thr His Tyr Thr Gln Met
   165 170 175
   Val Trp Ala Thr Ser Asn Arg Ile Gly Cys Ala Ile His Thr Cys Gln
   180 185 190
   Asn Met Asn Val Trp Gly Ser Val Trp Arg Arg Ala Val Tyr Leu Val
   195 200 205
   Cys Asn Tyr Ala Pro Lys Gly Asn Trp Ile Gly Glu Ala Pro Tyr Lys
   210 215 220
   Val Gly Val Pro Cys Ser Ser Cys Pro Pro Ser Tyr Gly Gly Ser Cys
   225 230 235 240
   Thr Asp Asn Leu Cys Phe Pro Gly Val Thr Ser Asn Tyr Leu Tyr Trp
   245 250 255
   Phe Lys
<210> 97
   <211> 253
   <212> PRT
   <213> Homo sapiens
<400> 97
   Met Pro Leu Leu Pro Ser Thr Val Gly Leu Ala Gly Leu Leu Phe Trp
   1 5 10 15
   Ala Gly Gln Ala Val Asn Ala Leu Ile Met Pro Asn Ala Thr Pro Ala
   20 25 30
   Pro Ala Gln Pro Glu Ser Thr Ala Met Arg Leu Leu Ser Gly Leu Glu
   35 40 45
   Val Pro Arg Tyr Arg Arg Lys Arg His Ile Ser Val Arg Asp Met Asn
   50 55 60
   Ala Leu Leu Asp Tyr His Asn His Ile Arg Ala Ser Val Tyr Pro Pro
   65 70 75 80
   Ala Ala Asn Met Glu Tyr Met Val Trp Asp Lys Arg Leu Ala Arg Ala
   85 90 95
   Ala Glu Ala Trp Ala Thr Gln Cys Ile Trp Ala His Gly Pro Ser Gln
   100 105 110
   Leu Met Arg Tyr Val Gly Gln Asn Leu Ser Ile His Ser Gly Gln Tyr
   115 120 125
   Arg Ser Val Val Asp Leu Met Lys Ser Trp Ser Glu Glu Lys Trp His
   130 135 140
   Tyr Leu Phe Pro Ala Pro Arg Asp Cys Asn Pro His Cys Pro Trp Arg
   145 150 155 160
   Cys Asp Gly Pro Thr Cys Ser His Tyr Thr Gln Met Val Trp Ala Ser
   165 170 175
   Ser Asn Arg Leu Gly Cys Ala Ile His Thr Cys Ser Ser Ile Ser Val
   180 185 190
   Trp Gly Asn Thr Trp His Arg Ala Ala Tyr Leu Val Cys Asn Tyr Ala
   195 200 205
   Ile Lys Gly Asn Trp Ile Gly Glu Ser Pro Tyr Lys Met Gly Lys Pro
   210 215 220
   Cys Ser Ser Cys Pro Pro Ser Tyr Gln Gly Ser Cys Asn Ser Asn Met
   225 230 235 240
   Cys Phe Lys Gly Leu Lys Ser Asn Lys Phe Thr Trp Phe
   245 250
<210> 98
   <211> 245
   <212> PRT
   <213> Homo sapiens
<400> 98

## Description

The invention relates generally to polynucleotides and polypeptides, as well as vectors, host cells, antibodies, and recombinant methods for producing these nucleic acids and polypeptides.

Database EMBL Online HSM802230, accession No. AL137500 (27 January 2000) describes a nucleic acid sequence and an amino acid sequence "homo sapiens mRNA, cDNA DKFZp761F171 (from clone DKFZp761F171); partial cds".

Database EMBL Online accession No. AF086607, (3 August 1999) describes a nucleic acid sequence and an amino acid sequence "rattus norvegicus Neurestin alpha mRNA, complete cds".

Database EMBL Online accession No. AB025411, (10 May 1999) describes a nucleic acid sequence and an amino acid sequence "mus musculus mRNA for Ten-m2, complete cdcs".

WO 00/75321 describes polynucleotides and membrane-bound polypeptides encoded thereby.

WO 01/19856 describes secreted polypeptides and corresponding polynucleotides and uses thereof.

Database EMBL Acc. No. AB032953 (11 November 1999) describes homo sapiens mRNA for KIAA1127 protein, partial CDs.

The present invention provides an isolated human polypeptide comprising a human amino acid sequence selected from the group consisting of: (a) a mature from of an amino acid sequence selected from the group consisting of SEQ ID NOS: 8 and 13; (b) a variant amino acid sequence having at least 95% identity to a mature form of an amino acid sequence selected from the group consisting of SEQ ID NOS: 8 and 13, over an amino acid sequence of identical size to said mature form; (c) an amino acid sequence selected from the group consisting of SEQ ID NOS: 8 and 13; and (d) a variant amino acid sequence having at least 95% identity to an amino acid sequence selected from the group consisting of SEQ ID NOS: 8 and 13, over an amino acid sequence of identical size to said amino acid sequence of SEQ ID NOS 8 or 13.

The present invention also provides an isolated human nucleic acid molecule comprising a human nucleic acid sequence encoding a polypeptide comprising an amino acid sequence selected from the group consisting of: (a) a mature form of an amino acid sequence selected from the group consisting of SEQ ID NOS: 8 and 13; (b) a variant amino acid sequence having at least 95% identity to a mature form of an amino acid sequence selected from the group consisting of SEQ ID NOS: 8 and 13, over an amino acid sequence of identical size to said mature form; (c) an amino acid sequence selected from the group consisting of SEQ ID NOS: 8 and 13; (d) a variant amino acid sequence having at least 95% identity to an amino acid sequence selected from the group consisting of SEQ ID NOS: 8 and 13, over an amino acid sequence of identical size to said amino acid sequence of SEO ID NOs: 8 or 13; or a nucleic acid molecule encoding the complement of (a), (b), (c) or (d).

The invention is thus based in part upon the discovery of novel nucleic acid sequences encoding novel polypeptides. The disclosed FCTR3 nucleic acids and polypeptides encoded therefrom, as well as derivatives, homologs, analogs and fragments thereof, will hereinafter be collectively designated as "FCTRX" nucleic acid or polypeptide sequences.

In one aspect, the invention provides an isolated FGTRX nucleic acid molecule encoding a FCTRX polypeptide that includes a nucleic acid sequence that has identity to the nucleic acids disclosed in SEQ ID NOS: 7, 9, 10 and 11. In some embodiments, the FCTRX nucleic acid molecule will hybridize under stringent conditions to a nucleic acid sequence complementary to a nucleic acid molecule that includes a protein-coding sequence of a FCTRX nucleic acid sequence. The invention also includes an isolated nucleic acid that encodes a FCTRX polypeptide, or a fragment, homolog, analog or derivative thereof. For example, the nucleic acid can encode a polypeptide at least 80% identical to a polypeptide comprising the amino acid sequences of SEQ ID NOS: 8 and 13. The nucleic acid can be, for example, a genomic DNA fragment or a cDNA molecule that includes the nucleic acid sequence of any of SEQ ID NOS: 7,9, 10 and 11.

Also included in the invention is an oligonucleotide, *e.g.,* an oligonucleotide which includes at least 6 contiguous nucleotides of a FCTRX nucleic acid (*e.g.,* SEQ ID NOS: 7, 9, 10 and 11) or a complement of said oligonucleotide.

Also included in the invention are substantially purified FCTRX polypeptides (SEQ ID NO: 8 and 13). In certain embodiments, the FCTRX polypeptides include an amino acid sequence that is substantially identical to the amino acid sequence of a human FCTRX polypeptide.

The invention also features antibodies that selectively-bind to FCTRX polypeptides, or fragments, homologs, analogs or derivatives thereof.

In another aspect, the invention includes pharmaceutical compositions that include therapeutically- or prophylactically-effective amounts of a therapeutic and a pharmaceutically-acceptable carrier. The therapeutic can be, *e.g.,* a FCTRX nucleic acid, a FCTRX polypeptide, or an antibody specific for a FCTRX polypeptide. In a further aspect, the invention includes, in one or more containers, a therapeutically- or prophylactically-effective amount of this pharmaceutical composition.

In a further aspect, the invention includes a method of producing a polypeptide by culturing a cell that includes a FCTRX nucleic acid, under conditions allowing for expression of the FCTRX polypeptide encoded by the DNA. If desired, the FCTRX polypeptide can then be recovered.

In another aspect, the invention includes a method of detecting the presence of a FCTRX polypeptide in a sample. In the method, a sample is contacted with a compound that selectively binds to the polypeptide under conditions allowing for formation of a complex between the polypeptide and the compound. The complex is detected, if present, thereby identifying the FCTRX polypeptide within the sample.

The invention also includes methods to identify specific cell or tissue types based on their expression of a FCTRX.

Also included in the invention is a method of detecting the presence of a PCTRX nucleic acid molecule in a sample by contacting the sample with a FCTRX nucleic acid probe or primer, and detecting whether the nucleic acid probe or primer bound to a FCTRX nucleic acid molecule in the sample.

In a further aspect, the invention provides a method for modulating the activity of a FCTRX polypeptide by contacting a cell sample that includes the PCTRX polypeptide with a compound that binds to the FCTRX polypeptide in an amount sufficient to modulate the activity of said polypeptide. The compound can be, *e*.*g*., a small molecule, such as a nucleic acid, peptide, polypeptide, peptidomimetic, carbohydrate, lipid or other organic (carbon containing) or inorganic molecule, as further described herein.

Also within the scope of the invention is the use of a Therapeutic in the manufacture of a medicament for treating or preventing disorders or syndromes including. *e*.*g*., Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain turnors, mammary tumors, human gliomas, astrocytomas, mixed glioma/aStrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance. The Therapeutic can be, *e.g.,* a FCTRX nucleic acid, a FCTRX polypeptide, or a FCTRX-specific antibody, or biologically-active derivatives or fragments thereof.

Also within the scope of the invention is the use of a Therapeutic in the manufacture of a medicament for treating or preventing disorders or syndromes including, *e.g.,* Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance. The invention further includes a method for screening for a modulator of the disorders or syndromes recited above. The method includes contacting a that compound with a FCTRX polypeptide and determining if the test compound binds to said FCTRX polypeptide. Binding of the test compound to the FCTRX polypeptide indicates the test compound is a modulator of activity, or of latency or predisposition to the aforementioned disorders or syndromes. Also within the scope of the invention is a method for screening for a modulator of activity, or of latency or predisposition to the aforementioned disorders or syndromes by administering a test compound to a test animal at increased risk for the aforementioned disorders of syndromes. The test animal expresses a recombinant polypeptide encoded by a FCTRX nucleic acid. Expression or activity of FCTRX polypeptide is then measured in the test animal, as is expression or activity of the protein in a control animal which recombinantly-expresses FCTRX polypeptide and is not at increased risk for the disorder or syndrome. Next, the expression of FCTRX polypeptide in both the test animal and the control animal is compared. A change in the activity of FCTRX polypeptide in the test animal relative to the control animal indicates the test compound is a modulator of latency of the disorder or syndrome.

In yet another aspect, the invention includes an in vitro method for determining the presence of or predisposition to a disease associated with altered levels of a FCTRX polypeptide, a FCTRX nucleic acid, or both, in a sobject (*e.g.,* a human subject). The method includes measuring the amount of the FCTRX polypeptide in a test sample from the subject and comparing the amount of the polypeptide in the test sample to the amount of the FCTRX polypeptide present in a control sample. An alteration in the level of the FCTRX polypeptide in the test sample as compared to the control sample indicates the presence of or predisposition to a disease in the subject. Preferably, the predisposition includes, *e.g.* Colorectal cancer, adenomatous polyposis coli, myclogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinorna, clear cell and granular cell carcinomas, autocrine/paracrine stimulation of tumor cell proliferation, autorine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance. Also, the expression levels of the new polypeptides of the invention can be used in a method to screen for various cancers as well as to determine the stage of cancers.

In a further aspect, the invention includes the use of a FCTRX polypeptide, a FCTRX nucleic acid, or a FCTRX-specific antibody for the manufacture of a medicament for treating or preventing disorders or syndromes including, *e.g.,* Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance.

In yet another aspect, the invention can be used in a method to identity the cellular receptors and downstream effectors of the invention by any one of a number of techniques commonly employed in the art. These include but are not limited to the two-hybrid system, affinity purification, co-precipitation with antibodies or other specific-interacting molecules.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In the case of conflict, the present specification, including definitions, will control. In addition, the materials, methods, and examples are illustrative only and not intended to be limiting.

Other features and advantages of the invention will be apparent from the following detailed description and claims.

The invention is based, in part, upon the discovery of novel nucleic acid sequences that encode novel polypeptides. The novel nucleic acids and their encoded polypeptides are referred to individually as FCTR3. The nucleic acids, and their encoded polypeptides, are collectively designated herein as "FCTRX".

The novel FCTRX nucleic acids of the invention include the nucleic acids whose sequences are provided in Tables 3C, 3E, 3F and 3G, inclusive ("Tables 3C-3G"), or a fragment, derivative, analog or homolog thereof. The novel FCTRX proteins of the invention include the protein fragments whose sequences are provided in Tables 3B and 3L inclusive ("Tables 3B+3I"). The individual FCTRX nucleic acids and proteins are described below. Within the scope of this invention is a method of using these nucleic acids and peptides in the treatment or prevention of a disorder related to cell signaling or metabolic pathway modulation.

### FCTR3

FCTR3, is an amino acid type II membrane, neurestin-like protein. The FCTR3a nucleic acid of 1430 nucleotides (also designated 10129612.0.118) is shown in Table 3A. An ORF was identified beginning with an ATG initiation codon at nucleotides 69-71 and ending with a TAG codon at nucleotides 1212-1214. A putative untranslated region upstream from the initiation codon and downstream from the termination codon is underlined in Table 3A, and the start and stop codons are in bold letters.

**Table 3A. FCTR3a Nucleotide Sequence (SEQ ID NO:5)**

| |
|---|
| |
| |

The FCTR3 polypeptide (SEQ ID NO: 6) encoded by SEQ ID NO:5 is 381 amino acid residues and is presented using the one-letter code in Table 3B.

**Table 3B. Encoded FCTR3a protein sequence (SEQ ID NO:6).**

| |
|---|
| |

In an alternative embodiment, the 5' end of the FCTR3a nucleic acid could be extended as it is in the 9826bp FCTR3b (also referred to herein as 10129612.0.405) shown in Table 3C. An ORF was identified beginning with an ATG initiation codon at nucleotides 280-282 and ending with a TAA codon at nucleotides 8479-8481. A putative untranslated region upstream from the initiation codon and downstream from the termination codon is underlined in Table 3C, and the start and stop codons are in bold letters. Italicized bases 1-201 refer to a variable 5' region that will be further discussed below.

**Table 3C. FCTR3b Nucleotide Sequence (SEQ ID NO:7)**

| |
|---|
| |
| |
| |

The FCTR3b polypeptide (SEQ ID NO:8) encoded by SEQ ID NO:7 is 2733 amino acid residues and is presented using the one-letter code in Table 3D. The protein has a predicted molecular weight of 303424.3 daltons.

**Table 3D. Encoded FCTR3b protein sequence (SEQ ID NO:8).**

| |
|---|
| |
| |

In further alternative embodiments the italicized bases in the 5' end of the FCTR3b sequence in table 3C is a variable region. This region can be substituted for in other embodiments of FCTR3. The nucleotide sequence for 9823bp FCTR3c (also referred to herein as 10129612.0.154) has the same nucleotide sequence as FCTR3b except that the italicized region is replaced with the 201 base sequence shown in Table 3E. An ORF for the total FCTR3c nucleotide sequence was identified beginning with an ATG initiation codon at nucleotides 277-280 and ending with a TAG codon at nucleotides 8473-8475. This is the same open reading frame that is shown in Table 3C, with the corresponding base numbers for FCTR3c. This open reading frame will translate the same amino acid sequence as shown in Table 3C for FCTR3b.

**Table 3E. Encoded FCTR3c 5'end nucleotide sequence (SEQ ID NO:9).**

| |
|---|
| |

In yet another embodiment, the italicized region shown in the 5' end of the sequence in Table 3C can be replaced with the sequence shown in Table 3F to form 9823bp FCTR3d (also referred to herein as 10129612.0.67). An ORF was identified beginning with an ATG initiation codon at nucleotides 277-280 and ending with a TAG codon at nucleotides 8473-8475. This is the same open reading frame that is shown in Table 3C, with the corresponding base numbers for FCTR3d. This open reading frame will translate the same amino acid sequence as shown in Table 3D for FCTR3b.

**Table 3F. Encoded FCTR3d 5'end nucleotide sequence (SEQ ID NO:10).**

| |
|---|
| |

In yet another embodiment, the italicized region shown in the 5' end of the sequence in Table 3C can be replaced with the sequence shown in Table 3G to form 9765 bp FCTR3e (also referred to as 10129612.0.258). An ORF was identified beginning with an ATG initiation codon at nucleotides 210-212 and ending with a TAG codon at nucleotides 8408-8410. This is the same open reading frame that is shown in Table 3C, with the corresponding base numbers for FCTR3e. This open reading frame will translate the same amino acid sequence as shown in Table 3D for FCTR3b.
**Table 3G. Encoded FCTR3e 5' end nucleotide sequence (SEQ ID NO:11).**

In yet another embodiment another FCTR3a homolog, FCTR3f (also referred to as 10129612.0.352) was found having the 9729bp sequence shown in Table 3H. An ORF was identified beginning with an ATG initiation codon at nucleotides 210-212 and ending with a TAG codon at nucleotides 8382-8384. A putative untranslated region upstream from the initiation codon and downstream from the termination codon is underlined in Table 3G, and the start and stop codons are in bold letters.

**Table 3H. Encoded FCTR3f nucleotide sequence (SEQ ID NO:12).**

| |
|---|
| |
| |
| |

The FCTR3f polypeptide (SEQ ID NO:13) encoded by SEQ ID NO:12 is 2724 amino acid residues long and is presented using the one-letter code in Table 3I. This sequence differs from FCTR3b in that it is missing amino acids 758-766 from that polypeptide.

**Table 3I. Encoded FCTR3f protein sequence (SEQ ID NO:13)**

| |
|---|
| |

In a BLASTN search it was found that the FCTR3a nucleic acid has homology to three fragments of *Mus musculus* odd Oz/ten-m homolog 2. It has 634 of 685 bases (92%) identical to bases 614-1298, 365 of 406 bases (89%) identical to bases 1420-1825, and 93 of 103 bases (90%) identical to bases 1823-1925 of *Mus musculus* odd Oz/ten-m homolog 2 (GenBank Acc: NM_011856.2) (Table 3J).

**Table 3J. BLASTN of FCTR3a against Mus musculus odd Oz/ten-m homolog 2 (SEQ ID NO:62)**

| |
|---|
| >GI17657414\|RBF\|NM 011856.21 MOS MUSCULUS ODD OZ/TEN-M HOMOLOG 2 (DROSOPHILA) |
| (ODZ2), MRNA |
| LENGTH = 8797 |
| SCORE = 954 BITS (481), EXPECT = 0.0 |
| IDENTITIES = 634/685 (92%) |
| STRAND = PLUS / PLUS |
| |
| |
| |
| |
| |
| |
| |
| |
| |
| |
| |
| |
| SCORE = 480 BITS (242), EXPECT = E-132 |
| IDENTITIES = 365/406 (89%) |
| STRAND - PLUS / PLUS |
| |
| |
| |
| |
| |
| |
| |
| SCORE - 125 BITS (63), EXPECT - 7E-26 |
| IDENTITIES = 93/103 (90%) |
| STRAND = PLUS / PLUS |
| |
| |

In another BLASTN search it was found that the FCTR3a nucleic acid has homology to three fragments of *Gallus gallus* mRNA for teneurin-2. It has 541 of 629 bases (86%) identical to bases 502-1130, 302 of 367 bases (82%) identical to bases 1330-1696, and 87 of 103 bases (84%) identical to bases 1711-1813 of *Gallus gallus* mRNA for teneurin-2 (EMBL Ace: AJ245711.1) (Table 3K).

**Table 3K. BLASTN of FCTR3a against Gallus gallus mRNA for teneurin-2 (SEQ ID NO:63)**

| |
|---|
| >GI\|6010048\|EMB\|AJ245711.1\|GGA245711 GALLUS GALLUS MRNA FOR TENEURIN-2, SHORT SPLICE |
| VARIANT (TEN2 GENE) |
| LENGTH = 2496 |
| SCORE = 549 BITS (277), EXPECT = E-153 |
| IDENTITIES = 541/629 (86%) |
| STRAND - PLUS / PLUS |
| |
| |
| |
| |
| |
| |
| |
| |
| |
| |
| |
| |
| SCORE = 212 BITS (107), EXPECT = 4E-52 |
| IDENTITIES = 302/367 (82%) |
| STRAND = PLUS / PLUS |
| |
| |
| |
| |
| |
| |
| |
| SCORE = 77.8 BITS (39), EXPECT = 1E-11 |
| IDENTITIES = 87/103 (84%) |
| STRAND = PLUS / PLUS |
| |
| |
| |

In this search it was also found that the fragments of FCTR3bcd and e nucleic acids had homology to three fragments of *Homo sapiens* mRNA for KIAA1127 protein. It has 5537 of 5538 bases (99%) identical to bases 1-5538, 705 of 714 bases (98%) identical to bases 5609-6322, and 176 of 176 bases (100%) identical to bases 6385-6560 of *Homo sapiens* mRNA for KIAA1127 protein (GenBank Ace: AB032953) (Table 3L).

**Table 3L. BLASTN of FCTR3b, c, d, and e against Homo sapiens KIAA1127 mRNA (SEQ ID NO:64)**

| |
|---|
| >GI\|6329762\|DBJ\|A8032953.1\|AB032953 HOMO SAPIENS MRNA FOR KIAA1127 PROTEIN, PARTIAL |
| CDS |
| LENGTH = 6560 |
| SCORE - 1.097E+04 BITS (5534), EXPECT - 0.0 |
| IDENTITIES = 5537/5538 (99%) |
| STRAND = PLUS / PLUS |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| SCORE = 1362 BITS (687), EXPECT - 0.0 |
| IDENTITIES - 705/714 (98%) |
| STRAND = PLUS / FLUS |
| |
| |
| |
| |
| |
| |
| |
| |
| |
| |
| |
| |
| |
| SCORE = 349 BITS (176), EXPECT = 2E-92 |
| IDENTITIES = 176/176 (100%) |
| STRAND = PLUS / PLUS |
| |
| |
| |

In this search it was also found that the FCTR3bcd and e nucleic acids had homology to five fragments of *Mus musculus* mRNA for Ten-m2. It has 5498 of 6108 bases (90%) identical to bases 2504-8610, 1095 of 1196 bases (91%) identical to bases 103-1298,1000 of 1088 bases (91%) identical to bases 1420-2540, 81 of 89 bases (91%) identical to bases 8655-8743, and 30 of 32 bases (93%) identical to bases 7-38 of *Mus musculus* mRNA for Ten-m2 (Table 3M).

**Table 3M. BLASTN of FCTR3b, c, d, and e against Mus musculus mRNA for Ten-m2 Mrna (SEQ ID NO:65)**

| |
|---|
| >GI\|4760777\|DBJ\|AB025411.1\|AB025411 MUS MUSCULUS MRNA FOR TEN-M2, COMPLETE CDS |
| LENGTH - 8797 |
| SCORE = 7263 BITS (3664), EXPECT - 0.0 |
| IDENTITIES = 5498/6108 (90%), GAPS - 1/6108 (0%) |
| STRAND = PLUS / PLUS |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| SCORE - 1570 BITS (792), EXPECT - 0.0 |
| IDENTITIES - 1095/1196 (91%) |
| STRAND - PLUS / PLUS |
| |
| |
| |
| |
| |
| |
| |
| |
| |
| |
| |
| |
| |
| |
| |
| |
| |
| |
| |
| |
| |
| SCORE = 1455 BITS (734), EXPECT - 0.0 |
| IDENTITIES = 1000/1088 (91%), GAPS = 3/1088 (0%) |
| STRAND = PLUS / PLUS |
| |
| |
| |
| |
| |
| |
| |
| |
| |
| |
| |
| |
| |
| |
| |
| |
| |
| |
| |
| |
| SCORE = 105 BITS (53), EXPECT = 5E-19 |
| IDENTITIES = 81/89 (91%), GAPS = 1/89 (1%) |
| STRAND = PLUS / PLUS |
| |
| |
| SCORE = 48.1 BITS (24), EXPECT = 0.093 |
| IDENTITIES = 30/32 (93%) |
| STRAND = PLUS / PLUS |
| |

In this search it was also found that the FCTR3bcd and e nucleic acids had homology to three fragments of *Rattus norvegicus* neurestin alpha. It has 5498 of 6132 bases (89%) identical to bases 2527-8658,1081 of 1196 bases (90%) identical to bases 123-1318, 996 of 1088 bases (91%) identical to bases 1440-2527 of *Rattus norvegicus* neurestin alpha (GenRank Acc:NM_020088.1) (Table 3N).

**Table 3N. BLASTN of FCTR3b, c, d, and e against Rattus norvegicus Neurestin alpha mRNA (SEQ ID NO:66)**

| |
|---|
| >GI\|99\|0329\|FEF\|NM 020088.1\| RATTUS NORVEGICUS NEURESTIN ALPHA (LOC56762), MRNA |
| LENGTH = 8689 |
| SCORE - 7129 BITS (3596), EXPECT = 0.0 |
| IDENTITIES - 5498/6132 (89%) |
| STRAND - PLUS / PLUS |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| SCORE - 1459 BITS (736), EXPECT - 0.0 |
| IDENTITIES = 1081/1196 (90%) |
| STRAND = PLUS / PLUS |
| |
| |
| |
| |
| |
| |
| |
| |
| |
| |
| |
| |
| |
| |
| |
| |
| |
| |
| |
| |
| SCORE - 1427 BITS (720), EXPECT = 0.0 |
| IDENTITIES = 996/1088 (91%) |
| STRAND = PLUS / PLUS |
| |
| |
| |
| |
| |
| |
| |
| |
| |
| |
| |
| |
| |
| |
| |
| |
| |
| |
| |
| |

In this search it was also found that the FCTR3bcd and e nucleic acid had homology to six fragments of *Gallus gallus* partial mRNA for teneurin-2. It has 2780 of 3449 bases (80%) identical to bases 3386-6834, 1553 of 1862 bases (83%) identical to bases 1414-3275, 540 of 628 bases (85%) identical to bases 587-1214, 593 of 725 bases (81%) identical to bases 7084-7808, 429 of 515 bases (83%) identical to bases 7895-8409, and 397 of 475 bases (83%) identical to bases 20-494 of *Gallus gallus* partial mRNA for teneurin-2. (EMBL Acc: GGA278031) (Table 30).

**Table 3O. BLASTN of FCTR3b, c, d, and e against Gallus gallus Teneurin-2 mRNA (SEQ ID NO:67)**

| |
|---|
| >GI\|10241573\|EMB\|AJ279031.1\|GGA279031 GALLUS GALLUS PARTIAL MRNA FOR TENEURIN-2 |
| (TEN2 GENE), LONG SPLICE |
| VARIANT |
| LENGTH = 8409 |
| SCORE = 1532 BITS (773), EXPECT = 0.0 |
| IDENTITIES = 2180/3449 (80%) |
| STRANd = PLUS / PLUS |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| SCORE - 1241 BITS (626), EXPECT = 0.0 |
| IDENTITIES - 1553/1862 (83%) |
| STRAND = PLUS / PLUS |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| SCORE = 547 BITS (276), EXPECT = E-152 |
| IDENTITIES = 540/628 (85%) |
| STRAND = PLUS / PLUS |
| |
| |
| |
| |
| |
| |
| |
| |
| |
| |
| |
| |
| SCORE = 391 BITS (191), EXPECT = E-105 |
| IDENTITIES = 593/725 (81%) |
| STRAND = PLUS / PLUS |
| |
| |
| |
| |
| |
| |
| |
| |
| |
| |
| |
| |
| |
| SCORE = 339 BITS (171), EXPECT = 2E-89 |
| IDENTITIES = 429/515 (83%) |
| STRAND = PLUS / PLUS |
| |
| |
| |
| |
| |
| |
| |
| |
| |
| SCORE = 323 BITS (163), EXPECT = 1E-84 |
| IDENTITIES = 397/475 (83%) |
| STRAND = PLUS / PLUS |
| |
| |
| |
| |
| |
| |
| |
| |
| |

The full FCTR3a amino acid sequence also has 342 of 383 amino acid residues (89%) identical to, and 342 of 383 residues (89%) positive with, the 276 amino acid residue Odd Qz/ten-m homolog 2 (*Drosophila*) (GenBank Acc: NP_035986.2) (SEQ ID NO:68) (Table 3P).

**Table 3P. BLASTP of FCTR3a against Odd Oz/ten-m homolog 2 - (SEQ ID NO:68)**

| |
|---|
| >GI\|7657415\|REF\|NP 035986.21 ODD OZ/TEN-M HOMOLOG 2 (DROSOPHILA); ODD OZ/TEN-M |
| HOMOLOG 3 |
| (DROSOPHILA) [MUS MUSCULUS] |
| GI\|4760778\|DBJ\|BAA77397.1\| (AB025411) TEN-M2 [MUS MUSCULUS] |
| LENGTH = 2764 |
| SCORE = 495 BITS (1274), EXPECT = E-139 |
| IDENTITIES = 342/383 (89%), POSITIVES = 342/383 (89%), GAPS = 41/383 (10%) |
| |
| |
| |
| |
| |
| |
| |

The full FCTR3b amino acid sequence has 2442 of 2802 amino acid residues (87%) identical to, and 2532 of 2802 residues (90%) positive with, the 2802 amino acid residue teneurin-2 [*Gallus gallus*] (GenBank Acc: AJ279031) (SEQ ID NO:69) (Table 3Q).

**Table 3Q. BLASTP of FCTR3a against Teneurin-2 - (SEQ ID NO:69**

| |
|---|
| >GI\|10241574\|EMB\|CAC09416.1\| (AJ279031) TENEORIN-2 [GALLUS GALLUS] |
| LENGTH = 2802 |
| SCORE = 4853 BITS (12589), EXPECT = 0.0 |
| IDENTITIES = 2510/2802 (87%), POSITIVES = 2600/2802 (90%), GAPS = 69/2802 (2%) |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |

The FCTR3bcde and f amino acid sequences have 1524 of 2352 amino acid residues (64%) identical to, and 1881 of 2532 residues (79%) positive with, the amino acid residues 429-2771, 93 of 157 residues (59%) identical to and 118 of 157 residues (74%) positive with amino acid residues 1-155, and 59 of 152 residues (38%) identical to and 68 of 152 residues (43%) positive with amino acid residues 211-361 of Ten-m4 [*Mus musculus*] (ptnr: GenBank Acc: BAA77399.1) (SEQ ID NO:70) (Table 3R).

**Table 3R. BLASTP of FCTR3b, c, d, e, and f against Mus musculus Ten-m4 - (SEQ ID NO:70)**

| |
|---|
| >GI\|4760782\|DBJ\|BAA77399.1\| (AB025413) TEN-M4 [MUS MUSCULUS] |
| LENGTH = 2771 |
| SCORE - 3089 BITS (8008), EXPECT = 0.0 |
| IDENTITIES - 1524/2352 (64%), POSITIVES - 1881/2352 (79%), GAPS - 28/2352 (1%) |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| SCORE = 161 BITS (407), EXPECT = 2E-37 |
| IDENTITIES = 93/157 (59%), POSITIVES = 118/157 (74%), GAPS = 4/157 (2%) |
| |
| |
| |
| SCORE = 72.1 BITS (176), EXPECT - 8E-11 |
| IDENTITIES = 59/152 (38%), POSITIVES = 68/152 (43%), GAPS = 42/152 (27%) |
| |
| |
| |

| |
|---|
| *FCTR3F DOES NOT CONTAIN THESE AMINO ACIDS |

The 997-2733 amino acid fragment of the FCTR3bcde and f protein was also found to have 1695 of 1737 amino acid residues (97%) identical to, and 1695 of 1737 residues (97%) positive with the amino a 1737 amino acid residue protein KIAA1127 protein [*Homo sapiens*] (GenBank Acc:(AB032953) (SEQ ID NO:71), (Table 3S).

**Table 3S. BLASTP of FCTR3b, c, d, e, and f against Homo sapiens KIAA1127 protein (SEQ ID NO:71)**

| |
|---|
| >GI\|6329763\|DBJ\|BAA86441.1\| (AB032953) KIAA1127 PROTEIN [HOMO SAPIENS] |
| LENGTH = 1737 |
| SCORE = 3295 BITS (8545), EXPECT - 0.0 |
| IDENTITIES = 1695/1737 (97%), POSITIVES = 1695/1737 (97%) |
| |
| |
| |
| |
| |
| |
| |
| |
| |
| |
| |
| |
| |
| |
| |
| |
| |
| |
| |
| |
| |
| |
| |
| |
| |
| |
| |
| |
| |

The amino acid sequences of the FCTR3bcde and f proteins were also found to have 2528 of 2774 amino acid residues (91%) identical to, and 2557 of 2774 residues (92%) positive with, the 2765 amino acid residue protein neurestin alpha [*Rattus norvegicus*] (GenBank Acc:AF086607) (SEQ ID NO:72), shown in Table 3T.

**Table 3T. BLASTP of FCTR3bcd and f against Rattus norvegicus Neurestin alpha (SEQ ID NO:72)**

| |
|---|
| >GI\|9910320\|IREP\|NP 064473.1\| NEURESTIN ALPHA [RATTUS NORVEGICUS] |
| GI\|5712201\|GB\|AAD47383.1\|AF086607 1 (AF086607) NEORESTIN ALPHA [RATTUS NORVEGICUS] |
| LENGTH - 2765 |
| SCORE - 4998 BITS (12938), EXPECT = 0.0 |
| IDENTITIES - 2528/2774 (91%), POSITIVES - 2557/2774 (92%), GAPS - 50/2774 (1%) |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |

| |
|---|
| * = KTR3F DOES NOT CONTAIN THESE AMINO ACIDS |

The amino acid sequences of the FCTR3bcde and f proteins were also found to have 2536 of 2774 amino acid residues (91%) identical to, and 2558 of 2774 residues (91%) positive with, the 2764 amino acid residue protein Odd Oz/ten-m homolog 2 *(Drosophila)* (GenBank Acc:NP_035986.2) (SEQ ID NO:65), shown in Table 3U.

**Table 3U. BLASTP of FCTR3bcde and f against Odd Oz/ten-m homolog 2 (SEQ ID NO:65)**

| |
|---|
| >GI\|7657415\|REF\|NP 035986.2\| ODD OZ/TEN-M HOMOLOG 2 (DROSOPHILA): ODD OZ/TEN-M |
| HOMOLOG 3 |
| (DROSOPHILA) [MUS MUSCULUS) |
| GI\|4760118\|DBJ\|BAA77391.1\| (AB025411) TEN-M2 [MUS MUSCULUS] |
| LENGTH = 2764 |
| SCORE = 4996 BITS (12961), EXPECT = 0.0 |
| IDENTITIES = 2536/2774 (91%), POSITIVES = 2558/2774 (91%), GAPS = 51/2774 (1%) |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |
| |

| |
|---|
| ***** = FCTR3F DOES NOT CONTAIN THESE AMINO ACIDS |

FCTR3 is related to rat neurestin, a gene implicated in neuronal development (Otaki JM, Firestein S Dev Biol 1999 Aug 1;212(1):165-81) Neurestin shows homology to human gamma-heregulin, a Drosophila receptor-type pair-rule gene product, Odd Oz (Odz) / Ten(m), and Ten(a). Neurestin has putative roles in synapse formation and brain morphogenesis. A mouse neurestin homolog, DOC4, has independently been isolated from the NIH-3T3 fibroblasts. DOC4 is also known as tenascin M (TNM), a *Drosophila* pair-rule gene homolog containing extracellular EGF-like repeats. The significant homology to these molecules and in particular, γ-heregulin, have important implications regarding the potential contribution of FCTR3 to disease progression. Heregulin is the ligand for HBR-2/ErbB2/NEU, a proto-oncogene receptor tyrosine kinase implicated in breast and prostate cancer progression that was originally identified in rat neuro/glioblastoma cell lines. Extopic expression of HER-2/ErbB2/NEU in MDA-MB-435 breast adenocarcinoma cells confers chemoresistance to Taxol-induced apoptosis relative to vector transfected control cells (Yu et al. Overexpression of ErbB2 blocks Taxol-induced apoptosis by up-regulation of p21Cip1, which inhibits p34Cdc2 kinase. Molec. Cell 2: 581-591,1998).

### FCTR3 related tenascins and cancer biology

As mentioned, FCTR3 also has significant homology to DOC4, (AKA tenascin M), a *Drosophila* pair-rule gene homolog containing extracellular EGF-like repeats. The tenascins are a growing family of extracellular matrix proteins that play prominent roles in tissue interactions critical to embryogenesis. Overexpression of tenascins has been described in multiple human solid malignancies.

The role of the tenascin family of related proteins is to regulate epithelial-stromal interactions, participate in fibronectin-dependent cell attachment and interaction. Indeed, tenascin-C (TN) is overexpressed in the stroma of malignant ovarian tumours particularly at the interface between epithelia and stroma leading to suggestions that it may be involved in the process of invasion (Wilson et al (1996) Br J Cancer 74: 999-1004). Tenascin-C is considered a therapeutic target for certain malignant brain tumors (Gladson CL : J Neuropathol Exp Neurol 1999 Oct;58(10):1029-40). Stromal or moderate to strong periductal Tenascin-C expression in DCIS (ductal carcinoma in situ) correlates with tumor cell invasion. (Jahkola et al. Eur J Cancer 1998 Oct;34(11):1687-92. Tenascin-C expression at the invasion border of early breast cancer is a useful predictor of local and distant recurrence. Jahkola T, et al. Br J Cancer. 1998 Dec;78(11):1507-13). Tenascin (TN) is an extracellular matrix protein found in areas of cell migration during development and expressed at high levels in migratory glioma cells. Treasurywala S, Berens ME Glia 1998 Oct;24(2):236-43 Migration arrest in glioma cells is dependent on the alphaV integrin subunit. Phillips GR, Krushel LA, Crossin KL J Cell Sci 1998 Apr,111 (Pt 8):1095-104 Domains of tenascin involved in glioma migration. Finally, tenascin expression in hormone-dependent tissues of breast and endometrium indicate that Tenascin expression reflects malignant progression and is down-regulated by antiprogestins during terminal differentiation of rat mammary tumors (Vollmer et al. Cancer Res 1992 Sep 1;52(17):4642-8)

### Potential role of FCTR3 in oncologic disease progression:

Based on the bioactivity described in the medical literature for related molecules, FCTR3 may play a role in one or more aspects of tumor cell biology that alter the interactions of tumor epithelial cells with stromal components. In consideration, FCTR3 may play a role in the following malignant properties:
Autocrine/paracrine stimulation of tumor cell proliferation
Autocrine/paracrine stimulation of tumor cell survival and tumor cell resistance to cytotoxic therapy
Local tissue remodeling, paranechmal and basement membrane invasion and motility of tumor cells thereby contributing to metastasis.

Tumor-mediated immunosuppression of T-cell mediated immune effector cells and pathways resulting in tumor escape from immune surveilance.

### Therapeutic intervention targeting FCTR3 in oncologic and central nervous system indications:

Predicted disease indications from expression profiling in 41 normal human tissues and 55 human cancer cell lines (see Example 2) include a subset of human gliomas, astrocytomas, mixed glioma/astrocytomas, renal cells carcinoma, breast adenocarcinoma, ovarian cancer, melanomas. Targeting of FCTR3 by human or humanized monoclonal antibodies designed to disrupt predicted interactions of FCTR3 with its cognate ligand may result in significant anti-tumorlanti-metastatic activity and the amelioration of associated symptomatology. Identification of small molecules that specifically/selectively interfere with downstream signaling components engaged by FCTR3/ligandinteractions would also be expected to result in significant anti-tumor/anti-metastatic activity and the amelioration of associated symptomatology. Likewise, modified antisense ribonucleotides or antisense gene expression constructs (plasmids, adenovirus, adeno-associated viruses, "naked" DNA approaches) designed to diminish the expression of FCTR3 transcripts/messenger RNA (mRNA) would be anticipated based on predicted properties of FCTR3 to have anti-tumor impact.

Based on the relatedness to neurestin and heregulins, as well as its high level expression in brain tissue, FCTR3 may also be used for remyelination in order to promote regeneration/repair/remyleination of injured central nervous system cells resulting from ischemia, brain trauma and various neurodegenerative diseases.. This postulate is based on reports indicating that neuregulin, glial growth factor 2, diminishes autoimmune demyelination and enhances remyelination in a chronic relapsing model for multiple sclerosis (Cannella et al.. Proc. Nat. Acad. Sci. 95:10100-10105,1998). The expression of the related molecule neurestin can be induced in external tufted cells during regeneration of olfactory sensory neurons.

**TABLE 8A: Summary Of Nucleic Acids And Proteins Of The Invention**

| **Name** | **Tables** | **Clone; Description of Homolog** | **Nucleic Acid SEQ IOD NO** | **Amino Acid SEQ ID NO** |
|---|---|---|---|---|
| FCTR3 | 3C, 3D | 10129612.0.405; neurestin-like protein | 7 | 8 |
| | 3E | 10129612.0.154; neurestin-like protein | 9 | |
| | 3F | 10129612.0.67; neurestin-like protein | 10 | |
| | 3G | 10129612.0258; neurestin-like protein | 11 | |
| | 3H, 3I | 10129612.0.352; neurestin-like protein | | 13 |

**TABLE 8B: Summary of Query Sequences Disclosed**

| **Table** | **Database** | **Acc. No.** | **Sequence Name** | **Species** | **SEQ ID NO.** |
|---|---|---|---|---|---|
| 3J | GenBank | NM_011856.2 | Odd Oz/ten-m homology 2 | Fruit fly | 62 |
| 3K | Embl | AJ245711.1 | Teneurin-2 cDNA, short splice variant | Chicken | 63 |
| 3L | GenBank | AB032953 | KIAA 1127 cDNA | Human | 64 |
| 3M, 3U | GenBank | AB025411 | Ten-m2 cDNA | Mouse | 65 |
| 3N | GenBank | NM_020088.1 | Neurestin alpha cDNA | Rat | 66 |
| 30 | Emb1 | GGA278031 | Teneurin-2 | Chicken | 67 |
| 3P | GenBank | NP_035986.2 | Odd Oz/ten-m homology 2 | Fruit fly | 68 |
| 3Q | Embl | CAC09416.1 | Teneurin-2 | Chicken | 69 |
| 3R | GenBank | BAA77399.1 | Ten-m4 | Mouse | 70 |
| 3S | GenBank | AB032953 | KIAA1127 protein | Human | 71 |
| 3T | GenBank | AF086607 | Neurestin alpha | Rat | 72 |
| 4C | SptrEmbl | 099233 | Hypothetical 10 kD protein | Trypanos | 73 |

### FCTRX Nucleic Acids and Polypeptides

One aspect of the invention pertains to isolated nucleic acid molecules that encode FCTRX polypeptides or biologically-active portions thereof. Also included in the invention are nucleic acid fragments sufficient for use as hybridization probes to identify FCTRX-encoding nucleic acids (*e.g.,* FCTRX mRNAs) and fragments for use as PCR primers for the amplification and/or mutation of FCTRX nucleic acid molecules. As used herein, the term "nucleic acid molecule" is intended to include DNA molecules (*e.g.,* cDNA or genomic DNA), RNA molecules (*e.g.,* mRNA), analogs of the DNA or RNA generated using nucleotide analogs, and derivatives, fragments and homologs thereof. The nucleic acid molecule may be single-stranded or double-stranded, but preferably is comprised double-stranded DNA.

An FCTRX nucleic acid can encode a mature FCTRX polypeptide. As used herein, a "mature" form of a polypeptide or protein disclosed in the present invention is the product of a naturally occurring polypeptide or precursor form or proprotein. The naturally occurring polypeptide, precursor or proprotein includes, by way of nonlimiting example, the full length gene product, encoded by the corresponding gene. Alternatively, it may be defined as the polypeptide, precursor or proprotein encoded by an ORF described herein. The product "mature" form arises, again by way of nonlimiting example, as a result of one or more naturally occurring processing steps as they may take place within the cell, or host cell, in which the gene product arises. Examples of such processing steps leading to a "mature" form of a polypeptide or protein include the cleavage of the N-terminal methionine residue encoded by the initiation codon of an ORF, or the proteolytic cleavage of a signal peptide or leader sequence. Thus a mature form arising from a precursor polypeptide or protein that has residues 1 to N, where residue 1 is the N-terminal methionine, would have residues 2 through N remaining after removal of the N-terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or protein may arise from a step of post-traaslational modification other than a proteolytic cleavage event Such additional processes include, by way of non-limiting example, glycosylation, myristoylation or phosphorylation. In general, a mature polypeptide or protein may result from the operation of only one of these processes, or a combination of any of them.

The term "probes", as utilized herein, refers to nucleic acid sequences of variable length, preferably between at least about 10 nucleotides (nt), 100 nt, or as many as approximately, *e.g.,* 6,000 nt, depending upon the specific use. Probes are used in the detection of identical, similar, or complementary nucleic acid sequences. Longer length probes are generally obtained from a natural or recombinant source, are highly specific, and much slower to hybridize than shorter-length oligomer probes. Probes may be single- or double-stranded and designed to have specificity in PCR, membrane-based hybridization technologies, or ELISA-like technologies.

The term "isolated" nucleic acid molecule, as utilized herein, is one which is separated from other nucleic acid molecules which are present in the natural source of the nucleic acid. Preferably, an "isolated" nucleic acid is free of sequences which naturally flank the nucleic acid (*i.e.,* sequences located at the 5'- and 3'-termini of the nucleic acid) in the genomic DNA of the organism from which the nucleic acid is derived. For example, in various embodiments, the isolated FCTRX nucleic acid molecules can contain less than about 5 kb, 4 kb, 3 kb, 2 kb, 1 kb, 0.5 kb or 0.1 kb of nucleotide sequences which naturally flank the nucleic acid molecule in genomic DNA of the cell/tissue from which the nucleic acid is derived (*e.g.,* brain, heart, liver, spleen, etc.). Moreover, an "isolated" nucleic acid molecule, such as a cDNA molecule, can be substantially free of other cellular material or culture medium when produced by recombinant techniques, or of chemical precursors or other chemicals when chemically synthesized.

A nucleic acid molecule of the invention, *e.g.,* a nucleic acid molecule having the nucleotide sequence of SEQ ID NOS: 7, 9, 10 or 11, or a complement of this aforementioned nucleotide sequence, can be isolated using standard molecular biology techniques and the sequence information provided herein. Using all or a portion of the nucleic acid sequence of SEQ ID NOS: 7, 9, 10 or 11 as a hybridization probe, FCTRX molecules can be isolated using standard hybridization and cloning techniques (*e.g.,* as described in Sambrook, *et al.,* (eds.), MOLECULAR CLOMNG: A LABORATORY MANUAL 2^{nd} Ed., Cold Spring Harbor Laboratory Press, Cold Spring Harbor, NY, 1989; and Ausubel, *et al.,* (eds.), CURRENT PROTOCOLS IN MOLECULAR BIOLOGY, John Wiley & Sons, New York, NY,1993.)

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to FCTRX nucleotide sequences can be prepared by standard synthetic techniques, *e.g.,* using an automated DNA synthesizer.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length, In one embodiment of the invention, an oligonucleotide comprising a nucleic acid molecule less than 100 nt in length would further comprise at least 6 contiguous nucleotides of SEQ ID NOS: 7, 9, 10 or 11, or a complement thereof. Oligonucleotides may be chemically synthesized and may also be used as probes.

In another embodiment, an isolated nucleic acid molecule of the invention comprises a nucleic acid molecule that is a complement of the nucleotide sequence shown in SEQ ID NOS: 7, 9, 10 or 11, or a portion of this nucleotide sequence (*e.g.,* a fragment that can be used as a probe or primer or a fragment encoding a biologically-active portion of an FCTRX polypeptide). A nucleic acid molecule that is complementary to the nucleotide sequence shown in SEQ ID NOS: 5, 7, 9, 10 or 11, is one that is sufficiently complementary to the nucleotide sequence shown in SEQ ID NOS: 7, 9, 10, or 11, that it can hydrogen bond with little or no mismatches to the nucleotide sequence shown in SEQ ID NOS: 7, 9, 10 or 11, thereby forming a stable duplex.

As used herein, the term "complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof. Binding includes ionic, non-ionic, van der Waals, hydrophobic interactions, and the like. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

Fragments provided herein are defined as sequences of at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full length sequence. Fragments may be derived from any contiguous portion of a nucleic acid or amino acid sequence of choice. Derivatives are nucleic acid sequences or amino acid sequences formed from the native compounds either directly or by modification or partial substitution. Analogs are nucleic acid sequences or amino acid sequences that have a structure similar to, but not identical to, the native compound but differs from it in respect to certain components or side chains. Analogs may be synthetic or from a different evolutionary origin and may have a similar or opposite metabolic activity compared to wild type. Homologs are nucleic acid sequences or amino acid sequences of a particular gene that are derived from different species.

Derivatives and analogs may be full length or other than full length, if the derivative or analog contains a modified nucleic acid or amino acid, as described below. Derivatives or analogs of the nucleic acids or proteins of the invention include, but are not limited to, molecules comprising regions that are substantially homologous to the nucleic acids or proteins of the invention, in various embodiments, by at least about 70%, 80%, or 95% identity (with a preferred identity of 80-95%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions. See *e.g*. Ausubel, *et al.,* CURRENT PROTOCOLS IN MOLECULAR BIOLOGY, John Wiley & Sons, New York, NY, 1993, and below.

A "homologous nucleic acid sequence" or "homologous amino acid sequence," or variations thereof, refer to sequences characterized by a homology at the nucleotide level or amino acid level as discussed above. Homologous nucleotide sequences encode those sequences coding for isoforms of FCTRX polypeptides. Isoforms can be expressed in different tissues of the same organism as a result of, for example, alternative splicing of RNA. Alternatively, isoforms can be encoded by different genes. In the invention, homologous nucleotide sequences include nucleotide sequences encoding for an FCTRX polypeptide of species other than humans, including, but not limited to: vertebrates, and thus can include, *e.g.,* frog, mouse, rat, rabbit, dog, cat cow, horse, and other organisms. Homologous nucleotide sequences also include, but are not limited to, naturally occurring allelic variations and mutations of the nucleotide sequences set forth herein. A homologous nucleotide sequence does not, however, include the exact nucleotide sequence encoding human FCTRX protein. Homologous nucleic acid sequences include those nucleic acid sequences that encode conservative amino acid substitutions (see below) in SEQ ID NOS: 8 or 13, as well as a polypeptide possessing FCTRX biological activity. Various biological activities of the FCTRX proteins are described below.

An FCTRX polypeptide is encoded by the open reading frame ("ORF") of an FCTRX nucleic acid. An ORF corresponds to a nucleotide sequence that could potentially be translated into a polypeptide. A stretch of nucleic acids comprising an ORF is uninterrupted by a stop codon. An ORF that represents the coding sequence for a full protein begins with an ATG "start" codon and terminates with one of the three "stop" codons, namely, TAA, TAG, or TGA. For the purposes of this invention, an ORF may be any part of a coding sequence, with or without a start codon, a stop codon, or both. For an ORF to be considered as a good candidate for coding for a *bona fide* cellular protein, a minimum size requirement is often set, *e.g.,* a stretch of DNA that would encode a protein of 50 amino acids or more.

The nucleotide sequences determined from the cloning of the human FCTRX genes allows for the generation of probes and primers designed for use in identifying and/or cloning FCTRX homologues in other cell types, *e.g.* from other tissues, as well as FCTRX homologues from other vertebrates. The probe/primer typically comprises substantially purified oligonucleotide. The oligonucleotide typically comprises a region of nucleotide sequence that hybridizes under stringent conditions to at least about 12, 25, 50, 100, 150, 200, 250, 300, 350 or 400 consecutive sense strand nucleotide sequence of SEQ ID NOS: 7, 9, 10 or 11; or an anti-sense strand nucleotide sequence of SEQ ID NOS: 5, 7, 9, 10 or 11; or of a naturally occurring mutant of SEQ ID NOS: 7, 9, 10 or 11.

Probes based on the human FCTRX nucleotide sequences can be used to detect transcripts or genomic sequences encoding the same or homologous proteins. In various embodiments, the probe further comprises a label group attached thereto, e.g. the label group can be a radioisotope, a fluorescent compound, an enzyme, or an enzyme co-factor. Such probes can be used as a part of a diagnostic test kit for identifying cells or tissues which mis-express an FCTRX protein, such as by measuring a level of an FCTRX-encoding nucleic acid in a sample of cells from a subject *e.g.,* detecting FCTRX mRNA levels or determining whether a genomic FCTRX gene has been mutated or deleted.

"A polypeptide having a biologically-active portion of an FCTRX polypeptide" refers to polypeptides exhibiting activity similar, but not necessarily identical to, an activity of a polypeptide of the invention, including mature forms, as measured in a particular biological assay, with or without dose dependency. A nucleic acid fragment encoding a "biologically-active portion of FCTRX" can be prepared by isolating a portion of SEQ ID NOS: 7, 9, 10 or 11, that encodes a polypeptide having an FCTRX biological activity (the biological activities of the FCTRX proteins are described below), expressing the encoded portion of FCTRX protein (*e.g*., by recombinant expression *in vitro*) and assessing the activity of the encoded portion of FCTRX.

### FCTRX Nucleic Acid and Polypeptide Variants

The invention further encompasses nucleic acid molecules that differ from the nucleotide sequences shown in SEQ ID NOS: 7, 9, 10 or 11, due to degeneracy of the genetic code and thus encode the same FCTRX proteins as that encoded by the nucleotide sequences shown in SEQ ID NO NOS: 7, 9 10 or 11. In another embodiment, an isolated nucleic acid molecule of the invention has a nucleotide sequence encoding a protein having an amino acid sequence shown in SEQ ID NOS: 8 or 13.

In addition to the human FCTRX nucleotide sequences shown in SEQ ID NOS: 7, 9, 10 or 11, it will be appreciated by those skilled in the art that DNA sequence polymorphisms that lead to changes in the amino acid sequences of the FCTRX polypeptides may exist within a population (*e.g.,* the human population). Such genetic polymorphism in the FCTRX genes may exist among individuals within a population due to natural allelic variation. As used herein, the terms "gene" and "recombinant gene" refer to nucleic acid molecules comprising an open reading frame (ORF) encoding an FCTRX protein, preferably a vertebrate FCTRX protein. Such natural allelic variations can typically result in 1-5% variance in the nucleotide sequence of the FCTRX genes. Any and all such nucleotide variations and resulting amino acid polymorphisms in the FCTRX polypeptides, which are the result of natural allelic variation and that do not alter the functional activity of the FCTRX polypeptides, are intended to be within the scope of the invention.

Moreover, nucleic acid molecules encoding FCTRX proteins from other species, and thus that have a nucleotide sequence that differs from the human sequence of SEQ ID NOS: 7, 9, 10 or 11, are intended to be within the scope of the invention. Nucleic acid molecules corresponding to natural allelic variants and homologues of the FCTRX cDNAs of the invention can be isolated based on their homology to the human FCTRX nucleic acids disclosed herein using the human cDNAs, or a portion thereof, as a hybridization probe according to standard hybridization techniques under stringent hybridisation conditions.

Accordingly, in another embodiment, an isolated nucleic acid molecule of the invention is at least 6 nucleotides in length and hybridizes under stringent conditions to the nucleic acid molecule comprising the nucleotide sequence of SEQ ID NOS: 7, 9, 10 or 11. In another embodiment, the nucleic acid is at least 10, 25, 50, 100, 250, 500, 750, 1000, 1500, or 2000 or more nucleotides in length. In yet another embodiment, an isolated nucleic acid molecule of the invention hybridizes to the coding region. As used herein, the term "hybridizes under stringent conditions" is intended to describe conditions for hybridization and washing under which nucleotide sequences at least 60% homologous to each other typically remain hybridized to each other.

Homologs (*i.e.,* nucleic acids encoding FCTRX proteins derived from species other than human) or other related sequences (*e.g.,* paralogs) can be obtained by low, moderate or high stringency hybridization with all or a portion of the particular human sequence as a probe using methods well known in the art for nucleic acid hybridization and cloning.

As used herein, the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5°C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (*e.g.,* 10 nt to 50 nt) and at least about 60°C for longer probes, primers and oligonucleotides. Stringent conditions may also be achieved with the addition of destabilizing agents, such as formamide.

Stringent conditions are known to those skilled in the art and can be found in Ausubel, *et al.,* (eds.), CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions are hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C, followed by one or more washes in 0.2X SSC, 0.01% BSA at 50°C. An isolated nucleic acid molecule of the invention that hybridizes under stringent conditions to the sequences of SEQ ID NOS: 7, 9, 10 or 11, corresponds to a naturally-occvrring nucleic acid molecule. As used herein, a "naturally-occurring" nucleic acid molecule refers to an RNA or DNA molecule having a nucleotide sequence that occurs in nature (*e.g.,* encodes a natural protein).

In a second embodiment, a nucleic acid sequence that is hybridizable to the nucleic acid molecule comprising the nucleotide sequence of SEQ ID NOS: 7, 9, 10, or 11, or fragments, analogs or derivatives thereof, under conditions of moderate stringency is provided. A non-limiting example of moderate stringency hybridization conditions are hybridization in 6X SSC, 5X Denhardt's solution, 0.5% SDS and 100 mg/ml denatured salmon sperm DNA at 55°C, followed by one or more washes in 1X SSC, 0.1% SDS at 37°C. Other conditions of moderate stringency that may be used are well-known within the art. See, *e.g*., Ausubel, et *al.* (eds.), 1993, CURRENT PROTOCOLS IN MOLECULAR BIOLOGY, John Wiley & Sons, NY, and Kriegler, 1990; GENE TRANSFER AND EXPRESSION, A LABORATORY MANUAL, Stockton Press, NY.

In a third embodiment, a nucleic acid that is hybridizable to the nucleic acid molecule comprising the nucleotide sequences of SEQ ID NOS: 7, 9, 10 or 11, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that may be used are well known in the art (*e.g.,* as employed for cross-species hybridizations). See, *e.g.,* Ausubel, *et al.* (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981. *Proc Natl Acad Sci USA* 78: 6789-6792.

### Conservative Mutations

In addition to naturally-occurring allelic variants ofFCTRX sequences that may exist in the population, the skilled artisan will further appreciate that changes can be introduced by mutation into the nucleotide sequences of SEQ ID NO NOS: 7, 9, 10 or 11, thereby leading to changes in the amino acid sequences of the encoded FCTRX proteins, without altering the functional ability of said FCTRX proteins. For example, nucleotide substitutions leading to amino acid substitutions at "non-essential" amino acid residues can be made in the sequence of SEQ ID NOS: 8 or 13. A "non-essential" amino acid residue is a residue that can be altered from the wild-type sequences of the FCTRX proteins without altering their biological activity, whereas an "essential" amino acid residue is required for such biological activity. For example, amino acid residues that are conserved among the FCTRX proteins of the invention are predicted to be particularly non-amenable to alteration. Amino acids for which conservative substitutions can be made are well-known within the art.

Another aspect of the invention pertains to nucleic acid molecules encoding FCTRX proteins that contain changes in amino acid residues that are not essential for activity. Such FCTRX proteins differ in amino acid sequence from SEQ ID NOS: 8 or 13, yet retain biological activity. In one embodiment, the isolated nucleic acid molecule comprises a nucleotide sequence encoding a protein, wherein the protein comprises an amino acid sequence at least about 45% homologous to the amino acid sequences of SEQ ID NOS: 8 or 13. Preferably, the protein encoded by the nucleic acid molecule is at least about 60% homologous to SEQ ID NOS: 8 or 13; more preferably at least about 70% homologous to SEQ ID NOS: 8 or 13; still more preferably at least about 80% homologous to SEQ ID NOS: 8 or 13; even more preferably at least about 90% homologous to SEQ ID NOS: 8 or 13; and most preferably at least about 95% homologous to SEQ ID NOS: 8 or 13.

An isolated nucleic acid molecule encoding an FCTRX protein homologous to the protein of SEQ ID NOS: 8 or 13, can be created by introducing one or more nucleotide substitutions, additions or deletions into the nucleotide sequence of SEQ ID NOS: 7, 9, 10 or 11, such that one or more amino acid substitutions, additions or deletions are introduced into the encoded protein.

Mutations can be introduced into SEQ ID NOS: 8 or 13, by standard techniques, such as site-directed mutagenesis and PCR-mediated mutagenesis. Preferably, conservative amino acid substitutions are made at one or more predicted, non-cssential amino acid residues. A "conservative amino acid substitution" is one in which the amino acid residue is replaced with an amino acid residue having a similar side chain. Families of amino acid residues having similar side chains have been defined within the art. These families include amino acids with basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine). Thus, a predicted non-essential amino acid residue in the FCTRX protein is replaced with another amino acid residue from the same side chain family. Alternatively, in another embodiment, mutations can be introduced randomly along all or part of an FCTRX coding sequence, such as by saturation mutagenesis, and the resultant mutants can be screened for FCTRX biological activity to identify mutants that retain activity. Following mutagenesis of SEQ ID NOS: 8 or 13, the encoded protein can be expressed by any recombinant technology known in the art and the activity of the protein can be determined.

The relatedness of amino acid families may also be determined based on side chain interactions. Substituted amino acids may be fully conserved "strong" residues or fully conserved "weak" residues. The "strong" group of conserved amino acid residues may be any one of the following groups: STA, NEQK, NHQK, NDEQ, QHRK, MILV, MBLF, HY, FYW, wherein the single letter amino acid codes are grouped by those amino acids that may be substituted for each other. Likewise, the "weak" group of conserved residues may be any one of the following: CSA, ATV, SAG, SINK, STPA, SGND, SNDBQK, NDEQHK, NEQHRK, VLIM, HFY, wherein the letters within each group represent the single letter amino acid code.

In one embodiment, a mutant FCTRX protein can be assayed for (*i*) the ability to form protein:protein interactions with other FCTRX proteins, other cell-surface proteins, or biologically active portions thereof, (*ii*) complex formation between a mutant FCTRX protein and an FCTRX ligand; or (*iii*) the ability of a mutant FCTRX protein to bind to an intracellular target protein or biologically-active portion thereof; (*e.g.* avidin pmteins).

In yet another embodiment, a mutant FCTRX protein can be assayed for the ability to regulate a specific biological function (*e.g.,* regulation of insulin release).

### Antisense Nucleic Acids

Another aspect of the invention pertains to isolated antisense nucleic acid molecules that are hybridizable to or complementary to the nucleic acid molecule comprising the nucleotide sequence of SEQ ID NOS: 7, 9, 10 or 11, or fragments, analogs or derivatives thereof. An "antisense" nucleic acid comprises a nucleotide sequence that is complementary to a "sense" nucleic acid encoding a protein (*e.g.,* complementary to the coding strand of a double-stranded cDNA molecule or complementary to an mRNA sequence). In specific aspects, antisense nucleic acid molecules are provided that comprise a sequence complementary to at least about 10, 25, 50,100, 250 or 500 nucleotides or an entire FCTRX coding strand, or to only a portion thereof. Nucleic acid molecules encoding fragments, homologs, derivatives and analogs of an FCTRX protein of SEQ ID NOS: 8 or 13; or antisense nucleic acids complementary to an FCTRX nucleic acid sequence of SEQ ID NOS: 7, 9, 10 or 11, are additionally provided.

In one embodiment, an antisense nucleic acid molecule is antisense to a "coding region" of the coding strand of a nucleotide sequence encoding an FCTRX protein. The term "coding region" refers to the region of the nucleotide sequence comprising codons which are translated into amino acid residues. In another embodiment, the antisense nucleic acid molecule is antisense to a "noncoding region" of the coding strand of a nucleotide sequence encoding the FCTRX protein. The term "noncoding region" refers to 5' and 3' sequences which flank the coding region that are not translated into amino acids (*i.e.,* also referred to as 5' and 3' untranslated regions).

Given the coding strand sequences encoding the FCTRX protein disclosed herein, antisense nucleic acids of the invention can be designed according to the rules of Watson and Crick or Hoogsteen base pairing. The antisense nucleic acid molecule can be complementary to the entire coding region of FCTRX mRNA, but more preferably is an oligonucleotide that is antisense to only a portion of the coding or noncoding region of FCTRX mRNA. For example, the antisense oligonucleotide can be complementary to the region surrounding the translation start site of FCTRX mRNA. An antisense oligonucleotide can be, for example, about 5,10,15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (*e.g.,* an antisense oligonucleotide) can be chemically synthesized using naturally-occurring nucleotides or variously modified nucleotides designed to increase the biological stability of the molecules or to increase the physical stability of the duplex formed between the antisense and sense nucleic acids (*e.g.,* phosphorothioate derivatives and acridine substituted nucleotides can be used).

Examples of modified nucleotides that can be used to generate the antisense nucleic acid include: 5-fluomuracil, 5-bromouracil, S-chlorouracil, S-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcybosine, 5-methylcytosine, N6-adenine, 7-methylguanine, 5-methylaminomethyluracil, 5-methoxyaominomethyl-2-thiowacil, beta-D-mannosylqueosine, 5'-methoxycarboxymethyluracil, 5-methoxyuracil, 2-methylthio-N6-isopeatenyladenine, uaacil-5-oxyacetic acid (v), wybutoxosine, pseudouracil, queosine, 2-thiocytosme, 5-methyl-2-thiouracil, 2-thiouracil, 4-thiouracil, 5-methyhiracil, utacil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i.e.,* RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding an FCTRX protein to thereby inhibit expression of the protein (*e.g.,* by inhibiting transcription and/or translation). The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface (*e.g.,* by linking the antisense nucleic acid molecules to peptides or antibodies that bind to cell surface receptors or antigens). The antisense nucleic acid molecules can also be delivered to cells using the vectors described herein. To achieve sufficient nucleic acid molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other. See, *e.g.,* Gaultier, *et al.,* 1987. *Nucl. Acids Res.* **15:** 6625-6641. The antisense nucleic acid molecule can also comprise a 2'-o-methylribonucleotide (see, *e.g.,* Inoue, *et al.* 1987. *Nucl. Acids Res.* **15**: 6131-6148) or a chimeric RNA-DNA analogue (see, *e.g.,* Inoue, *et al.,* 1987. *FEBS Lett.* **215**: 327-330.

### Ribozymes and PNA Moieties

Nucleic acid modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

In one embodiment, an antisense nucleic acid of the invention is a ribozyme. Ribozymes are catalytic RNA molecules with ribonuclease activity that are capable of cleaving a single-stranded nucleic acid, such as an mRNA, to which they have a complementary region. Thus, ribozymes (*e.g.,* hammerhead ribozymes as described in Haselhoff and Gerlach 1988. *Nature* 334: 585-591) can be used to catalytically cleave FCTRX mRNA transcripts to thereby inhibit translation of FCTRX mRNA. A ribozyme having specificity for an FCTRX-encoding nucleic acid can be designed based upon the nucleotide sequence of an FCTRX cDNA disclosed herein (*i.e.,* SEQ ID NOS: 7, 9, 10 or 11). For example, a derivative of a *Tetrahymena* L-19 IVS RNA can be constructed in which the nucleotide sequence of the active site is complementary to the nucleotide sequence to be cleaved in an FCTRX-encoding mRNA. See, *e.g*., U.S. Patent 4,987,071 to Cech, *et al.* and U.S. Patent 5,116,742 to Cech, *et al.* FCTRX mRNA can also be used to select a catalytic RNA having a specific ribonuclease activity from a pool of RNA molecules See, *e.g.,* Bartel *et al.,* (1993) *Science* 261:1411-1418.

Alternatively, FCTRX gene expression can be inhibited by targeting nucleotide sequences complementary to the regulatory region of the FCTRX nucleic acid (*e.g.,* the FCTRX promoter and/or enhancers) to form triple helical structures that prevent transcription of the FCTRX gene in target cells. *See, e.g.,* Helene, 1991. *Anticancer Drug Des.* 6: 569-84; Helene, *et al.* 1992. *Ann. N.Y. Acad. Sci.* 660: 27-36; Maher,1992. *Bioassays* 14: 807-15.

In various embodiments, the FCTRX nucleic acids can be modified at the base moiety, sugar moiety or phosphate backbone to improve, *e.g*., the stability, hybridization, or solubility of the molecule. For example, the deoxyribose phosphate backbone of the nucleic acids can be modified to generate peptide nucleic acids. See, *e.g.,* Hyrup, *et al.,* 1996. *Bioorg Med Chem* 4: 5-23. As used herein, the terms "peptide nucleic acids" or "PNAs" refer to nucleic acid mimics (*e.g.,* DNA mimics) in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup, *et al.,* 1996. *supra*; Perry-O'Keefe, *et al.,* 1996. *Proc. Natl. Acad Sci. USA* 93:14670-14675.

PNAs of FCTRX can be used in therapeutic and diagnostic applications. For example, PNAs can be used as antisense or antigene agents for sequence-specific modulation of gene expression by, *e.g.,* inducing transcription or translation arrest or inhibiting replication. PNAs of FCTRX can also be used, for example, in the analysis of single base pair mutations in a gene (*e.g.,* PNA directed PCR clamping; as artificial restriction enzymes when used in combination with other enzymes, *e.g.,* S₁ nucleases *(see,* Hyrup, *et al.,* 1996. *supra*); or as probes or primers for DNA sequence and hybridization *(see,* Hyrup, *et al.,* 1996, *supra*; Perry-O'Keefe, *et al.,* 1996. *supra*).

In another embodiment, PNAs of FCTRX can be modified, *e.g.,* to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of FCTRX can be generated that may combine the advantageous properties ofPNA and DNA. Such chimeras allow DNA recognition enzymes (*e.g*., RNase H and DNA polymerases) to interact with the DNA portion while the PNA portion would provide high binding affinity and specificity. PNA-DNA chimeras can be linked using linkers of appropriate lengths selected in terms of base stacking, number of bonds between the nucleobases, and orientation *(see,* Hyrup, etal., 1996. *supra*). The synthesis of PNA-DNA chimeras can be performed as described in Hyrup, *et al., 1996. supra* and Finn, *et al.,* 1996. *Nucl Acids Res* 24: 3357-3363. For example, a DNA chain can be synthesized on a solid support using standard phosphoramidite coupling chemistry, and modified nucleoside analogs, *e.g*., 5'-(4-methoxytntyl)amino-5'-deoxy-thymidine phosphoramidite, can be used between the PNA and the 5' end of DNA. *See, e.g.,* Mag, *et al.,* 1989. *Nucl Acid Res* 17: 5973-5988. PNA monomers are then coupled in a stepwise manner to produce a chimeric molecule with a 5' PNA segment and a 3' DNA segment. *See, e.g.,* Finn, *et al.,* 1996. *supra.* Alternatively, chimeric molecules can be synthesized with a 5' DNA segment and a 3' PNA segment. *See, e.g.,* Petersen, *et al.,* 1975. *Bioorg. Med. Chem. Lett.* 5: 1119-11124.

In other embodiments, the oligonucleotide may include other appended groups such as peptides (*e.g.,* for targeting host cell receptors *in vivo*)*,* or agents facilitating transport across the cell membrane (*see, e.g.,* Letsinger, *et al.,* 1989. *Proc. Natl. Acad. Sci. U.S.A.* 86: 6553-6556; Lemaitre, *et al.,* 1987. *Proc. Natl. Acad. Sci.* 84: 648-652; PCT Publication No. WO88/09810) or the blood-brain barrier (*see, e.g.,* PCT Publication No. WO 89/10134). In addition, oligonucleotides can be modified with hybridization triggered cleavage agents (*see, e.g.,* Krol, *et al.,* 1988. *BioTechniques* 6:958-976) or intercalating agents (*see, e.g.,* Zon, 1988. *Pharm. Res.* 5: 539-549). To this end, the oligonucleotide may be conjugated to another molecule, *e.g.,* a peptide, a hybridization triggered cross-linking agent, a transport agent, a hybridization-triggered cleavage agent, and the like.

### FCTRX Polypeptides

A polypeptide according to the invention includes a polypeptide including the amino acid sequence of FCTRX polypeptides whose sequences are provided in SEQ ID NOS: 8 or 13. The invention also includes a mutant or variant protein any of whose residues may be changed from the corresponding residues shown in SEQ ID NOS: 8 or 13, while still encoding a protein that maintains its FCTRX activities and physiological functions, or a functional fragment thereof.

In general, an FCTRX variant that preserves FCTRX-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

One aspect of the invention pertains to isolated FCTRX proteins, and biologically-active portions thereof, or derivatives, fragments, analogs or homologs thereof. Also provided are polypeptide fragments suitable for use as immunogens to raise anti-FCTRX antibodies. In one embodiment, native FCTRX proteins can be isolated from cells or tissue sources by an appropriate purification scheme using standard protein purification techniques. In another embodiment, FCTRX proteins are produced by recombinant DNA techniques. Alternative to recombinant expression, an FCTRX protein or polypeptide can be synthesized chemically using standard peptide synthesis techniques.

An "isolated" or "purified" polypeptide or protein or biologically-active portion thereof is substantially free of cellular material or other contaminating proteins from the cell or tissue source from which the FCTRX protein is derived, or substantially free from chemical precursors or other chemicals when chemically synthesized. The language "substantially free of cellular material" includes preparations of FCTRX proteins in which the protein is separated from cellular components of the cells from which it is isolated or recombinantly-produced. In one embodiment, the language "substantially free of cellular material" includes preparations of FCTRX proteins having less than about 30% (by dry weight) of non-FCTRX proteins (also referred to herein as a "contaminating protein"), more preferably less than about 20% of non-FCTRX proteins, still more preferably less than about 10% of non-FCTRX proteins, and most preferably less than about 5% of non-FCTRX proteins. When the FCTRX protein or biologically-active portion thereof is recombinantly-produced, it is also preferably substantially free of culture medium, *i.e.,* culture medium represents less than about 20%, more preferably less than about 10%, and most preferably less than about 5% of the volume of the FCTRX protein preparation.

The language "substantially free of chemical precursors or other chemicals" includes preparations of FGTRX proteins in which the protein is separated from chemical precursors or other chemicals that are involved in the synthesis of the protein. In one embodiment, the language "substantially free of chemical precursors or other chemicals" includes preparations of FCTRX proteins having less than about 30% (by dry weight) of chemical precursors or non-FCTRX chemicals, more preferably less than about 20% chemical precursors or non-FCTRX chemicals, still more preferably less man about 10% chemical precursors or non-FCTRX chemicals, and most preferably less than about 5% chemical precursors or non-FCTRX chemicals.

Biologically-active portions of FCTRX proteins include peptides comprising amino acid sequences sufficiently homologous to or derived from the amino acid sequences of the FCTRX proteins (*e.g*., the amino acid sequence shown in SEQ ID NOS: 8 or 13), that include fewer amino acids than the full-length FCTRX proteins, and exhibit at least one activity of an FCTRX protein. Typically, biologically-active portions comprise a domain or motif with at least one activity of the FCTRX protein. A biologically-active portion of an FCTRX protein can be a polypeptide which is, for example, 10, 25, 50, 100 or more amino acid residues in length.

Moreover, other biologically-active portions, in which other regions of the protein are deleted, can be prepared by recombinant techniques and evaluated for one or more of the functional activities of a native FCTRX protein.

In an embodiment, the FCTRX protein has an amino acid sequence shown in SEQ ID NOS: 8 or 13. In other embodiments, the FCTRX protein is substantially homologous to SEQ ID NOS: 8 or 13, and retains the functional activity of the protein of SEQ ID NOS: 8 or 13, yet differs in amino acid sequence due to natural allelic variation or mutagenesis, as described in detail, below. Accordingly, in another embodiment, the FCTRX protein is a protein that comprises an amino acid sequence at least about 45% homologous to the amino acid sequence of SEQ ID NOS: 8 or 13, and retains the functional activity of the FCTRX proteins of SEQ ID NOS: 8 or 13.

### Determining Homology Between Two or More Sequences

To determine the percent homology of two amino acid sequences or of two nucleic acids, the sequences are aligned for optimal comparison purposes (*e.g.,* gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i.e.,* as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. *See*, Needleman and Wunsch, 1970. *J Mol Biol* 48:443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the CDS (encoding) part of the DNA-sequence shown in SEQ ID NOS: 7, 9, 10 or 11.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e.g.,* A, T, C, G, U, or I, in the case of nucleic acids) occurs in both sequences to yield the number of matched positions, dividing the number of matched positions by the total number of positions in the region of comparison (*i.e.,* the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region.

### Chimeric and Fusion Proteins

The invention also provides FCTRX chimeric or fusion proteins. As used herein, an FCTRX "chimeric protein" or "fusion protein" comprises an FCTRX polypeptide operatively-linked to a non-FCTRX polypeptide. An "FCTRX polypeptide" refers to a polypeptide having an amino acid sequence corresponding to an FCTRX protein (SEQ ID NOS: 8 or 13), whereas a "non-FCTRX polypeptide" refers to a polypeptide having an amino acid sequence corresponding to a protein that is not substantially homologous to the FCTRX protein, *e.g.,* a protein that is different from the FCTRX protein and that is derived from the same or a different organism. Within an FCTRX fusion protein the FCTRX polypeptide can correspond to all or a portion of an FCTRX protein. In one embodiment, an FCTRX fusion protein comprises at least one biologically-active portion of an FCTRX protein. In another embodiment, an FCTRX fusion protein comprises at least two biologically-active portions of an FCTRX protein. In yet another embodiment, an FCTRX fusion protein comprises at least three biologically-active portions of an FCTRX protein. Within the fusion protein, the term "operatively-linked" is intended to indicate that the FCTRX polypeptide and the non-FCTRX polypeptide are fused in-frame with one another. The non-FCTRX polypeptide can be fused to the N-tenninus or C-terminus of the FCTRX polypeptide.

In one embodiment, the fusion protein is a GST-FCTRX fusion protein in which the FCTRX sequences are fused to the C-terminus of the GST (glutathione S-transferase) sequences. Such fusion proteins can facilitate the purification of recombinant FCTRX polypeptides.

In another embodiment, the fusion protein is an FCTRX protein containing a heterologous signal sequence at its N-terminus. In certain host cells (*e.g.,* mammalian host cells), expression and/or secretion of FCTRX can be increased through use of a heterologous signal sequence.

In yet another embodiment, the fusion protein is an FCTRX-immunoglobulin fusion protein in which the FCTRX sequences are fused to sequences derived from a member of the immunoglobulin protein family. The FCTRX-immunoglobulin fusion proteins of the invention can be incorporated into pharmaceutical compositions and administered to a subject to inhibit an interaction between an FCTRX ligand and an FCTRX protein on the surface of a cell, to thereby suppress FCTRX-mediated signal transduction *in vivo.* The FCTRX-immunoglobulin fusion proteins can be used to affect the bioavailability of an FCTRX cognate ligand. Inhibition of the FCTRX ligand/FCTRX interaction may be useful therapeutically for both the treatment of proliferative and differentiative disorders, as well, as modulating (*e.g.* promoting or inhibiting) cell survival. Moreover, the FCTRX-immunoglobulin fusion proteins of the invention can be used as immunogens to produce anti-FCTRX antibodies in a subject, to purify FCTRX ligands, and in screening assays to identify molecules that inhibit the interaction of FCTRX with an FCTRX ligand.

An FCTRX chimeric or fusion protein of the invention can be produced by standard recombinant DNA techniques. For example, DNA fragments coding for the different polypeptide sequences are ligated together in-frame in accordance with conventional techniques, *e.g*., by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence *(see, e.g.,* Ausubel, *et al.* (eds.) CURRENT PROTOCOLS IN MOLECULAR BIOLOGY, John Wiley & Sons, 1992). Moreover, many expression vectors are commercially available that already encode a fusion moiety (*e.g.,* a GST polypeptide). An FCTRX-encoding nucleic acid can be cloned into such an expression vector such that the fusion moiety is linked in-frame to the FCTRX protein.

### FCTRX Agonists and Antagonists

The invention also pertains to variants of the FCTRX proteins that function as either FCTRX agonists (*i.e.,* mimetics) or as FCTRX antagonists. Variants of the FCTRX protein can be generated by mutagenesis (*e.g.,* discrete point mutation or truncation of the FCTRX protein). An agonist of the FCTRX protein can retain substantially the same, or a subset of, the biological activities of the naturally occurring form of the FCTRX protein. An antagonist of the FCTRX protein can inhibit one or more of the activities of the naturally occurring form of the FCTRX protein by, for example, competitively binding to a downstream or upstream member of a cellular signaling cascade which includes the FCTRX protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the FCTRX proteins.

Variants of the FCTRX proteins that function as either FCTRX agonists (*i.e.,* mimetics) or as FCTRX antagonists can be identified by screening combinatorial libraries of mutants (*e.g.,* truncation mutants) of the FCTRX proteins for FCTRX protein agonist or antagonist activity. In one embodiment, a variegated library of FCTRX variants is generated by combinatorial mutagenesis at the nucleic acid level and is encoded by a variegated gene library. A variegated library of FCTRX variants can be produced by, for example, enzymatically ligating a mixture of synthetic oligonucleotides into gene sequences such that a degenerate set of potential FCTRX sequences is expressible as individual polypeptides, or alternatively, as a set of larger fusion proteins (*e.g.,* for phage display) containing the set of FCTRX sequences therein. There are a variety of methods which can be used to produce libraries of potential FCTRX variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential FCTRX sequences. Methods for synthesizing degenerate oligonucleotides are well-known within the art. *See, e.g.,* Narang, 1983. *Tetrahedron* 39: 3; Itakura, *et al.,* 1984. *Annu. Rev. Biochem.* 53: 323; Itakura, *et al.,* 1984. *Science* 198: 1056; Ike, *et al.,* 1983. *Nucl. Acids Res.* 11: 477.

### Polypeptide Libraries

In addition, libraries of fragments of the FCTRX protein coding sequences can be used to generate a variegated population of FCTRX fragments for screening and subsequent selection of variants of an FCTRX protein. In one embodiment, a library of coding sequence fragments can be generated by treating a double stranded PCR fragment of an FCTRX coding sequence with a nuclease under conditions wherein nicking occurs only about once per molecule, denaturing the double stranded DNA, renaturing the DNA to form double-stranded DNA that can include sense/antisense pairs from different nicked products, removing single stranded portions from reformed duplexes by treatment with S₁ nuclease, and ligating the resulting fragment library into an expression vector. By this method, expression libraries can be derived which encodes N-terminal and internal fragments of various sizes of the FCTRX proteins.

Various techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of FCTRX proteins. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify FCTRX variants. *See, e.g.,* Arkin and Yourvan, 1992. *Proc. Natl. Acad. Sci. USA* 89:7811-7815; Delgrave, *et al.,* 1993. *Protein Engineering* 6:327-331.

### Anti-FCTRX Antibodies

The invention encompasses antibodies and antibody fragments, such as F_{ab} or (F_{ab})₂, that bind specifically to any of the FCTRX polypeptides of said invention.

An isolated FCTRX protein, or a portion or fragment thereof, can be used as an immunogen to generate antibodies that bind to FCTRX polypeptides using standard techniques for polyclonal and monoclonal antibody preparation. The full-length FCTRX proteins can be used or, alternatively, the invention provides antigenic peptide fragments of FCTRX proteins for use as immunogens. The antigenic FCTRX peptides comprises at least 4 amino acid residues of the amino acid sequence shown in SEQ ID NO NOS: 8 or 13, and encompasses an epitope of FCTRX such that an antibody raised against the peptide forms a specific immune complex with FCTRX. Preferably, the antigenic peptide comprises at least 6, 8, 10, 15, 20, or 30 amino acid residues. Longer antigenic peptides are sometimes preferable over shorter antigenic peptides, depending on use and according to methods well known to someone skilled in the art.

In certain embodiments of the invention, at least one epitope encompassed by the antigenic peptide is a region of FCTRX that is located on the surface of the protein (*e.g.,* a hydrophilic region). As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation (*see, e.g.,* Hopp and Woods, 1981. *Proc. Nat. Acad Sci. USA* 78: 3824-3828; Kyte and Doolittle, 1982. *J. Mol. Biol.* 157:105-142, each incorporated herein by reference in their entirety).

As disclosed herein, FCTRX protein sequences of SEQ ID NOS 8 or 13, or derivatives, fragments, analogs or homologs thereof, may be utilized as immunogens in the generation of antibodies that specifically-bind these protein components. The term "antibody" as used herein refers to immunoglobulin molecules and immunologically-active portions of immunoglobulin molecules, *i.e.,* molecules that contain an antigen binding site that specifically-binds (immunoreacts with) an antigen, such as FCTRX. Such antibodies include, but are not limited to, polyclonal, monoclonal, chimeric, single chain, F_{ab} and F_{(ab')2} fragments, and an F_{ab} expression library. In a specific embodiment, antibodies to human FCTRX proteins are disclosed. Various procedures known within the art may be used for the production of polyclonal or monoclonal antibodies to an FCTRX protein sequence of SEQ ID NOS: 8 or 13, or a derivative, fragment, analog or homolog thereof. Some of these proteins are discussed below.

For the production of polyclonal antibodies, various suitable host animals (*e.g*., rabbit, goat, mouse or other mammal) may be immunized by injection with the native protein, or a synthetic variant thereof, or a derivative of the foregoing. An appropriate immunogenic preparation can contain, for example, recombinantly-expressed FCTRX protein or a chemically-synthesized FCTRX polypeptide. The preparation can further include an adjuvant. Various adjuvants used to increase the immunological response include, but are not limited to, Freund's (complete and incomplete), mineral gels (*e.g*., aluminum hydroxide), surface active substances (*e.g.,* lysolecithin, pluronic polyols, polyanions, peptides, oil emulsions, dinitrophenol, etc.), human adjuvants such as *Bacille Calmette-Guerin* and *Corynebacterium parvum,* or similar immunostimulatory agents. If desired, the antibody molecules directed against FCTRX can be isolated from the mammal (*e.g*., from the blood) and further purified by well known techniques, such as protein A chromatography to obtain the IgG fraction.

The term "monoclonal antibody" or "monoclonal antibody composition", as used herein, refers to a population of antibody molecules that contain only one species of an antigen binding site capable of immunoreacting with a particular epitope of FCTRX. A monoclonal antibody composition thus typically displays a single binding affinity for a particular FCTRX protein with which it immunoreacts. For preparation of monoclonal antibodies directed towards a particular FCTRX protein, or derivatives, fragments, analogs or homologs thereof, any technique that provides for the production of antibody molecules by continuous cell line culture may be utilized. Such techniques include, but are not limited to, the hybridoma technique (*see, e.g.,* Kohler & Milstein, 1975. *Nature* 256:495-497); the trioma technique; the human B-cell hybridoma technique *(see, e.g.,* Kozbor, *et al.,* 1983. *Immunol. Today* 4: 72) and the EBV hybridoma technique to produce human monoclonal antibodies (*see, e.g.,* Cole, *et al.,* 1985. In: MONOCLONAL ANTIBODIES AND CANCER THERAPY, Alan R. Liss, Inc., pp. 77-96). Human monoclonal antibodies may be utilized in the practice of the invention and may be produced by using human hybridomas *(see, e.g.,* Cote, *et al.,* 1983. *Proc Natl Acad Sci USA* 80: 2026-2030) or by transforming human B-cells with Epstein Barr Virus *in vitro* (*see, e.g.,* Cole, *et al.,* 1985. In: MONOCLONAL ANTIBODIES AND CANCER THERAPY, Alan R. Liss, Inc., pp. 77-96). Each of the above citations is incorporated herein by reference in their entirety.

According to the invention, techniques can be adapted for the production of single-chain antibodies specific to an FCTRX protein *(see, e.g.,* U.S. Patent No. 4,946,778). In addition, methods can be adapted for the construction of F_{ab} expression libraries (*see, e.g.,* Huse, *et al.,* 1989. *Science* 246:1275-1281) to allow rapid and effective identification of monoclonal F_{ab} fragments with the desired specificity for an FCTRX protein or derivatives, fragments, analogs or homologs thereof. Non-human antibodies can be "humanized" by techniques well known in the art. *See, e.g.,* U.S. Patent No. 5,225,539. Antibody fragments that contain the idiotypes to an FCTRX protein may be produced by techniques known in the art including, but not limited to: (*i*) an F_{(ab')2} fragment produced by pepsin digestion of an antibody molecule; (*ii*) an F_{ab} fragment generated by reducing the disulfide bridges of an F_{(ab')2} fragment; (*iii*) an F_{ab} fragment generated by the treatment of the antibody molecule with Papain and a reducing agent; and (*iv*) Fᵥ fragments.

Additionally, recombinant anti-FCTRX antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in International Application No. PCT/US86/02269; European Patent Application No. 184,187; European Patent Application No. 171,496; European Patent Application No. 173,494; PCT International Publication No. WO 86/01533; U.S. Patent No. 4,816,567; U.S. Pat. No. 5,225,539; European Patent Application No. 125,023; Better, *et.al.,* 1988. *Science* 240: 1041-1043; Liu, *et al.,* 1987. *Proc. Natl. Acad Sci. USA* 84: 3439-3443; Liu, *et al.,* 1987. *J. Immunol.* 139: 3521-3526; Sun, *et al.,* 1987. *Proc. Natl. Acad Sci. USA* 84: 214-218; Nishimura, *et al.,* 1987. *Cancer Res*. 47: 999-1005; Wood, *et al.,* 1985. *Nature* 314 :446-449; Shaw, *et al.,* 1988. *J. Natl. Cancer Inst.* 80: 1553-1559); Morrison(1985) *Science* 229:1202-1207; Oi, *et al.* (1986) *BioTechniques* 4:214; Jones, *et al.,* 1986. *Nature* 321: 552-525; Verhoeyan, *et al.,* 1988. *Science* 239: 1534; and Beidler, *et al.,* 1988. *J. Immunol.* 141: 4053-4060. Each of the above citations are incorporated herein by reference in their entirety.

In one embodiment, methods for the screening of antibodies that possess the desired specificity include, but are not limited to, enzyme-linked immunosorbent assay (ELISA) and other immunologically-mediated techniques known within the art. In a specific embodiment, selection of antibodies that are specific to a particular domain of an FCTRX protein is facilitated by generation of hybridomas that bind to the fragment of an FCTRX protein possessing such a domain. Thus, antibodies that are specific for a desired domain within an FCTRX protein, or derivatives, fragments, analogs or homologs thereof, are also provided herein.

Anti-FCTRX antibodies may be used in methods known within the art relating to the localization and/or quantitation of an FCTRX protein (*e.g.,* for use in measuring levels of the FCTRX protein within appropriate physiological samples, for use in diagnostic methods, for use in imaging the protein, and the like). In a given embodiment, antibodies for FCTRX proteins, or derivatives, fragments, analogs or homologs thereof, that contain the antibody derived binding domain, are utilized as phannacologically-active compounds (hereinafter "Therapeutics").

An anti-FCTRX antibody (*e.g.,* monoclonal antibody) can be used to isolate an FCTRX polypeptide by standard techniques, such as affinity chromatography or immunoprecipitation. An and-FCTRX antibody can facilitate the purification of natural FCTRX polypeptide from cells and of recombinantly-produced FCTRX polypeptide expressed in host cells. Moreover, an anti-FCIRX antibody can be used to detect FCTRX protein (*e.g.,* in a cellular lysate or cell supernatant) in order to evaluate the abundance and pattern of expression of the FCTRX protein. Anti-FCTRX antibodies can be used diagnostically to monitor protein levels in tissue as part of a clinical testing procedure, *e.g.,* to, for example, determine the efficacy of a given treatment regimen. Detection can be facilitated by coupling (*i.e*., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I. ³⁵S or ³H.

### FCTRX Recombinant Expression Vectors and Host Cells

Another aspect of the invention pertains to vectors, preferably expression vectors, containing a nucleic acid encoding an FCTRX protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e*.*g*., bacterial vectors having a bacterial origin of replication and episomal mammalian vectors). Other vectors (*e.g.,* non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g.,* replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-finked to the nucleic acid sequence to be expressed. Within a recombinant expression vector, "operaMy-Iinked" is intended to mean that the nucleotide sequence of interest is linked to the regulatory sequence(s) in a manner that allows for expression of the nucleotide sequence (*e.g.,* in an *in vitro* transcription/translation system or in a host cell when the vector is introduced into the host cell).

The term "regulatory sequence" is intended to includes promoters, enhancers and other expression control elements (*e.g.,* polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e.g.,* tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e.g.,* FCTRX proteins, mutant forms of FCTRX proteins, fusion proteins, etc.).

The recombinant expression vectors of the invention can be designed for expression of FCTRX proteins in prokaryotic or eukaryotic cells. For example, FCTRX proteins can be expressed in bacterial cells such as *Escherichia coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro;* for example using T7 promoter regulatory sequences and T7 polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (*i*) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988. *Gene* 67:31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann *et al.,* (1988) *Gene* 69:301-315) and pET 11d (Studier *et al.,* GENE EXPRESSION TECHNOLOGY: METHODS IN ENZYMOLOGY 185, Academic Press, San Diego, Calif. (1990) 60-89).

One strategy to maximize recombinant protein expression in *E*. *coli* is to express the protein in a host bacteria with an impaired capacity to proteolytically cleave the recombinant protein. *See, e.g.,* Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E*. *coli* (*see, e.g.,* Wada, *et al.,* 1992. *Nucl. Acids Res.* 20:2111-2118). Such alteration of nucleic acid sequences of the invention can be carried out by standard DNA synthesis techniques.

In another embodiment, the FCTRX expression vector is a yeast expression vector. Examples of vectors for expression in yeast *Saccharomyces cerivisae* include pYepSec1 (Baldari, *et al.,* 1987. *EMBO J.* 6: 229-234), pMFa (Kurjan and Herskowitz, 1982. *Cell* 30: 933-943), pJRY88 (Schultz *et al.,* 1987. *Gene* 54: 113-123), pYES2 (Invitrogen Corporation, San Diego, Calif.), and picZ (In Vitrogen Corp, San Diego, Calif).

Alternatively, FCTRX can be expressed in insect cells using baculovirus expression vectors. Baculovirus vectors available for expression of proteins in cultured insect cells (*e.g.,* SF9 cells) include the pAc series (Smith, *et al.,* 1983. *Mol. Cell. Biol.* 3: 2156-2165) and the pVL series (Lucklow and Summers, 1989. *Virology* 170:31-39).

In yet another embodiment, a nucleic acid of the invention is expressed in mammalian cells using a mammalian expression vector. Examples of mammalian expression vectors include pCDM8 (Seed,1987. *Nature* 329: 840) and pMT2PC (Kaufman, *et al.,* 1987. EMBO *J.* 6: 187-195). When used in mammalian cells, the expression vector's control functions are often provided by viral regulatory elements. For example, commonly used promoters are derived from polyoma, adenovirus 2, cytomegalovirus, and simian virus 40. For other suitable expression systems for both prokaryotic and eukaryotic cells see, *e.g*., Chapters 16 and 17 of Sambrook, *et al.,* MOLECULAR CLONING: A LABORATORY MANUAL. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type (*e.g.,* tissue-specific regulatory elements are used to express the nucleic acid). Tissue-specific regulatory elements are known in the art. Non-limiting examples of suitable tissue-specific promoters include the albumin promoter (liver-specific; Pinkert, *et al.,* 1987. *Genes Dev.* 1: 268-277), lymphoid-specific promoters (Calame and Eaton, 1988. *Adv. Immunol.* 43: 235-275), in particular promoters of T cell receptors (Winoto and Baltimore, 1989. *EMBO J.* 8: 729-733) and immunoglobulins (Banerji, *et al.,* 1983. *Cell* 33: 729-740; Queen and Baltimore, 1983. *Cell* 33:741-748), neuron-specific promoters (*e.g.,* the neurofilament promoter; Byrne and Ruddle, 1989. *Proc. Natl. Acad. Sci. USA* 86: 5473-5477), pancreas-specific promoters (Edlund, *et al.,* 1985. *Science* 230: 912-916), and mammary gland-specific promoters (*e.g.,* milk whey promoter; U.S. Pat. No. 4,873,316 and European Application Publication No. 264,166). Developmentally-regulated promoters are also encompassed, *e.g.,* the murine hox promoters (Kessel and Gruss, 1990. *Science* 249: 374-379) and the α-fetoprotein promoter (Campes and Tilghman, 1989. *Genes Dev*. 3:537-546).

The invention further provides a recombinant expression vector comprising a DNA molecule of the invention cloned into the expression vector in an antisense orientation. That is, the DNA molecule is operatively-linked to a regulatory sequence in a manner that allows for expression (by transcription of the DNA molecule) of an RNA molecule that is antisense to FCTRX mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes *see, e.g.,* Weintraub, *et al.,* "Antisense RNA as a molecular tool for genetic analysis," *Reviews-Trends in Genetics,* Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, FCTRX protein can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g.,* DNA) into a host cell, including calcium phosphate or calcium chloride co-precipitation, DEAE-dextiran-mediated transfection, lipofection, or electroporation. Suitable methods for transforming or transfecting host cells can be found in Sambrook, *et al.* (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g.,* resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding FCTRX or can be introduced on a separate vector. Cells stably transfected with the introduced nucleic acid can be identified by drug selection (*e.g.,* cells that have incorporated the selectable marker gene will survive, while the other cells die).

A host cell of the invention, such as a prokaryotic or eukaryotic host cell in culture, can be used to produce (*i.e.,* express) FCTRX protein. Accordingly, the invention further provides methods for producing FCTRX protein using the host cells of the invention. In one embodiment, the method comprises culturing the host cell of invention (into which a recombinant expression vector encoding FCTRX protein has been introduced) in a suitable medium such that FCTRX protein is produced. In another embodiment, the method further comprises isolating FCTRX protein from the medium or the host cell.

### Transgenic FCTRX Animals

The host cells of the invention can also be used to produce non-human transgenic animals. For example, in one embodiment, a host cell of the invention is a fertilized oocyte or an embryonic stem cell into which FCTRX protein-coding sequences have been introduced. Such host cells can then be used to create non-human transgenic animals in which exogenous FCTRX sequences have been introduced into their genome or homologous recombinant animals in which endogenous FCTRX sequences have been altered. Such animals are useful for studying the function and/or activity of FCTRX protein and for identifying and/or evaluating modulators of FCTRX protein activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA that is integrated into the genome of a cell from which a transgenic animal develops and that remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous FCTRX gene has been altered by homologous recombination between the endogenous gene and an exogenous DNA molecule introduced into a cell of the animal, *e.g.,* an embryonic cell of the animal, prior to development of the animal.

A transgenic animal of the invention can be created by introducing FCTRX-encoding nucleic acid into the male pronuclei of a fertilized oocyte (*e*.*g*., by microinjection, retroviral infection) and allowing the oocyte to develop in a pseudopregnant female foster animal. The human FCTRX cDNA sequences of SEQ ID NOS: 7, 9, 10 or 11, can be introduced as a transgene into the genome of a non-human animal. Alternatively, a non-human homologue of the human FCTRX gene, such as a mouse FCTRX gene, can be isolated based on hybridization to the human FCTRX cDNA (described further *supra*) and used as a transgene. Intronic sequences and polyadenylation signals can also be included in the transgene to increase the efficiency of expression of the transgene. A tissue-specific regulatory sequence(s) can be operably-linked to the FCTRX transgene to direct expression of FCTRX protein to particular cells. Methods for generating transgenic animals via embryo manipulation and microinjection, particularly animals such as mice, have become conventional in the art and are described, for example, in U.S. Patent Nos. 4,736,866; 4,870,009; and 4,873,191; and Hogan, 1986. In: MANIPULATING THE MOUSE EMBRYO, Cold Spring Harbor Laboratory Press, Cold Spring Harbor, N.Y. Similar methods are used for production of other transgenic animals. A transgenic founder animal can be identified based upon the presence of the FCTRX transgene in its genome and/or expression ofFCTRX mRNA in tissues or cells of the animals. A transgenic founder animal can then be used to breed additional animals carrying the transgene. Moreover, transgenic animals carrying a transgene-encoding FCTRX protein can further be bred to other transgenic animals carrying other transgenes.

To create a homologous recombinant animal, a vector is prepared which contains at least a portion of an FCTRX gene into which a deletion, addition or substitution has been introduced to thereby alter, *e.g.,* functionally disrupt, the FCTRX gene. The FCTRX gene can be a human gene (*e.g.,* the cDNA of SEQ ID NOS: 7, 9, 10 or 11), but more preferably, is a non-human homologue of a human FCTRX gene. For example, a mouse homologue of human FCTRX gene of SEQ ID NOS: 7, 9, 10 or 11, can be used to construct a homologous recombination vector suitable for altering an endogenous FCTRX gene in the mouse genome. In one embodiment, the vector is designed such that, upon homologous recombination, the endogenous FCTRX gene is functionally disrupted (*i.e.,* no longer encodes a functional protein; also referred to as a "knock out" vector).

Alternatively, the vector can be designed such that, upon homologous recombination, the endogenous FCTRX gene is mutated or otherwise altered but still encodes functional protein (*e.g.,* the upstream regulatory region can be altered to thereby alter the expression of the endogenous FCTRX protein). In the homologous recombination vector, the altered portion of the FCTRX gene is flanked at its 5'- and 3'-termini by additional nucleic acid of the FCTRX gene to allow for homologous recombination to occur between the exogenous FCTRX gene carried by the vector and an endogenous FCTRX gene in an embryonic stem cell. The additional flanking FCTRX nucleic acid is of sufficient length for successful homologous recombination with the endogenous gene. Typically, several kilobases of flanking DNA (both at the 5'- and 3'-termini) are included in the vector. *See, e.g.,* Thomas, *et al.,* 1987. *Cell* 51: 503 for a description of homologous recombination vectors. The vector is ten introduced into an embryonic stem cell line (*e.g.,* by electroporation) and cells in which the introduced FCTRX gene has homologously-recombined with the endogenous FCTRX gene are selected. *See, e.g., Li, et al.,* 1992. *Cell* 69: 915.

The selected cells are then injected into a blastocyst of an animal (*e.g.,* a mouse) to form aggregation chimeras. *See, e.g.,* Bradley, 1987. In: TERATOCARCINOMAS AND EMBRYONIC STEM CELLS: A PRACTICAL APPROACH, Robertson, ed. IRL, Oxford, pp. 113-152. A chimeric embryo can then be implanted into a suitable pseudopregnant female foster animal and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described further in Bradley, 1991. *Curr. Opin. Biotechnol.* 2: 823-829; PCT International Publication Nos.: WO 90/11354; WO 91/01140; WO 92/0968; and WO 93/04169.

In another embodiment, transgenic non-humans animals can be produced that contain selected systems that allow for regulated expression of the transgene. One example of such a system is the cre/loxP recombinase system of bacteriophage P1. For a description of the cre/loxP recombinase system, *See, e.g.,* Lakso, *et al.,* 1992. *Proc. Natl. Acad. Sci. USA* 89: 6232-6236. Another example of a recombinase system is the FLP recombinase system of *Saccharomyces cerevisiae. See,* O'Gorman, *et al.,* 1991. *Science* 251:1351-1355. If a cre/loxP recombinase system is used to regulate expression of the transgene, animals containing transgenes encoding both the Cre recombinase and a selected protein are required. Such animals can be provided through the construction of "double" transgenic animals, *e.g.,* by mating two transgenic animals, one containing a transgene encoding a selected protein and the other containing a transgene encoding a recombinase.

Clones of the non-human transgenic animals described herein can also be produced according to the methods described in Wilmut, *et al.,* 1997. *Nature* 385: 810-813. In brief, a cell (*e.g.,* a somatic cell) from the transgenic animal can be isolated and induced to exit the growth cycle and enter Go phase. The quiescent cell can then be fused, *e.g.,* through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell (*e.g.,* the somatic cell) is isolated.

### Pharmaceutical Compositions

The FCTRX nucleic acid molecules, FCTRX proteins, and anti-FCTRX antibodies (also referred to herein as "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e.g.,* intravenous, intradermal, subcutaneous, oral (*e.g.,* inhalation), transdermal (*i.e.,* topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e.g.,* an FCTRX protein or anti-FCTRX antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g.,* with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (*see, e.g.,* U.S. Patent No. 5,328,470) or by stereotactic injection (*see, e.g.,* Chen, *et al.,* 1994. *Proc. Natl. Acad. Sci. USA* 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.,* retroviral vectors, the pharmaceutical preparation can include one or more cells that produce the gene delivery system.

The pharmaceutical compositions can be included in a container, pack, or dispenser together with instructions for administration.

### Screening and Detection Methods

The isolated nucleic acid molecules of the invention can be used to express FCTRX protein (*e.g.,* via a recombinant expression vector in a host cell in gene therapy applications), to detect FCTRX mRNA (*e.g.,* in a biological sample) or a genetic lesion in an FCTRX gene, and to modulate FCTRX activity, as described further, below. In addition, the FCTRX proteins can be used to screen drugs or compounds that modulate the FCTRX protein activity or expression as well as to treat disorders characterized by insufficient or excessive production of FCTRX protein or production of FCTRX protein forms that have decreased or aberrant activity compared to FCTRX wild-type protein (*e.g.*; diabetes (regulates insulin release); obesity (binds and transport lipids); metabolic disturbances associated with obesity, the metabolic syndrome X as well as anorexia and wasting disorders associated with chronic diseases and various cancers, and infectious disease(possesses anti-microbial activity) and the various dyslipidemias. In addition, the anti-FCT'RX antibodies of the invention can be used to detect and isolate FCTRX proteins and modulate FCTRX activity. In yet a further aspect, the invention can be used in methods to influence appetite, absorption of nutrients and the disposition of metabolic substrates in both a positive and negative fashion.

The invention further pertains to novel agents identified by the screening assays described herein and uses thereof for treatments as described, *supra.*

### Screening Assays

The invention provides a method (also referred to herein as a "screening assay") for identifying modulators, *i.e.,* candidate or test compounds or agents (*e.g.,* peptides, peptidomimetics, small molecules or other drugs) that bind to FCTRX proteins or have a stimulatory or inhibitory effect on, *e.g.,* FCTRX protein expression or FCTRX protein activity. The invention also includes compounds identified in the screening assays described herein.

In one embodiment, the invention provides assays for screening candidate or test compounds which bind to or modulate the activity of the membrane-bound form of an FCTRX protein or polypeptide or biologically-active portion thereof. The test compounds of the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds. *See, e.g.,* Lam, 1997. *Anticancer Drug Design* 12: 145.

A "small molecule" as used herein, is meant to refer to a composition that has a molecular weight of less than about 5 kD and most preferably less than about 4 kD. Small molecules can be, *e.g.,* nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention.

Examples of methods for the synthesis of molecular libraries can be found in the art, for example in: DeWitt, *et al.,* 1993. *Proc. Natl. Acad. Sci. U.S.A.* 90: 6909; Erb, *et al.,* 1994. *Proc. Natl. Acad. Sci. U.S.A.* 91: 11422; Zuckermann, *et al.,* 1994. *J. Med. Chem.* 37: 2678; Cho, *et al.,* 1993. *Science* 261: 1303; Carrell, *et al.,* 1994. *Angew. Chem. Int. Ed. Engl.* 33: 2059; Carell, *et al.,* 1994. *Angew. Chem. Int. Ed. Engl.* 33: 2061; and Gallop, *et al.,* 1994. *J. Med. Chem.* 37:1233.

Libraries of compounds may be presented in solution (*e.g.,* Houghten, 1992. *Biotechniques* 13: 412-421), or on beads (Lam, 1991. *Nature* 354: 82-84), on chips (Fodor, *1993. Nature* 364: 555-556), bacteria (Ladner, U.S. Patent No. 5,223,409), spores (Ladner, U.S. Patent 5,233,409), plasmids (Cull, *et al.,* 1992. *Proc. Natl. Acad Set. USA* 89: 1865-1869) or on phage (Scott and Smith, 1990. *Science* 249:386-390; Devlin, 1990. *Science* 249: 404-406; Cwirla, *et al.,* 1990. *Proc. Natl. Acad Sci. U.S.A.* 87: 6378-6382; Felici, 1991. *J. Mol. Biol.* 222: 301-310; Ladner, U.S. PatentNo. 5,233,409.).

In one embodiment, an assay is an in vitro cell-based assay in which a cell which expresses a membrane-bound form of FCTRX protein, or a biologically-active portion thereof, on the cell surface is contacted with a test compound and the ability of the test compound to bind to an FCTRX protein determined. The cell, for example, can of mammalian origin or a yeast cell. Determining the ability of the test compound to bind to the FCTRX protein can be accomplished, for example, by coupling the test compound with a radioisotope or enzymatic label such that binding of the test compound to the FCTRX protein or biologically-active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemission or by scintillation counting. Alternatively, test compounds can be enzymatically-labeled with, for example, horseradish peroxidase, alkaline phosphatase, or luciferase, and the enzymatic label detected by determination of conversion of an appropriate substrate to product. In one embodiment, the assay comprises contacting a cell which expresses a membrane-bound form of FGTRX protein, or a biologically-active portion thereof, on the cell surface with a known compound which binds FCTRX to form an assay mixture, contacting the assay mixture with a test compound, and determining the ability of the test compound to interact with an FCTRX protein, wherein determining the ability of the test compound to interact with an FCTRX protein comprises determining the ability of the test compound to preferentially bind to FCTRX protein or a biologically-active portion thereof as compared to the known compound.

In another embodiment, an assay is an in vitro cell-based assay comprising contacting a cell expressing a membrane-bound form of FCTRX protein, or a biologically-active portion thereof, on the cell surface with a test compound and determining the ability of the test compound to modulate (*e.g.,* stimulate or inhibit) the activity of the FCTRX protein or biologically active portion thereof. Determining the ability of the test compound to modulate the activity of FCTRX or a biologically-active portion thereof can be accomplished, for example, by determining the ability of the FCTRX protein to bind to or interact with an FCTRX target molecule. As used herein, a "target molecule" is a molecule with which an FCTRX protein binds or interacts in nature, for example, a molecule on the surface of a cell which expresses an FCTRX interacting protein, a molecule on the surface of a second cell, a molecule in the extracellular milieu, a molecule associated with the internal surface of a cell membrane or a cytoplasmic molecule. An FCTRX target molecule can be a non-FCTRX molecule or an FCTRX protein or polypeptide of the invention. In one embodiment, an FCTRX target molecule is a component of a signal transduction pathway that facilitates transduction of an extracellular signal (*e.g.* a signal generated by binding of a compound to a membrane-bound FCTRX molecule) through the cell membrane and into the cell. The target, for example, can be a second intercellular protein that has catalytic activity or a protein that facilitates the association of downstream signaling molecules with FCTRX.

Determining the ability of the FCTRX protein to bind to or interact with an FCTRX target molecule can be accomplished by one of the methods described above for determining direct binding. In one embodiment, determining the ability of the FCTRX protein to bind to or interact with an FCTRX target molecule can be accomplished by determining the activity of the target molecule. For example, the activity of the target molecule can be determined by detecting induction of a cellular second messenger of the target (*i.e*. intracellular Ca²⁺, diacylglycerol, IP₃, etc.), detecting catalytic/enzymatic activity of the target an appropriate substrate, detecting the induction of a reporter gene (comprising an FCTRX-responsive regulatory element operatively linked to a nucleic acid encoding a detectable marker, *e.g.,* luciferase), or detecting a cellular response, for example, cell survival, cellular differentiation, or cell proliferation.

In yet another embodiment, an assay of the invention is an in vitro cell-free assay comprising contacting an FCTRX protein or biologically-active portion thereof with a test compound and determining the ability of the test compound to bind to the FCTRX protein or biologically-active portion thereof. Binding of the test compound to the FCTRX protein can be determined either directly or indirectly as described above. In one such embodiment, the assay comprises contacting the FCTRX protein or biologically-active portion thereof with a known compound which binds FCTRX to form an assay mixture, contacting the assay mixture with a test compound, and determining the ability of the test compound to interact with an FCTRX protein, wherein determining the ability of the test compound to interact with an FCTRX protein comprises determining the ability of the test compound to preferentially bind to FCTRX or biologically-active portion thereof as compared to the known compound.

In still another embodiment, an assay is an in vitro cell-free assay comprising contacting FCTRX protein or biologically-active portion thereof with a test compound and determining the ability of the test compound to modulate (*e.g.* stimulate or inhibit) the activity of the FCTRX protein or biologically-active portion thereof. Determining the ability of the test compound to modulate the activity of FCTRX can be accomplished, for example, by determining the ability of the FCTRX protein to bind to an FCTRX target molecule by one of the methods described above for determining direct binding. In an alternative embodiment, determining the ability of the test compound to modulate the activity of FCTRX protein can be accomplished by determining the ability of the FCTRX protein further modulate an FCTRX target molecule. For example, the catalytic/enzymatic activity of the target molecule on an appropriate substrate can be determined as described, *supra.*

In yet another embodiment, the in vitro cell-free assay comprises contacting the FCTRX protein or biologically-active portion thereof with a known compound which binds FCTRX protein to form an assay mixture, contacting the assay mixture with a test compound, and determining the ability of the test compound to interact with an FCTRX protein, wherein determining the ability of the test compound to interact with an FCTRX protein comprises determining the ability of the FCTRX protein to preferentially bind to or modulate the activity of an FCTRX target molecule.

The in vitro cell-free assays of the invention are amenable to use of both the soluble form or the membrane-bound form of FCTRX protein. In the case of cell-free assays comprising the membrane-bound form of FCTRX protein, it may be desirable to utilize a solubilizing agent such that the membrane-bound form of FCTRX protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, N-dodecyl-N,N-dimethyl-3-ammonio-1-propane sulfonate, 3-(3-cholamidopropyl) dimethylamminiol-1-propane sulfonate (CHAPS), or 3-(3-cholamidopropyl)dimethylamminiol-2-hydroxy-1-propane sulfonate (CHAPSO).

In more than one embodiment of the above assay methods of the invention, it may be desirable to immobilize either FCTRX protein or its target molecule to facilitate separation of complexed from uncomplexed forms of one or both of the proteins, as well as to accommodate automation of the assay. Binding of a test compound to FCTRX protein, or interaction of FCTRX protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-FCTRX fusion proteins or GST-target fusion proteins can be adsorbed onto glutathione sepharose beads (Sigma Chemical, St. Louis, MO) or glutathione derivatized microtiter plates, that are then combined with the test compound or the test compound and either the non-adsorbed target protein or FCTRX protein, and the mixture is incubated under conditions conducive to complex formation (*e.g.,* at physiological conditions for salt and pH). Following incubation, the beads or microtiter plate wells are washed to remove any unbound components, the matrix immobilized in the case of beads, complex determined either directly or indirectly, for example, as described, *supra.* Alternatively, the complexes can be dissociated from the matrix, and the level of FCTRX protein binding or activity determined using standard techniques.

Other techniques for immobilizing proteins on matrices can also be used in the screening assays of the invention. For example, either the FCTRX protein or its target molecule can be immobilized utilizing conjugation of biotin and streptavidin. Biotinylated FCTRX protein or target molecules can be prepared from biotin-NHS (N-hydroxy-succinimide) using techniques well-known within the art (*e.g.,* biotinylation kit, Pierce Chemicals, Rockford, Ill.), and immobilized in the wells of streptavidin-coated 96 well plates (Pierce Chemical). Alternatively, antibodies reactive with FCTRX protein or target molecules, but which do not interfere with binding of the FCTRX protein to its target molecule, can be derivatized to the wells of the plate, and unbound target or FCTRX protein trapped in the wells by antibody conjugation. Methods for detecting such complexes, in addition to those described above for the GST-immobilized complexes, include immunodetection of complexes using antibodies reactive with the FCTRX protein or target molecule, as well as enzyme-linked assays that rely on detecting an enzymatic activity associated with the FCTRX protein or target molecule.

In another embodiment, modulators of FCTRX protein expression are identified in a method wherein a cell is contacted in vitro with a candidate compound and the expression of FCTRX mRNA or protein in the cell is determined. The level of expression of FCTRX mRNA or protein in the presence of the candidate compound is compared to the level of expression of FCTRX mRNA or protein in the absence of the candidate compound. The candidate compound can then be identified as a modulator of FCTRX mRNA or protein expression based upon this comparison. For example, when expression of FCTRX mRNA or protein is greater (*i.e.,* statistically significantly greater) in the presence of the candidate compound than in its absence, the candidate compound is identified as a stimulator of FCTRX mRNA or protein expression. Alternatively, when expression of FCTRX mRNA or protein is less (statistically significantly less) in the presence of the candidate compound than in its absence, the candidate compound is identified as an inhibitor of FCTRX mRNA or protein expression. The level of FCTRX mRNA or protein expression in the cells can be determined by methods described herein for detecting FCTRX mRNA or protein.

In yet another aspect of the invention, the FCTRX proteins can be used as "bait proteins" in a two-hybrid assay or three hybrid assay (*see, e.g.,* U.S. Patent No. 5,283,317; Zervos, *et al.,* 1993. *Cell* 72: 223-232; Madura, *et al.,* 1993. *J. Biol. Chem.* 268: 12046-12054; Bartel, *et al.,* 1993. *Biotechniques* 14: 920-924; Iwabuchi, *et al.,* 1993. *Oncogene* 8: 1693-1696; and Brent WO 94/10300), to identify other proteins that bind to or interact with FCTRX ("FCTRX-binding proteins" or "FCTRX-bp") and modulate FCTRX activity. Such FCTRX-binding proteins are also likely to be involved in the propagation of signals by the FCTRX proteins as, for example, upstream or downstream elements of the FCTRX pathway.

The two-hybrid system is based on the modular nature of most transcription factors, which consist of separable DNA-binding and activation domains. Briefly, the assay utilizes two different DNA constructs. In one construct, the gene that codes for FCTRX is fused to a gene encoding the DNA binding domain of a known transcription factor (*e.g.,* GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming an FCTRX-dependent complex, the DNA-binding and activation domains of the transcription factor are brought into close proximity. This proximity allows transcription of a reporter gene (*e.g.,* LacZ) that is operably linked to a transcriptional regulatory site responsive to the transcription factor. Expression of the reporter gene can be detected and cell colonies containing the functional transcription factor can be isolated and used to obtain the cloned gene that encodes the protein which interacts with FCTRX.

The invention further pertains to novel agents identified by the aforementioned screening assays and uses thereof for treatments as described herein.

### Detection Assays

Portions or fragments of the cDNA sequences identified herein (and the corresponding complete gene sequences) can be used in numerous ways as polynucleotide reagents. By way of example, and not of limitation, these sequences can be used to: (*i*) map their respective genes on a chromosome; and, thus, locate gene regions associated with genetic disease; (*ii*) identify an individual from a minute biological sample (tissue typing); and (*iii*) aid in forensic identification of a biological sample. Some of these applications are described in the subsections, below.

### Chromosome Mapping

Once the sequence (or a portion of the sequence) of a gene has been isolated, this sequence can be used to map the location of the gene on a chromosome. This process is called chromosome mapping. Accordingly, portions or fragments of the FCTRX sequences, SEQ ID NOS: 7, 9, 10 or 11, or fragments or derivatives thereof, can be used to map the location of the FCTRX genes, respectively, on a chromosome. The mapping of the FCTRX sequences to chromosomes is an important first step in correlating these sequences with genes associated with disease.

Briefly, FCTRX genes can be mapped to chromosomes by preparing PCR primers (preferably 15-25 bp in length) from the FCTRX sequences. Computer analysis of the FCTRX, sequences can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the FCTRX sequences will yield an amplified fragment.

Somatic cell hybrids are prepared by fusing somatic cells from different mammals (*e.g.,* human and mouse cells). As hybrids of human and mouse cells grow and divide, they gradually lose human chromosomes in random order, but retain the mouse chromosomes. By using media in which mouse cells cannot grow, because they lack a particular enzyme, but in which human cells can, the one human chromosome that contains the gene encoding the needed enzyme will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. *See, e.g.,* D'Bustachio, *et al.,* 1983. *Science* 220: 919-924. Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the FCTRX sequences to design oligonucleotide primers, sub-localization can be achieved with panels of fragments from specific chromosomes.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical like colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases, will suffice to get good results at a reasonable amount of time. For a review of this technique, *see,* Verma, *et al.,* HUMAN CHROMOSOMES: A MANUAL OF BASIC TECHNIQUES (Pergamon Press, New York 1988).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. Such data are found, *e.g*., in McKusick, MENDEUAN INHERITANCE IN MAN, available on-line through Johns Hopkins University Welch Medical Library). The relationship between genes and disease, mapped to the same chromosomal region, can then be identified through linkage analysis (co-inheritance of physically adjacent genes), described in, *e.g.,* Egeland, *et al.,* 1987. *Nature,* 325: 783-787.

Moreover, differences in the DNA sequences between individuals affected and unaffected with a disease associated with the FCTRX gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

The FCTRX sequences of the invention can also be used to identify individuals from minute biological samples. In this technique, an individual's genomic DNA is digested with one or more restriction enzymes, and probed on a Southern blot to yield unique bands for identification. The sequences of the invention are useful as additional DNA markers for RFLP ("restriction fragment length polymorphisms," described in U.S. Patent No. 5,272,057).

Furthermore, the sequences of the invention can be used to provide an alternative technique that determines the actual base-by-base DNA sequence of selected portions of an individual's genome. Thus, the FCTRX sequences described herein can be used to prepare two PCR primers from the 5'- and 3'-termini of the sequences. These primers can then be used to amplify an individual's DNA and subsequently sequence it

Panels of corresponding DNA sequences from individuals, prepared in this manner, can provide unique individual identifications, as each individual will have a unique set of such DNA sequences due to allelic differences. The sequences of the invention can be used to obtain such identification sequences from individuals and from tissue. The FCTRX sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Much of the allelic variation is due to single nucleotide polymorphisms (SNPs), which include restriction fragment length polymorphisms (RFLPs).

Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences can comfortably provide positive individual identification with a panel of perhaps 10 to 1,000 primers that each yield a noncoding amplified sequence of 100 bases. If predicted coding sequences, such as those in SEQ ID NOS: 7, 9, 10 or 11, are used, a more appropriate number of primers for positive individual identification would be 500-2,000.

### Predictive Medicine

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining FCTRX protein and/or nucleic acid expression as well as FCTRX activity, in the context of a biological sample (*e.g.,* blood, serum, cells, tissue) to thereby determine whether an individual is afflicted with a disease or disorder, or is at risk of developing a disorder, associated with aberrant FCTRX expression or activity. The disorders include *e.g.,* Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance. The invention also provides for prognostic (or predictive) assays for determining whether an individual is at risk of developing a disorder associated with FCTRX protein, nucleic acid expression or activity. For example, mutations in an FCTRX gene can be assayed in a biological sample. Such assays can be used for prognostic or predictive purpose to thereby prophylactically treat an individual prior to the onset of a disorder characterized by or associated with FCTRX protein, nucleic acid expression, or biological activity.

Another aspect of the invention provides methods for determining FCTRX protein, nucleic acid expression or activity in an individual to thereby select appropriate therapeutic or prophylactic agents for that individual (referred to herein as "pharmacogenomics"). Pharmacogenomics allows for the selection of agents (*e.g.,* drugs) for therapeutic or prophylactic treatment of an individual based on the genotype of the individual (*e.g.,* the genotype of the individual examined to determine the ability of the individual to respond to a particular agent.)

Yet another aspect of the invention pertains to monitoring the influence of agents (*e.g.,* drugs, compounds) on the expression or activity of FCTRX in clinical trials.

These and other agents are described in further detail in the following sections.

### Diagnostic Assays

An exemplary method for detecting the presence or absence of FCTRX in a biological sample involves contacting a biological sample from a test subject with a compound or an agent capable of detecting FCTRX protein or nucleic acid (*e.g.,* mRNA, genomic DNA) that encodes FCTRX protein such that the presence of FCTRX is detected in the biological sample. An agent for detecting FCTRX mRNA or genomic DNA is a labeled nucleic acid probe capable of hybridizing to FCTRX mRNA or genomic DNA. The nucleic acid probe can be, for example, a full-length FCTRX nucleic acid, such as the nucleic acid of SEQ ID NOS: 7, 9, 10 or 11, or a portion thereof, such as an oligonucleotide of at least 15, 30, 50, 100, 250 or 500 nucleotides in length and sufficient to specifically hybridize under stringent conditions to FCTRX mRNA or genomic DNA Other suitable probes for use in the diagnostic assays of the invention are described herein.

An agent for detecting FCTRX protein is an antibody capable of binding to FCTRX protein, preferably an antibody with a detectable label. Antibodies can be polyclonal, or more preferably, monoclonal. An intact antibody, or a fragment thereof (*e.g*., Fab or F(ab')₂) can be used. The term "labeled", with regard to the probe or antibody, is intended to encompass direct labeling of the probe or antibody by coupling (*i.e.,* physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. That is, the detection method of the invention can be used to detect FCTRX mRNA, protein, or genomic DNA in a biological sample *in vitro* as well as *in vivo.* For example, *in vitro* techniques for detection of FCTRX mRNA include Northern hybridizations and *in situ* hybridizations. *In vitro* techniques for detection of FCTRX protein include enzyme linked immunosorbent assays (ELISAs), Western blots, immunoprecipitations, and immunofluorescence. *In vitro* techniques for detection of FCTRX genomic DNA include Southern hybridizations. Furthermore, *in vivo* techniques for detection of FCTRX protein include introducing into a subject a labeled and-FCTRX antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In another embodiment, the methods further involve contacting a control sample from a control subject with a compound or agent capable of detecting FCTRX protein, mRNA, or genomic DNA, such that the presence of FCTRX protein, mRNA or genomic DNA is detected in the biological sample, and comparing the presence of FCTRX protein, mRNA or genomic DNA in the control sample with the presence of FCTRX protein, mRNA or genomic DNA in the test sample.

The invention also encompasses kits for detecting the presence of FCTRX in a biological sample. For example, the kit can comprise: a labeled compound or agent capable of detecting FCTRX protein or mRNA in a biological sample; means for determining the amount of FCTRX in the sample; and means for comparing the amount of FCTRX in the sample with a standard. The compound or agent can be packaged in a suitable container. The kit can further comprise instructions for using the kit to detect FCTRX protein or nucleic acid

### Prognostic Assays

The diagnostic methods described herein can furthermore be utilized to identify subjects having or at risk of developing a disease or disorder associated with aberrant FCTRX expression or activity. For example, the assays described herein, such as the preceding diagnostic assays or the following assays, can be utilized to identify a subject having or at risk of developing a disorder associated with FCTRX protein, nucleic acid expression or activity. Alternatively, the prognostic assays can be utilized to identify a subject having or at risk for developing a disease or disorder. Thus, the invention provides a method for identifying a disease or disorder associated with aberrant FCTRX expression or activity in which a test sample is obtained from a subject and FCTRX protein or nucleic acid (*e.g*., mRNA, genomic DNA) is detected, wherein the presence of FCTRX protein or nucleic acid is diagnostic for a subject having or at risk of developing a disease or disorder associated with aberrant FCTRX expression or activity. As used herein, a "test sample" refers to a biological sample from a subject of interest. For example, a test sample can be a biological fluid (*e.g.,* serum), cell sample, or tissue.

Furthermore, the prognostic assays described herein can be used to determine whether a subject can be administered an agent (*e.g.,* an agonist, antagonist, peptidomimetic, protein, peptide, nucleic acid, small molecule, or other drug candidate) to treat a disease or disorder associated with aberrant FCTRX expression or activity. For example, such methods can be used to determine whether a subject can be effectively treated with an agent for a disorder. Thus, the invention provides methods for determining whether a subject can be effectively treated with an agent for a disorder associated with aberrant FCTRX expression or activity in which FCTRX protein or nucleic acid is detected in a test sample (*e*.*g*., wherein the presence of FCTRX protein or nucleic acid is diagnostic for a subject that can be administered the agent to treat a disorder associated with aberrant FCTRX expression or activity).

The methods of the invention can also be used to detect genetic lesions in an FCTRX gene, thereby determining if a subject with the lesioned gene is at risk for a disorder characterized by aberrant cell proliferation and/or differentiation. In various embodiments, the methods include detecting, in a sample of cells from the subject, the presence or absence of a genetic lesion characterized by at least one of an alteration affecting the integrity of a gene encoding an FCTRX-protein, or the misexpression of the FCTRX gene. For example, such genetic lesions can be detected by ascertaining the existence of at least one of: (*i*) a deletion of one or more nucleotides from an FCTRX gene; (*ii*) an addition of one or more nucleotides to an FCTRX gene; (*iii*) a substitution of one or more nucleotides of an FCTRX gene, (*iv*) a chromosomal rearrangement of an FCTRX gene; (*v*) an alteration in the level of a messenger RNA transcript of an FCTRX gene, (*vi*) aberrant modification of an FCTRX gene, such as of the methylation pattern of the genomic DNA, (*vii*) the presence of a non-wild-type splicing pattern of a messenger RNA transcript of an FCTRX gene, (*viii*) a non-wild-type level of an FCTRX protein, (*ix*) allelic loss of an FCTRX gene, and (*x*) inappropriate post-translational modification of an FCTRX protein. As described herein, there are a large number of assay techniques known in the art which can be used for detecting lesions in an FCTRX gene. A preferred biological sample is a peripheral blood leukocyte sample isolated by conventional means from a subject. However, any biological sample containing nucleated cells may be used, including, for example, buccal mucosal cells.

In certain embodiments, detection of the lesion involves the use of a probe/primer in a polymerase chain reaction (PCR) (*see, e*.*g*., U.S. Patent Nos. 4,683,195 and 4**,**683,202), such as anchor PCR or RACE PCR, or, alternatively, in a ligation chain reaction (LCR) (*see, e.g.,* Landegran, *et al.,* 1988. *Science* 241: 1077-1080; and Nakazawa, *et al.,* 1994. *Proc. Natl. Acad. Sci. USA* 91: 360-364), the latter of which can be particularly useful for detecting point mutations in the FCTRX-gene *(see,* Abravaya, *et al.,* 1995. *Nucl. Acids Res.* 23: 675-682). This method can include the steps of collecting a sample of cells from a patient, isolating nucleic acid (*e.g.,* genomic, mRNA or both) from the cells of the sample, contacting the nucleic acid sample with one or more primers that specifically hybridize to an FCTRX gene under conditions such that hybridization and amplification of the FCTRX gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (*see*, Guatelli, *et al.,* 1990. *Proc. Natl. Acad Sci. USA* 87: 1874-1878), transcriptional amplification system (*see,* Kwoh, *et al.,* 1989. *Proc. Natl. Acad. Sci. USA* 86:1173-1177); Qβ Replicase *(see,* Lizardi, *et al,* 1988. *BioTechnology* 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in an FCTRX gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (*see, e.g.,* U.S. Patent No. 5,493,531) can be used to score for the presence of specific mutations by development or loss of a ribozyme cleavage site.

In other embodiments, genetic mutations in FCTRX can be identified by hybridizing a sample and control nucleic acids, *e.g*., DNA or RNA, to high-density arrays containing hundreds or thousands of oligonucleotides probes. *See, e.g.,* Cronin, *et al.,* 1996. *Human Mutation* 7: 244-255; Kozal, *et al.,* 1996. *Nat. Med.* 2: 753-759. For example, genetic mutations in FCTRX can be identified in two dimensional arrays containing light-generated DNA probes as described in Cronin, *et al., supra.* Briefly, a first hybridization array of probes can be used to scan through long stretches of DNA in a sample and control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the FCTRX gene and detect mutations by comparing the sequence of the sample FCTRX with the corresponding wild-type (control) sequence. Examples of sequencing reactions include those based on techniques developed by Maxim and Gilbert, 1977. *Proc. Natl. Acad. Sci. USA* 74: 560 or Sanger, 1977. *Proc. Natl. Acad. Sci. USA* 74: 5463. It is also contemplated that any of a variety of automated sequencing procedures can be utilized when performing the diagnostic assays *(see, e.g.,* Naeve, *et al.,* 1995. *Biotechniques* 19: 448), including sequencing by mass spectrometry (*see*, *e.g.,* PCT International Publication No. WO 94/16101; Cohen, *et al.,* 1996. *Adv. Chromatography* 36: 127-162; and Griffin, *et al.,* 1993. *Appl. Biochem. Biotechnol.* 38: 147-159).

Other methods for detecting mutations in the FCTRX gene include methods in which protection from cleavage agents is used to detect mismatched bases in RNA/RNA or RNA/DNA heteroduplexes. *See, e.g.,* Myers, *et al.,* 1985. *Science* 230: 1242. In general, the art technique of "mismatch cleavage" starts by providing heteroduplexes of formed by hybridizing (labeled) RNA or DNA containing the wild-type FCTRX sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S₁ nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation *See, e.g.,* Cotton, *et al.,* 1988. *Proc. Natl. Acad. Sci. USA* 85: 4397; Saleeba, *et al.,* 1992. *Methods Enzymol.* 217: 286-295. In an embodiment, the control DNA or RNA can be labeled for detection

In still another embodiment, the mismatch cleavage reaction employs one or more proteins that recognize mismatched base pairs in double-stranded DNA (so called "DNA mismatch repair" enzymes) in defined systems for detecting and mapping point mutations in FCTRX cDNAs obtained from samples of cells. For example, the mutY enzyme of *E. coli* cleaves A at G/A mismatches and the thymidine DNA glycosylase from HeLa cells cleaves T at G/T mismatches. *See, e.g.,* Hsu, *et al.,* 1994. *Carcinogenesis* 15*:* 1657-1662. According to an exemplary embodiment, a probe based on an FCTRX sequence, *e.g.,* a wild-type FCTRX sequence, is hybridized to a cDNA or other DNA product from a test cell(s). The duplex is treated with a DNA mismatch repair enzyme, and the cleavage products, if any, can be detected from electrophoresis protocols or the like. *See, e.g.,* U.S. Patent No. 5,459,039.

In other embodiments, alterations in electrophoretic mobility will be used to identify mutations in FCTRX genes. For example, single strand conformation polymorphism (SSCP) may be used to detect differences in electrophoretic mobility between mutant and wild type nucleic acids. *See, e.g.,* Orita, *et al.,* 1989. *Proc. Natl. Acad Sci. USA*: 86: 2766; Cotton, 1993. *Mutat. Res.* 285: 125-144; Hayashi, 1992. *Genet. Anal. Tech. Appl.* 9: 73-79. Single-stranded DNA fragments of sample and control FCTRX nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility. *See, e.g.,* Keen, *et al.,* 1991. *Trends Genet.* 7: 5.

In yet another embodiment, the movement of mutant or wild-type fragments in polyacrylamide gels containing a gradient of denaturant is assayed using denaturing gradient gel electrophoresis (DGGE). *See, e.g.,* Myers, *et al.,* 1985. *Nature* 313: 495. When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA. *See, e.g.,* Rosenbaum and Reissner, 1987. *Biophys. Chem.* 265: 12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found. *See, e.g.,* Saiki, *et al.,* 1986. *Nature* 324: 163; Saiki, *et al.,* 1989. *Proc. Natl. Acad Sci. USA* 86: 6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology that depends on selective PCR amplification may be used in conjunction with the instant invention. Oligonucleotides used as primers for specific amplification may carry the mutation of interest in the center of the molecule (so that amplification depends on differential hybridization; *see, e.g.,* Gibbs, *et al.,* 1989. *Nucl. Acids Res.* 17: 2437-2448) or at the extreme 3'-terminus of one primer where, under appropriate conditions, mismatch can prevent, or reduce polymerase extension (*see, e.g.,* Prossner, 1993. *Tibtech.* 11: 238). In addition it may be desirable to introduce a novel restriction site in the region of the mutation to create cleavage-based detection. *See, e.g.,* Gasparini, *et al.,* 1992. *Mol. Cell Probes* 6: 1. It is anticipated that in certain embodiments amplification may also be performed using *Taq* ligase for amplification. *See, e.g.,* Barany, 1991. *Proc. Natl. Acad. Sci. USA* 88: 189. In such cases, ligation will occur only if there is a perfect match at the 3'-terminus of the 5' sequence, making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, *e.g.,* in clinical settings to diagnose patients exhibiting symptoms or family history of a disease or illness involving an FCTRX gene.

Furthermore, any cell type or tissue, preferably peripheral blood leukocytes, in which FCTRX is expressed may be utilized in the prognostic assays described herein. However, any biological sample containing nucleated cells may be used, including, for example, buccal mucosal cells.

### Pharmacogenomics

Agents, or modulators that have a stimulatory or inhibitory effect on FCTRX activity (*e.g.,* FCTRX gene expression), as identified by a screening assay described herein can be to treat (prophylactically or therapeutically) disorders used for the manufacture of a medicament or syndromes including, *e.g.,* Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance. In conjunction with such treatment, the pharmacogenomics (*i.e.,* the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (*e.g.,* drugs) for prophylactic or therapeutic treatments based on a consideration of the individual's genotype. Such pharmacogenomics can further be used to determine appropriate dosages and therapeutic regimens. Accordingly, the activity of FCTRX protein, expression of FCTRX nucleic acid, or mutation content of FCTRX genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See *e.g.,* Eichelbaum, 1996. *Clin. Exp. Pharmacol. Physiol.,* 23: 983-985; Linder, 1997. *Clin. Chem.,* 43: 254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucoso-6-phosphate dehydrogenase (G6PD) deficiency is a common inherited enzymopathy in which the main clinical complication is hemolysis after ingestion of oxidant drugs (anti-malarials, sulfonamides, analgesics; nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e.g*., N-acetyltransferase 2 (NAT 2) and cytochrome P450 enzymes CYP2D6 and CYP2C19) has provided an exphanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. At the other extreme are the so called ultra-rapid metabolizers who do not respond to standard doses. Recently, the molecular basis of ultra-rapid metabolism has been identified to be due to CYP2D6 gene amplification.

Thus, the activity of FCTRX protein, expression of FCTRX nucleic acid, or mutation content ofFCTRX genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with an FCTRX modulator, such as a modulator identified by one of the exemplary screening assays described herein.

### Monitoring of Effects During Clinical Trials

Monitoring the influence of agents (*e.g.,* drugs, compounds) on the expression or activity of FCTRX (*e.g.,* the ability to modulate aberrant cell proliferation and/or differentiation) can be applied not only in basic drug screening, but also in clinical trials. For example, the effectiveness of an agent determined by a screening assay as described herein to increase FCTRX gene expression, protein levels, or upregulate FCTRX activity, can be monitored in clinical trails of subjects exhibiting decreased FCTRX gene expression, protein levels, or downregulated FCTRX activity. Alternatively, the effectiveness of an agent determined by a screening assay to decrease FCTRX gene expression, protein levels, or downregulate FCTRX activity, can be monitored in clinical trails of subjects exhibiting increased FCTRX gene expression, protein levels, or upregulated FCTRX activity. In such clinical trials, the expression or activity of FCTRX and, preferably, other genes that have been implicated in, for example, a cellular proliferation or immune disorder can be used as a "read out" or markers of the immune responsiveness of a particular cell.

By way of example, and not of limitation, genes, including FCTRX, that are modulated in cells by treatment with an agent (*e.g.,* compound, drug or small molecule) that modulates FCTRX activity (*e.g.,* identified in a screening assay as described herein) can be identified. Thus, to study the effect of agents on cellular proliferation disorders, for example, in a clinical trial, cells can be isolated and RNA prepared and analyzed for the levels of expression of FCTRX and other genes implicated in the disorder. The levels of gene expression (*i.e.,* a gene expression pattern) can be quantised by Northern blot analysis or RT-PCR, as described herein, or alternatively by measuring the amount of protein produced, by one of the methods as described herein, or by measuring the levels of activity ofFCTRX or other genes. In this manner, the gene expression pattern can serve as a marker, indicative of the physiological response of the cells to the agent. Accordingly, this response state may be determined before, and at various points during, treatment of the individual with the agent.

In one embodiment, the invention provides a method for monitoring the effectiveness of treatment of a subject with an agent (*e.g.,* an agonist, antagonist, protein, peptide, peptidomimetic, nucleic acid, small molecule, or other drug candidate identified by the screening assays described herein) comprising the steps of (*i*) detecting the level of expression of an FCTRX protein, mRNA, or genomic DNA in a preadministration sample from a subject, (ii) detecting the level of expression or activity of the FCTRX protein, mRNA, or genomic DNA in one or more post-administration samples form the subject (iii) comparing the level of expression or activity of the FCTRX protein, mRNA, or genomic DNA in the pre-administration sample with the FCTRX protein, mRNA, or genomic DNA in the post administration sample or samples; and (iv) such that administration of the agent to the subject may be altered accordingly. For example, increased administration of the agent may be desirable to increase the expression or activity of FCTRX to higher levels than detected, *i.e.,* to increase the effectiveness of the agent Alternatively, decreased administration of the agent may be desirable to decrease expression or activity of FCTRX to lower levels than detected, *i.e.,* to decrease the effectiveness of the agent.

### Uses for Treatment

The invention provides for both prophylactic and therapeutic uses for treating a subject at risk of (or susceptible to) a disorder or having a disorder associated with aberrant FCTRX expression or activity. The disorders include, adrenoleukodystrophy, congenital adrenal hyperplasia, prostate cancer, neoplasm; adenocarcinoma, lymphoma, uterus cancer, idiopathic thrombocytopenic purpura, and other diseases, disorders and conditions of the like.

These methods of treatment will be discussed more fully, below.

### Disease and Disorders

Therapeutics that antagonize (*i.e.,* reduce or inhibit) activity may be used for me manufacture of a medicament to treat. Diseases and disorders that are characterized by increased (relative to a subject not suffering from the disease or disorder) levels or biological activity. Therapeutics that antagonize activity may be used in a therapeutic or prophylactic manner. Therapeutics that may be utilized include, but are not limited to: (*i*) an aforementioned peptide, or analogs, derivatives, fragments or homologs thereof; (*ii*) antibodies to an aforementioned peptide; (*iii*) nucleic acids encoding an aforementioned peptide; (*iv*) administration of antisense nucleic acid and nucleic acids that are "dysfunctional" (*i.e.,* due to a heterologous insertion within the coding sequences of coding sequences to an aforementioned peptide) that are utilized to "knockout" endoggenous function of an aforementioned peptide by homologous recombination *(see, e.g.,* Capecchi, 1989. *Science* 244: 1288-1292); or (v) modulators (*i.e.,* inhibitors, agonists and antagonists, including additional peptide mimetic of the invention or antibodies specific to a peptide of the invention) that alter the interaction between an aforementioned peptide and its binding partner.

Diseases and disorders are characterized by decreased (relative to a subject not suffering from the disease or disorder) levels or biological activity. Therapeutics increase (*i.e.,* are agonists to) activity. Therapeutics that upregulate activity may be used in a therapeutic or prophylactic manner. Therapeutics that may be utilized include, but are not limited to, an aforementioned peptide, or analogs, derivatives, fragments or homologs thereof; or an agonist that increases bioavailability.

Increased or decreased levels can be readily detected by quantifying peptide and/or RNA, by obtaining a patient tissue sample (*e.g.,* from biopsy tissue) and assaying it *in vitro* for RNA or peptide levels, structure and/or activity of the expressed peptides (or mRNAs of an aforementioned peptide). Methods that are well-known within the art include, but are not limited to, immunoassays (*e.g.,* by Western blot analysis, immunoprecipitation followed by sodium dodecyl sulfate (SDS) polyacrylamide gel electrophoresis, immunocytochemistry, etc.) and/or hybridization assays to detect expression of mRNAs (*e.g.,* Northern assays, dot blots, *in situ* hybridization, and the like).

### Prophylactic Uses

In one aspect, the invention provides use of an agent that modulates FCTRX expression or at least one FCTRX activity for the manufacture of a medicament for preventing, in a subject, a disease or condition associated with an aberrant FCTRX expression or activity. Subjects at risk for a disease that is caused or contributed to by aberrant FCTRX expression or activity can be identified by, for example, any or a combination of diagnostic or prognostic assays as described herein. A prophylactic agent may be for administration prior to the manifestation of symptoms characteristic of the FCTRX aberrancy, such that a disease or disorder is prevented or, alternatively, delayed in its progression. Depending upon the type of FCTRX aberrancy, for example, an FCTRX agonist or FCTRX antagonist agent can be used. The appropriate agent can be determined based on screening assays described herein. The prophylactic methods of the invention are further discussed in the following subsections.

### Therapeutic Uses

Another aspect of the invention pertains to uses for modulating FCTRX expression or activity for therapeutic purposes. The modulatory uses of the invention involve contacting a cell with an agent that modulates one or more of the activities of FCTRX protein activity associated with the cell. An agent that modulates FCTRX protein activity can be an agent as described herein, such as a nucleic acid or a protein, a naturally-occurring cognate ligand of an FCTRX protein, a peptide, an FCTRX peptidomimetic, or other small molecule. In one embodiment, the agent stimulates one or more FCTRX protein activity. Examples of such stimulatory agents include active FCTRX protein and a nucleic acid molecule encoding FCTRX that has been introduced into the cell. In another embodiment, the agent inhibits one or more FCTRX protein activity. Examples of such inhibitory agents include antisense FCTRX nucleic acid molecules and anti-FCTRX antibodies. These modulatory methods can be performed *in vitro* (*e.g.,* by culturing the cell with the agent) or, alternatively, *in vivo* (*e.g.,* by administering the agent to a subject). As such, the invention provides uses for treating an individual afflicted with a disease or disorder characterized by aberrant expression or activity of an FCTRX protein or nucleic acid molecule. In one embodiment, the therapeutic use is of an agent (*e.g.,* an agent identified by a screening assay described herein), or combination of agents that modulates (*e.g.,* up-regulates or down-regulates) FCTRX expression or activity. In another embodiment, the therapeutic use is of an FCTRX protein or nucleic acid molecule as therapy to compensate for reduced or aberrant FCTRX expression or activity.

Stimulation of FCTRX activity is desirable in situations in which FCTRX is abnormally downregulated and/or in which increased FCTRX activity is likely to have a beneficial effect. One example of such a situation is where a subject has a disorder characterized by aberrant cell proliferation and/or differentiation (*e.g.,* cancer).

### Determination of the Biological Effect of the Therapeutic

In various embodiments of the invention, suitable *in vitro* or *in vivo* assays are performed to determine the effect of a specific Therapeutic and whether its use is indicated for treatment of the affected tissue.

In various specific embodiments, *in vitro* assays may be performed with representative cells of the type(s) involved in the patient's disorder, to determine if a given Therapeutic exerts the desired effect upon the cell type(s). Compounds for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

### Prophylactic and Therapeutic Uses of the Compositions of the Invention

The FCTRX nucleic acids and proteins of the invention are useful for the manufacture of a medicament for treating or preventing disorders or syndromes including, *e.g.,* Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression ofT-cell mediated immune effector cells and pathways resulting in turnor escape from immune surveilance.

As an example, a cDNA encoding the FCTRX protein of the invention may be useful in gene therapy, and the protein may be useful to a subject in need thereof. By way of non-limiting example, the compositions of the invention will have efficacy for the manufacture of a medicament for the treatment of patients suffering from disorders or syndromes including, *e.g.,* Colorectal cancer, adenomatous polyposis coli, myelogenous leukemia, congenital ceonatal alloimmune thrombocytopenia, multiple human solid malignancies, malignant ovarian tumours particularly at the interface between epithelia and stroma, malignant brain tumors, mammary tumors, human gliomas, astrocytomas, mixed glioma/astrocytomas, renal cells carcinoma, breast adenocarcinoma, ovarian cancer, melanomas, renal cell carcinoma, clear cell and granular cell carcinomas, autocrine/paracrine stimulation of tumor cell proliferation, autocrine/paracrine stimulation of tumor cell survival and tumor cell resistance to cytotoxic therapy, paranechmal and basement membrane invasion and motility of tumor cells thereby contributing to metastasis, tumor-mediated immunosuppression of T-cell mediated immune effector cells and pathways resulting in tumor escape from immune surveilance.

Both the novel nucleic acid encoding the FCTRX protein, and the FCTRX protein of the invention, or fragments thereof, may also be useful in diagnostic applications, wherein the presence or amount of the nucleic acid or the protein are to be assessed. A further use could be as an anti-bacterial molecule (*i.e.,* some peptides have been found to possess anti-bacterial properties). These materials are further useful in the generation of antibodies which

### EXAMPLES

### Example 1: Method of Identifying the Nucleic Acids

The novel nucleic acids of the invention were identified by TblastN using a proprietary sequence file, run against the Genomic Daily Files made available by GenBaak. The nucleic acids were further predicted by the proprietary software program GenScan^{™}, including selection of exons. These were further modified by means of similarities using BLAST searches. The sequences were then manually corrected for apparent inconsistencies, thereby obtaining the sequences encoding the full-length proteins.

### EQUIVALENTS

Although particular embodiments have been disclosed herein in detail, this has been done by way of example for purposes of illustration only, and is not intended to be limiting with respect to the scope of the appended claims which follow. In particular, it is contemplated by the inventors that various substitutions, alterations; and modifications may be made to the invention without departing from the scope of the invention as defined by the claims. The choice of nucleic acid starting material, clone of interest, or library type is believed to be a matter of routine for a person of ordinary skill in the art with knowledge of the embodiments described herein. Other aspects, advantages, and modifications considered to be within the scope of the following claims.

## Claims

1. An isolated human polypeptide comprising a human amino acid sequence selected from the group consisting of;
(a) a mature form of an amino acid sequence selected from the group consisting of SEQ ID NOS: 8 and 13;
(b) a variant amino acid sequence having at least 95% identity to a mature form of an amino acid sequence selected from the group consisting of SEQ ID NOS: 8 and 13, over an amino acid sequence of identical size to said mature form;
(c) an amino acid sequence selected from the group consisting of SEQ ID NOS: 8, and 13; and
(d) a variant amino acid sequence having at least 95% identity to an amino acid sequence selected from the group consisting of SEQ ID NOS: 8 and 13, over an amino acid sequence of identical size to said amino acid sequence of SEQ ID NOs: 8 or 13.

2. A polypeptide according to Claim 1, wherein said polypeptide comprises the amino acid sequence of a naturally-occurring allelic variant of an amino acid sequence selected from the group consisting of SEQ ID NOS: 8 and 13.

3. A polypeptide according to Claim 2, wherein said allelic variant comprises an amino acid sequence that is the translation of a nucleic acid sequence differing by a single nucleotide from a nucleic acid sequence selected from the group consisting of SEQ ID NOS: 7, 9, 10 and 11.

4. A polypeptide according to Claim 1, wherein the amino acid sequence of said variant comprises a conservative amino acid substitution.

5. An isolated human nucleic acid molecule comprising a human nucleic acid sequence encoding a polypeptide comprising an amino acid sequence selected from the group consisting of:
(a) a mature form of an amino acid sequence selected from the group consisting of SEQ ID NOS: 8 and 13;
(b) a variant amino acid sequence having at least 95% identity to a mature form of an amino acid sequence selected from the group consisting of SEQ ID NOS: 8 and 13, over an amino acid sequence of identical size to said mature form;
(c) an amino acid sequence selected from the group consisting of SEQ ID NOS: 8 and 13;
(d) a variant amino acid sequence having at least 95% identity to an amino acid sequence selected from the group consisting of SEQ ID NOS: 8 and 13, over an amino acid sequence of identical size to said amino acid sequence of SEQ ID NOs: 8 or 13;
or a nucleic acid molecule encoding the complement of (a), (b), (c) or (d).

6. A nucleic acid molecule according to Claim 5, wherein the nucleic acid molecule differs by a single nucleotide from a nucleic acid sequence selected from the group consisting of SEQ ID NOS: 7, 9, 10 and 11.

7. A nucleic acid molecule according to Claim 5, wherein said nucleic acid molecule comprises a nucleotide sequence selected from the group consisting of:
(a) a nucleotide sequence selected from the group consisting of SEQ ID NOS: 7, 9, 10 and 11; and
(b) a variant nucleotide sequence differing from a nucleotide sequence selected from the group consisting of SEQ ID NOS: 7, 9, 10 and 11 by no more than 20% of the nucleotides.

8. A nucleic acid molecule according to Claim 5, wherein said nucleic acid molecule hybridizes under high stringency conditions to a nucleotide sequence chosen from the group consisting of SEQ ID NOS: 7, 9, 10 and 11, or a complement of said nucleotide sequence.

9. A vector comprising a nucleic acid molecule according to Claim 8.

10. A vector according to Claim 9, further comprising a promoter operably-linked to said nucleic acid molecule.

11. A host cell comprising a vector according to Claim 9 or 10.

12. An antibody that binds specifically to a polypeptide according to Claim 1.

13. An antibody according to Claim 12, wherein said antibody is a monoclonal antibody.

14. An antibody according to Claim 12, wherein the antibody is a humanized antibody.

15. An *in vitro* method for determining the presence or amount of a polypeptide according to Claim 1 in a sample, the method comprising:
(a) contacting the sample *in vitro* with an antibody that binds specifically to the polypeptide; and
(b) determining the presence or amount of antibody bound to said polypeptide, thereby determining the presence or amount of polypeptide in said sample.

16. An *in vitro* method for determining the presence or amount of a nucleic acid molecule according to Claim 5 in a sample, the method comprising:
(a) contacting the sample *in vitro* with a probe that binds to said nucleic acid molecule; and
(b) determining the presence or amount of the probe bound to said nucleic acid molecule,
thereby determining the presence or amount of the nucleic molecule in said sample.

17. An *in vitro* method according to Claim 16 wherein the presence or amount of the nucleic acid molecule is used as a marker for cell or tissue type.

18. An *in vitro* method according to Claim 17 wherein the cell or tissue type is cancerous.

19. A method of identifying an agent that binds to a polypeptide according to Claim 1, the method comprising:
(a) contacting said polypeptide with said agent; and
(b) determining whether said agent binds to said polypeptide.

20. A method for identifying an agent that modulates the expression or activity of a polypeptide according to Claim 1, the method comprising:
(a) contacting a cell expressing said polypeptide with said agent *in vitro,* and
(b) determining whether the agent modulates expression or activity of said polypeptide,
whereby an alteration in expression or activity of said polypeptide indicates said agent modulates expression or activity of said polypeptide.

21. Use of a polypeptide according to Claim 1, a nucleic acid according to Claim 5, or an antibody according to Claim 12, for the manufacture of a medicament for treating or preventing cancer.

22. Use of a polypeptide according to Claim 1, a nucleic acid according to Claim 5, or an antibody according to Claim 12, for the manufacture of a medicament for treating or preventing colorectal cancer, adenomatous polyposis coli, myelogenous leukemia, congenital ceonatal alloimmune thrombocytopenia, multiple human solid malignancies, malignant ovarian tumours particularly at the interface between the epithelial and stroma, malignant brain tumours, mammary tumours, human gliomas, astrocytomas, mixed glioma/astrocytomas, renal cell carcinoma, clear cell and granular cell carcinomas, autocrine/paracrine stimulation of tumour cell survival and tumour cell resistance to cytotoxic therapy, parenchymal and basement membrane invasion and motility of tumour cells thereby contributing to metastasis and tumour-mediated immunosuppression of T-cell mediated immune effector cells and pathways resulting in tumour escape from immune surveillance.

23. Use of a polypeptide according to Claim 1, a nucleic acid according to Claim 5 or an antibody according to Claim 12, for the manufacture of a medicament for treating or preventing gliomas, astrocytomas, mixed glioma/astrocytomas, renal cell carcinomas, breast adenocarcinomas, ovarian cancer or melanomas.

24. Use according to any of Claims 21-23, wherein the subject to be treated is a human.

25. A pharmaceutical composition comprising a polypeptide according to Claim 1, or a nucleic acid molecule according to Claim 5, or an antibody according to Claim 12, and a pharmaceutically-acceptable carrier.

26. A kit comprising, in one or more containers, a pharmaceutical composition according to Claim 25.

27. An *in vitro* method for determining the presence of or predisposition to a disease associated with altered levels of a polypeptide according to Claim 1 in a first mammalian subject, the method comprising:
(a) measuring the level of expression of the polypeptide *in vitro* in a sample from the first mammalian subject; and
(b) comparing the amount of said polypeptide in the sample of step (a) to the amount of the polypeptide present in a control sample from a second mammalian subject known not to have, or not to be predisposed to, said disease;
wherein an alteration in the expression level of the polypeptide first subject as compared to the control sample indicates the presence of or predisposition to said disease.

28. An *in vitro* method for determining the presence of or predisposition to a disease associated with altered levels of the nucleic acid molecule of Claim 5 in a first mammalian subject, the method comprising:
(a) measuring the amount of the nucleic acid *in vitro* in a sample from the first mammalian subject; and
(b) comparing the amount of said nucleic acid in the sample of step (a) to the amount of the nucleic acid present in a control sample from a second mammalian subject known not to have, or not be predisposed to, the disease;
wherein an alteration in the level of the nucleic acid in the first subject as compared to the control sample indicates the presence of or predisposition to the disease.

29. An *in vitro* method according to Claim 27 or 28 wherein the predisposition is to cancers.

## Patentansprüche

1. Isoliertes humanes Polypeptid, umfassend eine humane Aminosäuresequenz, ausgewählt aus der Gruppe, bestehend aus:
a) einer reifen Form einer Aminosäuresequenz, ausgewählt aus der Gruppe, bestehend aus SEQ ID NOS: 8 und 13;
b) einer Aminosäuresequenz-Variante, die mindestens 95% Identität zu einer reifen Form einer Aminosäuresequenz, ausgewählt aus der Gruppe, bestehend aus SEQ ID NOS: 8 und 13, über eine Aminosäuresequenz identischer Größe zu der reifen Form aufweist;
c) einer Aminosäuresequenz, ausgewählt aus der Gruppe, bestehend aus SEQ ID NOS: 8 und 13; und
d) einer Aminosäuresequenz-Variante, die mindestens 95% Identität zu einer Aminosäuresequenz, ausgewählt aus der Gruppe, bestehend aus SEQ ID NOS: 8 und 13, über eine Aminosäuresequenz identischer Größe zu der Aminosäuresequenz von SEQ ID NOS: 8 oder 13 aufweist.

2. Polypeptid gemäß Anspruch 1, wobei das Polypeptid die Aminosäuresequenz einer natürlich vorkommenden allelischen Variante einer Aminosäuresequenz, ausgewählt aus der Gruppe, bestehend aus SEQ ID NOS: 8 und 13, umfasst.

3. Polypeptid gemäß Anspruch 2, wobei die allelische Variante eine Aminosäuresequenz umfasst, die die Translation einer Nukleinsäuresequenz darstellt, die sich durch ein einzelnes Nukleotid von der Nukleinsäuresequenz, ausgewählt aus der Gruppe, bestehend aus SEQ ID NOS: 7, 9, 10 und 11, unterscheidet.

4. Polypeptid gemäß Anspruch 1, wobei die Aminosäuresequenz der Variante eine konservative Aminosäure-Substitution umfasst.

5. Isoliertes humanes Nukleinsäure-Molekül, umfassend eine humane Nukleinsäuresequenz, kodierend für ein Polypeptid, umfassend eine Aminosäuresequenz, ausgewählt aus der Gruppe, bestehend aus:
a) einer reifen Form einer Aminosäuresequenz, ausgewählt aus der Gruppe, bestehend aus SEQ ID NOS: 8 und 13;
b) einer Aminosäuresequenz-Variante, die mindestens 95% Identität zu einer reifen Form einer Aminosäuresequenz, ausgewählt aus der Gruppe, bestehend aus SEQ ID NOS: 8 und 13, über eine Aminosäuresequenz identischer Größe zu der reifen Form aufweist;
c) einer Aminosäuresequenz, ausgewählt aus der Gruppe, bestehend aus SEQ ID NOS: 8 und 13;
d) einer Aminosäuresequenz-Variante, die mindestens 95% Identität zu einer Aminosäuresequenz, ausgewählt aus der Gruppe, bestehend aus SEQ ID NOS: 8 und 13, über eine Aminosäuresequenz identischer Größe zu der Aminosäuresequenz von SEQ ID NOS: 8 oder 13 aufweist;
oder ein Nukleinsäure-Molekül, kodierend für das Komplementäre von a), b), c) oder d).

6. Nukleinsäure-Molekül gemäß Anspruch 5, wobei das Nukleinsäure-Molekül sich durch ein einzelnes Nukleotid von der Nukleinsäuresequenz, ausgewählt aus der Gruppe, bestehend aus SEQ ID NOS: 7, 9, 10 und 11, unterscheidet.

7. Nukleinsäure-Molekül gemäß Anspruch 5, wobei das Nukleinsäure-Molekül eine Nukleotidsequenz umfasst, ausgewählt aus der Gruppe, bestehend aus:
a) einer Nukleotidsequenz, ausgewählt aus der Gruppe, bestehend aus SEQ ID NOS: 7, 9, 10 und 11; und
b) einer Nukleotidsequenz-Variante, die sich von der Nukleotidsequenz, ausgewählt aus der Gruppe, bestehend aus SEQ ID NOS: 7, 9, 10 und 11, durch nicht mehr als 20% der Nukleotide unterscheidet.

8. Nukleinsäure-Molekül gemäß Anspruch 5, wobei das Nukleinsäure-Molekül unter hohen Stringenz-Bedingungen an eine Nukleotidsequenz, ausgewählt aus der Gruppe, bestehend aus SEQ ID NOS: 7, 9, 10 und 11, oder an ein Komplementäres der Nukleotidsequenz hybridisiert.

9. Vektor, umfassend ein Nukleinsäure-Molekül gemäß Anspruch 8.

10. Vektor gemäß Anspruch 9, weiterhin umfassend einen Promotor, der mit dem Nukleinsäure-Molekül operabel verknüpft ist.

11. Wirtszelle, umfassend einen Vektor gemäß Anspruch 9 oder 10.

12. Antikörper, der spezifisch an ein Polypeptid gemäß Anspruch 1 bindet.

13. Antikörper gemäß Anspruch 12, wobei der Antikörper ein monoklonaler Antikörper isL

14. Antikörper gemäß Anspruch 12, wobei der Antikörper ein humanisierter Antikörper ist

15. *In-Vitro-*Verfahren zur Bestimmung der Anwesenheit oder Menge eines Polypeptids gemäß Anspruch 1 in einer Probe, wobei das Verfahren umfasst:
a) In-Kontakt-Bringen der Probe *in vitro* mit einem Antikörper, der spezifisch an das Polypeptid bindet; und
b) Bestimmen der Anwesenheit oder Menge des Antikörpers, der an das Polypeptid gebunden ist,
wodurch die Anwesenheit oder Menge des Polypeptids in der Probe bestimmt wird.

16. *In-Vitro-*Verfahren zur Bestimmung der Anwesenheit oder Menge eines Nukleinsäure-Moleküls gemäß Anspruch 5 in einer Probe, wobei das Verfahren umfasst:
a) In-Kontakt-Bringen der Probe *in vitro* mit einer Sonde, die an das Nukleinsäure-Molekül bindet; und
b) Bestimmen der Anwesenheit oder Menge der Sonde, die an das Nukleinsäure-Molekül gebunden ist,
wodurch die Anwesenheit oder Menge des Nukleinsäure-Moleküls in der Probe bestimmt wird.

17. *In-Vitro*-Verfahren gemäß Anspruch 16, wobei die Anwesenheit oder Menge des Nukleinsäure-Moleküls als ein Marker für den Zell- oder Gewebs-Typ verwendet wird.

18. *In-Vitro-*Verfahren gemäß Anspruch 17, wobei der Zell- oder Gewebs-Typ kanzerös ist.

19. Verfahren zur Identifizierung eines Mittels, das an ein Polypeptid gemäß Anspruch 1 bindet, wobei das Verfahren umfasst:
a) In-Kontakt-Bringen des Polypeptids mit dem Mittel und
b) Bestimmen, ob das Mittel an das Polypeptid bindet.

20. Verfahren zur Identifizierung eines Mittels, das die Expression oder Aktivität eines Polypeptids gemäß Anspruch 1 moduliert, wobei das Verfahren umfasst
a) In-Kontakt-Bringen einer Zelle, die das Polypeptid exprimiert, mit dem Mittel *in vitro* und
b) Bestimmen, ob das Mittel die Expression oder Aktivität des Polypeptids moduliert,
wobei eine Änderung in der Expression oder Aktivität des Polypeptids darauf hinweist, dass das Mittel die Expression oder Aktivität des Polypeptids moduliert

21. Verwendung eines Polypeptids gemäß Anspruch 1, einer Nukleinsäure gemäß Anspruch 5 oder eines Antikörpers gemäß Anspruch 12 zur Herstellung eines Medikaments zur Behandlung oder Verhinderung von Krebs.

22. Verwendung eines Polypeptids gemäß Anspruch 1, einer Nukleinsäure gemäß Anspruch 5 oder eines Antikörpers gemäß Anspruch 12 zur Herstellung eines Medikaments zur Behandlung oder Verhinderung von kolorektalem Krebs, adenomatöser Colon-Polyposis, myelogener Leukämie, kongenitaler keonataler AIloimmun-Thrombozytopänie, multiplen humanen soliden Malignitäten, malignen Ovarial-Tumoren, insbesondere an der Grenzfläche zwischen Epithel und Bindegewebe, malignen Gehirntumoren, Brusttumoren, humanen Gliomen, Astrozytomen, gemischten Gliom/Astrozytomen, Nierenzell-Karzinom, hellzelligen und kömerzelligen Karzinomen, autokriner/parakriner Stimulation des Tumorzell-Überlebens und der Tumorzell-Resisitenz gegenüber zytotoxischer Therapie, parenchymaler und basalmembraner Tumorzell-Invasion und -Beweglichkeit, die zur Metastase beiträgt, Tumor-vermittelter Immunsuppression T-Zell-vermittelter Immun-Effektorzellen und von Pfaden, die zum Tumor-Escape aus der Immunüberwachung führen.

23. Verwendung eines Polypeptids gemäß Anspruch 1, einer Nukleinsäure gemäß Anspruch 5 oder eines Antikörpers gemäß Anspruch 12 zur Herstellung eines Medikaments zur Behandlung oder Verhinderung von Gliomen, Astrozytomen gemischten Gliom/Astrozytomen, Nierenzell-Karzinomen, Brust-Adenokarzinomen, Ovarial-Krebs oder Melanomen.

24. Verwendung gemäß jedem beliebigen der Ansprüche 21-23, wobei der zu behandelnde Organismus ein Mensch ist

25. Pharmazeutische Zusammensetzung, umfassend ein Polypeptid gemäß Anspruch 1, ein Nukleinsäure-Molekül gemäß Anspruch 5 oder einen Antikörper gemäß Anspruch 12 und einen pharmazeutisch verträglichen Träger.

26. Kit, umfassend eine pharmazeutische Zusammensetzung gemäß Anspruch 25 in einem oder mehr Behälter/n.

27. *In-Vitro-*Verfahren zur Bestimmung der Anwesenheit von oder Prädisposition für eine/r Erkrankung, die mit veränderten Niveaus eines Polypeptids gemäß Anspruch 1 assoziiert ist, in einem ersten Säugetier-Organismus, wobei das Verfahren umfasst:
a) Messung des Expressions-Niveaus des Polypeptids *in vitro* in einer Probe aus einem ersten Säugetier-Organismus; und
b) Vergleich der Menge des Polypeptids in der Probe von Schritt a) mit der Menge des Polypeptids, die in einer Kontrollprobe aus einem zweiten Säugetier-Organismus vorliegt, für den bekannt ist, dass dieser die Erkrankung nicht aufweist oder für diese nicht prädisponiert ist;
wobei eine Änderung in dem Expressions-Niveau des Polypeptids im ersten Organismus im Vergleich zur Kontrollprobe auf die Anwesenheit von oder Prädisposition für der/die Erkrankung hinweist.

28. *In*-*Vitro*-Verfahren zur Bestimmung der Anwesenheit von oder Prädisposition für eine/r Erkrankung, die mit veränderten Niveaus des Nukleinsäure-Moleküls gemäB Anspruch 5 assoziiert ist, in einem ersten Säugetier-Organismus, wobei das Verfahren umfasst:
a) Messung der Menge der Nukleinsäure *in vitro* in einer Probe aus einem ersten Säugetier-Organismus; und
b) Vergleich der Menge der Nukleinsäure in der Probe von Schritt a) mit der Menge der Nukleinsäure, die in einer Kontrollprobe eines zweiten Säugetier-Organismus vorliegt, für den bekannt ist, dass dieser die Erkrankung nicht aufweist oder für diese nicht prädisponiert ist;
wobei eine Änderung in dem Nukleinsäure-Niveau im ersten Organismus im Vergleich zur Kontrollprobe auf die Anwesenheit von oder Prädisposition für der/die Erkrankung hinweist.

29. *In-Vitro*-Verfahren gemäß Anspruch 27 oder 28, wobei die Prädisposition die Prädisposition für Krebs ist.

## Revendications

1. Polypeptide humain isolé comprenant une séquence humaine d'acides aminés choisie parmi le groupe constitué par :
(a) une forme mature d'une séquence d'acides aminés choisie parmi le groupe constitué par les SEQ ID NO: 8 et 13 ;
(b) une autre séquence d'acides aminés ayant une identité d'au moins 95 % avec une forme mature d'une séquence d'acides aminés choisie parmi le groupe constitué par les SEQ ID NO: 8 et 13, supérieure à une séquence d'acides aminés de taille identique à ladite forme mature ;
(c) une séquence d'acides aminés choisie parmi le groupe constituée par les SEQ ID NO: 8 et 13 ; et
(d) une autre séquence d'acides aminés ayant une identité d'au moins 95 % avec une séquence d'acides aminés choisie parmi le groupe constituée par les SEQ ID NO: 8 et 13, supérieure à une séquence d'acides aminés de taille identique à ladite séquence d'acides aminés de SEQ ID NO: 8 ou 13.

2. Polypeptide selon la revendication 1, où ledit polypeptide comprend la séquence d'acides aminés d'une variante allélique apparaissant naturellement d'une séquence d'acides aminés choisie parmi le groupe constituée par les SEQ ID NO: 8 et 13.

3. Polypeptide selon la revendication 2, où ledit variant allélique comprend une séquence d'acides aminés qui est la traduction d'une séquence d'acide nucléique qui diffère d'un seul nucléotide par rapport à la séquence d'acide nucléique choisie parmi le groupe constitué par les SEQ ID NO: 7, 9, 10 et 11.

4. Polypeptide selon la revendication 1, où la séquence d'acides aminés dudit variant comprend une substitution conservative en acide aminé.

5. Molécule d'acide nucléique humaine isolée comprenant une séquence d'acide nucléique humaine codant pour un polypeptide comprenant une séquence d'acides aminés choisie parmi le groupe constituée par :
(a) une forme mature d'une séquence d'acides aminés choisie parmi le groupe constitué par les SEQ ID NO: 8 et 13 ;
(b) une autre séquence d'acides aminés ayant une identité d'au moins 95 % avec une forme mature d'une séquence d'acides aminés choisie parmi le groupe constitué par les SEQ ID NO: 8 et 13, supérieure à une séquence d'acides aminés de taille identique à ladite forme mature ;
(c) une séquence d'acides aminés choisie parmi le groupe constituée par les SEQ ID NO: 8 et 13 ; et
(d) une autre séquence d'acides aminés ayant une identité d'au moins 95 % avec une séquence d'acides aminés choisie parmi le groupe constitué par les SEQ ID NO: 8 et 13, supérieure à une séquence d'acides aminés de taille identique à ladite séquence d'acides aminés de SEQ ID NO: 8 ou 13 ; ou une molécule d'acide nucléique codant pour le complément de (a), (b), (c) ou (d).

6. Molécule d'acide nucléique selon la revendication 5, où la molécule d'acide nucléique diffère d'un seul nucléotide par rapport à la séquence d'acide nucléique choisie parmi le groupe constitué par les SEQ ID NO: 7, 9, 10 et 11.

7. Molécule d'acide nucléique selon la revendication 5, où ladite molécule d'acide nucléique comprend une séquence nucléotidique choisie parmi le groupe constitué par :
(a) une séquence nucléotidique choisie parmi le groupe constitué par les SEQ ID NO: 7, 9, 10 et 11 ; et
(b) un variant de séquence nucléotidique qui diffère par rapport à une séquence nucléotidique choisie parmi le groupe constitué par les SEQ ID NO: 7, 9, 10 et 11 de 20 % en nucléotides au maximum.

8. Molécule d'acide nucléique selon la revendication 5, où ladite molécule d'acide nucléique s'hybride dans des conditions de forte stringence à une séquence nucléotidique choisie parmi le groupe constitué par les SEQ ID NO: 7, 9, 10 et 11, ou un complément de ladite séquence nucléotidique.

9. Vecteur comprenant une molécule d'acide nucléique selon la revendication 8.

10. Vecteur selon la revendication 9, comprenant en outre un promoteur lié opérationnellement à ladite molécule d'acide nucléique.

11. Cellule hôte comprenant un vecteur selon la revendication 9 ou 10.

12. Anticorps qui se lie spécifiquement à un polypeptide selon la revendication 1.

13. Anticorps selon la revendication 12, où ledit anticorps est un anticorps monoclonal.

14. Anticorps selon la revendication 12, où l'anticorps est un anticorps humanisé.

15. Méthode *in vitro* permettant de déterminer la présence ou la quantité d'un polypeptide selon la revendication 1 dans un échantillon, la méthode comprenant :
(a) la mise en contact de l'échantillon *in vitro* avec un anticorps qui se lie spécifiquement au polypeptide ; et
(b) la détermination de la présence ou de la quantité de l'anticorps lié audit polypeptide, déterminant de ce fait la présence ou la quantité du polypeptide dans ledit échantillon.

16. Méthode *in vitro* pour déterminer la présence ou la quantité d'une molécule d'acide nucléique selon la revendication 5 dans un échantillon, la méthode comprenant :
(a) la mise en contact de l'échantillon *in vitro* avec une sonde qui se lie à ladite molécule d'acide nucléique ; et
(b) la détermination de la présence ou de la quantité de la sonde liée à ladite molécule d'acide nucléique,
déterminant ainsi la présence ou la quantité de la molécule nucléique dans ledit échantillon.

17. Méthode *in vitro* selon la revendication 16, dans laquelle la présence ou la quantité de la molécule d'acide nucléique est utilisée en tant que marqueur pour un type de cellule ou de tissu.

18. Méthode *in vitro* selon la revendication 17, dans laquelle le type de cellule ou de tissu est cancéreux.

19. Méthode d'identification d'un agent qui se lie à un polypeptide selon la revendication 1, la méthode comprenant :
(a) la mise en contact dudit polypeptide avec ledit agent ; et
(b) la détermination de si ledit agent se lit audit polypeptide.

20. Méthode d'identification d'un agent qui module l'expression ou l'activité d'un polypeptide selon la revendication 1, la méthode comprenant :
(a) la mise en contact d'une cellule exprimant ledit polypeptide avec ledit agent *in vitro,* et
(b) la détermination de si l'agent module l'expression ou l'activité dudit polypeptide, moyennant quoi une modification de l'expression ou de l'activité dudit polypeptide indique que ledit agent module l'expression ou l'activité dudit polypeptide.

21. Utilisation d'un polypeptide selon la revendication 1, d'un acide nucléique selon la revendication 5, ou d'un anticorps selon la revendication 12, pour la fabrication d'un médicament permettant de traiter ou de prévenir le cancer.

22. Utilisation d'un polypeptide selon la revendication 1, d'un acide nucléique selon la revendication 5, ou d'un anticorps selon la revendication 12, pour la fabrication d'un médicament permettant de traiter ou de prévenir le cancer colorectal, la polypose adénomateuse colique, la leucémie myélogène, la thrombocytopénie alloimmune néonatale congénitale, de multiples tumeurs solides malignes humaines, les tumeurs ovariennes malignes en particulier à l'interface entre l'épithélium et le stroma, les tumeurs cérébrales malignes, les tumeurs mammaires, les gliomes humains, les astrocytomes, les gliomes/astrocytomes mixtes, le carcinome cellulaire rénal, l'adénocarcinome à cellules claires et le folliculome, la stimulation autocrine/paracrine de la survie et de la résistance d'une cellule tumorale au traitement cytotoxique, l'invasion de la membrane basale ou du parenchyme et la motilité des cellules tumorales contribuant ainsi aux métastases et à l'immunosuppression à médiation tumorale des voies et des cellules effectrices de la réponse immune médiée par les lymphocytes T dans l'absence de détection par le système de surveillance immunitaire.

23. Utilisation d'un polypeptide selon la revendication 1, d'un acide nucléique selon la revendication 5 ou d'un anticorps selon la revendication 12, pour la fabrication d'un médicament pour traiter ou prévenir les gliomes, les astrocytomes, les gliomes/astrocytomes mixtes, les carcinomes cellulaires rénaux, les adénocarcinomes du sein, le cancer de l'ovaire ou les mélanomes.

24. Utilisation selon l'une quelconque des revendications 21-23, où le sujet à traiter est un être humain.

25. Composition pharmaceutique comprenant un polypeptide selon la revendication 1, ou une molécule d'acide nucléique selon la revendication 5, ou un anticorps selon la revendication 12, et un vecteur pharmaceutiquement acceptable.

26. Trousse comprenant, dans un ou plusieurs conteneurs, une composition pharmaceutique selon la revendication 25.

27. Méthode *in vitro* de détermination de la présence ou d'une prédisposition à une maladie associée à des taux anormaux d'un polypeptide selon la revendication 1 chez un premier sujet mammifère, la méthode comprenant :
(a) la mesure du taux d'expression du polypeptide *in vitro* dans un échantillon issu du premier sujet mammifère ; et
(b) la comparaison de la quantité dudit polypeptide dans l'échantillon de l'étape (a) à la quantité du polypeptide présent dans un échantillon contrôle issu d'un second sujet mammifère connu pour ne pas avoir ladite maladie ou de ne pas y être prédisposé ;
dans laquelle une modification du taux d'expression du polypeptide du premier sujet par rapport à l'échantillon contrôle indique la présence ou la prédisposition à ladite maladie.

28. Méthode *in vitro* pour déterminer la présence ou la prédisposition à une maladie associée à des taux anormaux de la molécule d'acide nucléique de la revendication 5 chez un premier sujet mammifère, la méthode comprenant :
(a) la mesure de la quantité d'acide nucléique *in vitro* dans un échantillon issu du premier sujet mammifère ; et
(b) la comparaison de la quantité dudit acide nucléique dans l'échantillon de l'étape (a) avec la quantité d'acide nucléique présente dans un échantillon contrôle issu d'un second sujet mammifère connu pour ne pas avoir la maladie ou ne pas y être prédisposé ;
dans laquelle une modification du taux d'acide nucléique chez le premier sujet par rapport à l'échantillon contrôle indique la présence ou la prédisposition à la maladie.

29. Méthode *in vitro* selon la revendication 27 ou 28, dans laquelle la prédisposition concerne les cancers.
